(19) Europäisches Patentamt
European Patent Office
Office européen des brevets

(11) EP 4 512 482 A2

(12) EUROPEAN PATENT APPLICATION

(43) Date of publication:
**26.02.2025 Bulletin 2025/09**

(21) Application number: **24213388.2**

(22) Date of filing: **12.03.2021**

(51) International Patent Classification (IPC):
***A61P 37/02*** *(2006.01)*

(52) Cooperative Patent Classification (CPC):
(C-Sets available)
**A61P 37/04; A61K 39/02; A61K 39/0208; A61K 39/0258; A61K 39/0275; A61K 39/095; A61K 39/099; A61K 39/104; A61K 39/39; A61K 39/39558; A61P 37/02; C07K 16/2887;**
A61K 2039/55505; A61K 2039/55572; A61K 2039/804; (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.03.2020 EP 20305264**

(62) Document number(s) of the earlier application(s) in accordance with Art. 76 EPC:
**21710521.2 / 4 117 790**

(71) Applicant: **Hephaistos-Pharma**
**91400 Orsay (FR)**

(72) Inventors:
- **CAROFF, Martine**
**91400 Orsay (FR)**
- **NOVIKOV, Alexey**
**91400 Orsay (FR)**

(74) Representative: **Icosa**
**83 avenue Denfert-Rochereau**
**75014 Paris (FR)**

Remarks:
This application was filed on 15-11-2024 as a divisional application to the application mentioned under INID code 62.

# (54) DETOXIFIED LIPOPOLYSACCHARIDES (LPS), NATURALLY NON-TOXIC LPS, AND USES THEREOF

(57) The invention relates to an enriched population of modified lipopolysaccharide (LPS) molecular species being:
- devoid of phosphate group at position C1 of the reducing end of their lipid A domain; and
- substituted at position C6' of the non-reducing end of their lipid A domain by a hydrophilic moiety, with the proviso that said hydrophilic moiety is not a hydroxyl group.

It also relates to compositions comprising the enriched population of modified LPS; and uses of naturally-occurring LPS molecular species and/or enriched population of modified LPS molecular species for treating and/or preventing cancer, inflammatory diseases or infectious diseases, and for stimulating an immune response or vaccinating a subject.

EP 4 512 482 A2

(52) Cooperative Patent Classification (CPC): (Cont.)
Y02A 50/30

C-Sets
**A61K 39/02, A61K 2300/00;**
**A61K 39/099, A61K 2300/00;**
**A61K 39/39558, A61K 2300/00**

## Description

### FIELD OF INVENTION

The invention relates to an enriched population of modified lipopolysaccharide (LPS) molecular species being:

- devoid of phosphate group at position C1 of the reducing end of their lipid A domain; and
- substituted at position C6' of the non-reducing end of their lipid A domain by a hydrophilic moiety, with the proviso that said hydrophilic moiety is not a hydroxyl group.

It also relates to compositions comprising the enriched population of modified LPS; and uses of naturally-occurring LPS molecular species and/or enriched population of modified LPS molecular species for treating and/or preventing cancer, inflammatory diseases or infectious diseases, and for stimulating an immune response or vaccinating a subject.

### BACKGROUND OF INVENTION

Antigens in vaccine compositions are often poorly immunogenic and require additional components to help stimulate protective immunity based on antibodies and effector T cell functions. These additional components, termed adjuvants, provide the help needed to enhance the immunogenicity of vaccine antigens.

For decades, adjuvants used in vaccines and immunotherapies have been developed empirically, without understanding their mechanism of action. It is the case for the widely used Freund's adjuvant (a mixture of paraffin oil and surfactant with heat-killed *Mycobacterium tuberculosis* or *Mycobacterium butryicum* in which aqueous antigen solutions are emulsified), alum adjuvants (typically, precipitates of aluminum phosphate and aluminium hydroxide to which antigens are adsorbed), oil-in-water emulsions (such as MF59 from Novartis or AS03 from GlaxoSmithKline, both based on squalene, in which aqueous antigen solutions are emulsified), or saponin-based adjuvants (such as the so-called "immunostimulatory complexes" or ISCOMs, which are cage-like nanoparticles of saponin formulated with cholesterol, phospholipid and antigens). However, these empirically-designed adjuvants are far from offering optimal responses. Plus, there remain substantial groups of people which do not achieve adequate seroconversion rates or protective antibody titers, not to mention the decline of vaccine responsiveness in healthy adults after 40-50 years of age or in diseased patients.

More recently, researches have suggested that most adjuvants enhance T and B cell responses by engaging components of the innate immune system, rather than by direct effects on lymphocytes. A focus has been to develop natural or synthetic agonists - either obtained by chemical synthesis or by directed molecular biology - for well-defined pattern recognition receptors (PRRs) as adjuvants. Members of nearly all of the PRR families are potential target for adjuvants - including Toll-like receptors (TLRs) which have been intensively studied and are of particular interest in the present invention. A particular TLR is Toll-like receptor 4 (TLR4, also known as CD284), a transmembrane protein which, in complex with the MD-2 and CD14 co-receptors, constitutes a mammalian receptor for bacterial lipopolysaccharides (LPS).

LPS are phosphoglycolipids present in the external membrane of Gram-negative bacteria, spirochaetes (such as, e.g., Leptospira), cyanobacteria and some Gram-positive bacteria (such as, *e.g.,* Negativicutes). LPS are made of two distinct regions: an inner phospholipid comprising a disaccharide with a high number - typically from 2 to 9 - of long-chain fatty acids, called **"lipid A",** and which carries most of the biological properties of LPS; and a polysaccharide moiety, comprising a central "core **oligosaccharide"** domain (with a nine-, ten-sugar, or so, branched oligosaccharide) and an outer **"O-antigen"** domain (made of a repetitive carbohydrate polymer, linear or branched, with a highly variable number, nature and spatial arrangement of sugar residues) - although LPS of some bacteria do not possess the O-antigen domain and their "polysaccharide" moiety is restricted to an "oligosaccharide" comprising the core oligosaccharide domain only (which may however, in some cases, be capped with a capping antigen, such as, *e.g.,* in the case of *Bordetella pertussis,* with a distal trisaccharide which is biosynthetically and genetically distinguished from the O-antigen), hence these LPS are sometimes referred to as LOS, for lipooligosaccharides.

The lipid A domain comprises a disaccharide (most often a β-1,6-glucosamine [GlcN] disaccharide, a β-1,6-diaminoglucose [DAG] disaccharide, or a β-1,6-disaccharide comprising one GlcN residue and one DAG residue), with a high number of long-chain fatty acids serving as a hydrophobic anchor of LPS to the outer membrane. Typically, the disaccharide of lipid A is substituted with phosphate groups at the C1 and C4' positions, and with branched or linear fatty acid chains - from 10 to 18 carbons in most cases, some being hydroxylated at position C3, the so-called β-hydroxy fatty acids - linked at C2, C3, C2', and C3' positions. This 3-OH group, when present, can further be esterified with other fatty acids (such as, *e.g.,* with non-hydroxylated, 2-hydroxylated and sometimes 3-hydroxylated fatty acids, or even with a lipid moiety). These secondary fatty acids are also known as acyl-oxy-acyl fatty acids. The total number of fatty acids per 1 lipid

A molecule can be thus from 2 to 9. The lipid A domain is further substituted at position C6' by the polysaccharide moiety (comprising a full or truncated core oligosaccharide domain, and optionally, an O-antigen domain and/or any other capping antigen), through a glycosidic bound with, in most common cases, a 3-deoxy-*D*-manno-oct-2-ulosonic acid (Kdo) molecule.

Upon activation, TLR4 induces an intracellular signaling pathways NF-κB and TRIF, proinflammatory cytokine secretion (such as IL-1, TNFα, IFNγ and IL-12), and expression of co-stimulatory molecules. This response is activated by small amounts of LPS, ultimately activating the innate and then priming the adaptive immune system by, *e.g.,* maturating antigen presenting cells (such as dendritic cells, macrophages and B cells), or amplifying T and B cells functions. It is for these reasons that LPS have been long recognized as potent adjuvants.

However, LPS, when used as adjuvants, are not devoid of drawbacks:

(1) LPS are mostly naturally toxic and pyrogenic, as they can trigger strong immune responses in the host resulting in fever, uncontrolled production of proinflammatory cytokines, which can often lead to septic shock. This toxicity has been shown to originate from the lipid A domain, in particular from the phosphate at C1 and C4' positions. As a result, the EMA, FDA and other regulatory agencies have stringent requirements for the elimination of the toxicity of LPS in pharmaceutical products;

(2) Although the lipid A domain has been long time known to be responsible for TLR4 activation, evidences suggest that the core oligosaccharide domain and, when present, O-antigen domain, are also involved in the activation of TLR4. Their presence (1) improves LPS solubility comparatively to isolated lipid A and thus its bioavailability, and (2) maintain native tri-dimensional lipid A conformation. Moreover, the core oligosaccharide domain, and, when present, O-antigen domain, define antigenicity of the molecule. They can also bind to sugar-dependent receptors such as C-type lectin receptors (CLRs). However, these domains are often removed, wittingly or unwittingly, when preparing LPS-based adjuvants.

A variety of LPS-based molecules have been developed, showing fewer toxic effects than "wild type" LPS. Among these, monophosphoryl lipid A (MPL) is the most known LPS-based adjuvant. MPL, produced by GlaxoSmithKline, is obtained through controlled hydrolysis of *Salmonella enterica enterica* serovar *Minnesota* R595 LPS, by removal of (1) the core oligosaccharide domain at C6' position of the lipid A domain, (2) the phosphate at C1 position of the lipid A domain, and (3) the acyl chain at C3 position of the lipid A domain. MPL is currently used in so-called "adjuvant systems" in a number of vaccines (Garçon & Di Pasquale, 2017. Hum Vaccin Immunother. 13(1): 19-33):

- AS01: a liposome-based combination of MPL and saponin QS-21 - in the Mosquirix® vaccine against malaria caused by the parasite *Plasmodium falciparum;*
- AS02: an oil-in-water emulsion of MPL and saponin QS-21;
- AS04: an adsorption of MPL on alum - in the Fendrix® and Cervarix® vaccines against hepatitis B virus (HBV) and human papillomavirus (HPV), respectively;
- AS15: a liposome-based combination of MPL, saponin QS-21 and CpG 7909.

Other LPS-based immune adjuvants include synthetic lipid A analogues. Some of these molecules exhibit reduced toxicity in respect to "wild-type" lipid A and promote protective immune responses after vaccination. Plus, their synthesis results in higher molecular homogeneity and batch reproducibility as compared to chemically-obtained MPL. Examples of such synthetic lipid A analogues include RC-529, E6020, glucopyranosyl lipid A (GLA), ONO-4007, E5564 and CRX-526.

However, while being less toxic, these molecules are still not optimum because (1) they lack their core oligosaccharide domain and, when applicable, O-antigen domain; (2) they do not react properly with the TLR4 diversity in humans, which takes advantage of the natural heterogeneity and diversity of lipid A in the bacterial world; and (3) they have only limited solubility in common organic solvents and poor solubility in aqueous media, with a tendency to aggregate - even at very low concentration ($< 10^{-8}$ M), hence making their formulation complicated.

There remains thus a need for low-toxicity LPS molecules, derived from naturally-occurring LPS (for a natural heterogeneity and diversity of lipid A structures) and retaining their core oligosaccharide domain and, when applicable, O-antigen domain (for an improved bioactivity).

Here, the Inventors provide modified LPS molecular species devoid of their phosphate at C1 position of the lipid A domain, but retaining their polysaccharide moiety (core oligosaccharide domain and, when applicable, O-antigen domain). These modified LPS molecular species may also exhibit a liberation of fatty acids at secondary positions like acyl-oxy-acyl at C2 and C2', but also at C3 and C3' when present.

The combination of these features strongly diminishes the toxicity of the different LPS and LOS tested. In some cases, detoxification can be further amplified by additional release of primary ester-linked fatty acids.

These modified, low-toxicity LPS and LOS molecules are therefore the nearest-natural LPS molecular species,

in terms of composition and three-dimensional structure. Not only does this improve their binding to TLR4 receptors, but also their properties as compared to MPL and its synthetic analogs, whether in terms of biological activity or solubility.

## SUMMARY

The present invention relates to an enriched population of modified lipopolysaccharide (LPS) molecular species, comprising at least 80 % of modified LPS molecular species, as defined in the claims.

It further relates to a composition comprising an enriched population of modified LPS molecular species, as defined in the claims.

It further relates to a naturally-occurring non-toxic LPS molecular species and/or an enriched population of modified LPS molecular species, or a composition comprising the same, for use in treating and/or preventing cancer, an inflammatory disease or an infectious disease in a subject in need thereof; or for use in stimulating an immune response in a subject in need thereof; or for use in vaccinating a subject in need thereof, as defined in the claims.

It further relates to a kit-of-parts, as defined in the claims.

## DEFINITIONS

In the present invention, the following terms have the following meanings:

As used herein, the terms **"lipopolysaccharide"** or **"LPS"** refer to phosphoglycolipids present in the external membrane of Gram-negative bacteria, spirochaetes (such as, *e.g.,* Leptospira), cyanobacteria and some Gram-positive bacteria (such as, *e.g.,* Negativicutes). LPS are typically made of two distinct regions: an inner phospholipid comprising a disaccharide with a high number - typically from 2 to 9 - of long-chain fatty acids, called **"lipid A",** and which carries most of the biological properties of LPS; and a polysaccharide moiety, comprising a central **"core oligosaccharide"** domain (with a nine-, ten-sugar, or so, branched oligosaccharide) and an outer antigen domain, such as, *e.g.,* and **"O-antigen"** domain (made of a repetitive carbohydrate polymer, linear or branched, with a highly variable number, nature and spatial arrangement of sugar residues), and/or a **"capping antigen"** domain. LPS of some bacteria do not possess an O-antigen domain and their "polysaccharide" moiety is restricted to an "oligosaccharide" comprising the core oligosaccharide domain only, hence these LPS are sometimes referred to as LOS, for lipooligosaccharides. However, LPS of some bacteria, while not possessing an O-antigen domain, are capped with a capping antigen, such as, *e.g.,* in the case of *Bordetella pertussis,* with a distal trisaccharide which is biosynthetically and genetically distinguished from the O-antigen.

As used herein, the term **"lipid A"** refers to the hydrophobic portion of lipopolysaccharide (LPS) molecules. It is typically a β-1,6-glucosamine (GlcN) disaccharide, a β-1,6-diaminoglucose (DAG) disaccharide or a mixed β-1,6-glucosamine-diaminoglucose disaccharide, typically phosphorylated at positions C1 and C4', and acylated with four to seven fatty acids at positions C2, C3, C2' and/or C3'. The numbering of carbons of the disaccharide forming the lipid A is well-known in the art and conventionally used by the one skilled in the art. Briefly, numbering without an apostrophe (') appended to the carbon number refers to carbons of the saccharide forming the reducing side of the disaccharide of the lipid A domain. Numbering with an apostrophe (') appended to the carbon number refers to carbons of the saccharide forming the non-reducing side of the disaccharide of the lipid A domain. The term **"lipid A",** as used herein, also encompasses precursors and altered structures of the classical disaccharide lipid A forming the hydrophobic portion of LPS molecules. Such precursors and altered structures may be naturally found in LPS molecule of some bacterial species or may be obtained upon modification, and include, e.g., lipid X, and other lipid A backbones comprising a monosaccharide backbone retaining the saccharide forming the reducing side of the lipid A domain and optionally a portion of the saccharide forming the non-reducing side of the lipid A domain.

As used herein, the terms **"fatty acid"** or **"fatty acid residue"** refer to aliphatic monocarboxylic acids which often, but not always, have an even number of carbon atoms. A fatty acid molecule has two distinct regions: a long hydrocarbon chain (which is hydrophobic and not very reactive chemically), and a carboxylic acid group (which is extremely hydrophilic and readily forms ester and amide linkages). In the context of LPS, fatty acids may be **"primary fatty acids"** which are esterified or amidated at positions C2, C3, C2' and C3' of the lipid A domain. Fatty acids can also be **"secondary fatty acids"** (also termed **"estolide-bound fatty acid"** or **"acyl-oxy-acyl fatty acid"),** *i.e.,* fatty acids esterified on other fatty acids, such as, *e.g.,* on a 3-OH group of a primary fatty acid. Fatty acids can be classified according to the length of their aliphatic chain, into short-chain, medium-chain, long-chain and very long chain fatty acids. As used herein, the terms **"short-chain fatty acids"** or **"SCFA"** refer to fatty acids with aliphatic tails of five or fewer carbons, such as 1, 2, 3, 4 or 5 carbons. As used herein, the terms **"medium-chain fatty acids"** or **"MCFA"** refer to fatty acids with aliphatic tails of 6 to 12 carbons, such as 6, 7, 8, 9, 10, 11 or 12 carbons. As used herein, the terms **"long-chain fatty acids"** or **"LCFA"** refer to fatty acids with aliphatic tails of 13 to 21 carbons, such as 13, 14, 15, 16, 17, 18, 19, 20 or 21 carbons. As used herein, the terms **"very long chain fatty acids"** or **"VLCFA"** refer to fatty acids with aliphatic tails of 22 or more carbons, such as 22, 23, 24, 25, 26, 27, 28, 29, 30 or more carbons. Fatty acids can additionally or alternatively be classified according to their degree of saturation, into saturated or unsaturated fatty acids. As used herein, the term **"saturated fatty**

**acid"** refers to a fatty acid which does not comprise any double -C=C- bond in its aliphatic chain. As used herein, the term **"unsaturated fatty acid"** refers to a fatty acid which comprises at least one or several double -C=C- bonds in its aliphatic chain. In such case, the double -C=C- bond may either be in *cis* or in *trans.*

As used herein, the terms **"core oligosaccharide"** or **"core-OS"** refer to a chain of sugar residues found in Gram-negative lipopolysaccharide (LPS). The composition of the core-OS domain is highly diverse among bacterial species and strains. The core-OS is typically divided into two regions, termed **"inner core"** and **"outer core",** the inner core attaching directly at position C6' of the lipid A domain (corresponding to $R_3$ of **Formula I)** through a ($\alpha$2→6) ketosidic bond.

As used herein, the term **"inner core"** refers to the first 3 to 6 sugar residues, which may be linear or branched. Typically, the inner core comprises, from a proximal to distal direction from position C6' of the lipid A domain (corresponding to $R_3$ of **Formula I),** a first 3-deoxy-D-*manno*-oct-2-ulosonic acid (Kdo) residue or D-glycero-D-*talo*-oct-2-ulosonic acid (Ko), typically substituted by one or two further sugar residues, in a linear or branched fashion, including, without limitation, 3-deoxy-D-*manno*-oct-2-ulosonic acid (Kdo), D-glycero-D-*talo*-oct-2-ulosonic acid (Ko) or 4-amino-4-deoxy-L-arabinose (Ara4N) residues, or a combination thereof. The most distal of these residues, or a single Kdo molecule, may be modified with a phosphate group or ethanolamine group. Distally from these residues, further hexose residues may be attached, including, but not limited to, from 2 to 3 heptose residues, optionally phosphorylated. A LPS molecular species comprising only a lipid A domain and an inner core region is conventionally referred to as **"deep-rough LPS".**

As used herein, the terms **"3-deoxy-D-*manno*-oct-2-ulosonic acid"** or **"Kdo"** refer to an ulosonic acid of a 2-ketooctose, with the following formula (and carbon numbering indicated in italics):

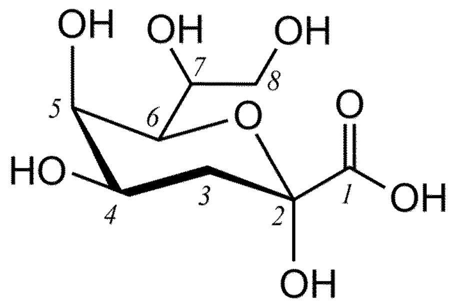

As used herein, the term **"outer core"** refers to a linear or branched hexose chain that is attached to the most distal sugar residue of the inner core, typically a heptose residue. This hexose chain typically comprises from 1 to 6 or more hexose residues, including, without limitation, glucose, mannose, galactose, and combinations thereof. A LPS molecular species that comprises a lipid A domain and a complete core-OS domain (*i.e.*, with an inner core and an outer core region) is conventionally referred to as **"rough-type LPS".**

As used herein, the term **"O-antigen"** refers to a polysaccharide typically contained within LPS molecular species, and attached to the core-OS domain. The composition of the O-antigen domain is highly diverse among bacterial species and strains. A LPS molecular species that comprises a lipid A domain, a complete core-OS domain (*i.e.*, with an inner core and an outer core region) and a O-antigen domain is conventionally referred to as **"smooth-type LPS".**

As used herein, the terms **"capping antigen"** or **"capping sugar"** refer to a saccharide moiety typically contained within LPS molecular species, and attached to the core-OS domain *in lieu* or in addition of the O-antigen domain. Such capping antigens are biosynthetically and genetically distinguished from a classical O-antigen domain. The composition of the capping antigen is highly diverse among bacterial species and strains.

As used herein, the term **"toxicity"** or **"pyrogenicity",** when referring to a LPS molecular species, means the ability of said LPS molecular species to elicit an acute or a chronic inflammatory response when administered to a subject. An acute or a chronic inflammatory response may be characterized by a production of inflammatory mediators including, without limitation, IL-6, IL-1$\beta$ and TNF-$\alpha$. According to the amount and structural characteristics of the administered LPS molecular species, these acute or a chronic inflammatory response can range from fever and chills to irreversible and fatal septic shock. Means and methods to assess the toxicity and/or pyrogenicity of a LPS molecular species are well-known in the art. Examples of such means and methods include, but are not limited to, *in vivo* tests such as, without limitation, rabbit pyrogen test (RPT), $C_{50}$ lethal dose in chicken embryo, $C_{50}$ lethal dose in GalN sensitized mouse, and acute toxicity test in normal mouse; and *in vitro* tests such as, without limitation, monocyte activation test (MAT).

The rabbit pyrogen test (RPT), performed according to European Pharmacopeia (Ph.Eur. 5.1.10, this section being incorporated herein by reference), involves measuring the rise in temperature of three rabbits following the intravenous injection of the test solution (such as, a composition comprising LPS to be tested). Temperature is first measured before injection of the test solution; the test solution is then injected and body temperature of each rabbit is measured for 3 hours. If the sum of the temperature of the three rabbits increases by less than 1.15°C, the test solution is

considered to be non-pyrogenic; if the sum of the temperature of the three rabbits increases by more than 2.65°C, the test solution is considered to be pyrogenic; if the sum of the temperature of the three rabbits increases between 1.15°C and 2.65°C, the test should be repeated to confirm the results.

The monocyte activation test (MAT) has been described as a compendial method for Pyrogen Detection in the European Pharmacopeia (January 2010 - Ph. Eur. 9.8 section 2.6.30, this section being incorporated herein by reference). MAT is based on the activation of monocytes by pyrogens, leading to the production of cytokines that are detected in an immunological assay (such as, *e.g.,* an ELISA). There are different variants of the MAT available depending on (1) the source of monocytes, including whole blood, isolated primary monocytes (such as, *e.g.,* PBMC) or monocytic cell line; and (2) the ELISA read-out, including IL-6, IL-1β and TNF-α. All of them mimic the human fever reaction *in vitro.*

For more details on means and methods to assess the toxicity and/or pyrogenicity of a LPS molecular species, see chapter 5.1.10 and chapter 2.6.30 of the European Pharmacopoeia, these sections being incorporated herein by reference.

**DETAILED DESCRIPTION**

The present invention relates to modified LPS molecular species and enriched populations comprising the same.

In one embodiment, the enriched population of modified LPS molecular species comprises at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or more modified LPS molecular species according to the present invention out of the total amount of LPS molecular species in the population.

According to the present invention, the modified LPS molecular species are devoid of phosphate group at position C1 of the reducing end of their lipid A domain; and are substituted at position C6' of the non-reducing end of their lipid A domain by a monosaccharide, an oligosaccharide or a polysaccharide moiety.

According to the present invention, the modified LPS molecular species may alternatively be devoid of phosphate group at position C4' of the non-reducing end of their lipid A domain; and be substituted at position C6' of the non-reducing end of their lipid A domain by a monosaccharide, an oligosaccharide or a polysaccharide moiety.

According to the present invention, the modified LPS molecular species may alternatively be devoid of phosphate group at positions C1 of the reducing end and C4' of the non-reducing end of their lipid A domain; and be substituted at position C6' of the non-reducing end of their lipid A domain by a monosaccharide, an oligosaccharide or a polysaccharide moiety.

In one embodiment, the modified LPS molecular species further comprise a smaller or greater number of fatty acid chains as compared to classical (*i.e.,* non-modified) *Escherichia coli* or *Salmonella enterica* LPS molecules.

In one embodiment, the modified LPS molecular species further comprise fatty acid chains of shorter and/or longer length as compared to classical (*i.e.,* non-modified) *Escherichia coli* or *Salmonella enterica* LPS molecules.

In one embodiment, the modified LPS molecular species are of general **Formula A:**

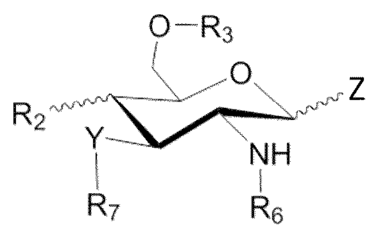

**Formula A**

wherein:

- Yis -O- or -NH-;
- $R_2$ is a phosphate group ($-OPO_3H_2$) or a hydroxyl group (-OH); optionally substituted;
- $R_3$ is a monosaccharide, an oligosaccharide or a polysaccharide; optionally substituted; preferably $R_3$ is a core oligosaccharide (*i.e.,* an inner core, or an inner and outer core) optionally substituted with a O-antigen domain;
- $R_6$ is a fatty acid residue; optionally substituted;
- $R_7$ is a hydrogen or a fatty acid residue; optionally substituted;
- Z is a hydroxyl group (-OH), optionally substituted, with the proviso that, if substituted, the substitution does not consist of a phosphate group ($-OPO_3H_2$), or

Z is

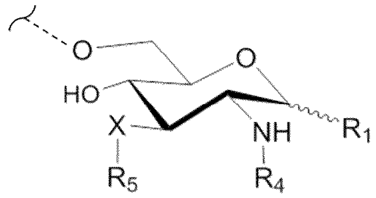

,

wherein
X is -O- or -NH-;
$R_1$ is a hydroxyl group (-OH), optionally substituted, with the proviso that, if substituted, the substitution does not consist of a phosphate group ($-OPO_3H_2$);
$R_4$ is a fatty acid residue; optionally substituted; and
$R_5$ is a hydrogen or a fatty acid residue; optionally substituted.

In one embodiment, the modified LPS molecular species are of general **Formula A:**

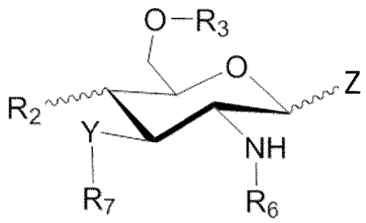

**Formula A**

wherein:

Y is -O- or -NH-;
$R_2$ is a hydroxyl group (-OH), optionally substituted, with the proviso that, if substituted, the substitution does not consist of a phosphate group ($-OPO_3H_2$);
$R_3$ is a monosaccharide, an oligosaccharide or a polysaccharide; optionally substituted; preferably $R_3$ is a core oligosaccharide (*i.e.,* an inner core, or an inner and outer core) optionally substituted with a O-antigen domain;
$R_6$ is a fatty acid residue; optionally substituted;
$R_7$ is a hydrogen or a fatty acid residue; optionally substituted;
Z is a hydroxyl group (-OH), optionally substituted, with the proviso that, if substituted, the substitution does not consist of a phosphate group ($-OPO_3H_2$), or
Z is

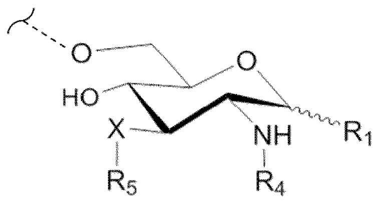

,

wherein
X is -O- or -NH-;
$R_1$ is a hydroxyl group (-OH), optionally substituted, with the proviso that, if substituted, the substitution does not consist of a phosphate group ($-OPO_3H_2$);
$R_4$ is a fatty acid residue; optionally substituted; and
$R_5$ is a hydrogen or a fatty acid residue; optionally substituted.

In one embodiment, the modified LPS molecular species are of general **Formula B:**

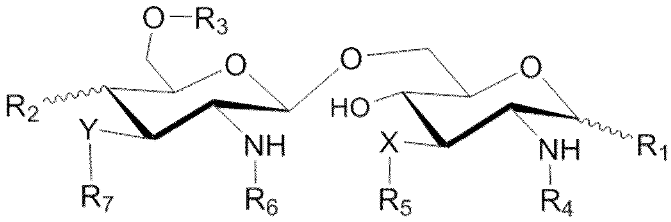

**Formula B**

wherein:

X and Y are, independently from each other, -O- or -NH-;
$R_1$ is a hydroxyl group (-OH), optionally substituted, with the proviso that, if substituted, the substitution does not consist of a phosphate group (-$OPO_3H_2$);
$R_2$ is a phosphate group (-$OPO_3H_2$) or a hydroxyl group (-OH); optionally substituted;
$R_3$ is a monosaccharide, an oligosaccharide or a polysaccharide; optionally substituted; preferably $R_3$ is a core oligosaccharide (*i.e.,* an inner core, or an inner and outer core) optionally substituted with a O-antigen domain;
$R_4$ and $R_6$ are, independently from each other, a fatty acid residue; optionally substituted; and
$R_5$ and $R_7$ are, independently from each other, a hydrogen or a fatty acid residue;
optionally substituted.

In one embodiment, the modified LPS molecular species are of general **Formula B:**

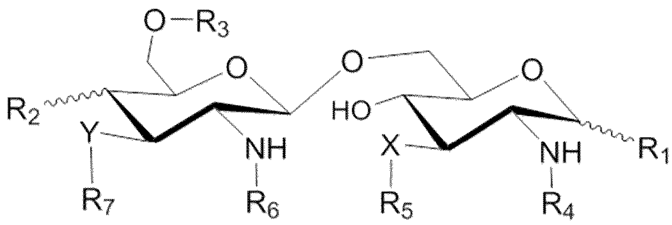

**Formula B**

wherein:

X and Y are, independently from each other, -O- or -NH-;
$R_1$ is a phosphate group (-$OPO_3H_2$) or a hydroxyl group (-OH); optionally substituted;
$R_2$ is a hydroxyl group (-OH), optionally substituted, with the proviso that, if substituted, the substitution does not consist of a phosphate group (-$OPO_3H_2$);
$R_3$ is a monosaccharide, an oligosaccharide or a polysaccharide; optionally substituted; preferably $R_3$ is a core oligosaccharide (*i.e.,* an inner core, or an inner and outer core) optionally substituted with a O-antigen domain;
$R_4$ and $R_6$ are, independently from each other, a fatty acid residue; optionally substituted; and
$R_5$ and $R_7$ are, independently from each other, a hydrogen or a fatty acid residue;
optionally substituted.

In one embodiment, the modified LPS molecular species are of general **Formula B:**

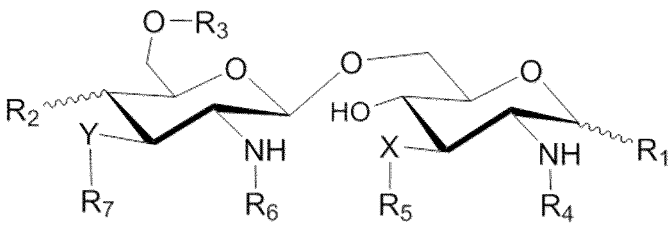

**Formula B**

wherein:

X and Y are, independently from each other, -O- or -NH-;
$R_1$ and $R_2$ are a hydroxyl group (-OH), optionally substituted, with the proviso that, if substituted, the substitution does not consist of a phosphate group ($-OPO_3H_2$);
$R_3$ is a monosaccharide, an oligosaccharide or a polysaccharide; optionally substituted; preferably $R_3$ is a core oligosaccharide (*i.e.,* an inner core, or an inner and outer core) optionally substituted with a O-antigen domain;
$R_4$ and $R_6$ are, independently from each other, a fatty acid residue; optionally substituted; and
$R_5$ and $R_7$ are, independently from each other, a hydrogen or a fatty acid residue;
optionally substituted.

In one embodiment, the modified LPS molecular species are of general **Formula A:**

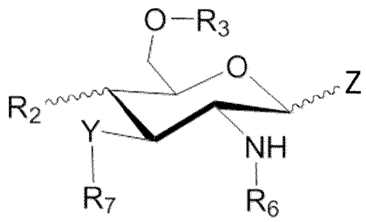

**Formula A**

wherein:

Y is -O- or -NH-;
$R_2$ is a phosphate group ($-OPO_3H_2$) or a hydroxyl group (-OH); optionally substituted;
$R_3$ is a monosaccharide, an oligosaccharide or a polysaccharide; optionally substituted; preferably $R_3$ is a core oligosaccharide (*i.e.,* an inner core, or an inner and outer core) optionally substituted with a O-antigen domain;
$R_6$ is a fatty acid residue; optionally substituted;
$R_7$ is a hydrogen or a fatty acid residue; optionally substituted;
Z is a hydroxyl group (-OH).

In one embodiment, X is -O- and Y is -O-. In this embodiment, the disaccharide of the lipid A domain is therefore a β-1,6-glucosamine [GlcN] disaccharide.
In one embodiment, X is -NH- and Y is -NH-. In this embodiment, the disaccharide of the lipid A domain is therefore a β-1,6-diaminoglucose [DAG] disaccharide.
In one embodiment, X is -O- and Y is -NH-. In an alternative embodiment, X is -NH-and Y is -O-. In these embodiments, the disaccharide of the lipid A domain is therefore a β-1,6-disaccharide comprising one GlcN residue and one DAG residue.
In one embodiment, Z is a hydroxyl group (-OH).
In one embodiment, $R_2$ is a hydroxyl group (-OH). In one embodiment, Z and $R_2$ are a hydroxyl group (-OH).
In one embodiment, Z is a hydroxyl group (-OH), optionally substituted, with the proviso that, if substituted, the substitution does not consist of a phosphate group ($-OPO_3H_2$). In one embodiment, $R_2$ is a hydroxyl group (-OH), optionally substituted. In one embodiment, Z and $R_2$ are a hydroxyl group (-OH), optionally substituted, with the proviso that, if substituted, the substitution of Z does not consist of a phosphate group ($-OPO_3H_2$).
In one embodiment, Z is a phosphate group ($-OPO_3H_2$), with the proviso that $R_2$ is not a phosphate group. In one embodiment, $R_2$ is a phosphate group ($-OPO_3H_2$), with the proviso that Z is not a phosphate group.
In one embodiment, Z is

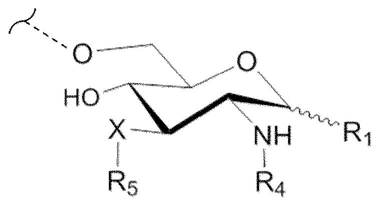

,

wherein X is as defined above and wherein $R_1$, $R_4$, $R_5$ are as defined below.

In one embodiment, $R_1$ is a hydroxyl group (-OH). In one embodiment, $R_2$ is a hydroxyl group (-OH). In one embodiment, $R_1$ and $R_2$ are a hydroxyl group (-OH).

In one embodiment, $R_1$ is a hydroxyl group (-OH), optionally substituted, with the proviso that, if substituted, the substitution does not consist of a phosphate group ($-OPO_3H_2$). In one embodiment, $R_2$ is a hydroxyl group (-OH), optionally substituted. In one embodiment, $R_1$ and $R_2$ are a hydroxyl group (-OH), optionally substituted, with the proviso that, if substituted, the substitution of $R_1$ does not consist of a phosphate group ($-OPO_3H_2$).

In one embodiment, $R_1$ is a phosphate group ($-OPO_3H_2$), with the proviso that $R_2$ is not a phosphate group. In one embodiment, $R_2$ is a phosphate group ($-OPO_3H_2$), with the proviso that $R_1$ is not a phosphate group.

In one embodiment, Z is a hydroxyl group (-OH), substituted with further substituents, with the proviso that the substitution does not consist of a phosphate group ($-OPO_3H_2$). In one embodiment, $R_1$ is a hydroxyl group (-OH), substituted with further substituents, with the proviso that the substitution does not consist of a phosphate group ($-OPO_3H_2$). In one embodiment, $R_2$ is a hydroxyl group (-OH), substituted with further substituents.

In one embodiment, Z is a phosphate group ($-OPO_3H_2$), substituted with further substituents. In one embodiment, $R_1$ is a phosphate group ($-OPO_3H_2$), substituted with further substituents. In one embodiment, $R_2$ is a phosphate group ($-OPO_3H_2$), substituted with further substituents.

Such further substituents include, but are not limited to, an ethanolamine group "EA" ($-CH_2-CH_2-NH_2$), a phosphoryl ethanolamine group "PEA" ($-OPO_3H-CH_2-CH_2-NH_2$), a diphosphoryl ethanolamine group "PPEA" ($-OPO_3H_2-PO_3H-CH_2-CH_2-NH_2$), a 4-amino-4-deoxy-L-arabinose (LAra*p*4N) residue, a glucosamine residue, a galactosamine residue, and the like. Additionally, such further substituents include, but are not limited to, an alkyl group, an aminoalkyl group, an aryl group, a carboxyalkyl group, a cycloalkyl group, an ether or polyether group, an haloalkyl group, an heteroaryl group, a nitrooxyalkyl group, and the like.

The term **"alkyl"** as used herein by itself or as part of another substituent refers to a hydrocarbyl radical of formula $C_nH_{2n+1}$ wherein n is a number greater than or equal to 1. In one embodiment, alkyl groups comprise from 1 to 8 carbon atoms, preferably from 1 to 6 carbon atoms, more preferably from 1 to 4 carbon atoms, more preferably from 1 to 3 carbon atoms. Alkyl groups may be linear or branched. Examples of alkyl groups include, but are not limited to, methyl, ethyl, propyl, butyl, pentyl, hexyl, heptyl, octyl, and the like.

The term **"aminoalkyl"** as used herein by itself or as part of another substituent refers to a group $-alkyl-NH_2$ wherein alkyl is as herein defined.

The term **"aryl"** as used herein by itself or as part of another substituent refers to a polyunsaturated, aromatic hydrocarbyl group having a single ring (*i.e.,* phenyl) or multiple aromatic rings fused together (*e.g.,* naphtyl), typically containing 5 to 12 atoms; preferably 6 to 10. Non-limiting examples of aryl comprise phenyl, naphthalenyl, and the like.

The term **"carboxyalkyl"** as used herein by itself or as part of another substituent refers to a group -alkyl-COOH wherein alkyl is as herein defined.

The term **"cycloalkyl"** as used herein by itself or as part of another substituent refers to a saturated cyclic hydrocarbyl radical of formula $C_nH_{2n-1}$ wherein n is a number greater than or equal to 1, or to multiple saturated cyclic hydrocarbyl rings fused together. In one embodiment, cycloalkyl groups comprise from 1 to 8 carbon atoms, preferably from 1 to 6 carbon atoms, more preferably from 1 to 5 carbon atoms.

The term **"ether"** as used herein by itself or as part of another substituent refers to a hydrocarbyl radical having an oxygen atom bonded to two carbon atoms. When Ethers can be linear or branched. Examples of ethers include, but are not limited to, dimethyl ether, diethyl ether, dipropyl ether, dibutyl ether, dipentyl ether, dihexyl ether, diheptyl ether, dioctyl ether, and the like. Where the ether group is repeated in a polymeric fashion, one refers to **"polyether".** Examples of polyethers include, but are not limited to, polyethylene glycol ($(-CH_2CH_2O-)_n$), polypropylene glycol ($(-CH_2CH(CH_3)O-)_n$), polytetramethylene glycol ($(-CH_2CH_2CH_2CH_2O-)_n$), paraformaldehyde ($(-CH_2O-)_n$), and the like.

The term **"haloalkyl"** itself or as part of another substituent, refers to an alkyl radical as herein defined wherein one or more hydrogens are replaced with a halogen as herein defined. The term **"halogen"** means fluoro, chloro, bromo, or iodo. Non-limiting examples of haloalkyl include fluoromethyl, difluoromethyl, trifluoromethyl, and the like.

The term **"heteroaryl"** as used herein by itself or as part of another substituent refers to an aryl group as herein defined wherein at least one carbon atom is replaced with a heteroatom. In other words, it refers to 5 to 12 carbon-atom aromatic single rings or ring systems containing 2 rings which are fused together, typically containing 5 to 6 atoms; in which one or more carbon atoms is replaced by oxygen, nitrogen and/or sulfur atoms where the nitrogen and sulfur heteroatoms may optionally be oxidized and the nitrogen heteroatoms may optionally be quaternized. Non-limiting examples of such heteroaryl, include: oxazolyl, thiazolyl, imidazolyl, furanyl and pyrrolyl.

The term **"nitrooxyalkyl"** as used herein by itself or as part of another substituent refers to a group $-alkyl-ONO_2$ wherein alkyl is as herein defined.

In one embodiment, Z is a hydroxyl group (-OH), substituted with a therapeutic agent. In one embodiment, $R_1$ is a hydroxyl group (-OH), substituted with a therapeutic agent. In one embodiment, $R_2$ is a hydroxyl group (-OH), substituted with a therapeutic agent. In this embodiment, any suitable therapeutic agent may be conjugated to LPS molecular species,

either directly or through a suitable linker.

Means and methods to conjugate therapeutic agents with LPS molecular species are well-known in the art. Examples of suitable therapeutic agents to be conjugated with LPS molecular species include, but are not limited to, peptides, polypeptides, proteins, polymers, nucleic acid molecules, small molecules, mimetic agents, synthetic drugs, inorganic molecules, organic molecules, radioisotopes, nanoparticles, vectors, and the like.

Alternatively, or additionally, examples of suitable therapeutic agents to be conjugated with LPS molecular species include, but are not limited to, chemotherapeutic agents, targeted therapy agents, cytotoxic agents, antibiotics, antivirals, cell cycle-synchronizing agents, ligands for cellular receptor(s), immunomodulatory agents, pro-apoptotic agents, anti-angiogenic agents, cytokines, growth factors, antibodies or antigen-binding fragments thereof, antigens, and the like.

It will be apparent that some therapeutic agents may fall into more than one category.

In one embodiment, the therapeutic agent is a chemotherapeutic agent.

As used herein, the term **"chemotherapeutic agent"** refers to any molecule that is effective in inhibiting tumor growth.

Suitable examples of chemotherapeutic agents include those described under subgroup L01 of the Anatomical Therapeutic Chemical Classification System.

Suitable examples of chemotherapeutic agents include, but are not limited to:

i. alkylating agents, such as, *e.g.:*

- nitrogen mustards, including chlormethine, cyclophosphamide, ifosfamide, trofosfamide, chlorambucil, melphalan, prednimustine, bendamustine, uramustine, chlomaphazine, cholophosphamide, estramustine, mechlorethamine, mechlorethamine oxide hydrochloride, novembichin, phenesterine, uracil mustard and the like;
- nitrosoureas, including carmustine, lomustine, semustine, fotemustine, nimustine, ranimustine, streptozocin, chlorozotocin, and the like;
- alkyl sulfonates, including busulfan, mannosulfan, treosulfan, and the like;
- aziridines, including carboquone, thiotepa, triaziquone, triethylenemelamine, benzodopa, meturedopa, uredopa, and the like; hydrazines, including procarbazine, and the like;
- triazenes, including dacarbazine, temozolomide, and the like; ethylenimines and methylamelamines, including altretamine, triethylenemelamine, trietylenephosphoramide, triethylenethiophosphaoramide, trimethylolomelamine and the like;
- and others, including mitobronitol, pipobroman, actinomycin, bleomycin, mitomycins (including mitomycin C, and the like), plicamycin, and the like;

ii. acetogenins, such as, *e.g.,* bullatacin, bullatacinone, and the like;

iii. benzodiazepines, such as, *e.g.,* 2-oxoquazepam, 3-hydroxyphenazepam, bromazepam, camazepam, carburazepam, chlordiazepoxide, cinazepam, cinolazepam, clonazepam, cloniprazepam, clorazepate, cyprazepam, delorazepam, demoxepam, desmethylflunitrazepam, devazepide, diazepam, diclazepam, difludiazepam, doxefazepam, elfazepam, ethyl carfluzepate, ethyl dirazepate, ethyl loflazepate, flubromazepam, fletazepam, fludiazepam, flunitrazepam, flurazepam, flutemazepam, flutoprazepam, fosazepam, gidazepam, halazepam, iclazepam, irazepine, kenazepine, ketazolam, lorazepam, lormetazepam, lufuradom, meclonazepam, medazepam, menitrazepam, metaclazepam, motrazepam, N-desalkylflurazepam, nifoxipam, nimetazepam, nitemazepam, nitrazepam, nitrazepate, nordazepam, nortetrazepam, oxazepam, phenazepam, pinazepam, pivoxazepam, prazepam, proflazepam, quazepam, QH-II-66, reclazepam, RO4491533, Ro5-4864, SH-I-048A, sulazepam, temazepam, tetrazepam, tifluadom, tolufazepam, triflunordazepam, tuclazepam, uldazepam, arfendazam, clobazam, CP-1414S, lofendazam, triflubazam, girisopam, GYKI-52466, GYKI-52895, nerisopam, talampanel, tofisopam, adinazolam, alprazolam, bromazolam, clonazolam, estazolam, flualprazolam, flubromazolam, flunitrazolam, nitrazolam, pyrazolam, triazolam, bretazenil, climazolam, EVT-201, FG-8205, flumazenil, GL-II-73, imidazenil, $^{123}$I-iomazenil, L-655,708, loprazolam, midazolam, PWZ-029, remimazolam, Ro15-4513, Ro48-6791, Ro48-8684, Ro4938581, sarmazenil, SH-053-R-CH3_2'F, cloxazolam, flutazolam, haloxazolam, mexazolam, oxazolam, bentazepam, clotiazepam, brotizolam, ciclotizolam, deschloroetizolam, etizolam, fluclotizolam, israpafant, JQ1, metizolam, olanzapine, telenzepine, lopirazepam, zapizolam, razobazam, ripazepam, zolazepam, zomebazam, zometapine, premazepam, clazolam, anthramycin, avizafone, rilmazafone, and the like;

iv. antimetabolites, such as, *e.g.:*

- antifolates, including aminopterin, methotrexate, pemetrexed, pralatrexate, pteropterin, raltitrexed, denopterin, trimetrexate, pemetrexed, and the like;
- purine analogues, including pentostatin, cladribine, clofarabine, fludarabine, nelarabine, tioguanine, mercaptopurine, and the like;

- pyrimidine analogues, including fluorouracil, capecitabine, doxifluridine, tegafur, tegafur/gimeracil/oteracil, carmofur, floxuridine, cytarabine, gemcitabine, azacytidine, decitabine, and the like; and
- hydroxycarbamide;

v. androgens, such as, *e.g.,* calusterone, dromostanolone propionate, epitiostanol, mepitiostane, testolactone, and the like;
vi. anti-adrenals, such as, *e.g.,* aminoglutethimide, mitotane, trilostane, and the like;
vii. folic acid replenishers, such as, *e.g.,* frolinic acid, and the like;
viii. maytansinoids, such as, *e.g.,* maytansine, ansamitocins, and the like;
ix. platinum analogs, such as, *e.g.,* platinum, carboplatin, cisplatin, dicycloplatin, nedaplatin, oxaliplatin, satraplatin, and the like;
x. antihormonal agents, such as, *e.g.:*

- anti-estrogens, including tamoxifen, raloxifene, aromatase inhibiting 4(5)-imidazoles, 4-hydroxytamoxifen, trioxifene, keoxifene, LY117018, onapristone, toremifene, and the like;
- anti-androgens, including flutamide, nilutamide, bicalutamide, leuprolide, goserelin, and the like;

xi. trichothecenes, such as, *e.g.,* T-2 toxin, verracurin A, roridinA, anguidine and the like;
xii. toxoids, such as, *e.g.,* cabazitaxel, docetaxel, larotaxel, ortataxel, paclitaxel, tesetaxel, and the like;
xiii. others, such as, *e.g.,* camptothecin (including the synthetic analogue topotecan); bryostatin; callystatin; CC-1065 (including its adozelesin, carzelesin and bizelesin synthetic analogues); cryptophycins (including cryptophycin 1 and cryptophycin 8); dolastatin; duocarmycin (including its synthetic analogues KW-2189 and CBI-TMI); eleutherobin; pancratistatin; sarcodictyin; spongistatin; aclacinomysins; authramycin; azaserine; bleomycin; cactinomycin; carabicin; canninomycin; carzinophilin; chromomycins; dactinomycin; daunorubicin; detorubicin; 6-diazo-5-oxo-L-norleucine; doxorubicin (including morpholino-doxorubicin, cyanomorpholino-doxorubicin, 2-pyrrolino-doxorubicin, deoxydoxorubicin, and the like); epirubicin; esorubicin; idanrbicin; marcellomycin; mycophenolic acid; nogalarnycin; olivomycins; peplomycin; potfiromycin; puromycin; quelamycin; rodorubicin; streptomgrin; streptozocin; tubercidin; ubenimex; zinostatin; zorubicin; aceglatone; aldophospharnide glycoside; aminolevulinic acid; amsacrine; bestrabucil; bisantrene; edatraxate; defofamine; demecolcine; diaziquone; elfomithine; elliptinium acetate; epothilone; etoglucid; gallium nitrate; hydroxyurea; lentinan; lonidamine; mitoguazone; mitoxantrone; mopidamol; nitracrine; phenamet; pirarubicin; podophyllinic acid; 2-ethylhydrazide; PSK®; razoxane; rhizoxin; sizofiran; spirogennanium; tenuazonic acid; 2,2',2"-trichlorotriethylamine; urethan; vindesine; dacarbazine; mannomustine; mitobromtol; mitolactol; pipobroman; gacytosine; arabinoside; 6-thioguanine; vinblastine; etoposide; vincristine; vinorelbine; navelbine; novantrone; teniposide; daunomycin; xeloda; ibandronate; CPT-11; topoisomerase inhibitor RFS 2000; topoisomerase I inhibitor SN38; difluoromethylomithine; retinoic acid; and the like.

In one embodiment, the therapeutic agent is a targeted therapy agent.

As used herein, the term **"targeted therapy agent"** refers to any molecule which aims at one or more particular target molecules (such as, *e.g.,* proteins) involved in tumor genesis, tumor progression, tumor metastasis, tumor cell proliferation, cell repair, and the like.

Suitable examples of targeted therapy agents include, but are not limited to, tyrosine-kinase inhibitors, serine/threonine kinase inhibitors, monoclonal antibodies and the like.

Suitable examples of targeted therapy agents include, but are not limited to, HER1/EGFR inhibitors (such as, *e.g.,* brigatinib, erlotinib, gefitinib, olmutinib, osimertinib, rociletinib, vandetanib, and the like); HER2/neu inhibitors (such as, *e.g.,* afatinib, lapatinib, neratinib, and the like); C-kit and PDGFR inhibitors (such as, *e.g.,* axitinib, masitinib, pazopanib, sunitinib, sorafenib, toceranib, and the like); FLT3 inhibitors (such as, *e.g.,* lestaurtinib, and the like); VEGFR inhibitors (such as, *e.g.,* axitinib, cediranib, lenvatinib, nintedanib, pazopanib, regorafenib, semaxanib, sorafenib, sunitinib, tivozanib, toceranib, vandetanib, and the like); RET inhibitors (such as, *e.g.,* vandetanib, entrectinib, and the like); c-MET inhibitors (such as, *e.g.,* cabozantinib, and the like); bcr-abl inhibitors (such as, *e.g.,* imatinib, dasatinib, nilotinib, ponatinib, radotinib, and the like); Src inhibitors (such as, *e.g.,* bosutinib, dasatinib, and the like); Janus kinase inhibitors (such as, *e.g.,* lestaurtinib, momelotinib, ruxolitinib, pacritinib, and the like); MAP2K inhibitors (such as, *e.g.,* cobimetinib, selumetinib, trametinib, binimetinib, and the like); EML4-ALK inhibitors (such as, *e.g.,* alectinib, brigatinib, ceritinib, crizotinib, and the like); Bruton's inhibitors (such as, *e.g.,* ibrutinib, and the like); mTOR inhibitors (such as, *e.g.,* everolimus, temsirolimus, and the like); hedgehog inhibitors (such as, *e.g.,* sonidegib, vismodegib, and the like); CDK inhibitors (such as, *e.g.,* palbociclib, ribociclib, and the like); anti-HER1/EGFR monoclonal antibodies (such as, *e.g.,* cetuximab, necitumumab, panitumumab, and the like); anti-HER2/neu monoclonal antibodies (such as, *e.g.,* ado-trastuzumab emtansine, pertuzumab, trastuzumab, trastuzumab-dkst, and the like); anti-EpCAM monoclonal antibodies

(such as, *e.g.,* catumaxomab, edrecolomab, and the like); anti-VEGF monoclonal antibodies (such as, *e.g.,* bevacizumab, bevacizumab-awwb, and the like); anti-CD20 monoclonal antibodies (such as, *e.g.,* ibritumomab, obinutuzumab, ocrelizumab, ofatumumab, rituximab, tositumomab, and the like); anti-CD30 monoclonal antibodies (such as, *e.g.,* brentuximab, and the like); anti-CD33 monoclonal antibodies (such as, *e.g.,* gemtuzumab, and the like); anti-CD52 monoclonal antibodies (such as, *e.g.,* alemtuzumab, and the like); and antigen-binding fragments thereof.

In one embodiment, the therapeutic agent is a cytotoxic agent.

As used herein, the term **"cytotoxic agent"** refers to any molecule that results in cell death by any mechanism.

Suitable examples of cytotoxic agents include, but are not limited to, taxanes, anthracyclines, alkylating agents, vinca alkaloids, antimetabolites, platinum agents, steroids, and chemotherapeutic agents.

Suitable examples of taxanes include, but are not limited to, cabazitaxel, docetaxel, larotaxel, ortataxel, paclitaxel and tesetaxel.

Suitable examples of anthracyclines include, but are not limited to, aclarubicin, amrubicin, daunorubicin, doxorubicin, epirubicin, idarubicin, pirarubicin, valrubicin and zorubicin.

Suitable examples of alkylating agent include, but are not limited to, nitrogen mustards (such as, *e.g.,* chlormethine, cyclophosphamide, ifosfamide, trofosfamide, chlorambucil, melphalan, prednimustine, bendamustine, uramustine, and the like), nitrosoureas (such as, *e.g.,* carmustine, lomustine, semustine, fotemustine, nimustine, ranimustine, streptozocin, and the like), alkyl sulfonates (such as, *e.g.,* busulfan, mannosulfan, treosulfan, and the like), aziridines (such as, *e.g.,* carboquone, thiotepa, triaziquone, triethylenemelamine, benzodopa, meturedopa, uredopa, and the like), hydrazines (such as, *e.g.,* procarbazine, and the like), triazenes (such as, *e.g.,* dacarbazine, temozolomide, and the like), altretamine, mitobronitol, pipobroman, actinomycin, bleomycin, mitomycins and plicamycin.

Suitable examples of vinca alkaloids include, but are not limited to, vinblastine, vincristine, vinflunine, vindesine and vinorelbine.

Suitable examples of antimetabolites include, but are not limited to, antifolates (such as, aminopterin, methotrexate, pemetrexed, pralatrexate, raltitrexed, pemetrexed, and the like), purine analogues (such as, *e.g.,* pentostatin, cladribine, clofarabine, fludarabine, nelarabine, tioguanine, mercaptopurine, and the like), pyrimidine analogues (such as, *e.g.,* fluorouracil, capecitabine, doxifluridine, tegafur, tegafur/gimeracil/oteracil, carmofur, floxuridine, cytarabine, gemcitabine, azacytidine, decitabine, and the like), and hydroxycarbamide.

Suitable examples of platinum agents include, but are not limited to, carboplatin, cisplatin, dicycloplatin, nedaplatin, oxaliplatin and satraplatin.

Suitable examples of steroids include, but are not limited to, estrogen receptor modulators, androgen receptor modulators and progesterone receptor modulators.

Suitable examples of chemotherapeutic agents have been described hereinabove.

In one embodiment, the therapeutic agent is an antibiotic.

Suitable examples of antibiotics include those described under subgroup J01 of the Anatomical Therapeutic Chemical Classification System.

Suitable examples of antibiotics include, but are not limited to:

i. aminoglycosides, such as, *e.g.,* amikacin, gentamicin, kanamycin, neomycin, netilmicin, streptomycin, tobramycin, paromycin, and the like;
ii. ansamycins, such as, *e.g.,* geldanamycin, herbimycin and the like;
iii. carbacephems, such as, *e.g.,* loracarbef and the like;
iv. carbapenems, such as, *e.g.,* ertapenum, doripenem, imipenem, cilastatin, meropenem, and the like;
v. first generation cephalosporins, such as, *e.g.,* cefadroxil, cefazolin, cefalotin, cephalexin, and the like;
vi. second generation cephalosporins, such as, *e.g.,* ceflaclor, cefamandole, cefoxitin, cefprozil, cefuroxime, and the like;
vii. third generation cephalosporins, such as, *e.g.,* cefixime, cefdinir, cefditoren, cefoperazone, cefotaxime, cefpodoxime, ceftazidime, ceftibuten, ceftizoxime, ceftriaxone, and the like;
viii. fourth generation cephalosporins, such as, *e.g.,* cefepime and the like;
ix. fifth generation cephalosporins, such as, *e.g.,* ceftobiprole, and the like;
x. glycopeptides, such as, *e.g.,* teicoplanin, vancomycin, and the like;
xi. macrolides, such as, *e.g.,* axithromycin, clarithromycin, dirithromycine, erythromycin, roxithromycin, troleandomycin, telithromycin, spectinomycin, and the like;
xii. monobactams, such as, *e.g.,* axtreonam, and the like;
xiii. penicilins, such as, *e.g.,* amoxicillin, ampicillin, axlocillin, carbenicillin, cloxacillin, dicloxacillin, flucloxacillin, mezlocillin, meticillin, nafcilin, oxacillin, penicillin, peperacillin, ticarcillin, and the like;
xiv. antibiotic polypeptides, such as, *e.g.,* bacitracin, colistin, polymyxin B, and the like;
xv. quinolones, such as, *e.g.,* ciprofloxacin, enoxacin, gatifloxacin, levofloxacin, lemefloxacin, moxifloxacin, norfloxacin, orfloxacin, trovafloxacin, and the like;

xvi. sulfonamides, such as, *e.g.,* mafenide, prontosil, sulfacetamide, sulfamethizole, sulfanilamide, sulfasalazine, sulfisoxazole, trimethoprim, trimethoprim-sulfamethoxazole, and the like;
xvii. tetracyclines, such as, *e.g.,* demeclocycline, doxycycline, minocycline, oxytetracycline, tetracycline, and the like; and
xviii. others such as, *e.g.,* arspenamine, chloramphenicol, clindamycin, lincomycin, ethambutol, fosfomycin, fusidic acid, furazolidone, isoniazid, linezolid, metronidazole, mupirocin, nitrofurantoin, platensimycin, pyrazinamide, quinupristin/dalfopristin, rifampin/rifampicin, tinidazole, and the like.

In one embodiment, the therapeutic agent is an antiviral.

Suitable examples of antivirals include those described under subgroup J05 of the Anatomical Therapeutic Chemical Classification System.

Suitable examples of antivirals include, but are not limited to, acemannan, acyclovir, acyclovir sodium, adamantanamine, adefovir, adenine arabinoside, alovudine, alvircept sudotox, amantadine hydrochloride, aranotin, arildone, atevirdine mesylate, avridine, cidofovir, cipamfylline, cytarabine hydrochloride, BMS 806, C31G, carrageenan, zinc salts, cellulose sulfate, cyclodextrins, dapivirine, delavirdine mesylate, desciclovir, dextrin 2-sulfate, didanosine, disoxaril, dolutegravir, edoxudine, enviradene, envirozime, etravirine, famciclovir, famotine hydrochloride, fiacitabine, fialuridine, fosarilate, foscarnet sodium, fosfonet sodium, FTC, ganciclovir, ganciclovir sodium, GSK 1265744, 9-2-hydroxy-ethoxy methylguanine, ibalizumab, idoxuridine, interferon, 5-iodo-2'-deoxyuridine, IQP-0528, kethoxal, lamivudine, lobucavir, maraviroc, memotine pirodavir, penciclovir, raltegravir, ribavirin, rimantadine hydrochloride, rilpivirine (TMC-278), saquinavir mesylate, SCH-C, SCH-D, somantadine hydrochloride, sorivudine, statolon, stavudine, T20, tilorone hydrochloride, TMC120, TMC125, trifluridine, trifluorothymidine, tenofovir, tenofovir alefenamide, tenofovir disoproxyl fumarate, prodrugs of tenofovir, UC-781, UK-427, UK-857, valacyclovir, valacyclovir hydrochloride, vidarabine, vidarabine phosphate, vidarabine sodium phosphate, viroxime, zalcitabene, zidovudine, and zinviroxime.

In one embodiment, the therapeutic agent is a cell cycle-synchronizing agent.

As used herein, the term **"cell cycle-synchronizing agent"** refers to any molecule able to for unify the cell cycle of a population of cells to the same phase upon administration.

Suitable examples of cell cycle-synchronizing agents include, but are not limited to, aphidicolin, butyrolactone I, colchicine, cycloheximide, demecolcine, dimethyl sulfoxide, 5-fluorodeoxyuridine, Hoechst 33342, mimosine, nocodazole, roscovitine, and thymidine.

In one embodiment, the therapeutic agent is a ligand for a cellular receptor.

As used herein, the term **"ligand for a cellular receptor"** refers to any molecule binding to a cellular receptor (such as a cell surface receptor, an intracellular receptor or a co-receptor, including transcription factors and the like), including agonists and antagonists, as well as partial agonists, inverse agonists, and allosteric modulators.

Suitable examples of ligands for cellular receptors include, but are not limited to, ligands binding to the AATYK receptors, the acetylcholine receptors, the ADGRG receptors, the adiponectin receptors, the adrenergic α1 receptors, the adrenergic α2 receptors, the adrenergic β1 receptors, the adrenergic β2 receptors, the adrenergic β3 receptors, the adrenomedullin receptor, the AMPA receptors, the anaphylatoxin receptors, the angiopoietin receptors, the angiotensin receptors, the anti-Müllerian hormone receptor, the apelin receptor, the asialoglycoprotein receptors, the AXL receptors, the benzodiazepine receptor, the bile acid receptor, the bombesin receptors, the bone morphogenetic protein receptors , the bradykinin receptors, the brain-specific angiogenesis inhibitors, the cadherin receptors, the calcitonin receptor, the calcitonin receptor-like receptor, the calcium-sensing receptor, the cannabinoid receptors, the CD97 receptor, the chemokine receptors, the cholecystokinin receptors, the complement receptors, the corticotropin-releasing hormone receptors, the CysLT receptors, the cytokine receptors, the DDR receptors, the dopamine receptors, the EBI2 receptor, the ectodysplasin A receptor, the EGF module-containing mucin-like hormone receptors, the EGF receptors, the endothelin receptors, the EPH receptors, the estrogen receptor, the FGF receptors, the free fatty acid receptors, the frizzled receptors, the FSH receptor, the GABAB receptors, the galanin receptors, the GHB receptor, the ghrelin receptor, the glucagon receptors, the glucagon-like peptide receptors, the glutamate receptors, the glycine receptors, the gonadotropin receptors, the gonadotropin-releasing hormone receptors, the GPRC6A receptor, the growth factor receptors, the growth hormone receptors, the growth-hormone-releasing hormone receptor, the guanylate cyclase-coupled receptors, the HGF receptors, the histamine receptors, the hydroxycarboxylic acids receptors, the immunoglobulin immune receptors, the insulin receptors, the kainate receptors, the KiSS1-derived peptide receptor, the latrophilin receptors, the leptin receptor, the leukotriene B4 receptors, the lipoprotein receptor-related protein receptors, the LTK receptors, the luteinizing hormone/choriogonadotropin receptor, the lysophosphatidic acid receptors, the lysophospholipid receptors, the mannose receptor, the MAS receptors, the melanin-concentrating hormone receptors, the melanocortin receptors, the melatonin receptors, the methuselah-like proteins receptors, the motilin receptor, the MuSK receptors, the N-acetylglucosamine receptor, the neuromedin receptors, the neuropeptide B/W receptors, the neuropeptide FF receptors, the neuropeptide S receptor, the neuropeptide Y receptors, the neuropilins receptor, the neurotensin receptors, the N-formyl peptide receptor, the nicotinic acetylcholine receptors, the NMDA receptors, the nuclear receptors, the olfactory receptor, the opioid

receptors, the opsin receptors, the orexin receptors, the oxoeicosanoid receptor, the oxoglutarate receptor, the oxytocin receptor, the parathyroid hormone receptors, the PDGF receptors, the pituitary adenylate cyclase-activating polypeptide type I receptor, the platelet-activating factor receptor, the progestin and adipoQ receptors, the prokineticin receptors, the prolactin receptor, the prolactin-releasing peptide receptor, the prostacyclin receptor, the prostaglandin receptors, the protease-activated receptor, the PTK7 receptors, the purinergic adenosine receptors, the purinergic P2X receptors, the purinergic P2Y receptors, the relaxin receptors, the RET receptors, the retinoic acid-inducible orphan G-protein-coupled receptors, the ROR receptors, the ROS receptors, the RYK receptors, the scavenger receptors, the secretin receptor, the serine/threonine-specific protein kinase receptors, the serotonine receptors, the smoothened receptor, the somatostatin receptors, the sphingosine-1-phosphate receptors, the SREB receptors, the stimulator of interferon genes (STING) receptor, the succinate receptor, the tachykinin receptors, the thromboxane receptor, the thyrotropin receptor, the thyrotropin-releasing hormone receptor, the toll-like receptors, the trace-amine associated receptors, the transferrin receptor, the Trk receptors, the tumor necrosis factor receptors, the tyrosine phosphatase receptors, the urotensin-II receptor, the vasoactive intestinal peptide receptors, the vasoactive intestine peptide receptors, the vasopressin receptors, the VEGF receptors, the vomeronasal receptor, and the zinc-activated ion channel receptor.

In one embodiment, the therapeutic agent is an immunomodulatory agent.

Suitable examples of immunomodulatory agents include, but are not limited to, immunostimulatory agents and immunosuppressor agents.

Suitable examples of immunostimulatory agents include those described under subgroup L03 of the Anatomical Therapeutic Chemical Classification System.

Suitable examples of immunostimulatory agents include, but are not limited to, cytokines (such as, e.g., filgrastim, pegfilgrastim, lenograstim, molgramostim, sargramostim, ancestim, albinterferon, interferon alfa natural, interferon alfa 2a, peginterferon alfa-2a, interferon alfa 2b, peginterferon alfa-2b, interferon alfa n1, interferon alfacon-1, interferon alpha-n3, interferon beta natural, interferon beta 1a, interferon beta 1b, interferon gamma, aldesleukin, oprelvekin, and the like); immune checkpoint inhibitors (such as, *e.g.,* inhibitors of CTLA4, PD-1, PD-L1, LAG-3, B7-H3, B7-H4, TIM3, A2AR, GITR, CD47, TNFR2, CD19, and/or IDO, including nivolumab, pembrolizumab, cemiplimab, spartalizumab, camrelizumab, sintilimab, tislelizumab, toripalimab, AMP-224, AMP-514, pidilizumab, MPDL3280A, MDX-1105, MEDI-4736, arelumab, ipilimumab, tremelimumab, pidilizumab, IMP321, MGA271, BMS-986016, lirilumab, urelumab, PF-05082566, IPH2101, MEDI-6469, CP-870,893, mogamulizumab, varlilumab, avelumab, galiximab, AUNP 12, indoximod, NLG-919, INCB024360, and the like); toll-like receptor agonists (such as, *e.g.,* buprenorphine, carbamazepine, ethanol, fentanyl, GS-9620, imiqimod, lefitolimod, levorphanol, methadone, morphine, (+)-morphine, morphine-3-glucuronide, oxcarbazepine, oxycodone, pethidine, resiquimod, SD-101, tapentadol, tilsotolimod, VTX-2337, glucuronoxylomannan from *Cryptococcus,* MALP-2 from *Mycoplasma,* MALP-404 from *Mycoplasma,* OspA from *Borrelia,* porin from *Neisseria* or *Haemophilus,* hsp60, hemmaglutinin, LcrV from *Yersinia,* bacterial flagellin, lipopolysaccharide, lipoteichoic acid, lipomannan from *Mycobacterium,* glycosylphosphatidylinositol, lysophosphatidylserine, lipophosphoglycan from *Leishmania,* zymosan from *Saccharomyces,* Pam2CGDPKHPKSF, Pam3CSK4, CpG oligodeoxynucleotides, poly(LC) nucleic acid sequences, poly(A:U) nucleic acid sequences, double-stranded viral RNA, and the like); STING receptor agonists (such as, *e.g.,* those described in WO2017100305, vadimezan, CL656, ADU-S100, 3'3'-cGAMP, 2'3'-cGAMP, ML RR-S2 CDG, ML RR-S2 cGAMP, cyclic di-GMP, DMXAA, DiABZI, and the like); CD1 ligands; growth hormone; immunocyanin; pegademase; prolactin; tasonermin; female sex steroids; histamine dihydrochloride; poly ICLC; vitamin D; lentinan; plerixafor; roquinimex; mifamurtide; glatiramer acetate; thymopentin; thymosin $\alpha$1; thymulin; polyinosinic:polycytidylic acid; pidotimod; Bacillus Calmette-Guérin; melanoma vaccine; sipuleucel-T; and the like.

Suitable examples of immunosuppressor agents include those described under subgroup L04 of the Anatomical Therapeutic Chemical Classification System.

Suitable examples of immunosuppressor agents include, but are not limited to:

i. antimetabolites, such as, *e.g.:*

- antifolates, including aminopterin, methotrexate, pemetrexed, pralatrexate, pteropterin, raltitrexed, denopterin, trimetrexate, pemetrexed, and the like;
- purine analogues, including pentostatin, cladribine, clofarabine, fludarabine, nelarabine, tioguanine, mercaptopurine, and the like;
- pyrimidine analogues, including fluorouracil, capecitabine, doxifluridine, tegafur, tegafur/gimeracil/oteracil, carmofur, floxuridine, cytarabine, gemcitabine, azacytidine, decitabine, and the like; and
- hydroxycarbamide);

ii. macrolides, such as, *e.g.,* tacrolimus, ciclosporin, pimecrolimus, abetimus, gusperimus, and the like;
iii. immunomodulatory imide drugs, such as, *e.g.,* lenalidomide, pomalidomide, thalidomide, apremilast, and the like;

iv. IL-1 receptor antagonists, such as, *e.g.,* anakinra, and the like);
v. mTOR inhibitors, such as, *e.g.,* sirolimus, everolimus, ridaforolimus, temsirolimus, umirolimus, zotarolimus, and the like);
vi. serum-targeting antibodies, such as, *e.g.,* eculizumab, adalimumab, afelimomab, certolizumab pegol, golimumab, infliximab, nerelimomab, mepolizumab, omalizumab, faralimomab, elsilimomab, lebrikizumab, ustekinumab, secukinumab, and the like;
vii. cell-targeting antibodies, such as, *e.g.,* muromonab-CD3, otelixizumab, teplizumab, visilizumab, clenoliximab, keliximab, zanolimumab, efalizumab, erlizumab, obinutuzumab, rituximab, ocrelizumab, pascolizumab, gomiliximab, lumiliximab, teneliximab, toralizumab, aselizumab, galiximab, gavilimomab, ruplizumab, belimumab, blisibimod, ipilimumab, tremelimumab, bertilimumab, lerdelimumab, metelimumab, natalizumab, tocilizumab, odulimomab, basifiximab, daclizumab, inolimomab, zolimomab aritox, atorolimumab, cedelizumab, fontolizumab, maslimomab, morolimumab, pexelizumab, reslizumab, rovelizumab, siplizumab, talizumab, telimomab aritox, vapaliximab, vepalimomab, and the like;
viii. fusion antibodies, such as, *e.g.,* abatacept, belatacept, etanercept, pegsunercept, aflibercept, alefacept, rilonacept and the like.

In one embodiment, the therapeutic agent is a pro-apoptotic agent.
As used herein, the term **"pro-apoptotic agent"** refers to any molecule able to induce apoptosis or programmed cell death in a cell upon administration.
Suitable examples of pro-apoptotic agents include, but are not limited to, histone deacetylase inhibitors (such as, *e.g.,* sodium butyrate, depsipeptide and the like), bortezomib, deguelin, favopiridol, fenretinide, fludarabine, kaempferol, miltefosine, narciclasine, obatoclax, oblimersen, and oncrasin.
In one embodiment, the therapeutic agent is an anti-angiogenic agent.
As used herein, the term **"anti-angiogenic agent"** refers to a molecule that reduces or prevents angiogenesis, which is responsible for the growth and development of blood vessels.
Suitable examples of anti-angiogenic agents include, but are not limited to, inhibitors of any of the vascular endothelial growth factor VEGF-A, VEGF-B, VEGF-C, or VEGF-D, which are major inducers of angiogenesis in normal and pathological conditions, and are essential in embryonic vasculogenesis.
Additionally or alternatively, an anti-angiogenic agent also can inhibit other angiogenic factors, such as, without limitation, a member of the fibroblast growth factor (FGF) family such as FGF-1 (acidic), FGF-2 (basic), FGF-4 or FGF-5; or angiopoietin-1, a factor that signals through the endothelial cell-specific Tie2 receptor tyrosine kinase; or the receptor of any of these angiogenic factors.
In one embodiment, the therapeutic agent is a cytokine.
Suitable examples of cytokines include, but are not limited to, chemokines, tumor necrosis factors, interleukins, and colony-stimulating factors.
Suitable examples of chemokines include, but are not limited to, chemokine C-C motif ligand (CCL) 1, CCL2, CCL3, CCL4, CCL5, CCL6, CCL7, CCL8, CCL9, CCL11, CCL12, CCL13, CCL14, CCL15, CCL16, CCL17, CCL18, CCL19, CCL20, CCL21, CCL22, CCL23, CCL24, CCL25, CCL26, CCL27, CCL28, chemokine C-X-C motif ligand (CXCL) 1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCL17, fractalkine, chemokine C motif ligand (XCL) 1, and XCL2.
Suitable examples of tumor necrosis factors include, but are not limited to, tumor necrosis factor (TNF) $\alpha$, lymphotoxin, OX40L, CD40LG, Fas ligand, CD70, CD153, 4-1BB ligand, TNF-related apoptosis-inducing ligand (TRAIL), receptor activator of nuclear factor $\kappa$-B ligand (RANKL), a proliferation-inducing ligand (APRIL), B-cell activating factor (BAFF), and ectodysplasin A (EDA).
Suitable examples of interleukins include, but are not limited to, interleukin- (IL-) 1$\alpha$, IL-1$\beta$, IL-1Ra, IL-2, IL-3, IL-4, IL-5, IL-6, IL-7, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35, IL-36$\alpha$, IE-36$\beta$, IL-36$\gamma$, IL-36Ra, IL-37, IL-38, interferon (IFN) $\alpha$, IFN$\beta$, IFN$\kappa$, and IFN$\omega$.
Suitable examples of colony-stimulating factors include, but are not limited to, granulocyte-macrophage colony-stimulating factor (GM-CSF) (including granulocyte-colony stimulating factor (G-CSF) and macrophage colony-stimulating factor (M-CSF)), haematopoietin, and thrombopoietin.
In one embodiment, the therapeutic agent is a growth factor.
Suitable examples of growth factors include, but are not limited to, fibroblast growth factor (FGF) 1, FGF2, FGF3, FGF4, FGF5, FGF6, FGF7, FGF8, FGF9, FGF10, FGF11, FGF12, FGF13, FGF14, FGF16, FGF17, FGF18, FGF19, FGF20, FGF21, FGF23, transforming growth factor (TGF) $\alpha$, epidermal growth factor (EGF), heparin-binding EGF-like growth factor (HB-EGF), transforming growth factor (TGF) $\beta$, insulin-like growth factor (IGF) 1, IGF2, Platelet-derived growth factor (PDGF) subunit A (PDGFA), PDGF subunit B (PDGFB), PDGF subunit C (PDGFC), PDGF subunit D (PDGFD), vascular endothelial growth factor (VEGF)-A, VEGF-B, VEGF-C, VEGF-D, placental growth factor (PGF),

nerve growth factor (NGF), and hepatocyte growth factor (HGF).

In one embodiment, the therapeutic agent is an antibody or an antigen-binding fragment thereof.

Suitable examples of antibodies or antigen-binding fragments thereof include, but are not limited to, monoclonal antibodies, polyclonal antibodies, bispecific antibodies, multispecific antibodies, antibody fragments, and antibody mimetics, such as, *e.g.,* scFv, di-scFv, tri-scFv, single domain antibodies, nanobodies, bispecific T-cell engagers (BiTEs), Fab, F(ab')2, Fab', chemically linked Fab, X-Link Fab, tandem-scFv/BiTE, diabodies, tandem diabodies, diabody-Fc fusions, tandem diabody-Fc fusion, tandem diabody-CH3 fusion, tetra scFv-Fc fusion, dual variable domain immunoglobulin, knob-hole, strand exchange engineered domain, CrossMab, quadroma-derived bispecific antibody, single domain based antibody, affibodies, affilins, affimers, affitins, alphabodies, anticalins, avimer, DARPins, Kunitz domain peptides, monobodies and nanoCLAMPs.

In one embodiment, the therapeutic agent is an antigen.

As used herein, the term **"antigen"** refers to any substance that induces a state of sensitivity and/or immune responsiveness after any latent period (normally, days to weeks in humans) and that reacts in a demonstrable way with antibodies and/or immune cells of the sensitized subject *in vivo* or *in vitro.*

Suitable examples of antigens include, but are not limited to, pathogen-related antigens (such as, *e.g.,* antigens of viruses, fungi or bacteria, or immunogenic molecules derived from them), self-antigens (such as, *e.g.,* cellular antigens including cells containing normal transplantation antigens and/or tumor-related antigens, RR-Rh antigens, and antigens characteristic of, or specific to particular cells or tissues or body fluids), allergen-related antigens (such as, *e.g.,* those associated with environmental allergens, including grasses, pollens, molds, dust, insects, dander, venoms, and the like; occupational allergens, including latex, dander, urethanes, epoxy resins, and the like; food, including shellfish, peanuts, eggs, milk products, and the like; and drugs, including antibiotics, anesthetics, and the like), neoantigens, and vaccines.

Examples of antigens include, but are not limited to, 17-IA, 4-1BB, 4Dc, 6-keto-PGF1a, 8-iso-PGF2a, 8-oxo-dG, A1 adenosine receptor, A33, ACE, ACE-2, activin, activin A, activin AB, activin B, activin C, activin RIA, activin RIA ALK-2, activin RIB ALK-4, activin RIIA, activin RIIB, ADAM, ADAM10, ADAM12, ADAM15, ADAM17/TACE, ADAM8, ADAM9, ADAMTS, ADAMTS4, ADAMTS5, addressin, aFGF, ALCAM, ALK, ALK-1, ALK-7, alpha-1-antitrypsin, alpha-V/beta-1 antagonist, ANG, Ang, APAF-1, APE, APJ, APP, APRIL, AR, ARC, ART, artemin, anti-Id, ASPARTIC, atrial natriuretic peptide, av/b3 integrin, Axl, b2M, B7-1, B7-2, B7-H, B-lymphocyte stimulating factor (BlyS), BACE, BACE-1, Bad, BAFF, BAFF-R, Bag-1, BAK, Bax, BCA-1, BCAM, Bcl, BCMA, BDNF, b-ECGF, bFGF, BID, Bik, BIM, BLC, BL-CAM, BLK, BMP, BMP-2 BMP-2a, BMP-3 osteogenin, BMP-4 BMP-2b, BMP-5, BMP-6 Vgr-1, BMP-7 (OP-1), BMP-8 (BMP-8a, OP-2), BMPR, BMPR-IA (ALK-3), BMPR-IB (ALK-6), BRK-2, RPK-1, BMPR-II (BRK-3), BMP, b-NGF, BOK, bombesin, bone-derived neurotrophic factor, BPDE, BPDE-DNA, BTC, complement factor 3 (C3), C3a, C4, C5, C5a, C10, CA125, CAD-8, calcitonin, cAMP, carcinoembryonic antigen (CEA), cancer associated antigen, cathepsin A, cathepsin B, cathepsin C/DPPI, cathepsin D, cathepsin E, cathepsin H, cathepsin L, cathepsin O, cathepsin S, cathepsin V, cathepsin X/Z/P, CBL, CCI, CCK2, CCL, CCL1, CCL11, CCL12, CCL13, CCL14, CCL15, CCL16, CCL17, CCL18, CCL19, CCL2, CCL20, CCL21, CCL22, CCL23, CCL24, CCL25, CCL26, CCL27, CCL28, CCL3, CCL4, CCL5, CCL6, CCL7, CCL8, CCL9/10, CCR, CCR1, CCR10, CCR10, CCR2, CCR3, CCR4, CCR5, CCR6, CCR7, CCR8, CCR9, CD1, CD2, CD3, CD3E, CD4, CD5, CD6, CD7, CD8, CD10, CD11a, CD11b, CD11c, CD13, CD14, CD15, CD16, CD18, CD19, CD20, CD21, CD22, CD23, CD25, CD27L, CD28, CD29, CD30, CD30L, CD32, CD33 (p67 protein), CD34, CD38, CD40, CD40L, CD44, CD45, CD46, CD49a, CD52, CD54, CD55, CD56, CD61, CD64, CD66e, CD74, CD80 (B7-1), CD89, CD95, CD123, CD137, CD138, CD140a, CD146, CD147, CD148, CD152, CD164, CEACAM5, CFTR, cGMP, CINC, Botulinum toxin, Clostridium perfringens toxin, CKb8-1, CLC, CMV, CMV UL, CNTF, CNTN-1, COX, C-Ret, CRG-2, CT-1, CTACK, CTGF, CTLA-4, CX3CL1, CX3CR1, CXCL, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCR, CXCR1, CXCR2, CXCR3, CXCR4, CXCR5, CXCR6,cytokeratin tumor associated antigen, DAN, DCC, DcR3, DC-SIGN, complement regulatory factor (Decay accelerating factor), des (1-3)-IGF-I (brain IGF-1), Dhh, digoxin, DNAM-1, Dnase, Dpp, DPPIV/CD26, Dtk, ECAD, EDA, EDA-A1, EDA-A2, EDAR, EGF, EGFR (ErbB-1), EMA, EMMPRIN, ENA, endothelin receptor, enkephalinase, eNOS, Eot, eotaxin 1, EpCAM, ephrin B2/EphB4, EPO, ERCC, E-selectin, ET-1, factor IIa, factor VII, factor VIIIc, factor IX, fibroblast activation protein (FAP), Fas, FcR1, FEN-1, ferritin, FGF, FGF-19, FGF-2, FGF3, FGF-8, FGFR, FGFR-3, fibrin, FL, FLIP, Flt-3, Flt-4, follicle stimulating hormone, fractalkine, FZD1, FZD2, FZD3, FZD4, FZD5, FZD6, FZD7, FZD8, FZD9, FZD10, G250, Gas6, GCP-2, GCSF, GD2, GD3, GDF, GDF-1, GDF-3 (Vgr-2), GDF-5 (BMP-14, CDMP-1), GDF-6 (BMP-13, CDMP-2), GDF-7 (BMP-12, CDMP-3), GDF-8 (myostatin), GDF-9, GDF-15 (MIC-1), GDNF, GDNF, GFAP, GFRa-1, GFR-alpha1, GFR-alpha2, GFR-alpha3, GITR, glucagon, Glut4, glycoprotein IIb/IIIa (GPIIb/IIIa), GM-CSF, gp130, gp72, GRO, growth hormone releasing hormone, hapten (NP-cap or NIP-cap), HB-EGF, HCC, HCMV gB envelope glycoprotein, HCMV gH envelope glycoprotein, HCMV UL, hematopoietic growth factor (HGF), Hep B gp120, heparanase, Her2, Her2/neu (ErbB-2), Her3 (ErbB-3), Her4 (ErbB-4), herpes simplex virus (HSV) gB glycoprotein, HSV gD glycoprotein, HGFA, high molecular weight melanoma-associated antigen (HMW-MAA), HIV gp120, HIV IIIB gp 120 V3 loop, HLA, HLA-DR, HM1.24, HMFG PEM, HRG, Hrk, human cardiac myosin, human cytomegalovirus (HCMV), human growth hormone (HGH), HVEM, 1-309, IAP, ICAM, ICAM-1, ICAM-3, ICE, ICOS, IFNg, Ig, IgA receptor, IgE, IGF, IGF

binding protein, IGF-1R, IGFBP, IGF-I, IGF-II, IL, IL-1, IL-1R, IL-2, IL-2R, IL-4, IL-4R, IL-5, IL-5R, IL-6, IL-6R, IL-8, IL-9, IL-10, IL-12, IL-13, IL-15, IL-18, IL-18R, IL-23, interferon (INF)-alpha, INF-beta, INF-gamma, inhibin, iNOS, insulin A chain, insulin B chain, insulin-like growth factor1, integrin alpha2, integrin alpha3, integrin alpha4, integrin alpha4/beta1, integrin alpha4/beta7, integrin alpha5 (alpha V), integrin alpha5/beta1, integrin alpha5/beta3, integrin alpha6, integrin beta1, integrin beta2,interferon gamma, IP-10, I-TAC, JE, kallikrein 2, kallikrein 5, kallikrein 6, kallikrein 11, kallikrein 12, kallikrein 14, kallikrein 15, kallikrein L1, kallikrein L2, kallikrein L3, kallikrein L4, KC, KDR, keratinocyte growth factor (KGF), laminin 5, LAMP, LAP, LAP (TGF-1), latent TGF-1, latent TGF-1 bp1, LBP, LDGF, LECT2, lefty, Lewis-Y antigen, Lewis-Y associated antigen, LFA-1, LFA-3, Lfo, LIF, LIGHT, lipoprotein, LIX, LKN, Lptn, L-selectin, LT-a, LT-b, LTB4, LTBP-1, lung surface, luteinizing hormone, lymphotoxin beta receptor, Mac-1, MAdCAM, MAG, MAP2, MARC, MCAM, MCAM, MCK-2, MCP, M-CSF, MDC, Mer, METALLOPROTEASES, MGDF receptor, MGMT, MHC (HLA-DR), MIF, MIG, MIP, MIP-1-alpha, MK, MMAC1, MMP, MMP-1, MMP-10, MMP-11, MMP-12, MMP-13, MMP-14, MMP-15, MMP-2, MMP-24, MMP-3, MMP-7, MMP-8, MMP-9, MPIF, Mpo, MSK, MSP, mucin (Muc1), MUC18, Mullerian-inhibiting substance, Mug, MuSK, NAIP, NAP, NCAD, N-C adherin, NCA 90, NCAM, NCAM, neprilysin, neurotrophin-3, -4, or -6, neurturin, nerve growth factor (NGF), NGFR, NGF-beta, nNOS, NO, NOS, Npn, NRG-3, NT, NTN, OB, OGG1, OPG, OPN, OSM, OX40L, OX40R, p150, p95, PADPr, parathyroid hormone, PARC, PARP, PBR, PBSF, PCAD, P-cadherin, PCNA, PDGF, PDGF, PDK-1, PECAM, PEM, PF4, PGE, PGF, PGI2, PGJ2, PIN, PLA2, placental alkaline phosphatase (PLAP), PIGF, PLP, PP14, proinsulin, prorelaxin, protein C, PS, PSA, PSCA, prostate-specific membrane antigen (PSMA), PTEN, PTHrp, Ptk, PTN, R51, RANK, RANKL, RANTES, RANTES, relaxin A chain, relaxin B chain, renin, respiratory syncytial virus (RSV) F, RSV Fgp, Ret, Rheumatoid factor, RLIP76, RPA2, RSK, S100, SCF/KL, SDF-1, SERINE, serum albumin, sFRP-3, Shh, SIGIRR, SK-1, SLAM, SLPI, SMAC, SMDF, SMOH, SOD, SPARC, Stat, STEAP, STEAP-II, TACE, TACI, TAG-72 (tumor-associated glycoprotein-72), TARC, TCA-3, T-cell receptor (for example, T-cell receptor alpha/beta), TdT, TECK, TEM1, TEM5, TEM7, TEM8, TERT, testis PLAP-like alkaline phosphatase, TfR, TGF, TGF-alpha, TGF-beta, TGF-beta Pan Specific, TGF-betaRI (ALK-5), TGF-betaRII, TGF-betaRIIb, TGF-betaRIII, TGF-beta1, TGF-beta2, TGF-beta3, TGF-beta4, TGF-beta5, thrombin, thymus Ck-1, thyroid-stimulating hormone, Tie, TIMP, TIQ, tissue factor, TMEFF2, Tmpo, TMPRSS2, TNF, TNF-alpha, TNF-alphabeta, TNF-beta2, TNFc, TNF-RI, TNF-RII, TNFRSF10A (TRAIL R1 Apo-2, DR4), TNFRSF10B (TRAIL R2 DR5, KILLER, TRICK-2A, TRICK-B), TNFRSF10C (TRAIL R3 DcR1, LIT, TRID), TNFRSF10D (TRAIL R4 DcR2, TRUNDD), TNFRSF11A (RANK ODF R, TRANCE R), TNFRSF11B (OPG OCIF, TR1), TNFRSF12 (TWEAK R FN14), TNFRSF13B (TACI), TNFRSF13C (BAFF R), TNFRSF14 (HVEM ATAR, HveA, LIGHT R, TR2), TNFRSF16 (NGFR p75NTR), TNFRSF17 (BCMA), TNFRSF18 (GITR AITR), TNFRSF19 (TROY TAJ, TRADE), TNFRSF19L (RELT), TNFRSF1A (TNF RI CD120a, p55-60), TNFRSF1B (TNF RII CD120b, p75-80), TNFRSF26 (TNFRH3), TNFRSF3 (LTbR TNF RIII, TNFC R), TNFRSF4 (OX40 ACT35, TXGP1 R), TNFRSF5 (CD40 p50), TNFRSF6 (Fas Apo-1, APT1, CD95), TNFRSF6B (DcR3 M68, TR6), TNFRSF7 (CD27), TNFRSF8 (CD30), TNFRSF9 (4-1BB CD137, ILA), TNFRSF21 (DR6), TNFRSF22 (DcTRAIL R2 TNFRH2), TNFRST23 (DcTRAIL R1 TNFRH1), TNFRSF25 (DR3 Apo-3, LARD, TR-3, TRAMP, WSL-1), TNFSF10 (TRAIL Apo-2 ligand, TL2), TNFSF11 (TRANCE/RANK ligand ODF, OPG ligand), TNFSF12 (TWEAK Apo-3 ligand, DR3 ligand), TNFSF13 (APRIL TALL2), TNFSF13B (BAFF BLYS, TALL1, THANK, TNFSF20), TNFSF14 (LIGHT HVEM ligand, LTg), TNFSF15 (TL1A/VEGI), TNFSF18 (GITR ligand AITR ligand, TL6), TNFSF1A (TNF-a Conectin, DIF, TNFSF2), TNFSF1B (TNF-b LTa, TNFSF1), TNFSF3 (LTb TNFC, p33), TNFSF4 (OX40 ligand gp34, TXGP1), TNFSF5 (CD40 ligand CD154, gp39, HIGM1, IMD3, TRAP), TNFSF6 (Fas ligand Apo-1 ligand, APT1 ligand), TNFSF7 (CD27 ligand CD70), TNFSF8 (CD30 ligand CD153), TNFSF9 (4-1BB ligand CD137 ligand), TP-1, t-PA, Tpo, TRAIL, TRAIL R, TRAIL-R1, TRAIL-R2, TRANCE, transferrin receptor, TRF, Trk, TROP-2, TSG, TSLP, tumor associated antigen CA125, tumor-associated antigen expressing Lewis-Y associated carbohydrates, TWEAK, TXB2, Ung, uPAR, uPAR-1, urokinase, VCAM, VCAM-1, VECAD, VE-Cadherin, VE-cadherin-2, VEFGR-1 (flt-1), VEGF, VEGFR, VEGFR-3 (flt-4), VEGI, VIM, virus antigen, VLA, VLA-1, VLA-4, VNR integrin, von Willebrand factor, WIF-1, WNT1, WNT2, WNT2B/13, WNT3, WNT3A, WNT4, WNT5A, WNT5B, WNT6, WNT7A, WNT7B, WNT8A, WNT8B, WNT9A, WNT9A, WNT9B, WNT10A, WNT10B, WNT11, WNT16, XCL1, XCL2, XCR1, XCR1, XEDAR, XIAP, XPD, HMGB1, IgA, Aβ, CD81, CD97, CD98, DDR1, DKK1, EREG, Hsp90, IL-17/IL-17R, IL-20/IL-20R, oxidized LDL, PCSK9, prekallikrein, RON, TMEM16F, SOD1, Chromogranin A, Chromogranin B, tau, VAP1, high molecular weight kininogen, IL-31, IL-31R, Nav1.1, Nav1.2, Nav1.3, Nav1.4, Nav1.5, Nav1.6, Nav1.7, Nav1.8, Nav1.9, EPCR, C1, C1q, C1r, C1s, C2, C2a, C2b, C3, C3a, C3b, C4, C4a, C4b, C5, C5a, C5b, C6, C7, C8, C9, factor B, factor D, factor H, properdin, sclerostin, fibrinogen, fibrin, prothrombin, thrombin, tissue factor, factor V, factor Va, factor VII, factor VIIa, factor VIII, factor VIIIa, factor IX, factor IXa, factor X, factor Xa, factor XI, factor XIa, factor XII, factor XIIa, factor XIII, factor XIIIa, TFPI, antithrombin III, EPCR, thrombomodulin, TAPI, tPA, plasminogen, plasmin, PAI-1, PAI-2, GPC3, Syndecan-1, Syndecan-2, Syndecan-3, Syndecan-4, LPA, SIP, Acetylcholine receptor, AdipoR1, AdipoR2, ADP ribosyl cyclase-1, alpha-4/beta-7 integrin, alpha-5/beta-1 integrin, alpha-v/beta-6 integrin, alphavbeta1 integrin, Angiopoietin ligand-2, Angpt12, Anthrax, Cadherin, Carbonic anhydrase-IX, CD105, CD155, CD158a, CD37, CD49b, CD51, CD70, CD72, Claudin 18, Clostridium difficile toxin, CS1, Delta-like protein ligand 4, DHICA oxidase, Dickkopf-1 ligand, Dipeptidyl peptidase IV, EPOR, F protein of RSV, Factor Ia, FasL, Folate receptor alpha, Glucagon receptor, Glucagon-like peptide 1 receptor, Glutamate carboxypeptidase II, GMCSFR, Hepatitis C virus E2 glycoprotein, Hepcidin, IL-17 receptor,

IL-22 receptor, IL-23 receptor, IL-3 receptor, Kit tyrosine kinase, Leucine Rich Alpha-2-Glycoprotein 1 (LRG1), Lysosphingolipid receptor, Membrane glycoprotein OX2, Mesothelin, MET, MICA, MUC-16, Myelin associated glycoprotein, Neuropilin-1, Neuropilin-2, Nogo receptor, PLXNA1, PLXNA2, PLXNA3, PLXNA4A, PLXNA4B, PLXNB1, PLXNB2, PLXNB3, PLXNC1, PLXND1, Programmed cell death ligand 1, Proprotein convertase PC9, P-selectin glycoprotein ligand-1, RAGE, Reticulon 4, RF, RON-8, SEMA3A, SEMA3B, SEMA3C, SEMA3D, SEMA3E, SEMA3F, SEMA3G, SEMA4A, SEMA4B, SEMA4C, SEMA4D, SEMA4F, SEMA4G, SEMA5A, SEMA5B, SEMA6A, SEMA6B, SEMA6C, SEMA6D, SEMA7A, Shiga like toxin II, Sphingosine-1-phosphate receptor-1, ST2, Staphylococcal lipoteichoic acid, Tenascin, TG2, Thymic stromal lymphoprotein receptor, TNF superfamily receptor 12A, Transmembrane glycoprotein NMB, TREM-1, TREM-2, Trophoblast glycoprotein, TSH receptor, TTR, Tubulin, and ULBP2, and receptors for growth factors and hormones, molecules that exist in their soluble form and are not anchored to cells in the body fluid of organisms.

Suitable examples of pathogen-related antigens include, but are not limited to, antigens derived from vaccinia, avipox virus, turkey influenza virus, bovine leukemia virus, feline leukemia virus, avian influenza, chicken pneumovirosis virus, canine parvovirus, equine influenza, FHV, Newcastle Disease Virus (NDV), Chicken/Pennsylvania/1/83 influenza virus, infectious bronchitis virus, Dengue virus, measles virus, Rubella virus, pseudorabies, Epstein-Barr Virus, HIV, SIV, EHV, BHV, HCMV, Hantaan, C. tetani, mumps, Morbillivirus, Herpes Simplex Virus type 1, Herpes Simplex Virus type 2, Human cytomegalovirus, Hepatitis A Virus, Hepatitis B Virus, Hepatitis C Virus, Hepatitis E Virus, Respiratory Syncytial Virus, Human Papilloma Virus, Influenza Virus, *Bordetella, Vitreoscilla, Salmonella, Neisseria, Borrelia, Chlamydia, Plasmodium, Toxoplasma, Cryptococcus, Streptococcus, Staphylococcus, Haemophilus, Diptheria, Tetanus, Escherichia, Candida, Aspergillus, Actinobacilus, Burkholderia, Moraxella, Pseudomonas, Vibrio, Entamoeba, Giardia,* and *Trypanasoma.*

Suitable examples of self-antigens include, but are not limited to, lupus autoantigen, Smith, Ro, La, U1-RNP, fibrillin, nuclear antigens, histones, glycoprotein gp70, ribosomal proteins, pyruvate dehydrogenase, dehydrolipoamide acetyltransferase (PCD-E2), hair follicle antigens, human tropomyosin isoform 5 (hTM5), proinsulin, insulin, IA2, GAD65, collagen type II, human cartilage gp 39 (HCgp39), gp130-RAPS, dnaJp1, citrullinated proteins and peptides (including citrullinated type II collagen, citrullinated vimentin and citrullinated fibrinogen), myelin basic protein, proteolipid protein (PLP), myelin oligodendrocyte glycoprotein (MOG), thyroid stimulating factor receptor (TSH-R), acetylcholine receptor (AchR), gliadin, PLP, glucose-6-phosphate isomerase, thyroglobulin, various tRNA synthetases, proteinase-3, and myeloperoxidase, and the like, including fragments thereof.

Suitable examples of tumor-related antigens include, but are not limited to, MART-1/Melan-A, gplOO, dipeptidyl peptidase IV (DPPIV), adenosine deaminase-binding protein (ADAbp), cyclophilin b, colorectal associated antigen (CRC)-C017-1A/GA733, carcinoembryonic antigen (CEA) and its immunogenic epitopes CAP-1 and CAP-2, etv6, amll, prostate specific antigen (PSA) and its immunogenic epitopes PSA-1, PSA-2, and PSA-3, prostate-specific membrane antigen (PSMA), T-cell receptor/CD3-zeta chain, MAGE-family of tumor antigens (*e.g.,* MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5), GAGE-family of tumor antigens (*e.g.,* GAGE-1, GAGE-2, GAGE-3, GAGE-4, GAGE- 5, GAGE-6, GAGE-7, GAGE-8, GAGE-9), BAGE, RAGE, LAGE-1, NAG, GnT-V, MUM-1, CDK4, tyrosinase, p53, MUC family (*e.g.* MUC1, MUC16, etc.), HER2/neu, p21ras, RCASl, alpha-fetoprotein, E-cadherin, alpha-catenin, beta-catenin and gamma-catenin, pl20ctn, gpl00.sup.Pmelll7, PRAME, NY-ESO-1, cdc27, adenomatous polyposis coli protein (APC), fodrin, connexin 37, Ig-idiotype, pl5, gp75, GM2 and GD2 gangliosides, Smad family of cancer antigens brain glycogen phosphorylase, SSX-1, SSX-2 (HOM-MEL-40), SSX-1, SSX-4, SSX-5, SCP-1 and CT-7, and c-erbB-2 and viral antigens such as the HPV-16 and HPV-18 E6 and E7 antigens and the EBV-encoded nuclear antigen (EBNA)-1, and the like, including fragments thereof. Further examples of tumor-related antigens are described in, *e.g.,* Li et al., 2004. Cancer Immunol Immunother. 53(3):139-43; Novellino et al., 2005. Cancer Immunol Immunother. 54(3):187-20; which are herein incorporated by reference in their entirety.

In one embodiment, $R_3$ is a hydrophilic moiety, with the proviso that said hydrophilic moiety is not a hydroxyl group.

Examples of hydrophilic moieties include, but are not limited to, methoxy, phenol, carboxylic acids and salts thereof, methyl and ethyl esters of carboxylic acids, amides, amino, cyano, ammonium salts, sulfonium salts, phosphonium salts, epoxy groups, acrylates, sulfonamides, nitro, guanidinium, aminate, acrylamide and pyridinium groups.

Further examples of hydrophilic moieties include, but are not limited to, hydrophilic polymers.

In one embodiment, $R_3$ is a hydrophilic polymer. Examples of hydrophilic polymers include, but are not limited to, polystyrene sulfonic acid, poly-N-alkylvinylpyridinium halogenide, poly(meth)acrylic acid, poly-*N*-vinylpyrrolidone, polyhydroxyethyl methacrylate, polyvinylether, polyethylene glycol, polyethylene imine, polypropylene oxide, and (poly) peptides.

Further examples of hydrophilic moieties include, but are not limited to, saccharides, including mono-, di-, oligo- and polysaccharides.

In one embodiment, $R_3$ is a saccharide selected from the group comprising or consisting of agarose, dextran,

starch, cellulose, amylose, and amylopectin.

In one embodiment, $R_3$ is a monosaccharide.

In one embodiment, $R_3$ is a 3-deoxy-D-*manno*-oct-2-ulosonic acid (Kdo) residue or a D-glycero-D-*talo*-oct-2-ulosonic acid (Ko).

In one embodiment, $R_3$ is a Kdo residue or a Ko residue, substituted with further substituents.

Such further substituents include, but are not limited to, a mono-, or an oligo- or poly-saccharide, either linear or branched, preferably comprising from 1 to 200 sugar residues.

Additionally, or alternatively, further substituents may include, without limitation, a phosphate group, a diphosphate group, a phosphoryl ethanolamine group "PEA" ($-OPO_3H-CH_2-CH_2-NH_2$), a diphosphoryl ethanolamine group "PPEA" ($-OPO_3H_2-PO_3H-CH_2-CH_2-NH_2$), a Kdo residue, a $Kdo_2$ residue, a Ko residue, an aminoarabinose (AraN) residue, a galactosamine (GalN) residue, a glucosamine (GlcN) residue, and the like.

In one embodiment, $R_3$ is a core oligosaccharide domain (*i.e.,* an inner core, or an inner and outer core). In one embodiment, the core oligosaccharide domain typically comprises from 1 to 12 sugar residues. In one embodiment, the core oligosaccharide domain comprises more than 2 sugar residues, such as, from 3, 4, 5, or more, to 12 sugar residues.

In one embodiment, the core oligosaccharide domain may comprise in its main chain one or several non-carbohydrate structural elements.

In one embodiment, $R_3$ is a core oligosaccharide domain, wherein one or more sugar residues, and/or one or more non-carbohydrate structural elements, when applicable, are substituted with further substituents.

Such further substituents include, but are not limited to, a phosphate group, a phosphoryl ethanolamine group "PEA" ($-OPO_3H-CH_2-CH_2-NH_2$), a diphosphoryl ethanolamine group "PPEA" ($-OPO_3H_2-PO_3H-CH_2-CH_2-NH_2$), an acetate group, a methyl group, a formyl group, a choline, a phospho-choline, an amino acid, and the like.

In one embodiment, $R_3$ is a core oligosaccharide domain, wherein one or more sugar residues, and/or one or more non-carbohydrate structural elements, when applicable, are substituted with a therapeutic agent. Suitable examples of such therapeutic agents have been described hereinabove as suitable agents for conjugation at positions Z, $R_1$ and/or $R_2$ of **Formula A** and **Formula B.**

Additionally or alternatively, further substituents may include, without limitation, a mono-, an oligo- and a polysaccharide, either linear or branched.

In one embodiment, $R_3$ is a core oligosaccharide domain further substituted, either directly or through a linker domain, with an antigen, such as, without limitation, an O-antigen domain and/or a capping antigen. In one embodiment, the core oligosaccharide domain and/or antigen domain comprises, either independently from each other or together, from 1 to 200 sugar residues. In one embodiment, the core oligosaccharide domain and/or the antigen domain may comprise in their main chain one or several non-carbohydrate structural elements.

In one embodiment, $R_3$ is a core oligosaccharide domain further substituted with an antigen domain, such as, without limitation, an O-antigen domain and/or a capping antigen, wherein one or more sugar residues are substituted with further substituents.

Such further substituents include, but are not limited to, a phosphate group, a phosphoryl ethanolamine group "PEA" ($-OPO_3H-CH_2-CH_2-NH_2$), a diphosphoryl ethanolamine group "PPEA" ($-OPO_3H_2-PO_3H-CH_2-CH_2-NH_2$), an acetate group, a methyl group, a formyl group, a choline or phospho-choline, an amino acid, and the like.

In one embodiment, $R_3$ is a core oligosaccharide domain further substituted with an antigen domain, such as, without limitation, an O-antigen domain and/or a capping antigen, wherein one or more sugar residues are substituted with a therapeutic agent. Suitable examples of such therapeutic agents have been described hereinabove as suitable agents for conjugation at positions Z, $R_1$ and/or $R_2$ of **Formula A** and **Formula B.**

In one embodiment, $R_5$ is a hydrogen. In this embodiment, X-$R_5$ may therefore be a hydroxyl group (-OH) or an amine group ($-NH_2$).

In one embodiment, $R_7$ is a hydrogen. In this embodiment, Y-$R_7$ may therefore be a hydroxyl group (-OH) or an amine group ($-NH_2$).

In one embodiment, X-$R_5$ is a hydroxyl group (-OH) or an amine group ($-NH_2$), substituted with further substituents. In one embodiment, Y-$R_7$ may therefore be a hydroxyl group (-OH) or an amine group ($-NH_2$), substituted with further substituents.

In one embodiment, $R_4$ is a primary fatty acid residue. In one embodiment, $R_5$ is a primary fatty acid residue. In one embodiment, $R_6$ is a primary fatty acid residue. In one embodiment, $R_7$ is a primary fatty acid residue.

In one embodiment, primary fatty acids are independently selected from short-chain fatty acids (SCFA), medium-chain fatty acids (MCFA), long-chain fatty acids (LCFA) and very long chain fatty acids (VLCFA).

In one embodiment, the primary fatty acid residues may independently be saturated or unsaturated.

In one embodiment, primary fatty acid residues may independently be linear or branched.

In one embodiment, the primary fatty acid residues may independently comprise a hydroxyl group (-OH) at position C2 of the fatty acid chain, *i.e.,* the primary fatty acid residues may independently be 2-hydroxy fatty acid residues.

In one embodiment, the primary fatty acid residues may independently comprise a hydroxyl group (-OH) at

position C3 of the fatty acid chain, *i.e.,* the primary fatty acid residues may independently be 3-hydroxy fatty acid residues.

In one embodiment where a primary fatty acid residue comprises a hydroxyl group (-OH) at position C3 of the fatty acid chain, the primary fatty acid residue may be esterified at position C3 with a secondary fatty acid residue (*i.e.,* with an estolide-bound fatty acid residue, also termed acyl-oxy-acyl fatty acid).

In one embodiment, secondary fatty acids are independently selected from short-chain fatty acids (SCFA), medium-chain fatty acids (MCFA), long-chain fatty acids (LCFA) and very long chain fatty acids (VLCFA).

In one embodiment, secondary fatty acid residues may independently be saturated or unsaturated.

In one embodiment, secondary fatty acid residues may independently be linear or branched.

In one embodiment, secondary fatty acid residues may independently comprise a hydroxyl group (-OH) at position C2 of the fatty acid chain, *i.e.,* the secondary fatty acid residues may independently be 2-hydroxy fatty acid residues.

In one embodiment, the secondary fatty acid residues may independently comprise a hydroxyl group (-OH) at position C3 of the fatty acid chain, *i.e.*, the secondary fatty acid residues may independently be 3-hydroxy fatty acid residues.

In one embodiment where a secondary fatty acid residue comprises a hydroxyl group (-OH) at position C3 of the fatty acid chain, the secondary fatty acid residue may be substituted at this position.

In one embodiment, the modified LPS molecular species may comprise:

i. a smaller or greater number of fatty acid chains as compared to classical *Escherichia coli* or *Salmonella enterica* LPS molecules; and/or

ii. fatty acid chains of shorter and/or longer length as compared to classical *Escherichia coli* or *Salmonella enterica* LPS molecules;

classical *Escherichia coli* or *Salmonella enterica* LPS molecules comprising six fatty acid chains, among which four primary fatty acid chains are $C_{14}$, one secondary fatty acid chain is $C_{14}$ and one secondary fatty acid chain is $C_{12}$.

In one embodiment, the modified LPS molecular species comprise 4 primary fatty acid residues, among which the 4 primary fatty acid residues are esterified at position C3 with a secondary fatty acid residue. In one embodiment, the modified LPS molecular species comprise 4 primary fatty acid residues, among which at least 3 primary fatty acid residues are esterified at position C3 with a secondary fatty acid residue. In one embodiment, the modified LPS molecular species comprise 4 primary fatty acid residues, among which at least 2 primary fatty acid residues are esterified at position C3 with a secondary fatty acid residue. In one embodiment, the modified LPS molecular species comprise at most 4 primary fatty acid residues, among which at most 1 primary fatty acid residue is esterified at position C3 with a secondary fatty acid residue. In one embodiment, the modified LPS molecular species comprise at most 3 primary fatty acid residues, among which at most 2 primary fatty acid residues are esterified at position C3 with a secondary fatty acid residue. In one embodiment, the modified LPS molecular species comprise at most 3 primary fatty acid residues, among which at most 1 primary fatty acid residue is esterified at position C3 with a secondary fatty acid residue. In one embodiment, the modified LPS molecular species comprise at most 2 primary fatty acid residues, among which at most 2 primary fatty acids residues are esterified at position C3 with a secondary fatty acid residue. In one embodiment, the modified LPS molecular species comprise at most 2 fatty acid residues, among which at most 1 fatty acid is esterified at position 3 with a secondary fatty acid residue.

In one embodiment, the modified LPS molecular species comprise primary fatty acid residues comprising at most 18 carbons, 17 carbons, 16 carbons, 15 carbons, 14 carbons, 13 carbons, 12 carbons, 11 carbons, 10 carbons, 9 carbons, 8 carbons, 7 carbons, 6 carbons, 5 carbons, 4 carbons, or less; preferably at most 14 carbons.

Additionally or alternatively, in one embodiment where the modified LPS molecular species comprise four primary fatty acid residues, at least one of said four primary fatty acid residues, such as one, two, three or four, does not comprise 14 carbons. In one embodiment where the modified LPS molecular species comprise four primary fatty acid residues, at least one of said four primary fatty acid residues, such as one, two, three or four, comprises 18 carbons, 17 carbons, 16 carbons, 15 carbons, 13 carbons, 12 carbons, 11 carbons, 10 carbons, 9 carbons, 8 carbons, 7 carbons, 6 carbons, 5 carbons, 4 carbons, or less.

In one embodiment, the modified LPS molecular species comprise secondary fatty acid residues comprising at most 32 carbons, 31 carbons, 30 carbons, 29 carbons, 28 carbons, 27 carbons, 26 carbons, 25 carbons, 24 carbons, 23 carbons, 22 carbons, 21 carbons, 20 carbons, 19 carbons, 18 carbons, 17 carbons, 16 carbons, 15 carbons, 14 carbons, 13 carbons, 12 carbons, 11 carbons, 10 carbons, 9 carbons, 8 carbons, 7 carbons, 6 carbons, 5 carbons, 4 carbons, or less; preferably at most 14 carbons.

Additionally or alternatively, in one embodiment where the modified LPS molecular species comprises two secondary fatty acid residues, at least one of said two secondary fatty acid residues, such as one or two, does not comprise 12 carbons. In one embodiment, the modified LPS molecular species comprise secondary fatty acid residues, at least one of said two secondary fatty acid residues, such as one or two, comprises 18 carbons, 17 carbons, 16 carbons, 15 carbons,

14 carbons, 13 carbons, 11 carbons, 10 carbons, 9 carbons, 8 carbons, 7 carbons, 6 carbons, 5 carbons, 4 carbons, or less.

Additionally or alternatively, in one embodiment where the modified LPS molecular species comprises two secondary fatty acid residues, at least one of said two secondary fatty acid residues, such as one or two, does not comprise 14 carbons. In one embodiment, the modified LPS molecular species comprise secondary fatty acid residues, at least one of said two secondary fatty acid residues, such as one or two, comprises 18 carbons, 17 carbons, 16 carbons, 15 carbons, 13 carbons, 12 carbons, 11 carbons, 10 carbons, 9 carbons, 8 carbons, 7 carbons, 6 carbons, 5 carbons, 4 carbons, or less.

In one embodiment, the modified LPS molecular species may be derived from any Gram-negative bacteria, and/or from any Gram-positive bacteria comprising LPS in their membrane.

Examples of Gram-negative bacteria include, but are not limited to, bacteria of the *Acidobacteria, Aquificae, Armatimonadetes, Bacteroidetes, Caldiserica, Chlamydiae, Chlorobi, Chloroflexi, Chrysiogenetes, Cyanobacteria, Deferribacteres, Deinococcus-Thermus, Dictyoglomi, Elusimicrobia, Fibrobacteres, Fusobacteria, Gemmatimonadetes, Lentisphaerae, Nitrospirae, Planctomycetes, Proteobacteria, Spirochaetes, Synergistetes, Tenericutes, Thermodesulfobacteria, Thermomicrobia, Thermotogae,* and *Verrucomicrobia* phylum, including any subtaxons thereof.

In one embodiment, the modified LPS molecular species may be derived from a Gram-negative bacterium of the *Proteobacteria* phylum, including, but not limited to, bacteria of the *Alphaproteobacteria, Betaproteobacteria, Gammaproteobacteria, Deltaproteobacteria, Epsilonproteobacteria, Zetaproteobacteria, Acidithiobacillia, Hydrogenophilalia,* and *Oligoflexia* classes.

Examples of Gram-positive bacteria comprising LPS in their membrane include, but are not limited to, *Listeria monocytogenes* (from the *Firmicutes* phylum).

In one embodiment, the modified LPS molecular species are derived from the *Bordetella* genus (from the *Proteobacteria* phylum), including, but not limited to, *Bordetella ansorpii, Bordetella avium, Bordetella bronchialis, Bordetella bronchiseptica, Bordetellaflabilis, Bordetella hinzii, Bordetella holmesii, Bordetella muralis, Bordetella parapertussis, Bordetella pertussis, Bordetella petrii, Bordetella pseudohinzii, Bordetella sputigena, Bordetella trematum, Bordetella tumbae,* and *Bordetella tumulicola.* In one embodiment, the modified LPS molecular species are derived from the bacterial species *Bordetella pertussis, Bordetella hinzii,* or *Bordetella trematum.*

In one embodiment, the modified LPS molecular species are derived from the *Vitreoscilla* genus (from the *Proteobacteria* phylum), including, but not limited to, *Vitreoscilla beggiatoides, Vitreoscilla filiformis,* and *Vitreoscilla stercoraria.* In one embodiment, the modified LPS molecular species are derived from the bacterial species *Vitreoscilla filiformis.*

In one embodiment, the modified LPS molecular species are derived from the *Salmonella* genus (from the *Proteobacteria* phylum), including, but not limited to, *Salmonella bongori,* and *Salmonella enterica (*further including, but not limited to, *Salmonella enterica enterica, Salmonella enterica salamae, Salmonella enterica arizonae, Salmonella enterica diarizonae, Salmonella enterica houtenae,* and *Salmonella enterica indica).* In one embodiment, the modified LPS molecular species are derived from the bacterial species *Salmonella enterica,* preferably from the bacterial subspecies *Salmonella enterica enterica,* more preferably from the bacterial serovar *Salmonella enterica enterica* serovar *Minnesota* or *Salmonella enterica enterica* serovar *Typhimurium.*

In one embodiment, the modified LPS molecular species are derived from the *Escherichia* genus (from the *Proteobacteria* phylum), including, but not limited to, *Escherichia albertii, Escherichia coli, Escherichia fergusonii, Escherichia hermannii, Escherichia marmotae,* and *Escherichia vulneris.* In one embodiment, the modified LPS molecular species are derived from the bacterial species *Escherichia coli,* preferably from the bacterial strain *Escherichia coli* K12 or *Escherichia coli* J5.

In one embodiment, the modified LPS molecular species are derived from the *Actinobacillus* genus (from the *Proteobacteria* phylum), including, but not limited to, *Actinobacillus actinomycetemcomitans, Actinobacillus arthritidis, Actinobacillus capsulatus, Actinobacillus delphinicola, Actinobacillus equuli, Actinobacillus hominis, Actinobacillus indolicus, Actinobacillus* lignieresii, *Actinobacillus* minor, *Actinobacillus muris, Actinobacillus pleuropneumoniae, Actinobacillus porcinus, Actinobacillus rossii, Actinobacillus scotiae, Actinobacillus seminis, Actinobacillus succinogenes, Actinobacillus suis,* and *Actinobacillus ureae.* In one embodiment, the modified LPS molecular species are derived from the bacterial species *Actinobacillus pleuropneumoniae.*

In one embodiment, the modified LPS molecular species are derived from the *Burkholderia* genus (from the *Proteobacteria* phylum), including, but not limited to, *Burkholderia alpine, Burkholderia ambifaria, Burkholderia anthina, Burkholderia arboris, Burkholderia cenocepacia, Burkholderia cepacian, Burkholderia contaminans, Burkholderia diffusa, Burkholderia dolosa, Burkholderia gladioli, Burkholderia glumae, Burkholderia humptydooensis, Burkholderia lata, Burkholderia latens, Burkholderia mallei, Burkholderia metallica, Burkholderia multivorans, Burkholderia oklahomensis, Burkholderia plantarii, Burkholderia pseudomallei, Burkholderia pseudomultivorans, Burkholderia puraquae, Burkholderia pyrrocinia, Burkholderia seminalis, Burkholderia singaporensis, Burkholderia singularis, Burkholderia stabilis, Burkholderia stagnalis, Burkholderia territorii, Burkholderia thailandensis, Burkholderia ubonensis,* and *Burkholderia vietnamiensis.* In one embodiment, the modified LPS molecular species are derived from the bacterial species *Burkholderia pseudomallei.*

In one embodiment, the modified LPS molecular species are derived from the *Moraxella* genus (from the *Proteobacteria* phylum), including, but not limited to, *Moraxella atlantae, Moraxella boevrei, Moraxella bovis, Moraxella bovoculi, Moraxella canis, Moraxella caprae, Moraxella catarrhalis, Moraxella caviae, Moraxella cuniculi, Moraxella equi, Moraxella lacunata, Moraxella lincolnii, Moraxella nonliquefaciens, Moraxella oblonga, Moraxella osloensis, Moraxella pluranimalium, Moraxella porci,* and *Moraxella saccharolytica*. In one embodiment, the modified LPS molecular species are derived from the bacterial species *Moraxella catarrhalis.*

In one embodiment, the modified LPS molecular species are derived from the *Neisseria* genus (from the *Proteobacteria* phylum), including, but not limited to, *Neisseria animalis, Neisseria animaloris, Neisseria bacilliformis, Neisseria canis, Neisseria cinerea, Neisseria dentiae, Neisseria elongata, Neisseria flava, Neisseria flavescens, Neisseria gonorrhoeae, Neisseria iguanae, Neisseria lactamica, Neisseria macacae, Neisseria meningitidis, Neisseria mucosa, Neisseria oralis, Neisseria perflava, Neisseria pharyngis, Neisseria polysaccharea, Neisseria shayeganii, Neisseria sicca, Neisseria subflava, Neisseria wadsworthii, Neisseria weaveri,* and *Neisseria zoodegmatis.* In one embodiment, the modified LPS molecular species are derived from the bacterial species *Neisseria sicca.*

In one embodiment, the modified LPS molecular species are derived from the *Pseudomonas* genus (from the *Proteobacteria* phylum), including, but not limited to, *Pseudomonas aeruginosa, Pseudomonas alcaligenes, Pseudomonas amygdali, Pseudomonas anguilliseptica, Pseudomonas antarctica, Pseudomonas argentinensis, Pseudomonas asplenii, Pseudomonas aurantiaca, Pseudomonas aureofaciens, Pseudomonas avellanae, Pseudomonas azotoformans, Pseudomonas balearica, Pseudomonas blatchfordae, Pseudomonas borbori, Pseudomonas brassicacearum, Pseudomonas brenneri, Pseudomonas caricapapayae, Pseudomonas cedrina, Pseudomonas chlororaphis, Pseudomonas cichorii, Pseudomonas citronellolis, Pseudomonas coronafaciens, Pseudomonas corrugata, Pseudomonas corrugata, Pseudomonas cremoricolorata, Pseudomonas denitrificans, Pseudomonas entomophila, Pseudomonas ficuserectae, Pseudomonas flavescens, Pseudomonas fluorescens, Pseudomonas fragi, Pseudomonas fulva, Pseudomonas gessardii, Pseudomonas helianthi, Pseudomonas libanensis, Pseudomonas lundensis, Pseudomonas luteola, Pseudomonas mandelii, Pseudomonas marginalis, Pseudomonas mediterranea, Pseudomonas meliae, Pseudomonas mendocina, Pseudomonas meridiana, Pseudomonas migulae, Pseudomonas monteilii, Pseudomonas mosselii, Pseudomonas mucidolens, Pseudomonas nitroreducens, Pseudomonas oleovorans, Pseudomonas orientalis, Pseudomonas oryzihabitans, Pseudomonas panacis, Pseudomonas parafulva, Pseudomonas pertucinogena, Pseudomonas plecoglossicida, Pseudomonas protegens, Pseudomonas proteolytica, Pseudomonas pseudoalcaligenes, Pseudomonas putida, Pseudomonas resinovorans, Pseudomonas rhodesiae, Pseudomonas savastanoi, Pseudomonas straminea, Pseudomonas stutzeri, Pseudomonas synxantha, Pseudomonas syringae, Pseudomonas taetrolens, Pseudomonas thivervalensis, Pseudomonas tolaasii, Pseudomonas tomato, Pseudomonas veronii,* and *Pseudomonas viridiflava.* In one embodiment, the modified LPS molecular species are derived from the bacterial species *Pseudomonas aeruginosa* or *Pseudomonas fluorescens.*

In one embodiment, the modified LPS molecular species are derived from the *Vibrio* genus (from the *Proteobacteria* phylum), including, but not limited to, *Vibrio adaptatus, Vibrio aerogenes, Vibrio aestivus, Vibrio aestuarianus, Vibrio agarivorans, Vibrio albensis, Vibrio alfacsensis, Vibrio alginolyticus, Vibrio anguillarum, Vibrio areninigrae, Vibrio artabrorum, Vibrio atlanticus, Vibrio atypicus, Vibrio azureus, Vibrio brasiliensis, Vibrio bubulus, Vibrio calviensis, Vibrio campbellii, Vibrio casei, Vibrio chagasii, Vibrio cholerae, Vibrio cincinnatiensis, Vibrio coralliilyticus, Vibrio crassostreae, Vibrio cyclitrophicus, Vibrio diabolicus, Vibrio diazotrophicus, Vibrio ezurae, Vibrio fluvialis, Vibrio fortis, Vibrio fumissii, Vibrio gallicus, Vibrio gazogenes, Vibrio gigantis, Vibrio halioticoli, Vibrio harveyi, Vibrio hepatarius, Vibrio hippocampi, Vibrio hispanicus, Vibrio ichthyoenteri, Vibrio indicus, Vibrio kanaloae, Vibrio lentus, Vibrio litoralis, Vibrio logei, Vibrio mediterranei, Vibrio metschnikovii, Vibrio mimicus, Vibrio mytili, Vibrio natriegens, Vibrio navarrensis, Vibrio neonatus, Vibrio neptunius, Vibrio nereis, Vibrio nigripulchritudo, Vibrio ordalii, Vibrio orientalis, Vibrio pacinii, Vibrio parahaemolyticus, Vibrio pectenicida, Vibrio penaeicida, Vibrio pomeroyi, Vibrio ponticus, Vibrio proteolyticus, Vibrio rotiferianus, Vibrio ruber, Vibrio rumoiensis, Vibrio salmonicida, Vibrio scophthalmi, Vibrio splendidus, Vibrio superstes, Vibrio tapetis, Vibrio tasmaniensis, Vibrio tubiashii, Vibrio vulnificus, Vibrio wodanis,* and *Vibrio xuii.* In one embodiment, the modified LPS molecular species are derived from the bacterial species *Vibrio fischeri.*

In one embodiment, the modified LPS molecular species are of general **Formula A,** wherein:

Y is -O-;
Z is a hydroxyl group (-OH);
$R_2$ is a phosphate group ($-OPO_3H_2$);
$R_3$ is a monosaccharide, an oligosaccharide or a polysaccharide; optionally substituted; preferably $R_3$ is a core oligosaccharide (*i.e.,* an inner core, or an inner and outer core) optionally substituted with an antigen domain, such as, without limitation, an O-antigen domain and/or a capping antigen;
$R_6$ is a fatty acid residue; optionally substituted; and
$R_7$ is a hydrogen or a fatty acid residue; optionally substituted.

In one embodiment, the modified LPS molecular species are of general **Formula A,** wherein:

Y is -O-;
Z is a phosphate group ($-OPO_3H_2$);
$R_2$ is a hydroxyl group (-OH);
$R_3$ is a monosaccharide, an oligosaccharide or a polysaccharide; optionally substituted; preferably $R_3$ is a core oligosaccharide (*i.e.,* an inner core, or an inner and outer core) optionally substituted with an antigen domain, such as, without limitation, an O-antigen domain and/or a capping antigen;
$R_6$ is a fatty acid residue; optionally substituted; and
$R_7$ is a hydrogen or a fatty acid residue; optionally substituted.

In one embodiment, the modified LPS molecular species are of general **Formula A,** wherein:

Y is -O-;
Z is a hydroxyl group (-OH);
$R_2$ is a hydroxyl group (-OH);
$R_3$ is a monosaccharide, an oligosaccharide or a polysaccharide; optionally substituted; preferably $R_3$ is a core oligosaccharide (*i.e.*, an inner core, or an inner and outer core) optionally substituted with an antigen domain, such as, without limitation, an O-antigen domain and/or a capping antigen;
$R_6$ is a fatty acid residue; optionally substituted; and
$R_7$ is a hydrogen or a fatty acid residue; optionally substituted.

In one embodiment, the modified LPS molecular species are of general **Formula B,** wherein:

X and Y are -O-;
$R_1$ is a hydroxyl group (-OH);
$R_2$ is a phosphate group ($-OPO_3H_2$);
$R_3$ is a monosaccharide, an oligosaccharide or a polysaccharide; optionally substituted; preferably $R_3$ is a core oligosaccharide (*i.e.*, an inner core, or an inner and outer core) optionally substituted with an antigen domain, such as, without limitation, an O-antigen domain and/or a capping antigen;
$R_4$ and $R_6$ are, independently from each other, a fatty acid residue; optionally substituted; and
$R_5$ and $R_7$ are, independently from each other, a hydrogen or a fatty acid residue;
optionally substituted.

In one embodiment, the modified LPS molecular species are of general **Formula B,** wherein:

X and Y are -O-;
$R_1$ is a phosphate group ($-OPO_3H_2$);
$R_2$ is a hydroxyl group (-OH);
$R_3$ is a monosaccharide, an oligosaccharide or a polysaccharide; optionally substituted; preferably $R_3$ is a core oligosaccharide (*i.e.*, an inner core, or an inner and outer core) optionally substituted with an antigen domain, such as, without limitation, an O-antigen domain and/or a capping antigen;
$R_4$ and $R_6$ are, independently from each other, a fatty acid residue; optionally substituted; and
$R_5$ and $R_7$ are, independently from each other, a hydrogen or a fatty acid residue;
optionally substituted.

In one embodiment, the modified LPS molecular species are of general **Formula B,** wherein:

X and Y are -O-;
$R_1$ is a hydroxyl group (-OH);
$R_2$ is a hydroxyl group (-OH);
$R_3$ is a monosaccharide, an oligosaccharide or a polysaccharide; optionally substituted; preferably $R_3$ is a core oligosaccharide (*i.e.*, an inner core, or an inner and outer core) optionally substituted with an antigen domain, such as, without limitation, an O-antigen domain and/or a capping antigen;
$R_4$ and $R_6$ are, independently from each other, a fatty acid residue; optionally substituted; and
$R_5$ and $R_7$ are, independently from each other, a hydrogen or a fatty acid residue;
optionally substituted.

In one embodiment, the modified LPS molecular species are selected from the compounds of Formulas (I)(1)(a), (I)(1)(b), (I)(1)(c), (I)(1)(d), (I)(1)(e), (1)(1)(f), (I)(2)(a), (I)(2)(b), (I)(2)(c), (I)(3)(a), (I)(3)(b), (I)(3)(c), (I)(5)(a), (I)(5)(b), (I)(6)(a), (I)(6)(b), (I)(7)(a), (I)(7)(b), (I)(7)(c), (I)(7)(d), (I)(8)(a), (I)(8)(b), (I)(8)(c), (I)(9)(a), (I)(9)(b), (I)(9)(c), (I)(9)(d), (I)(9)(e), (I)(9)(f), (II)(1)(a), (II)(1)(b), (II)(1)(c), (II)(1)(d), (II)(2)(a), (II)(2)(b), (II)(2)(c), (II)(2)(d), (III)(1)(a), (III)(1)(b), (III)(1)(c), (III)(1)(d), (IV)(1)(a), (IV)(1)(b), (IV)(1)(c), (V)(1)(a), (V)(1)(b), (V)(1)(c), (V)(1)(d), (V)(1)(e), (V)(1)(f), (V)(1)(g), (V)(1)(h), (V)(1)(i), (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), (VI)(2)(a), (VI)(2)(b), (VI)(2)(c), (VII)(1)(a), (VII)(1)(b), (VII)(1)(c), (VIII)(1)(a), (VIII)(1)(b), (VIII)(1)(c), (VIII)(1)(d), (IX)(1)(a), (IX)(1)(b), (IX)(1)(c), (IX)(1)(d), (X)(1)(a), (X)(1)(b), (X)(1)(c), (X)(1)(d), (XI)(1)(a), (XI)(1)(b), (XI)(1)(c), (XI)(1)(d), (XII)(1), (XIII)(2)(a), (XIII)(2)(b), (XIII)(3)(a), (XIII)(3)(b), (XIII)(4)(a), (XIII)(4)(b), (XIII)(5)(a), (XIII)(5)(b), (XIII)(5)(c), (XIII)(5)(d), (XIII)(6)(a), (XIII)(6)(b), (XIII)(6)(c), (XIII)(6)(d), (XIII)(6)(e), (XIII)(6)(f), (XIII)(7)(a), (XIII)(7)(b), (XIII)(7)(c), (XIII)(7)(d), (XIII)(7)(e), (XIII)(7)(f), (XIII)(8)(a), (XIII)(8)(b), (XIII)(8)(c), (XIII)(8)(d), (XIII)(8)(e), (XIII)(8)(f), (XIII)(9)(a), (XIII)(9)(b), (XIII)(9)(c), (XIII)(9)(d), (XIII)(9)(e), (XIII)(9)(f), (XIII)(9)(g), (XIII)(9)(h), (XIII)(9)(i), (XIΠ)(9)(j), and a combination thereof, as seen in **Figures 2-4**, 6-17, 27, 29-33 and **36-43,** wherein **R** at position C6' is a mono-, oligo- or polysaccharidic moiety comprising a core oligosaccharide domain and optionally, an antigen domain, such as an O-antigen domain and/or a capping antigen; wherein **X** at position C1 is an ethyl moiety.

In one embodiment, the modified LPS molecular species are selected from the compounds of Formulas (I)(1)(a), (I)(1)(b), (I)(1)(c), (I)(1)(d), (I)(1)(e), (1)(1)(f), (I)(2)(a), (I)(2)(b), (I)(2)(c), (I)(3)(a), (I)(3)(b), (I)(3)(c), (I)(5)(a), (I)(5)(b), (I)(6)(a), (I)(6)(b), (I)(7)(a), (I)(7)(b), (I)(7)(c), (I)(7)(d), (I)(8)(a), (I)(8)(b), (I)(8)(c), (I)(9)(a), (I)(9)(b), (I)(9)(c), (I)(9)(d), (I)(9)(e), (I)(9)(f), (II)(2)(a), (II)(2)(b), (II)(2)(c), (II)(2)(d), (III)(1)(a), (III)(1)(b), (III)(1)(c), (III)(1)(d), (IV)(1)(a), (IV)(1)(b), (IV)(1)(c), (V)(1)(a), (V)(1)(b), (V)(1)(c), (V)(1)(d), (V)(1)(e), (V)(1)(f), (V)(1)(g), (V)(1)(h), (V)(1)(i), (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), (VI)(2)(a), (VI)(2)(b), (VI)(2)(c), (VII)(1)(a), (VII)(1)(b), (VII)(1)(c), (VIII)(1)(a), (VIII)(1)(b), (VIII)(1)(c), (VIII)(1)(d), (IX)(1)(a), (IX)(1)(b), (IX)(1)(c), (IX)(1)(d), (X)(1)(a), (X)(1)(b), (X)(1)(c), (X)(1)(d), (XI)(1)(a), (XI)(1)(b), (XI)(1)(c), (XI)(1)(d), (XIII)(2)(a), (XIII)(2)(b), (XIII)(3)(a), (XIII)(3)(b), (XIII)(4)(a), (XIII)(4)(b), (XIII)(5)(a), (XIII)(5)(b), (XIII)(5)(c), (XIII)(5)(d), (XIII)(6)(a), (XIII)(6)(b), (XIII)(6)(c),(XIII)(6)(d), (XIII)(6)(e), (XIII)(6)(f), (XIII)(7)(a), (XIII)(7)(b), (XIII)(7)(c), (XIII)(7)(d), (XIII)(7)(e), (XIII)(7)(f), (XIII)(8)(a), (XIII)(8)(b), (XIII)(8)(c), (XIII)(8)(d), (XIII)(8)(e), (XIII)(8)(f), (XIII)(9)(a), (XIII)(9)(b), (XIII)(9)(c), (XIII)(9)(d), (XIII)(9)(e), (XIII)(9)(f), (XIII)(9)(g), (XIII)(9)(h), (XIII)(9)(i), (XΠI)(9)(j), and a combination thereof, as seen in **Figures 2-4**,7-17, 29-33 and **36-43,** wherein **R** at position C6' is a mono-, oligo- or polysaccharidic moiety comprising a core oligosaccharide domain and optionally, an antigen domain, such as an O-antigen domain and/or a capping antigen; wherein **X** at position C1 is an ethyl moiety.

In one embodiment, the modified LPS molecular species are selected from the compounds of Formulas (I)(1)(a), (I)(1)(b), (I)(1)(c), (I)(1)(d), (I)(1)(e), (1)(1)(f), (I)(2)(a), (I)(2)(b), (I)(2)(c), (I)(3)(a), (I)(3)(b), (I)(3)(c), (II)(1)(a), (II)(1)(b), (II)(1)(c), (II)(1)(d), (II)(2)(a), (II)(2)(b), (II)(2)(c), (II)(2)(d), (III)(1)(a), (III)(1)(b), (III)(1)(c), (III)(1)(d), (IV)(1)(a), (IV)(1)(b), (IV)(1)(c), (V)(1)(a), (V)(1)(b), (V)(1)(c), (V)(1)(d), (V)(1)(e), (V)(1)(f), (V)(1)(g), (V)(1)(h), (V)(1)(i), (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), (VI)(2)(a), (VI)(2)(b), (VI)(2)(c), (VII)(1)(a), (VII)(1)(b), (VII)(1)(c), (VIII)(1)(a), (VIII)(1)(b), (VIII)(1)(c), (VIII)(1)(d), (IX)(1)(a), (IX)(1)(b), (IX)(1)(c), (IX)(1)(d), (X)(1)(a), (X)(1)(b), (X)(1)(c), (X)(1)(d), (XI)(1)(a), (XI)(1)(b), (XI)(1)(c), (XI)(1)(d), (XII)(1), and a combination thereof, as seen in **Figures 2-4, 6-17** and **27,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (I)(1)(a), (I)(1)(b), (I)(1)(c), (I)(1)(d), (I)(1)(e), (1)(1)(f), (I)(2)(a), (I)(2)(b), (I)(2)(c), (I)(3)(a), (I)(3)(b), (I)(3)(c), (II)(2)(a), (II)(2)(b), (II)(2)(c), (II)(2)(d), (III)(1)(a), (III)(1)(b), (III)(1)(c), (III)(1)(d), (IV)(1)(a), (IV)(1)(b), (IV)(1)(c), (V)(1)(a), (V)(1)(b), (V)(1)(c), (V)(1)(d), (V)(1)(e), (V)(1)(f), (V)(1)(g), (V)(1)(h), (V)(1)(i), (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), (VI)(2)(a), (VI)(2)(b), (VI)(2)(c), (VII)(1)(a), (VII)(1)(b), (VII)(1)(c), (VIII)(1)(a), (VIII)(1)(b), (VIII)(1)(c), (VIII)(1)(d), (IX)(1)(a), (IX)(1)(b), (IX)(1)(c), (IX)(1)(d), (X)(1)(a), (X)(1)(b), (X)(1)(c), (X)(1)(d), (XI)(1)(a), (XI)(1)(b), (XI)(1)(c), (XI)(1)(d)" and a combination thereof, as seen in **Figures 2-4** and **7-17,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (I)(1)(a), (I)(1)(b), (I)(1)(c), (I)(1)(d), (I)(1)(e), (1)(1)(f), (I)(2)(a), (I)(2)(b), (I)(2)(c), (I)(3)(a), (I)(3)(b), (I)(3)(c), (I)(5)(a), (I)(5)(b), (I)(6)(a), (I)(6)(b), (I)(7)(a), (I)(7)(b), (I)(7)(c), (I)(7)(d), (I)(8)(a), (I)(8)(b), (I)(8)(c), (I)(9)(a), (I)(9)(b), (I)(9)(c), (I)(9)(d), (I)(9)(e), (I)(9)(f), and a combination thereof, as seen in **Figures 2-4 and 29-33,** wherein **R** at position C6' is a mono-, oligo- or polysaccharidic moiety comprising a core oligosaccharide domain and optionally, an antigen domain, such as an O-antigen domain and/or a capping antigen; wherein **X** at position C1 is an ethyl moiety.

In one embodiment, the modified LPS molecular species are selected from the compounds of Formulas (I)(1)(a), (I)(1)(b), (I)(1)(c), (I)(1)(d), (I)(1)(e), (1)(1)(f), (I)(2)(a), (I)(2)(b), (I)(2)(c), (I)(3)(a), (I)(3)(b), (I)(3)(c), and a combination thereof, as seen in **Figures 2-4,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (I)(1)(a),

(I)(1)(b), (I)(1)(c), (I)(1)(d), (I)(1)(e), (1)(1)(f), and a combination thereof, as seen in **Figure 2,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (I)(2)(a), (I)(2)(b), (I)(2)(c), and a combination thereof, as seen in **Figure 3,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (I)(3)(a), (I)(3)(b), (I)(3)(c), and a combination thereof, as seen in **Figure 4,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (I)(5)(a), (I)(5)(b), and a combination thereof, as seen in **Figure 29,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (I)(6)(a), (I)(6)(b), and a combination thereof, as seen in **Figure 30,** wherein **R** at position C6' is a mono-, oligo- or polysaccharidic moiety comprising a core oligosaccharide domain and optionally, an antigen domain, such as an O-antigen domain and/or a capping antigen; wherein **X** at position C1 is an ethyl moiety.

In one embodiment, the modified LPS molecular species are selected from the compounds of Formulas (I)(7)(a), (I)(7)(b), (I)(7)(c), (I)(7)(d), and a combination thereof, as seen in **Figure 31,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (I)(8)(a), (I)(8)(b), (I)(8)(c), and a combination thereof, as seen in **Figure 32,** wherein **R** at position C6' is a mono-, oligo- or polysaccharidic moiety comprising a core oligosaccharide domain and optionally, an antigen domain, such as an O-antigen domain and/or a capping antigen; wherein **X** at position C1 is an ethyl moiety.

In one embodiment, the modified LPS molecular species are selected from the compounds of Formulas (I)(9)(a), (I)(9)(b), (I)(9)(c), (I)(9)(d), (I)(9)(e), (1)(9)(f), and a combination thereof, as seen in **Figure 33,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (II)(1)(a), (II)(1)(b), (II)(1)(c), (II)(1)(d), (II)(2)(a), (II)(2)(b), (II)(2)(c), (II)(2)(d), and a combination thereof, as seen in **Figures 6-7,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (II)(1)(a), (II)(1)(b), (II)(1)(c), (II)(1)(d), and a combination thereof, as seen in **Figure 6,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (II)(2)(a), (II)(2)(b), (II)(2)(c), (II)(2)(d), and a combination thereof, as seen in **Figure 7,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (III)(1)(a), (III)(1)(b), (III)(1)(c), (III)(1)(d), and a combination thereof, as seen in **Figure 8,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formula (XII)(1), and a combination thereof, as seen in **Figure 27,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (IV)(1)(a), (IV)(1)(b), (IV)(1)(c), and a combination thereof, as seen in **Figure 9,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (V)(1)(a), (V)(1)(b), (V)(1)(c), (V)(1)(d), (V)(1)(e), (V)(1)(f), (V)(1)(g), (V)(1)(h), (V)(1)(i), and a combination thereof, as seen in **Figure 10,** wherein **R** at position C6' is a mono-, oligo- or polysaccharidic moiety comprising a core oligosaccharide domain and

optionally, an antigen domain, such as an O-antigen domain and/or a capping antigen.

In one embodiment, the modified LPS molecular species are selected from the compounds of Formulas (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), (VI)(2)(a), (VI)(2)(b), (VI)(2)(c), and a combination thereof, as seen in **Figures 11-12,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), and a combination thereof, as seen in **Figure 11,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (VI)(2)(a), (VI)(2)(b), (VI)(2)(c), and a combination thereof, as seen in **Figure 12,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (VII)(1)(a), (VII)(1)(b), (VII)(1)(c), and a combination thereof, as seen in **Figure 13,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (VIII)(1)(a), (VIII)(1)(b), (VIII)(1)(c), (VIII)(1)(d), and a combination thereof, as seen in **Figure 14,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (IX)(1)(a), (IX)(1)(b), (IX)(1)(c), (IX)(1)(d), and a combination thereof, as seen in **Figure 15,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (X)(1)(a), (X)(1)(b), (X)(1)(c), (X)(1)(d), and a combination thereof, as seen in **Figure 16,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (XI)(1)(a), (XI)(1)(b), (XI)(1)(c), (XI)(1)(d), and a combination thereof, as seen in **Figure 17,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (XIII)(2)(a), (XIII)(2)(b), (XIII)(3)(a), (XIII)(3)(b), (XIII)(4)(a), (XIII)(4)(b), (XIII)(5)(a), (XIII)(5)(b), (XIII)(5)(c), (XIII)(5)(d), (XIII)(6)(a), (XIII)(6)(b), (XIII)(6)(c),(XIII)(6)(d), (XIII)(6)(e), (XIII)(6)(f), (XIII)(7)(a), (XIII)(7)(b), (XIII)(7)(c), (XIII)(7)(d), (XIII)(7)(e), (XIII)(7)(f), (XIII)(8)(a), (XIII)(8)(b), (XIII)(8)(c), (XIII)(8)(d), (XIII)(8)(e), (XIII)(8)(f), (XIII)(9)(a), (XIII)(9)(b), (XIII)(9)(c), (XIII)(9)(d), (XIII)(9)(e), (XIII)(9)(f), (XIII)(9)(g), (XIII)(9)(h), (XIII)(9)(i), (XΠI)(9)(j), and a combination thereof, as seen in **Figures 36-43,** wherein **R** at position C6' is a mono-, oligo- or polysaccharidic moiety comprising a core oligosaccharide domain and optionally, an antigen domain, such as an O-antigen domain and/or a capping antigen; wherein **X** at position C1 is an ethyl moiety.

In one embodiment, the modified LPS molecular species are selected from the compounds of Formulas (XIII)(2)(a), (XIII)(2)(b), and a combination thereof, as seen in **Figure 36,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (XIII)(3)(a), (XIII)(3)(b), and a combination thereof, as seen in **Figure 37,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (XIII)(4)(a), (XIII)(4)(b), and a combination thereof, as seen in **Figure 38,** wherein **R** at position C6' is a mono-, oligo- or polysaccharidic moiety comprising a core oligosaccharide domain and optionally, an antigen domain, such as an O-antigen domain and/or a capping antigen; wherein **X** at position C1 is an ethyl moiety.

In one embodiment, the modified LPS molecular species are selected from the compounds of Formulas (XIII)(5)(a),(XIII)(5)(b), (XIII)(5)(c), (XIII)(5)(d), and a combination thereof, as seen in **Figure 39,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (XIII)(6)(a), (XIII)(6)(b), (XIII)(6)(c), (XIII)(6)(d), (XIII)(6)(e), (XIII)(6)(f), and a combination thereof, as seen in **Figure 40,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (XIII)(7)(a), (XIII)(7)(b), (XIII)(7)(c), (XIII)(7)(d), (XIII)(7)(e), (XIII)(7)(f), and a combination thereof, as seen in **Figure 41,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are selected from the compounds of Formulas (XIII)(8)(a), (XIII)(8)(b), (XIII)(8)(c), (XIII)(8)(d), (XIII)(8)(e), (XIII)(8)(f), and a combination thereof, as seen in **Figure 42,** wherein **R** at position C6' is a mono-, oligo- or polysaccharidic moiety comprising a core oligosaccharide domain and optionally, an antigen domain, such as an O-antigen domain and/or a capping antigen; wherein X at position C1 is an ethyl moiety.

In one embodiment, the modified LPS molecular species are selected from the compounds of Formulas (XIII)(9)(a), (XIII)(9)(b), (XIII)(9)(c), (XIII)(9)(d), (XIII)(9)(e), (XIII)(9)(f), (XIII)(9)(g), (XIII)(9)(h), (XIII)(9)(i), (XΠI)(9)(j), and a combination thereof, as seen in **Figure 43,** wherein **R** at position C6' is a mono-, oligo- or polysaccharidic moiety comprising a core oligosaccharide domain and optionally, an antigen domain, such as an O-antigen domain and/or a capping antigen.

In one embodiment, the modified LPS molecular species are soluble in aqueous solutions. In one embodiment, the modified LPS molecular species do not precipitate in aqueous solutions. The term "aqueous solution" include any water-based solutions, such as, but not limited to, water, saline solutions, buffered solutions and the like.

In one embodiment, the modified LPS molecular species are soluble in water and/or in phosphate buffered saline (PBS). In one embodiment, the modified LPS molecular species do not precipitate in water and/or in PBS.

Solubility and/or precipitation of LPS molecular species can be readily assessed using techniques well-known to the one skilled in the art. Such techniques include, but are not limited to, dynamic light scattering (DLS), scanning electron microscope (SEM), Fourier transform infrared spectroscopy (FT-IR) and the like.

In one embodiment, the modified LPS molecular species form particles, preferably supramolecular particles, in aqueous solutions.

In one embodiment, particles of modified LPS molecular species according to the invention have an average size in aqueous solutions, preferably in water and/or in PBS, smaller than about 500 nm, preferably about 490 nm, about 480 nm, about 470 nm, about 460 nm, about 450 nm, about 440 nm, about 430 nm, about 420 nm, about 410 nm, about 400 nm, about 390 nm, about 380 nm, about 370 nm, about 360 nm, about 350 nm, about 340 nm, about 330 nm, about 320 nm, about 310 nm, about 300 nm, about 290 nm, about 280 nm, about 270 nm, about 260 nm, about 250 nm, about 240 nm, about 230 nm, about 220 nm, about 210 nm, about 200 nm, about 190 nm, about 180 nm, about 170 nm, about 160 nm, about 150 nm, about 140 nm, about 130 nm, about 120 nm, about 110 nm, about 100 nm, about 90 nm, about 80 nm, about 70 nm, about 60 nm, about 50 nm, about 40 nm, about 30 nm, about 20 nm in diameter; as can be assessed by, *e.g.,* DLS; preferably DLS with a scattering angle $\Theta = 90°$, incident laser wavelength $\lambda = 633$ nm and temperature at 25°C.

In one embodiment, particles of modified LPS molecular species according to the invention have an average size in aqueous solutions, preferably in water and/or in PBS, smaller than about 30 nm in diameter; as can be assessed by, *e.g.,* DLS; preferably DLS with a scattering angle $\Theta = 90°$, incident laser wavelength $\lambda = 633$ nm and temperature at 25°C.

In one embodiment, particles of modified LPS molecular species according to the invention have an average size in aqueous solutions, preferably in water and/or in PBS, ranging from about 6 nm to about 500 nm, from about 6 nm to about 450 nm, from about 6 nm to about 400 nm, from about 6 nm to about 350 nm, from about 6 nm to about 300 nm, from about 6 nm to about 250 nm, from about 6 nm to about 200 nm, from about 6 nm to about 150 nm, from about 6 nm to about 100 nm, from about 6 nm to about 75 nm, from about 6 nm to about 60 nm, from about 6 nm to about 50 nm, from about 6 nm to about 40 nm, from about 6 nm to about 35 nm, from about 6 nm to about 34 nm, from about 6 nm to about 33 nm, from about 6 nm to about 32 nm, from about 6 nm to about 31 nm, from about 6 nm to about 30 nm, from about 6 nm to about 29 nm, from about 6 nm to about 28 nm, from about 6 nm to about 27 nm, from about 6 nm to about 26 nm, from about 6 nm to about 25 nm, from about 6 nm to about 24 nm, from about 6 nm to about 23 nm, from about 6 nm to about 22 nm, from about 6 nm to about 21 nm, from about 6 nm to about 20 nm, from about 6 nm to about 19 nm, from about 6 nm to about 18 nm, from about 6 nm to about 17 nm, from about 6 nm to about 16 nm, from about 6 nm to about 15 nm, from about 6 nm to about 14 nm, from about 6 nm to about 13 nm, from about 6 nm to about 12 nm, from about 6 nm to about 11 nm, from about 6 nm to about 10 nm in diameter; as can be assessed by, *e.g.,* DLS; preferably DLS with a scattering angle $\Theta = 90°$, incident laser wavelength $\lambda =$ 633 nm and temperature at 25°C.

In one embodiment, particles of modified LPS molecular species according to the invention have an average size in aqueous solutions, preferably in water and/or in PBS, ranging from about 9 nm to about 500 nm, from about 9 nm to about 450 nm, from about 9 nm to about 400 nm, from about 9 nm to about 350 nm, from about 9 nm to about 300 nm, from about 9 nm to about 250 nm, from about 9 nm to about 200 nm, from about 9 nm to about 150 nm, from about 9 nm to about 100 nm, from about 9 nm to about 90 nm, from about 8 nm to about 80 nm, from about 9 nm to about 75 nm, from about 9 nm to about

50 nm, from about 9 nm to about 40 nm, from about 9 nm to about 35 nm, from about 9 nm to about 34 nm, from about 9 nm to about 33 nm, from about 9 nm to about 32 nm, from about 9 nm to about 31 nm, from about 9 nm to about 30 nm, from about 9 nm to about 29 nm, from about 9 nm to about 28 nm, from about 9 nm to about 27 nm, from about 9 nm to about 26 nm, from about 9 nm to about 25 nm, from about 9 nm to about 24 nm, from about 9 nm to about 23 nm, from about 9 nm to about 22 nm, from about 9 nm to about 21 nm, from about 9 nm to about 20 nm, from about 9 nm to about 19 nm, from about 9 nm to about 18 nm, from about 9 nm to about 17 nm, from about 9 nm to about 16 nm, from about 9 nm to about 15 nm, from about 9 nm to about 14 nm, from about 9 nm to about 13 nm, from about 9 nm to about 12 nm, from about 9 nm to about 11 nm, from about 9 nm to about 10 nm in diameter; as can be assessed by, *e.g.,* DLS; preferably DLS with a scattering angle Θ = 90°, incident laser wavelength λ = 633 nm and temperature at 25°C.

In one embodiment, particles of modified LPS molecular species according to the invention have an average size in aqueous solutions, preferably in water and/or in PBS, ranging from about 10 nm to about 500 nm, from about 10 nm to about 450 nm, from about 10 nm to about 400 nm, from about 10 nm to about 350 nm, from about 10 nm to about 300 nm, from about 10 nm to about 250 nm, from about 10 nm to about 200 nm, from about 10 nm to about 150 nm, from about 10 nm to about 100 nm, from about 10 nm to about 75 nm, from about 10 nm to about 50 nm, from about 10 nm to about 40 nm, from about 10 nm to about 35 nm, from about 10 nm to about 34 nm, from about 10 nm to about 33 nm, from about 10 nm to about 32 nm, from about 10 nm to about 31 nm, from about 10 nm to about 30 nm, from about 10 nm to about 29 nm, from about 10 nm to about 28 nm, from about 10 nm to about 27 nm, from about 10 nm to about 26 nm, from about 10 nm to about 25 nm, from about 10 nm to about 24 nm, from about 10 nm to about 23 nm, from about 10 nm to about 22 nm, from about 10 nm to about 21 nm, from about 10 nm to about 20 nm, from about 10 nm to about 19 nm, from about 10 nm to about 18 nm, from about 10 nm to about 17 nm, from about 10 nm to about 16 nm, from about 10 nm to about 15 nm, from about 10 nm to about 14 nm, from about 10 nm to about 13 nm, from about 10 nm to about 12 nm, from about 10 nm to about 11 nm in diameter; as can be assessed by, *e.g.,* DLS; preferably DLS with a scattering angle Θ = 90°, incident laser wavelength λ = 633 nm and temperature at 25°C.

In one embodiment, particles of modified LPS molecular species according to the invention have an average size in aqueous solutions, preferably in water and/or in PBS, ranging from about 14 nm to about 500 nm, from about 14 nm to about 450 nm, from about 14 nm to about 400 nm, from about 14 nm to about 350 nm, from about 14 nm to about 300 nm, from about 14 nm to about 250 nm, from about 14 nm to about 200 nm, from about 14 nm to about 150 nm, from about 14 nm to about 100 nm, from about 14 nm to about 75 nm, from about 14 nm to about 50 nm, from about 14 nm to about 40 nm, from about 14 nm to about 35 nm, from about 14 nm to about 34 nm, from about 14 nm to about 33 nm, from about 14 nm to about 32 nm, from about 14 nm to about 31 nm, from about 14 nm to about 30 nm, from about 14 nm to about 29 nm, from about 14 nm to about 28 nm, from about 14 nm to about 27 nm, from about 14 nm to about 26 nm, from about 14 nm to about 25 nm, from about 14 nm to about 24 nm, from about 14 nm to about 23 nm, from about 14 nm to about 22 nm, from about 14 nm to about 21 nm, from about 14 nm to about 20 nm, from about 14 nm to about 19 nm, from about 14 nm to about 18 nm, from about 14 nm to about 17 nm, from about 14 nm to about 16 nm, from about 14 nm to about 15 nm in diameter; as can be assessed by, *e.g.,* DLS; preferably DLS with a scattering angle Θ = 90°, incident laser wavelength λ = 633 nm and temperature at 25°C.

In one embodiment, the modified LPS molecular species are less toxic and/or pyrogenic than classical *Escherichia coli* or *Salmonella enterica* LPS molecules.

Means and methods to assess the toxicity and/or pyrogenicity of a LPS molecular species are well-known in the art.

Examples of such means and methods include, but are not limited to, *in vivo* tests such as, without limitation, rabbit pyrogen test (RPT), $C_{50}$ lethal dose in chicken embryo, $C_{50}$ lethal dose in GalN sensitized mouse, and acute toxicity test in normal mouse; and *in vitro* tests such as, without limitation, monocyte activation test (MAT).

The rabbit pyrogen test (RPT) involves measuring the rise in temperature of three rabbits following the intravenous injection of the test solution (such as, a composition comprising LPS to be tested). Temperature is first measured before injection of the test solution; the test solution is then injected and body temperature of each rabbit is measured for 3 hours. If the sum of the temperature of the three rabbits increases by less than 1.15°C, the test solution is considered to be non-pyrogenic; if the sum of the temperature of the three rabbits increases by more than 2.65°C, the test solution is considered to be pyrogenic; if the sum of the temperature of the three rabbits increases between 1.15°C and 2.65°C, the test should be repeated to confirm the results.

The monocyte activation test (MAT) is based on the activation of monocytes by pyrogens, leading to the production of cytokines that are detected in an immunological assay (such as, *e.g.,* an ELISA). There are different variants of the MAT available depending on (1) the source of monocytes, including whole blood, isolated primary monocytes (such as, *e.g.,* PBMC) or monocytic cell line; and (2) the ELISA read-out, including IL-6, IL-1β and TNF-$\alpha$. All of them mimic the human fever reaction *in vitro.*

For more details on means and methods to assess the toxicity and/or pyrogenicity of a LPS molecular species, see chapter 5.1.10 and chapter 2.6.30 of the European Pharmacopoeia.

In one embodiment, the modified LPS molecular species have an endotoxin activity below 5000 endotoxin

equivalent unit (EEU)/μg, preferably below 4000, 3000, 2000, 1500, 1000 EEU/pg; as can be assessed by, *e.g.*, monocyte activation test (MAT) based on peripheral blood mononuclear cells (PBMC) and IL-6 response.

In one embodiment, the modified LPS molecular species according to the present invention are modified by any means and method known to the one skilled in the art, including, but not limited to, chemically, enzymatically, physically, or genetically.

In one embodiment, the modified LPS molecular species according to the present invention are chemically-modified LPS molecular species.

In one embodiment, the modified LPS molecular species are obtainable by acidic hydrolysis of naturally-occurring LPS molecular species.

In one embodiment, the modified LPS molecular species are obtainable by acidic hydrolysis of naturally-occurring LPS molecular species at a temperature ranging from about 4°C to about 85°C.

In one embodiment, the modified LPS molecular species are obtainable by acidic hydrolysis of naturally-occurring LPS molecular species in presence of an alcohol, such as, e.g., methanol, ethanol, 1-propanol, 2-propanol, 1-butanol, 2-butanol, 2-methyl-1-propanol, 2-methyl-2-propanol, 1-pentanol, 3-methyl-1-butanol, 2,2-dimethyl-1-propanol, cyclopentanol, 1-hexanol, cyclohexanol, 1-heptanol, 1-octanol, 1-nonanol, 1-decanol, 2-propen-1-ol, phenylmethanol, diphenylmethanol, triphenylmethanol and the like.

In one embodiment, the modified LPS molecular species are obtainable by acidic hydrolysis of naturally-occurring LPS molecular species at a temperature ranging from about 4°C to about 85°C, in presence of an alcohol, such as, e.g., methanol, ethanol, 1-propanol, 2-propanol, 1-butanol, 2-butanol, 2-methyl-1-propanol, 2-methyl-2-propanol, 1-pentanol, 3-methyl-1-butanol, 2,2-dimethyl-1-propanol, cyclopentanol, 1-hexanol, cyclohexanol, 1-heptanol, 1-octanol, 1-nonanol, 1-decanol, 2-propen-1-ol, phenylmethanol, diphenylmethanol, triphenylmethanol and the like.

In one embodiment, the modified LPS molecular species are obtainable by acidic hydrolysis followed by alkaline hydrolysis of naturally-occurring LPS molecular species. In one embodiment, the modified LPS molecular species are obtainable by alkaline hydrolysis followed by acidic hydrolysis of naturally-occurring LPS molecular species.

In a preferred embodiment, the modified LPS molecular species are obtainable by alkaline hydrolysis followed by acidic hydrolysis of naturally-occurring LPS molecular species.

In one embodiment, the modified LPS molecular species are obtainable by acidic hydrolysis followed by alkaline hydrolysis of naturally-occurring LPS molecular species. According to this embodiment, it may be desirable to reduce the disaccharide of the lipid A domain, in particular the glucosamine, with sodium borohydride prior to alkaline hydrolysis. For example, reduction can be carried out at pH 8, in the presence of 0.5 M of NaBH (final concentration), for 12 hours at 52°C.

The present invention also relates to a method for obtaining modified LPS molecular species according to the present invention.

The innovative process is that dephosphorylation is targeted at the lipid A glycosidic phosphate, which is removed *in situ,* in the complete LPS molecular species, without splitting it in its lipid A and polysaccharide moieties.

In particular, it relates to a method for obtaining modified LPS molecular species which:

i. are devoid of phosphate group at position C1 of the reducing end of their lipid A domain, and are substituted at position C6' of the non-reducing end of their lipid A domain by a monosaccharide, an oligosaccharide or a polysaccharide moiety; and/or

ii. are devoid of phosphate group at position C4' of the non-reducing end of their lipid A domain, and are substituted at position C6' of the non-reducing end of their lipid A domain by a monosaccharide, an oligosaccharide or a polysaccharide moiety;

and

iii. comprise a smaller or greater number of fatty acid chains as compared to classical (*i.e.,* non-modified) *Escherichia coli* or *Salmonella enterica* LPS molecules - which comprise six fatty acid chains among which four are primary fatty acid chains and two are secondary fatty acid chains; and/or

iv. comprise fatty acid chains of shorter length and/or longer length as compared to classical (i.e., non-chemically modified) *Escherichia coli* or *Salmonella enterica* LPS molecules - which comprise four primary 3-OH-$C_{14}$ fatty acid chains, one secondary $C_{14}$ fatty acid chain and one secondary $C_{12}$ fatty acid chain.

In one embodiment, the method comprises a preliminary step of extracting naturally-occurring LPS molecular species.

In one embodiment, naturally-occurring LPS molecular species are extracted from bacterial cultures. All methods of bacterial growth can be used. Examples of bacterial cultures include, but are not limited to, fermentor-based bacterial cultures, flask cultures, solid medium cultures (*e.g.*, agar cultures), biofilm cultures grown on solid-liquid or liquid-gas interfaces, and any other methods known in the art. In one embodiment, naturally-occurring LPS molecular species are extracted from a bacterial pellet, a culture concentrate, a culture supernatant, an enriched membrane fraction, and/or an outer membrane vesicle (OMV) fraction.

In one embodiment, naturally-occurring LPS molecular species are extracted from naturally-occurring biomass samples, such as, *e.g.*, marine plankton or microbiota.

Any LPS extraction method using solvents, reagents and procedures compatible with production of pharmacological products, can be employed. LPS extraction is preferentially followed by LPS purification steps, necessary to reduce to acceptable levels the contaminants related to bacteria as well as to the fabrication process, such as, *e.g.*, biomass-production.

Methods for extracting naturally-occurring LPS molecular species are well-known in the art. Some tens of methods suitable for LPS extraction are described in the literature, see, *e.g.,* a review by Wang et al., 2010 (Purification and characterization of lipopolysaccharides. In Wang & Quinn (Eds.), Endotoxins: structure, function and recognition. 53:27-51. Springer). Other methods are also described in US patent 8,137,935.

In one embodiment, LPS are extracted according to the method described in US patent 8,137,935. According to this method, a bacterial pellet, a culture concentrate, a culture supernatant, an enriched membrane fraction, and/or an outer membrane vesicle (OMV) fraction is suspended in a mixture of solvents comprising from about 10 to about 90% of a solvent chosen from linear or branched aliphatic acids containing from 3 to 6 carbon atoms, and from about 90 to about 10% of a basic aqueous solution of an aliphatic amine containing from 0 to 12 carbon atoms. The entire mixture is stirred, to produce a suspension of bacterial LPS in the mixture of solvents containing bacterial cell debris. The LPS extract is recovered from the suspension by centrifugation and/or filtration in the form of, respectively, a supernatant and/or a filtrate. The particles in suspension are separated in the form of, respectively, a pellet and/or a retentate; and discarded. Finally, the solvents are eliminated from the supernatant and/or the filtrate, and the naturally-occurring LPS molecular species are usually lyophilized. Other means of conservation may be used, including, but not limited to, dried forms and suspensions in appropriate solvents.

In one embodiment, the method comprises a step of purifying the naturally-occurring LPS molecular species, as described, *e.g.,* in Tirsoaga et al., 2007 (Appl Environ Microbiol. 73(6):1803-8) to reduce to acceptable levels bacteria-related and process-related contaminants, as well as to assure TLR-grade purity of the final products (TLR4 ligands).

In one embodiment, the method comprises a step of treating naturally-occurring LPS molecular species by acidic hydrolysis.

Hydrolytic conditions known in the art and used for lipid A phosphate removal at position C1 of the reducing end of the lipid A domain fatally lead to the cleavage of the ketosidic bond at position C6' of the non-reducing end of the lipid A domain, between the lipid A and the saccharide moiety (position $R_3$ of **Formula A** or **Formula B).**

In one embodiment, the step of treating naturally-occurring LPS molecular species by acidic hydrolysis is carried out in acidic hydrolysis conditions allowing removal of glycosidic phosphate without cleavage of the ketosidic bond at position C6' of the non-reducing end of the lipid A domain.

In one embodiment, acidic hydrolysis is carried out at a temperature below 85°C, preferably ranging from about 4°C to about 85°C. Both mineral and organic acids can be used, in presence or absence of additional reagents, such as, *e. g.*, salts. The acidic hydrolysis can be carried out both in aqueous or organic solutions. Practical results can be obtained for hydrolysis duration from some minutes to some tens hours, preferentially from 1 to 10 hours.

In one embodiment, acidic hydrolysis is carried out in presence of an alcohol, such as, e.g., methanol, ethanol, 1-propanol, 2-propanol, 1-butanol, 2-butanol, 2-methyl-1-propanol, 2-methyl-2-propanol, 1-pentanol, 3-methyl-1-butanol, 2,2-dimethyl-1-propanol, cyclopentanol, 1-hexanol, cyclohexanol, 1-heptanol, 1-octanol, 1-nonanol, 1-decanol, 2-propen-1-ol, phenylmethanol, diphenylmethanol, triphenylmethanol and the like.

In one embodiment, acidic hydrolysis is carried out at a temperature below 85°C, preferably ranging from about 4°C to about 85°C, in presence of an alcohol, such as, e.g., methanol, ethanol, 1-propanol, 2-propanol, 1-butanol, 2-butanol, 2-methyl-1-propanol, 2-methyl-2-propanol, 1-pentanol, 3-methyl-1-butanol, 2,2-dimethyl-1-propanol, cyclopentanol, 1-hexanol, cyclohexanol, 1-heptanol, 1-octanol, 1-nonanol, 1-decanol, 2-propen-1-ol, phenylmethanol, diphenylmethanol, triphenylmethanol and the like.

In one embodiment, acidic hydrolysis also removes partially one or several secondary fatty acid residues, thus amplifying the degree of LPS detoxification.

In one embodiment, the method comprises an additional step of treating naturally-occurring LPS molecular species by alkaline hydrolysis, thereby removing one or several esterified fatty acid residues, thus amplifying the degree of LPS detoxification. For certain initial structures of naturally-occurring LPS molecular species, such as, *e.g.,* LPS molecular species comprising a phosphate group at position C4' of the non-reducing end of their lipid A domain substituted with an amino sugar (such as, *e.g.*, glucosamine, galactosamine, muramic acid and the like), alkaline hydrolysis can result in at least partial removal of the phosphate at C4' position.

In one embodiment, the steps of treating by acidic hydrolysis and treating by alkaline hydrolysis may be carried out in any order.

In one embodiment, the step of treating by acidic hydrolysis is carried out before the step of treating by alkaline hydrolysis. Alternatively, the step of treating by alkaline hydrolysis is carried out before the step of treating by acidic hydrolysis.

In a preferred embodiment, the step of treating by alkaline hydrolysis is carried out before the step of treating by acidic hydrolysis.

In one embodiment, the step of treating by acidic hydrolysis is carried out before the step of treating by alkaline hydrolysis. According to this embodiment, it may be desirable to reduce the disaccharide of the lipid A domain, in particular the glucosamine, with sodium borohydride prior to the step of treating by alkaline hydrolysis. For example, reduction can be carried out at pH 8, in the presence of 0.5 M of NaBH (final concentration), for 12 hours at 52°C.

Depending on the intended application, the one skilled in the art will readily recognize that the LPS molecular species should be purify to a certain degree of purity (*e.g.*, to GMP grade for an intended human application, or to a lesser degree of purity for veterinary applications). In particular, for veterinary applications, it is conceivable that the method for obtaining modified LPS molecular species according to the present invention does not comprise a step of purifying naturally-occurring LPS molecular species, *i.e.,* the method can comprise steps of treating naturally-occurring LPS directly from a bacterial pellet, a culture concentrate, a culture supernatant, an enriched membrane fraction, and/or an outer membrane vesicle (OMV) fraction.

The present invention also relates to compositions comprising at least one LPS molecular species.

In one embodiment, the at least one LPS molecular species is a modified LPS molecular species according to the present invention.

In one embodiment, the composition comprises at least one modified LPS molecular species of **Formula A** as described hereinabove.

In one embodiment, the composition comprises a mix of modified LPS molecular species of **Formula A** as described hereinabove.

In one embodiment, the composition comprises an enriched population of modified LPS molecular species of **Formula A** as described hereinabove.

In one embodiment, the composition comprises at least one modified LPS molecular species of **Formula B** as described hereinabove.

In one embodiment, the composition comprises a mix of modified LPS molecular species of **Formula B** as described hereinabove.

In one embodiment, the composition comprises an enriched population of modified LPS molecular species of **Formula B** as described hereinabove.

In one embodiment, the composition comprises a mix of at least 10%, at least 25%, at least 50%, at least 60%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or more modified LPS molecular species as described hereinabove out of the total amount of LPS molecular species in the population.

In one embodiment, the composition comprises a mix of modified LPS molecular species selected from the group comprising or consisting of the LPS molecular species of Formulas (I)(1)(a), (I)(1)(b), (I)(1)(c), (I)(1)(d), (I)(1)(e), (1)(1)(f), (I)(2)(a), (I)(2)(b), (I)(2)(c), (I)(3)(a), (I)(3)(b), (I)(3)(c), (I)(5)(a), (I)(5)(b), (I)(6)(a), (I)(6)(b), (I)(7)(a), (I)(7)(b), (I)(7)(c), (I)(7)(d), (I)(8)(a), (I)(8)(b), (I)(8)(c), (I)(9)(a), (I)(9)(b), (I)(9)(c), (I)(9)(d), (I)(9)(e), (I)(9)(f), (II)(1)(a), (II)(1)(b), (II)(1)(c), (II)(1)(d), (II)(2)(a), (II)(2)(b), (II)(2)(c), (II)(2)(d), (III)(1)(a), (III)(1)(b), (III)(1)(c), (III)(1)(d), (IV)(1)(a), (IV)(1)(b), (IV)(1)(c), (V)(1)(a), (V)(1)(b), (V)(1)(c), (V)(1)(d), (V)(1)(e), (V)(1)(f), (V)(1)(g), (V)(1)(h), (V)(1)(i), (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), (VI)(2)(a), (VI)(2)(b), (VI)(2)(c), (VII)(1)(a), (VII)(1)(b), (VII)(1)(c), (VIII)(1)(a), (VIII)(1)(b), (VIII)(1)(c), (VIII)(1)(d), (IX)(1)(a), (IX)(1)(b), (IX)(1)(c), (IX)(1)(d), (X)(1)(a), (X)(1)(b), (X)(1)(c), (X)(1)(d), (XI)(1)(a), (XI)(1)(b), (XI)(1)(c), (XI)(1)(d), (XII)(1), (XIII)(2)(a), (XIII)(2)(b), (XIII)(3)(a), (XIII)(3)(b), (XIII)(4)(a), (XIII)(4)(b), (XIII)(5)(a), (XIII)(5)(b), (XIII)(5)(c), (XIII)(5)(d), (XIII)(6)(a), (XIII)(6)(b), (XIII)(6)(c), (XIII)(6)(d), (XIII)(6)(e), (XIII)(6)(f), (XIII)(7)(a), (XIII)(7)(b), (XIII)(7)(c), (XIII)(7)(d), (XIII)(7)(e), (XIII)(7)(f), (XIII)(8)(a), (XIII)(8)(b), (XIII)(8)(c), (XIII)(8)(d), (XIII)(8)(e), (XIII)(8)(f), (XIII)(9)(a), (XIII)(9)(b), (XIII)(9)(c), (XIII)(9)(d), (XIII)(9)(e), (XIII)(9)(f), (XIII)(9)(g), (XIII)(9)(h), (XIII)(9)(i), (XΠI)(9)(j), and a combination thereof.

In one embodiment, the composition comprises a mix of modified LPS molecular species selected from the group comprising or consisting of the LPS molecular species of Formulas (I)(1)(a), (I)(1)(b), (I)(1)(c), (I)(1)(d), (I)(1)(e), (1)(1)(f), (I)(2)(a), (I)(2)(b), (I)(2)(c), (I)(3)(a), (I)(3)(b), (I)(3)(c), (I)(5)(a), (I)(5)(b), (I)(6)(a), (I)(6)(b), (I)(7)(a), (I)(7)(b), (I)(7)(c), (I)(7)(d), (I)(8)(a), (I)(8)(b), (I)(8)(c), (I)(9)(a), (I)(9)(b), (I)(9)(c), (I)(9)(d), (I)(9)(e), (I)(9)(f), (II)(2)(a), (II)(2)(b), (II)(2)(c), (II)(2)(d), (III)(1)(a), (III)(1)(b), (III)(1)(c), (III)(1)(d), (IV)(1)(a), (IV)(1)(b), (IV)(1)(c), (V)(1)(a), (V)(1)(b), (V)(1)(c), (V)(1)(d), (V)(1)(e), (V)(1)(f), (V)(1)(g), (V)(1)(h), (V)(1)(i), (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), (VI)(2)(a), (VI)(2)(b), (VI)(2)(c), (VII)(1)(a), (VII)(1)(b), (VII)(1)(c), (VIII)(1)(a), (VIII)(1)(b), (VIII)(1)(c), (VIII)(1)(d), (IX)(1)(a), (IX)(1)(b), (IX)(1)(c), (IX)(1)(d), (X)(1)(a), (X)(1)(b), (X)(1)(c), (X)(1)(d), (XI)(1)(a), (XI)(1)(b), (XI)(1)(c), (XI)(1)(d), (XIII)(2)(a), (XIII)(2)(b), (XIII)(3)(a), (XIII)(3)(b), (XIII)(4)(a), (XIII)(4)(b), (XIII)(5)(a), (XIII)(5)(b), (XIII)(5)(c), (XIII)(5)(d), (XIII)(6)(a), (XIII)(6)(b), (XIII)(6)(c), (XIII)(6)(d), (XIII)(6)(e), (XIII)(6)(f), (XIII)(7)(a), (XIII)(7)(b), (XIII)(7)(c), (XIII)(7)(d), (XIII)(7)(e), (XIII)(7)(f), (XIII)(8)(a), (XIII)(8)(b), (XIII)(8)(c), (XIII)(8)(d), (XIII)(8)(e), (XIII)(8)(f), (XIII)(9)(a), (XIII)(9)(b), (XIII)(9)(c), (XIII)(9)(d), (XIII)(9)(e), (XIII)(9)(f), (XIII)(9)(g), (XIII)(9)(h), (XIII)(9)(i), (XIΠ)(9)(j), and a combination

thereof.

In one embodiment, the composition comprises a mix of at least two, three, four, five, six, seven, eight, nine, ten, eleven, twelve, thirteen, fourteen, fifteen, sixteen seventeen, eighteen, nineteen, or twenty or more modified LPS molecular species selected from the group comprising or consisting of the LPS molecular species of Formulas (I)(1)(a), (I)(1)(b), (I)(1)(c), (I)(1)(d), (I)(1)(e), (I)(1)(f), (I)(2)(a), (I)(2)(b), (I)(2)(c), (I)(3)(a), (I)(3)(b), (I)(3)(c), (I)(5)(a), (I)(5)(b), (f)(6)(a), (I)(6)(b), (f)(7)(a), (I)(7)(b), (I)(7)(c), (I)(7)(d), (I)(8)(a), (I)(8)(b), (I)(8)(c), (I)(9)(a), (I)(9)(b), (I)(9)(c), (I)(9)(d), (I)(9)(e), (I)(9)(f), (II)(1)(a), (II)(1)(b), (II)(1)(c), (II)(1)(d), (II)(2)(a), (II)(2)(b), (II)(2)(c), (II)(2)(d), (III)(1)(a), (III)(1)(b), (III)(1)(c), (III)(1)(d), (IV)(1)(a), (IV)(1)(b), (IV)(1)(c), (V)(1)(a), (V)(1)(b), (V)(1)(c), (V)(1)(d), (V)(1)(e), (V)(1)(f), (V)(1)(g), (V)(1)(h), (V)(1)(i), (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), (VI)(2)(a), (VI)(2)(b), (VI)(2)(c), (VII)(1)(a), (VII)(1)(b), (VII)(1)(c), (VIII)(1)(a), (VIII)(1)(b), (VIII)(1)(c), (VIII)(1)(d), (IX)(1)(a), (IX)(1)(b), (IX)(1)(c), (IX)(1)(d), (X)(1)(a), (X)(1)(b), (X)(1)(c), (X)(1)(d), (XI)(1)(a), (XI)(1)(b), (XI)(1)(c), (XI)(1)(d), (XII)(1), (XIII)(2)(a), (XIII)(2)(b), (XIII)(3)(a), (XIII)(3)(b), (XIII)(4)(a), (XIII)(4)(b), (XIII)(5)(a), (XIII)(5)(b), (XIII)(5)(c), (XIII)(5)(d), (XIII)(6)(a), (XIII)(6)(b), (XIII)(6)(c), (XIII)(6)(d), (XIII)(6)(e), (XIII)(6)(f), (XIII)(7)(a), (XIII)(7)(b), (XIII)(7)(c), (XIII)(7)(d), (XIII)(7)(e), (XIII)(7)(f), (XIII)(8)(a), (XIII)(8)(b), (XIII)(8)(c), (XIII)(8)(d), (XIII)(8)(e), (XIII)(8)(f), (XIII)(9)(a), (XIII)(9)(b), (XIII)(9)(c), (XIII)(9)(d), (XIII)(9)(e), (XIII)(9)(f), (XIII)(9)(g), (XIII)(9)(h), (XIII)(9)(i), (XΠI)(9)(j), and a combination thereof.

In one embodiment, the composition comprises a mix of at least two, three, four, five, six, seven, eight, nine, ten, eleven, twelve, thirteen, fourteen, fifteen, sixteen seventeen, eighteen, nineteen, or twenty or more modified LPS molecular species selected from the group comprising or consisting of the LPS molecular species of Formulas (I)(1)(a), (I)(1)(b), (I)(1)(c), (I)(1)(d), (I)(1)(e), (1)(1)(f), (I)(2)(a), (I)(2)(b), (I)(2)(c), (I)(3)(a), (I)(3)(b), (I)(3)(c), (I)(5)(a), (I)(5)(b), (I)(6)(a), (I)(6)(b), (I)(7)(a), (I)(7)(b), (I)(7)(c), (I)(7)(d), (I)(8)(a), (I)(8)(b), (I)(8)(c), (I)(9)(a), (I)(9)(b), (I)(9)(c), (I)(9)(d), (I)(9)(e), (I)(9)(f), (II)(2)(a), (II)(2)(b), (II)(2)(c), (II)(2)(d), (III)(1)(a), (III)(1)(b), (III)(1)(c), (III)(1)(d), (IV)(1)(a), (IV)(1)(b), (IV)(1)(c), (V)(1)(a), (V)(1)(b), (V)(1)(c), (V)(1)(d), (V)(1)(e), (V)(1)(f), (V)(1)(g), (V)(1)(h), (V)(1)(i), (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), (VI)(2)(a), (VI)(2)(b), (VI)(2)(c), (VII)(1)(a), (VII)(1)(b), (VII)(1)(c), (VIII)(1)(a), (VIII)(1)(b), (VIII)(1)(c), (VIII)(1)(d), (IX)(1)(a), (IX)(1)(b), (IX)(1)(c), (IX)(1)(d), (X)(1)(a), (X)(1)(b), (X)(1)(c), (X)(1)(d), (XI)(1)(a), (XI)(1)(b), (XI)(1)(c), (XI)(1)(d), (XIII)(2)(a), (XIII)(2)(b), (XIII)(3)(a), (XIII)(3)(b), (XIII)(4)(a), (XIII)(4)(b), (XIII)(5)(a), (XIII)(5)(b), (XIII)(5)(c), (XIII)(5)(d), (XIII)(6)(a), (XIII)(6)(b), (XIII)(6)(c), (XIII)(6)(d), (XIII)(6)(e), (XIII)(6)(f), (XIII)(7)(a), (XIII)(7)(b), (XIII)(7)(c), (XIII)(7)(d), (XIII)(7)(e), (XIII)(7)(f), (XIII)(8)(a), (XIII)(8)(b), (XIII)(8)(c), (XIII)(8)(d), (XIII)(8)(e), (XIII)(8)(f), (XIII)(9)(a), (XIII)(9)(b), (XIII)(9)(c), (XIII)(9)(d), (XIII)(9)(e), (XIII)(9)(f), (XIII)(9)(g), (XIII)(9)(h), (XIII)(9)(i), (XIΠ)(9)(j), and a combination thereof.

In one embodiment, the composition comprises a mix of modified LPS molecular species selected from the group comprising or consisting of the LPS molecular species of Formulas (I)(1)(a), (I)(1)(b), (I)(1)(c), (I)(1)(d), (I)(1)(e), (1)(1)(f), (I)(2)(a), (I)(2)(b), (I)(2)(c), (I)(3)(a), (I)(3)(b), (I)(3)(c), (II)(1)(a), (II)(1)(b), (II)(1)(c), (II)(1)(d), (II)(2)(a), (II)(2)(b), (II)(2)(c), (II)(2)(d), (III)(1)(a), (III)(1)(b), (III)(1)(c), (III)(1)(d), (IV)(1)(a), (IV)(1)(b), (IV)(1)(c), (V)(1)(a), (V)(1)(b), (V)(1)(c), (V)(1)(d), (V)(1)(e), (V)(1)(f), (V)(1)(g), (V)(1)(h), (V)(1)(i), (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), (VI)(2)(a), (VI)(2)(b), (VI)(2)(c), (VII)(1)(a), (VII)(1)(b), (VII)(1)(c), (VIII)(1)(a), (VIII)(1)(b), (VIII)(1)(c), (VIII)(1)(d), (IX)(1)(a), (IX)(1)(b), (IX)(1)(c), (IX)(1)(d), (X)(1)(a), (X)(1)(b), (X)(1)(c), (X)(1)(d), (XI)(1)(a), (XI)(1)(b), (XI)(1)(c), (XI)(1)(d), (XII)(1), and a combination thereof.

In one embodiment, the composition comprises a mix of modified LPS molecular species selected from the group comprising or consisting of the LPS molecular species of Formulas (I)(1)(a), (I)(1)(b), (I)(1)(c), (I)(1)(d), (I)(1)(e), (I)(1)(f), (I)(2)(a), (I)(2)(b), (I)(2)(c), (I)(3)(a), (I)(3)(b), (I)(3)(c), (II)(2)(a), (II)(2)(b), (II)(2)(c), (II)(2)(d), (III)(1)(a), (III)(1)(b), (III)(1)(c), (III)(1)(d), (IV)(1)(a), (IV)(1)(b), (IV)(1)(c), (V)(1)(a), (V)(1)(b), (V)(1)(c), (V)(1)(d), (V)(1)(e), (V)(1)(f), (V)(1)(g), (V)(1)(h), (V)(1)(i), (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), (VI)(2)(a), (VI)(2)(b), (VI)(2)(c), (VII)(1)(a), (VII)(1)(b), (VII)(1)(c), (VIII)(1)(a), (VIII)(1)(b), (VIII)(1)(c), (VIII)(1)(d), (IX)(1)(a), (IX)(1)(b), (IX)(1)(c), (IX)(1)(d), (X)(1)(a), (X)(1)(b), (X)(1)(c), (X)(1)(d), (XI)(1)(a), (XI)(1)(b), (XI)(1)(c), (XI)(1)(d), and a combination thereof.

In one embodiment, the composition comprises a mix of at least two, three, four, five, six, seven, eight, nine, ten, eleven, twelve, thirteen, fourteen, fifteen, sixteen seventeen, eighteen, nineteen, or twenty or more modified LPS molecular species selected from the group comprising or consisting of the LPS molecular species of Formulas (I)(1)(a), (I)(1)(b), (I)(1)(c), (I)(1)(d), (I)(1)(e), (I)(1)(f), (I)(2)(a), (I)(2)(b), (I)(2)(c), (I)(3)(a), (I)(3)(b), (I)(3)(c), (II)(1)(a), (II)(1)(b), (II)(1)(c), (II)(1)(d), (II)(2)(a), (II)(2)(b), (II)(2)(c), (II)(2)(d), (III)(1)(a), (III)(1)(b), (III)(1)(c), (III)(1)(d), (IV)(1)(a), (IV)(1)(b), (IV)(1)(c), (V)(1)(a), (V)(1)(b), (V)(1)(c), (V)(1)(d), (V)(1)(e), (V)(1)(f), (V)(1)(g), (V)(1)(h), (V)(1)(i), (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), (VI)(2)(a), (VI)(2)(b), (VI)(2)(c), (VII)(1)(a), (VII)(1)(b), (VII)(1)(c), (VIII)(1)(a), (VIII)(1)(b), (VIII)(1)(c), (VIII)(1)(d), (IX)(1)(a), (IX)(1)(b), (IX)(1)(c), (IX)(1)(d), (X)(1)(a), (X)(1)(b), (X)(1)(c), (X)(1)(d), (XI)(1)(a), (XI)(1)(b), (XI)(1)(c), (XI)(1)(d), (XII)(1), and a combination thereof.

In one embodiment, the composition comprises a mix of at least two, three, four, five, six, seven, eight, nine, ten, eleven, twelve, thirteen, fourteen, fifteen, sixteen seventeen, eighteen, nineteen, or twenty or more modified LPS molecular species selected from the group comprising or consisting of the LPS molecular species of Formulas (I)(1)(a), (I)(1)(b), (I)(1)(c), (I)(1)(d), (I)(1)(e), (I)(1)(f), (I)(2)(a), (I)(2)(b), (I)(2)(c), (I)(3)(a), (I)(3)(b), (I)(3)(c), (II)(2)(a), (II)(2)(b),

(II)(2)(c), (II)(2)(d), (III)(1)(a), (III)(1)(b), (III)(1)(c), (III)(1)(d), (IV)(1)(a), (IV)(1)(b), (IV)(1)(c), (V)(1)(a), (V)(1)(b), (V)(1)(c), (V)(1)(d), (V)(1)(e), (V)(1)(f), (V)(1)(g), (V)(1)(h), (V)(1)(i), (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), (VI)(2)(a), (VI)(2)(b), (VI)(2)(c), (VII)(1)(a), (VII)(1)(b), (VII)(1)(c), (VIII)(1)(a), (VIII)(1)(b), (VIII)(1)(c), (VIII)(1)(d), (IX)(1)(a), (IX)(1)(b), (IX)(1)(c), (IX)(1)(d), (X)(1)(a), (X)(1)(b), (X)(1)(c), (X)(1)(d), (XI)(1)(a), (XI)(1)(b), (XI)(1)(c), (XI)(1)(d), and a combination thereof.

In one embodiment, the composition comprises a mix of modified LPS molecular species selected from the group comprising or consisting of the LPS molecular species of Formulas (I)(1)(a), (I)(1)(b), (I)(1)(c), (I)(1)(d), (I)(1)(e), (I)(1)(f), (I)(2)(a), (I)(2)(b), (I)(2)(c), (I)(3)(a), (I)(3)(b), (I)(3)(c), (I)(5)(a), (I)(5)(b), (I)(6)(a), (I)(6)(b), (I)(7)(a), (I)(7)(b), (I)(7)(c), (I)(7)(d), (I)(8)(a), (I)(8)(b), (I)(8)(c), (I)(9)(a), (I)(9)(b), (I)(9)(c), (I)(9)(d), (I)(9)(e), (I)(9)(f), and a combination thereof.

In one embodiment, the composition comprises a mix of at least two, three, four, five, six, seven, eight, nine, ten, eleven, twelve, thirteen, fourteen, fifteen, sixteen, seventeen, eighteen, nineteen, twenty, twenty-one, twenty-two, twenty-three, twenty-four, twenty-five, twenty-six, twenty-seven, twenty-eight, or twenty-nine modified LPS molecular species selected from the group comprising or consisting of the LPS molecular species of Formulas (I)(1)(a), (I)(1)(b), (I)(1)(c), (I)(1)(d), (I)(1)(e), (I)(1)(f), (I)(2)(a), (I)(2)(b), (I)(2)(c), (I)(3)(a), (I)(3)(b), (I)(3)(c), (I)(5)(a), (I)(5)(b), (I)(6)(a), (I)(6)(b), (I)(7)(a), (I)(7)(b), (I)(7)(c), (I)(7)(d), (I)(8)(a), (I)(8)(b), (I)(8)(c), (I)(9)(a), (I)(9)(b), (I)(9)(c), (I)(9)(d), (I)(9)(e), (I)(9)(f), and a combination thereof.

In one embodiment, the composition comprises a mix of modified LPS molecular species selected from the group comprising or consisting of the LPS molecular species of Formulas (I)(1)(a), (I)(1)(b), (I)(1)(c), (I)(1)(d), (I)(1)(e), (I)(1)(f), (I)(2)(a), (I)(2)(b), (I)(2)(c), (I)(3)(a), (I)(3)(b), (I)(3)(c), and a combination thereof.

In one embodiment, the composition comprises a mix of at least two, three, four, five, six, seven, eight, nine, ten, eleven, or twelve modified LPS molecular species selected from the group comprising or consisting of the LPS molecular species of Formulas (I)(1)(a), (I)(1)(b), (I)(1)(c), (I)(1)(d), (I)(1)(e), (I)(1)(f), (I)(2)(a), (I)(2)(b), (I)(2)(c), (I)(3)(a), (I)(3)(b), (I)(3)(c), and a combination thereof.

In one embodiment, the composition comprises a mix of modified LPS molecular species selected from the group comprising or consisting of the LPS molecular species of Formulas (I)(1)(a), (I)(1)(b), (I)(1)(c), (I)(1)(d), (I)(1)(e), (I)(1)(f), and a combination thereof.

In one embodiment, the composition comprises a mix of at least two, three, four, five, or six modified LPS molecular species selected from the group comprising or consisting of the LPS molecular species of Formulas (I)(1)(a), (I)(1)(b), (I)(1)(c), (I)(1)(d), (I)(1)(e), (I)(1)(f), and a combination thereof.

In one embodiment, the composition comprises a mix of modified LPS molecular species comprising or consisting of at least one LPS molecular species of Formula (I)(1)(a), at least one LPS molecular species of Formula (I)(1)(b), at least one LPS molecular species of Formula (I)(1)(c), at least one LPS molecular species of Formula (I)(1)(d), at least one LPS molecular species of Formula (I)(1)(e), and at least one LPS molecular species of Formula (1)(1)(f).

In one embodiment, the composition comprises a mix of modified LPS molecular species selected from the group comprising or consisting of the LPS molecular species of Formulas (I)(2)(a), (I)(2)(b), (I)(2)(c), and a combination thereof.

In one embodiment, the composition comprises a mix of at least two, or three modified LPS molecular species selected from the group comprising or consisting of the LPS molecular species of Formulas (I)(2)(a), (I)(2)(b), (I)(2)(c), and a combination thereof.

In one embodiment, the composition comprises a mix of modified LPS molecular species comprising or consisting of at least one LPS molecular species of Formula (I)(2)(a), at least one LPS molecular species of Formula (I)(2)(b), and at least one LPS molecular species of Formula (I)(2)(c).

In one embodiment, the composition comprises a mix of modified LPS molecular species selected from the group comprising or consisting of the LPS molecular species of Formulas (I)(3)(a), (I)(3)(b), (I)(3)(c), and a combination thereof.

In one embodiment, the composition comprises a mix of at least two, or three modified LPS molecular species selected from the group comprising or consisting of the LPS molecular species of Formulas (I)(3)(a), (I)(3)(b), (I)(3)(c), and a combination thereof.

In one embodiment, the composition comprises a mix of modified LPS molecular species comprising or consisting of at least one LPS molecular species of Formula (I)(3)(a), at least one LPS molecular species of Formula (I)(3)(b), and at least one LPS molecular species of Formula (I)(3)(c).

In one embodiment, the composition comprises a mix of modified LPS molecular species selected from the group comprising or consisting of the LPS molecular species of Formulas (I)(5)(a), (I)(5)(b), and a combination thereof.

In one embodiment, the composition comprises a mix of two modified LPS molecular species selected from the group comprising or consisting of the LPS molecular species of Formulas (I)(5)(a), (I)(5)(b), and a combination thereof.

In one embodiment, the composition comprises a mix of modified LPS molecular species comprising or consisting of at least one LPS molecular species of Formula (I)(5)(a), and at least one LPS molecular species of Formula (I)(5)(b).

In one embodiment, the composition comprises a mix of modified LPS molecular species selected from the group comprising or consisting of the LPS molecular species of Formulas (I)(6)(a), (I)(6)(b), and a combination thereof.

In one embodiment, the composition comprises a mix of two modified LPS molecular species selected from the group comprising or consisting of the LPS molecular species of Formulas (I)(6)(a), (I)(6)(b), and a combination thereof.

In one embodiment, the composition comprises a mix of modified LPS molecular species comprising or consisting of at least one LPS molecular species of Formula (I)(6)(a), and at least one LPS molecular species of Formula (I)(6)(b).

In one embodiment, the composition comprises a mix of modified LPS molecular species selected from the group comprising or consisting of the LPS molecular species of Formulas (I)(7)(a), (I)(7)(b), (I)(7)(c), (I)(7)(d), and a combination thereof.

In one embodiment, the composition comprises a mix of at least two, three, or four modified LPS molecular species selected from the group comprising or consisting of the LPS molecular species of Formulas (I)(7)(a), (I)(7)(b), (I)(7)(c), (I)(7)(d), and a combination thereof.

In one embodiment, the composition comprises a mix of modified LPS molecular species comprising or consisting of at least one LPS molecular species of Formula (I)(7)(a), at least one LPS molecular species of Formula (I)(7)(b), at least one LPS molecular species of Formula (I)(7)(c), and at least one LPS molecular species of Formula (I)(7)(d).

In one embodiment, the composition comprises a mix of modified LPS molecular species selected from the group comprising or consisting of the LPS molecular species of Formulas (I)(8)(a), (I)(8)(b), (I)(8)(c), and a combination thereof.

In one embodiment, the composition comprises a mix of at least two, or three modified LPS molecular species selected from the group comprising or consisting of the LPS molecular species of Formulas (I)(8)(a), (I)(8)(b), (I)(8)(c), and a combination thereof.

In one embodiment, the composition comprises a mix of modified LPS molecular species comprising or consisting of at least one LPS molecular species of Formula (I)(8)(a), at least one LPS molecular species of Formula (I)(8)(b), and at least one LPS molecular species of Formula (I)(8)(c).

In one embodiment, the composition comprises a mix of modified LPS molecular species selected from the group comprising or consisting of the LPS molecular species of Formulas (I)(9)(a), (I)(9)(b), (I)(9)(c), (I)(9)(d), (I)(9)(e), (I)(9)(f), and a combination thereof.

In one embodiment, the composition comprises a mix of at least two, three, four, five, or six modified LPS molecular species selected from the group comprising or consisting of the LPS molecular species of Formulas (I)(9)(a), (I)(9)(b), (I)(9)(c), (I)(9)(d), (I)(9)(e), (I)(9)(f), and a combination thereof.

In one embodiment, the composition comprises a mix of modified LPS molecular species comprising or consisting of at least one LPS molecular species of Formula (I)(9)(a), at least one LPS molecular species of Formula (I)(9)(b), at least one LPS molecular species of Formula (I)(9)(c), at least one LPS molecular species of Formula (I)(9)(d), at least one LPS molecular species of Formula (I)(9)(e), and at least one LPS molecular species of Formula (1)(9)(f).

In one embodiment, the composition comprises a mix of modified LPS molecular species selected from the group comprising or consisting of the LPS molecular species of Formulas (II)(1)(a), (II)(1)(b), (II)(1)(c), (II)(1)(d), (II)(2)(a), (II)(2)(b), (II)(2)(c), (II)(2)(d), and a combination thereof.

In one embodiment, the composition comprises a mix of at least two, three, four, five, six, seven, or eight modified LPS molecular species selected from the group comprising or consisting of the LPS molecular species of Formulas (II)(1)(a), (II)(1)(b), (II)(1)(c), (II)(1)(d), (II)(2)(a), (II)(2)(b), (II)(2)(c), (II)(2)(d), and a combination thereof.

In one embodiment, the composition comprises a mix of modified LPS molecular species selected from the group comprising or consisting of the LPS molecular species of Formulas (II)(1)(a), (II)(1)(b), (II)(1)(c), (II)(1)(d), and a combination thereof.

In one embodiment, the composition comprises a mix of at least two, three, or four modified LPS molecular species selected from the group comprising or consisting of the LPS molecular species of Formulas (II)(1)(a), (II)(1)(b), (II)(1)(c), (II)(1)(d), and a combination thereof.

In one embodiment, the composition comprises a mix of modified LPS molecular species comprising or consisting of at least one LPS molecular species of Formula (II)(1)(a), at least one LPS molecular species of Formula (II)(1)(b), at least one LPS molecular species of Formula (II)(1)(c), and at least one LPS molecular species of Formula (II)(1)(d).

In one embodiment, the composition comprises a mix of modified LPS molecular species selected from the group comprising or consisting of the LPS molecular species of Formulas (II)(2)(a), (II)(2)(b), (II)(2)(c), (II)(2)(d), and a combination thereof.

In one embodiment, the composition comprises a mix of at least two, three, or four modified LPS molecular species selected from the group comprising or consisting of the LPS molecular species of Formulas (II)(2)(a), (II)(2)(b), (II)(2)(c), (II)(2)(d), and a combination thereof.

In one embodiment, the composition comprises a mix of modified LPS molecular species comprising or consisting of at least one LPS molecular species of Formula (II)(2)(a), at least one LPS molecular species of Formula (II)(2)(b), at least one LPS molecular species of Formula (II)(2)(c), and at least one LPS molecular species of Formula (II)(2)(d).

In one embodiment, the composition comprises a mix of modified LPS molecular species of Formula (XII)(1).

In one embodiment, the composition comprises a mix of modified LPS molecular species selected from the group comprising or consisting of the LPS molecular species of Formulas (III)(1)(a), (III)(1)(b), (III)(1)(c), (III)(1)(d), and a combination thereof.

In one embodiment, the composition comprises a mix of at least two, three, or four modified LPS molecular species selected from the group comprising or consisting of the LPS molecular species of Formulas (III)(1)(a), (III)(1)(b), (III)(1)(c), (III)(1)(d), and a combination thereof.

In one embodiment, the composition comprises a mix of modified LPS molecular species comprising or consisting of at least one LPS molecular species of Formula (III)(1)(a), at least one LPS molecular species of Formula (III)(1)(b), at least one LPS molecular species of Formula (III)(1)(c), and at least one LPS molecular species of Formula (III)(1)(d).

In one embodiment, the composition comprises a mix of modified LPS molecular species selected from the group comprising or consisting of the LPS molecular species of Formula (XII)(1), and a combination thereof.

In one embodiment, the composition comprises a mix of modified LPS molecular species selected from the group comprising or consisting of the LPS molecular species of Formulas (IV)(1)(a), (IV)(1)(b), (IV)(1)(c), and a combination thereof.

In one embodiment, the composition comprises a mix of at least two, or three modified LPS molecular species selected from the group comprising or consisting of the LPS molecular species of Formulas (IV)(1)(a), (IV)(1)(b), (IV)(1)(c), and a combination thereof.

In one embodiment, the composition comprises a mix of modified LPS molecular species comprising or consisting of at least one LPS molecular species of Formula (IV)(1)(a), at least one LPS molecular species of Formula (IV)(1)(b), and at least one LPS molecular species of Formula (IV)(1)(c).

In one embodiment, the composition comprises a mix of modified LPS molecular species selected from the group comprising or consisting of the LPS molecular species of Formulas (V)(1)(a), (V)(1)(b), (V)(1)(c), (V)(1)(d), (V)(1)(e), (V)(1)(f), (V)(1)(g), (V)(1)(h), (V)(1)(i), and a combination thereof.

In one embodiment, the composition comprises a mix of at least two, three, four, five, six, seven, eight, or nine modified LPS molecular species selected from the group comprising or consisting of the LPS molecular species of Formulas (V)(1)(a), (V)(1)(b), (V)(1)(c), (V)(1)(d), (V)(1)(e), (V)(1)(f), (V)(1)(g), (V)(1)(h), (V)(1)(i), and a combination thereof.

In one embodiment, the composition comprises a mix of modified LPS molecular species comprising or consisting of at least one LPS molecular species of Formula (V)(1)(a), at least one LPS molecular species of Formula (V)(1)(b), at least one LPS molecular species of Formula (V)(1)(c), at least one LPS molecular species of Formula (V)(1)(d), at least one LPS molecular species of Formula (V)(1)(e), at least one LPS molecular species of Formula (V)(1)(f), at least one LPS molecular species of Formula (V)(1)(g), at least one LPS molecular species of Formula (V)(1)(h), and at least one LPS molecular species of Formula (V)(1)(i).

In one embodiment, the composition comprises a mix of modified LPS molecular species selected from the group comprising or consisting of the LPS molecular species of Formulas (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), (VI)(2)(a), (VI)(2)(b), (VI)(2)(c), and a combination thereof.

In one embodiment, the composition comprises a mix of at least two, three, four, five, six, seven, or eight modified LPS molecular species selected from the group comprising or consisting of the LPS molecular species of Formulas (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), (VI)(2)(a), (VI)(2)(b), (VI)(2)(c), and a combination thereof.

In one embodiment, the composition comprises a mix of modified LPS molecular species selected from the group comprising or consisting of the LPS molecular species of Formulas (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), and a combination thereof.

In one embodiment, the composition comprises a mix of at least two, three, four, five, six, seven, eight, nine, or ten modified LPS molecular species selected from the group comprising or consisting of the LPS molecular species of Formulas (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), and a combination thereof.

In one embodiment, the composition comprises a mix of modified LPS molecular species comprising or consisting of at least one LPS molecular species of Formula (VI)(1)(a), at least one LPS molecular species of Formula (VI)(1)(b), at least one LPS molecular species of Formula (VI)(1)(c), at least one LPS molecular species of Formula (VI)(1)(d), at least one LPS molecular species of Formula (VI)(1)(e), at least one LPS molecular species of Formula (VI)(1)(f), at least one LPS molecular species of Formula (VI)(1)(g), at least one LPS molecular species of Formula (VI)(1)(h), at least one LPS molecular species of Formula (VI)(1)(i), and at least one LPS molecular species of Formula (VI)(1)(j).

In one embodiment, the composition comprises a mix of modified LPS molecular species selected from the group comprising or consisting of the LPS molecular species of Formulas (VI)(2)(a), (VI)(2)(b), (VI)(2)(c), and a combination

thereof.

In one embodiment, the composition comprises a mix of at least two, or three modified LPS molecular species selected from the group comprising or consisting of the LPS molecular species of Formulas (VI)(2)(a), (VI)(2)(b), (VI)(2)(c), and a combination thereof.

In one embodiment, the composition comprises a mix of modified LPS molecular species comprising or consisting of at least one LPS molecular species of Formula (VI)(2)(a), at least one LPS molecular species of Formula (VI)(2)(b), and at least one LPS molecular species of Formula (VI)(2)(c).

In one embodiment, the composition comprises a mix of modified LPS molecular species selected from the group comprising or consisting of the LPS molecular species of Formulas (VII)(1)(a), (VII)(1)(b), (VII)(1)(c), and a combination thereof.

In one embodiment, the composition comprises a mix of at least two, or three modified LPS molecular species selected from the group comprising or consisting of the LPS molecular species of Formulas (VII)(1)(a), (VII)(1)(b), (VII)(1)(c), and a combination thereof.

In one embodiment, the composition comprises a mix of modified LPS molecular species comprising or consisting of at least one LPS molecular species of Formula (VII)(1)(a), at least one LPS molecular species of Formula (VII)(1)(b), and at least one LPS molecular species of Formula (VII)(1)(c).

In one embodiment, the composition comprises a mix of modified LPS molecular species selected from the group comprising or consisting of the LPS molecular species of Formulas (VIII)(1)(a), (VIII)(1)(b), (VIII)(1)(c), (VIII)(1)(d), and a combination thereof.

In one embodiment, the composition comprises a mix of at least two, three, or four modified LPS molecular species selected from the group comprising or consisting of the LPS molecular species of Formulas (VIII)(1)(a), (VIII)(1)(b), (VIII)(1)(c), (VIII)(1)(d), and a combination thereof.

In one embodiment, the composition comprises a mix of modified LPS molecular species comprising or consisting of at least one LPS molecular species of Formula (VIII)(1)(a), at least one LPS molecular species of Formula (VIII)(1)(b), at least one LPS molecular species of Formula (VIII)(1)(c), and at least one LPS molecular species of Formula (VIII)(1)(d).

In one embodiment, the composition comprises a mix of modified LPS molecular species selected from the group comprising or consisting of the LPS molecular species of Formulas (IX)(1)(a), (IX)(1)(b), (IX)(1)(c), (IX)(1)(d), and a combination thereof.

In one embodiment, the composition comprises a mix of at least two, three, or four modified LPS molecular species selected from the group comprising or consisting of the LPS molecular species of Formulas (IX)(1)(a), (IX)(1)(b), (IX)(1)(c), (IX)(1)(d), and a combination thereof.

In one embodiment, the composition comprises a mix of modified LPS molecular species comprising or consisting of at least one LPS molecular species of Formula (IX)(1)(a), at least one LPS molecular species of Formula (IX)(1)(b), at least one LPS molecular species of Formula (IX)(1)(c), and at least one LPS molecular species of Formula (IX)(1)(d).

In one embodiment, the composition comprises a mix of modified LPS molecular species selected from the group comprising or consisting of the LPS molecular species of Formulas (X)(1)(a), (X)(1)(b), (X)(1)(c), (X)(1)(d), and a combination thereof.

In one embodiment, the composition comprises a mix of at least two, three, or four modified LPS molecular species selected from the group comprising or consisting of the LPS molecular species of Formulas (X)(1)(a), (X)(1)(b), (X)(1)(c), (X)(1)(d), and a combination thereof.

In one embodiment, the composition comprises a mix of modified LPS molecular species comprising or consisting of at least one LPS molecular species of Formula (X)(1)(a), at least one LPS molecular species of Formula (X)(1)(b), at least one LPS molecular species of Formula (X)(1)(c), and at least one LPS molecular species of Formula (X)(1)(d).

In one embodiment, the composition comprises a mix of modified LPS molecular species selected from the group comprising or consisting of the LPS molecular species of Formulas (XI)(1)(a), (XI)(1)(b), (XI)(1)(c), (XI)(1)(d), and a combination thereof.

In one embodiment, the composition comprises a mix of at least two, three, or four modified LPS molecular species selected from the group comprising or consisting of the LPS molecular species of Formulas (XI)(1)(a), (XI)(1)(b), (XI)(1)(c), (XI)(1)(d), and a combination thereof.

In one embodiment, the composition comprises a mix of modified LPS molecular species comprising or consisting of at least one LPS molecular species of Formula (XI)(1)(a), at least one LPS molecular species of Formula (XI)(1)(b), at least one LPS molecular species of Formula (XI)(1)(c), and at least one LPS molecular species of Formula (XI)(1)(d).

In one embodiment, the composition comprises a mix of modified LPS molecular species selected from the group comprising or consisting of the LPS molecular species of Formulas (XIII)(2)(a), (XIII)(2)(b), (XIII)(3)(a), (XIII)(3)(b), (XIII)(4)(a), (XIII)(4)(b), (XIII)(5)(a), (XIII)(5)(b), (XIII)(5)(c), (XIII)(5)(d), (XIII)(6)(a), (XIII)(6)(b), (XIII)(6)(c), (XIII)(6)(d), (XIII)(6)(e), (XIII)(6)(f), (XIII)(7)(a), (XIII)(7)(b), (XIII)(7)(c), (XIII)(7)(d), (XIII)(7)(e), (XIII)(7)(f), (XIII)(8)(a), (XIII)(8)(b), (XIII)(8)(c), (XIII)(8)(d), (XIII)(8)(e), (XIII)(8)(f), (XIII)(9)(a), (XIII)(9)(b), (XIII)(9)(c), (XIII)(9)(d), (XIII)(9)(e), (XIII)(9)(f), (XIII)(9)(g), (XIII)(9)(h), (XIII)(9)(i), (XIΠ)(9)(j), and a combination thereof.

In one embodiment, the composition comprises a mix of at least two, three, four, five, six, seven, eight, nine, ten, eleven, twelve, thirteen, fourteen, fifteen, sixteen, seventeen, eighteen, nineteen, twenty, twenty-one, twenty-two, twenty-three, twenty-four, twenty-five, twenty-six, twenty-seven, twenty-eight, twenty-nine, thirty, thirty-one, thirty-two, thirty-three, thirty-four, thirty-five, thirty-five, thirty-six, thirty-seven, or thirty-eight, modified LPS molecular species selected from the group comprising or consisting of the LPS molecular species of Formulas (XIII)(2)(a), (XIII)(2)(b), (XIII)(3)(a), (XIII)(3)(b), (XIII)(4)(a), (XIII)(4)(b),(XIII)(5)(a), (XIII)(5)(b), (XIII)(5)(c), (XIII)(5)(d), (XIII)(6)(a), (XIII)(6)(b), (XIII)(6)(c), (XIII)(6)(d), (XIII)(6)(e), (XIII)(6)(f), (XIII)(7)(a), (XIII)(7)(b), (XIII)(7)(c), (XIII)(7)(d), (XIII)(7)(e), (XIII)(7)(f), (XIII)(8)(a), (XIII)(8)(b), (XIII)(8)(c), (XIII)(8)(d), (XIII)(8)(e), (XIII)(8)(f), (XIII)(9)(a), (XIII)(9)(b), (XIII)(9)(c), (XIII)(9)(d), (XIII)(9)(e), (XIII)(9)(f), (XIII)(9)(g), (XIII)(9)(h), (XIII)(9)(i), (XΠI)(9)(j), and a combination thereof.

In one embodiment, the composition comprises a mix of modified LPS molecular species selected from the group comprising or consisting of the LPS molecular species of Formulas (XIII)(2)(a), (XIII)(2)(b), and a combination thereof.

In one embodiment, the composition comprises a mix of two modified LPS molecular species selected from the group comprising or consisting of the LPS molecular species of Formulas (XIII)(2)(a), (XIII)(2)(b), and a combination thereof.

In one embodiment, the composition comprises a mix of modified LPS molecular species comprising or consisting of at least one LPS molecular species of Formula (XIII)(2)(a), and at least one LPS molecular species of Formula (XIII)(2)(b).

In one embodiment, the composition comprises a mix of modified LPS molecular species selected from the group comprising or consisting of the LPS molecular species of Formulas (XIII)(3)(a), (XIII)(3)(b), and a combination thereof.

In one embodiment, the composition comprises a mix of two modified LPS molecular species selected from the group comprising or consisting of the LPS molecular species of Formulas (XIII)(3)(a), (XIII)(3)(b), and a combination thereof.

In one embodiment, the composition comprises a mix of modified LPS molecular species comprising or consisting of at least one LPS molecular species of Formula (XIII)(3)(a), and at least one LPS molecular species of Formula (XIII)(3)(b).

In one embodiment, the composition comprises a mix of modified LPS molecular species selected from the group comprising or consisting of the LPS molecular species of Formulas (XIII)(4)(a), (XIII)(4)(b), and a combination thereof.

In one embodiment, the composition comprises a mix of two modified LPS molecular species selected from the group comprising or consisting of the LPS molecular species of Formulas (XIII)(4)(a), (XIII)(4)(b), and a combination thereof.

In one embodiment, the composition comprises a mix of modified LPS molecular species comprising or consisting of at least one LPS molecular species of Formula (XIII)(4)(a), and at least one LPS molecular species of Formula (XIII)(4)(b).

In one embodiment, the composition comprises a mix of modified LPS molecular species selected from the group comprising or consisting of the LPS molecular species of Formulas (XIII)(5)(a), (XIII)(5)(b), (XIII)(5)(c), (XIII)(5)(d), and a combination thereof.

In one embodiment, the composition comprises a mix of two modified LPS molecular species selected from the group comprising or consisting of the LPS molecular species of Formulas (XIII)(5)(a), (XIII)(5)(b), (XIII)(5)(c), (XIII)(5)(d), and a combination thereof.

In one embodiment, the composition comprises a mix of modified LPS molecular species comprising or consisting of at least one LPS molecular species of Formula (XIII)(5)(a), at least one LPS molecular species of Formula (XIII)(5)(b), at least one LPS molecular species of Formula (XIII)(5)(c), and at least one LPS molecular species of Formula (XIII)(5)(d).

In one embodiment, the composition comprises a mix of modified LPS molecular species selected from the group comprising or consisting of the LPS molecular species of Formulas (XIII)(6)(a), (XIII)(6)(b), (XIII)(6)(c), (XIII)(6)(d), (XIII)(6)(e), (XIII)(6)(f), and a combination thereof.

In one embodiment, the composition comprises a mix of two modified LPS molecular species selected from the group comprising or consisting of the LPS molecular species of Formulas (XIII)(6)(a), (XIII)(6)(b), (XIII)(6)(c), (XIII)(6)(d), (XIII)(6)(e), (XIII)(6)(f), and a combination thereof.

In one embodiment, the composition comprises a mix of modified LPS molecular species comprising or consisting of at least one LPS molecular species of Formula (XIII)(6)(a), at least one LPS molecular species of Formula (XIII)(6)(b), at least one LPS molecular species of Formula (XIII)(6)(c), at least one LPS molecular species of Formula (XIII)(6)(d), at least one LPS molecular species of Formula (XIII)(6)(e), and at least one LPS molecular species of Formula (XIII)(6)(f).

In one embodiment, the composition comprises a mix of modified LPS molecular species selected from the group comprising or consisting of the LPS molecular species of Formulas (XIII)(7)(a), (XIII)(7)(b), (XIII)(7)(c), (XIII)(7)(d), (XIII)(7)(e), (XIII)(7)(f), and a combination thereof.

In one embodiment, the composition comprises a mix of two modified LPS molecular species selected from the group comprising or consisting of the LPS molecular species of Formulas (XIII)(7)(a), (XIII)(7)(b), (XIII)(7)(c), (XIII)(7)(d), (XIII)(7)(e), (XIII)(7)(f), and a combination thereof.

In one embodiment, the composition comprises a mix of modified LPS molecular species comprising or consisting of at least one LPS molecular species of Formula (XIII)(7)(a), at least one LPS molecular species of Formula (XIII)(7)(b), at least one LPS molecular species of Formula (XIII)(7)(c), at least one LPS molecular species of Formula (XIII)(7)(d), at least one LPS molecular species of Formula (XIII)(7)(e), and at least one LPS molecular species of Formula (XIII)(7)(f).

In one embodiment, the composition comprises a mix of modified LPS molecular species selected from the group comprising or consisting of the LPS molecular species of Formulas (XIII)(8)(a), (XIII)(8)(b), (XIII)(8)(c), (XIII)(8)(d), (XIII)(8)(e), (XIII)(8)(f), and a combination thereof.

In one embodiment, the composition comprises a mix of two modified LPS molecular species selected from the group comprising or consisting of the LPS molecular species of Formulas (XIII)(8)(a), (XIII)(8)(b), (XIII)(8)(c), (XIII)(8)(d), (XIII)(8)(e), (XIII)(8)(f), and a combination thereof.

In one embodiment, the composition comprises a mix of modified LPS molecular species comprising or consisting of at least one LPS molecular species of Formula (XIII)(8)(a), at least one LPS molecular species of Formula (XIII)(8)(b), at least one LPS molecular species of Formula (XIII)(8)(c), at least one LPS molecular species of Formula (XIII)(8)(d), at least one LPS molecular species of Formula (XIII)(8)(e), and at least one LPS molecular species of Formula (XIII)(8)(f).

In one embodiment, the composition comprises a mix of modified LPS molecular species selected from the group comprising or consisting of the LPS molecular species of Formulas (XIII)(9)(a), (XIII)(9)(b), (XIII)(9)(c), (XIII)(9)(d), (XIII)(9)(e), (XIII)(9)(f), (XIII)(9)(g), (XIII)(9)(h), (XIII)(9)(i), (XΠI)(9)(j), and a combination thereof.

In one embodiment, the composition comprises a mix of two modified LPS molecular species selected from the group comprising or consisting of the LPS molecular species of Formulas (XIII)(9)(a), (XIII)(9)(b), (XIII)(9)(c), (XIII)(9)(d), (XIII)(9)(e), (XIII)(9)(f), (XIII)(9)(g), (XIII)(9)(h), (XIII)(9)(i), (XΠI)(9)(j), and a combination thereof.

In one embodiment, the composition comprises a mix of modified LPS molecular species comprising or consisting of at least one LPS molecular species of Formula (XIII)(9)(a), at least one LPS molecular species of Formula (XIII)(9)(b), at least one LPS molecular species of Formula (XIII)(9)(c), at least one LPS molecular species of Formula (XIII)(9)(d), at least one LPS molecular species of Formula (XIII)(9)(e), at least one LPS molecular species of Formula (XIII)(9)(f), at least one LPS molecular species of Formula (XIII)(9)(g), at least one LPS molecular species of Formula (XIII)(9)(h), at least one LPS molecular species of Formula (XIII)(9)(i), and at least one LPS molecular species of Formula (XIII)(9)(j).

In one embodiment, the at least one LPS molecular species is a naturally-occurring LPS molecular species. In one embodiment, the naturally-occurring LPS molecular species is non-toxic.

In one embodiment, the naturally-occurring LPS molecular species have an endotoxin activity below 5000 endotoxin equivalent unit (EEU)/$\mu$g, preferably below 4000, 3000, 2000, 1500, 1000 EEU/pg; as can be assessed by, *e.g.*, monocyte activation test (MAT) based on peripheral blood mononuclear cells (PBMC) and IL-6 response.

Bacteria were present on earth long before human beings, therefore the later have coevolved with bacteria being present mostly in the intestinal microbiome, as well as on the skin and all body surfaces. *Escherichia coli* has been living in symbiosis with human beings, providing the host with vitamins and helping digestion. It is therefore not surprising that the TLR4 LPS receptor present at the surface of different human cells recognizes exactly the structure of the *Escherichia coli* LPS molecular species like a glove would do for a hand. Consequently, LPS molecular species differentiating from the classical (i.e., non-modified) *Escherichia coli* LPS molecules, like by a smaller or greater number of fatty acid chains, and/or fatty acid chains of shorter and/or longer length. Differentiation can also be at the level of the phosphate groups at position C1 of the reducing end and/or position C4' of the non-reducing end of their lipid A domain, either absents or substituted for neutralization of the negative charge.

In this context, some LPS molecular species following such differentiations will not be recognized by the receptor as well as classical *Escherichia coli* LPS molecules, and will therefore not induce a cytokine release leading to high inflammation.

Naturally-occurring LPS molecular species which are non-toxic may include, but are not limited to, LPS molecular species which:

i. comprise at most one, such as one or zero, unsubstituted phosphate group at position C1 of the reducing end and/or position C4' of the non-reducing end of their lipid A domain; and/or

ii. comprise a smaller or greater number of fatty acid chains as compared to toxic LPS molecular species, such as, to *Escherichia coli* or *Salmonella enterica* LPS molecules - which comprise six fatty acid chains among which four are

primary fatty acid chains and two are secondary fatty acid chains; and/or

iii. comprise fatty acid chains of shorter and/or longer length as compared to toxic LPS molecular species, such as, to *Escherichia coli* or *Salmonella enterica* LPS molecules - which comprise four primary 3-OH-$C_{14}$ fatty acid chains, one secondary $C_{14}$ fatty acid chain and one secondary $C_{12}$ fatty acid chain.

In one embodiment, the naturally-occurring LPS molecular species which are non-toxic do not comprise two secondary $C_{12}$ and/or $C_{14}$ fatty acid chains simultaneously present at positions C2' and C3' of their lipid A domain. In one embodiment, the naturally-occurring LPS molecular species which are non-toxic do not comprise two secondary $C_{12}$ fatty acid chains simultaneously present at positions C2' and C3' of their lipid A domain. In one embodiment, the naturally-occurring LPS molecular species which are non-toxic do not comprise two secondary $C_{14}$ fatty acid chains simultaneously present at positions C2' and C3' of their lipid A domain. In one embodiment, the naturally-occurring LPS molecular species which are non-toxic do not comprise one secondary $C_{12}$ fatty acid chain and one secondary $C_{14}$ fatty acid chain simultaneously present at positions C2' and C3' of their lipid A domain.

In one embodiment, the naturally-occurring LPS molecular species which are non-toxic do not comprise four primary 3-OH-$C_{14}$ fatty acid chains, nor two secondary $C_{12}$ and/or $C_{14}$ fatty acid chains simultaneously present at positions C2' and C3' of their lipid A domain. In one embodiment, the naturally-occurring LPS molecular species which are non-toxic do not comprise four primary 3-OH-$C_{14}$ fatty acid chains, nor two secondary $C_{12}$ fatty acid chains simultaneously present at positions C2' and C3' of their lipid A domain. In one embodiment, the naturally-occurring LPS molecular species which are non-toxic do not comprise four primary 3-OH-$C_{14}$ fatty acid chains, nor two secondary $C_{14}$ fatty acid chains simultaneously present at positions C2' and C3' of their lipid A domain. In one embodiment, the naturally-occurring LPS molecular species which are non-toxic do not comprise four primary 3-OH-$C_{14}$ fatty acid chains, nor one secondary $C_{14}$ fatty acid chain and one secondary $C_{12}$ fatty acid chain simultaneously present at positions C2' and C3' of their lipid A domain.

In one embodiment, the naturally-occurring LPS molecular species which are non-toxic do not comprise a phosphate group at either of position C1 of the reducing end of its lipid A domain or position C4' of the non-reducing end of its lipid A domain, or both.

In one embodiment, the naturally-occurring LPS molecular species which are non-toxic comprise a lipid A domain comprising a disaccharide, such as, *e.g.,* a β-1,6-glucosamine [GlcN] disaccharide, a β-1,6-diaminoglucose [DAG] disaccharide or a β-1,6-disaccharide comprising one GlcN residue and one DAG residue.

However, naturally-occurring LPS molecular species which are non-toxic also encompass those LPS molecular species with a lipid A domain comprising only one saccharide, such as, *e.g.,* a glucosamine, an aminoglucose or a di-amino-hexose. These LPS molecular species are also encompassed in the present invention.

Examples of naturally-occurring LPS molecular species which are non-toxic include, but are not limited to LPS molecular species from *Bordetella* (such as, *e.g.,* LPS molecules from *Bordetella pertussis*), LPS molecular species from *Vitreoscilla* (such as, *e.g.,* LPS molecules from *Vitreoscilla filiformis*), LPS molecular species from *Neisseria* (such as, *e.g.,* LPS molecules from *Neisseria meningitidis*), LPS molecular species from *Vibrio* (such as, *e.g.,* LPS molecular species from *Vibrio cholerae*), LPS molecular species from *Helicobacter* (such as, *e.g.,* LPS molecular species from *Helicobacter pylori*), LPS molecular species from *Porphyromonas,* LPS molecular species from Leptospira (such as, *e.g., Leptospira interrogans*), LPS molecular species from *Flavobacterium meningosepticum* (such as, *e.g., Flavobacterium meningosepticum*), LPS molecular species from *Rhodobacter,* LPS molecular species from *Rhizobium,* LPS molecular species from *Bacteroides,* LPS molecular species from *Prevotella.*

In one embodiment, non-toxic LPS molecular species from *Bordetella* comprise four or less $C_{14}$ fatty acid chains and one $C_{10}$ fatty acid chain.

In one embodiment, non-toxic LPS molecular species from *Vitreoscilla* comprise two $C_{12}$ fatty acid chains and four or less $C_{10}$ fatty acid chains.

In one embodiment, non-toxic LPS molecular species from *Neisseria* comprise two $C_{14}$ fatty acid chains and four $C_{12}$ fatty acid chains.

In one embodiment, non-toxic LPS molecular species from *Vibrio* comprise three $C_{14}$ fatty acid chains and three $C_{12}$ fatty acid chain.

In one embodiment, non-toxic LPS molecular species from *Helicobacter* comprise one $C_{16}$ fatty acid chain and three Cis fatty acid chains.

Other examples of naturally-occurring LPS molecular species which are non-toxic include, but are not limited to, LPS molecular species selected from the compounds of Formulas (I)(0)(a), (I)(0)(b) as seen in **Figure 1,** compounds of Formula (II)(0) as seen in **Figure 5,** compounds of Formulas (XIII)(0)(a), (XIII)(0)(b) as seen in **Figure 34,** and combinations thereof.

Other examples of naturally-occurring LPS molecular species which are non-toxic include, but are not limited to, LPS molecular species selected from the compounds of Formulas (I)(0)(a), (I)(0)(b) as seen in **Figure 1,** compounds of Formula (II)(0) as seen in **Figure 5,** and combinations thereof.

In one embodiment, the composition comprises a naturally-occurring LPS molecular species selected from the group comprising or consisting of the LPS molecular species of Formulas (I)(1)(a), (I)(1)(b), and a combination thereof.

In one embodiment, the composition comprises a mix of at least two naturally-occurring LPS molecular species selected from the group comprising or consisting of the LPS molecular species of Formulas (I)(1)(a), (I)(1)(b), and a combination thereof.

In one embodiment, the composition comprises a mix of naturally-occurring LPS molecular species comprising or consisting of at least one LPS molecular species of Formula (I)(1)(a), and at least one LPS molecular species of Formula (I)(1)(b).

In one embodiment, the composition comprises naturally-occurring LPS molecular species of Formula (II)(0).

In one embodiment, the compositions according to the present invention comprise at least one modified LPS molecular species and at least naturally-occurring LPS molecular species as described hereinabove.

In one embodiment, the compositions according to the present invention comprise a mix of modified LPS molecular species and of naturally-occurring LPS molecular species as described hereinabove.

In one embodiment, the compositions according to the present invention comprise LPS molecular species which are monodisperse in aqueous solutions, preferably in water and/or in PBS, as can be assessed by, *e.g.,* DLS measurements; preferably DLS measurements with a scattering angle $\Theta$ = 90°, incident laser wavelength $\lambda$ = 633 nm and temperature at 25°C.

As used herein, the term **"monodisperse",** with reference to the LPS molecular species, refers to being substantially uniform in size in an aqueous solution (such as, *e.g.,* in water and/or in PBS).

By **"substantially uniform",** it is meant that the LPS molecular species have a narrow distribution of sizes around an average size. In one embodiment, the LPS molecular species in water and/or in PBS have sizes exhibiting a standard deviation of less than 20 % with respect to their average size, such as less than 10 %, or less than 5 %.

In one embodiment, the compositions according to the present invention comprise LPS molecular species formulated as liposomes. According to this embodiment, the liposomes may comprise, consist substantially of or exclusively of LPS molecular species according to the present invention.

**"Liposome",** as used herein, refers to vesicles in which a lipid phase comprising a bilayer of amphiphilic molecules, such as, *e.g.*, phospholipids or cholesterol, encages an internal, aqueous phase. Liposomes may be unilamellar (*i.e.*, comprising a single lipid bilayer), or multilamellar (*i.e.*, comprising at least two or more concentric lipid bilayers).

Phospholipids are lipids comprising a phosphoric acid group. They are composed of a polar, hydrophilic "head" and two aliphatic, hydrophobic "tails". Examples of phospholipids include, but are not limited to, phosphatidic acids and phosphoglycerides.

Liposomes may be prepared by various means and methods known in the art.

In one embodiment, LPS molecular species formulated as liposomes in the sense of the present invention may be in the form of (1) liposomes composed of any suitable amphiphilic molecule and encaging LPS molecular species according to the present invention; or (2) liposomes composed substantially of or exclusively of LPS molecular species according to the present invention.

In one embodiment, the compositions according to the present invention comprise LPS molecular species formulated as liposomes, and further comprise at least one additional therapeutic agent, either unconjugated or conjugated to the LPS molecular species.

In one embodiment, the compositions according to the present invention comprise LPS molecular species formulated as liposomes, and further comprise at least one additional therapeutic agent, either encapsulated or not in said liposomes.

Suitable examples of additional therapeutic agents include, but are not limited to, chemotherapeutic agents, targeted therapy agents, cytotoxic agents, antibiotics, antivirals, cell cycle-synchronizing agents, ligands for cellular receptor(s), immunomodulatory agents, pro-apoptotic agents, anti-angiogenic agents, cytokines, growth factors, antibodies or antigen-binding fragments thereof, cell therapy, antigens, and the like.

Detailed examples of these therapeutic agents have been described hereinabove as suitable agents for conjugation at positions Z, $R_1$ and/or $R_2$ of **Formula A** and **Formula B.** However, the one skilled in the art will readily understand that these therapeutic agents may additionally or alternatively be provided as unconjugated to the composition according to the present invention.

In one embodiment, the additional therapeutic agent is a targeted therapy agent as defined hereinabove, preferably said additional therapeutic agent is an anti-CD20 monoclonal antibody or an antigen-binding fragment thereof. In one embodiment, said anti-CD20 monoclonal antibody is selected from the group comprising or consisting of ibritumomab, obinutuzumab, ocrelizumab, ofatumumab, rituximab, tositumomab, or an antigen-binding fragment thereof; preferably said anti-CD20 monoclonal antibody is rituximab, or an antigen-binding fragment thereof.

In one embodiment, the additional therapeutic agent is an immunostimulatory agent as defined hereinabove, preferably said additional therapeutic agent is an immune checkpoint inhibitor. In one embodiment, said immune

checkpoint inhibitor is selected from the group comprising or consisting of inhibitors of CTLA4, PD-1, PD-L1, LAG-3, B7-H3, B7-H4, TIM3, A2AR, GITR, CD47, TNFR2, CD19, and/or IDO. In one embodiment, said immune checkpoint inhibitor is selected from the group comprising or consisting of nivolumab, pembrolizumab, cemiplimab, pidilizumab, AMP-224, AMP-514, MPDL3280A, MDX-1105, MEDI-4736, arelumab, ipilimumab, tremelimumab, pidilizumab, IMP321, MGA271, BMS-986016, lirilumab, urelumab, PF-05082566, IPH2101, MEDI-6469, CP-870,893, mogamulizumab, varlilumab, avelumab, galiximab, AUNP 12, indoximod, NLG-919, and INCB024360. In one embodiment, said immune checkpoint inhibitor is an inhibitor of PD-1 or PD-L1. Examples of PD-1 inhibitors include, but are not limited to, nivolumab, pembrolizumab, cemiplimab, spartalizumab, camrelizumab, sintilimab, tislelizumab, toripalimab, AMP-224, and AMP-514. Examples of PD-L1 inhibitors include, but are not limited to, atezolizumab, avelumab, durvalumab, KN035, CK-301, AUNP12, CA-170, and BMS-986189.

In one embodiment, the compositions according to the present invention are pharmaceutical compositions and further comprise at least one pharmaceutically acceptable excipient.

As used herein, the term **"pharmaceutically acceptable excipient"** refers to a solid, semi-solid or liquid component of a pharmaceutical composition or a vaccine composition that is not an active ingredient, and that does not produce an adverse, allergic or other untoward reaction when administered to an animal, preferably to a human. The most of these pharmaceutically acceptable excipients are described in detail in, *e.g.,* Allen (Ed.), 2017. Ansel's pharmaceutical dosage forms and drug delivery systems (11th ed.). Philadelphia, PA: Wolters Kluwer; Remington, Allen & Adeboye (Eds.), 2013. Remington: The science and practice of pharmacy (22nd ed.). London: Pharmaceutical Press; and Sheskey, Cook & Cable (Eds.), 2017. Handbook of pharmaceutical excipients (8th ed.). London: Pharmaceutical Press; each of which is herein incorporated by reference in its entirety.

Pharmaceutically acceptable excipients include, but are not limited to, water, saline, Ringer's solution, dextrose solution, and solutions of ethanol, glucose, sucrose, dextran, mannose, mannitol, sorbitol, polyethylene glycol (PEG), phosphate, acetate, gelatin, collagen, Carbopol®, vegetable oils, and the like. One may additionally include suitable preservatives, stabilizers, antioxidants, antimicrobials, and buffering agents, such as, *e.g.,* BHA, BHT, citric acid, ascorbic acid, tetracycline, and the like.

Other examples of pharmaceutically acceptable excipients that may be used in the composition of the invention include, but are not limited to, ion exchangers, alum such as aluminum phosphate or aluminium hydroxide, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

In addition, some pharmaceutically acceptable excipients may include, surfactants *(e.g.,* hydroxypropylcellulose); suitable carriers, such as, *e.g.,* solvents and dispersion media containing, *e.g.,* water, ethanol, polyol *(e.g.,* glycerol, propylene glycol, and liquid polyethylene glycol, and the like), suitable mixtures thereof, and vegetable oils, such as, *e.g.,* peanut oil and sesame oil; isotonic agents, such as, *e.g.,* sugars or sodium chloride; coating agents, such as, *e.g.,* lecithin; agents delaying absorption, such as, *e.g.,* aluminum monostearate and gelatin; preservatives, such as, *e.g.*, benzalkonium chloride, benzethonium chloride, chlorobutanol, thimerosal and the like; buffers, such as, *e.g.,* boric acid, sodium and potassium bicarbonate, sodium and potassium borates, sodium and potassium carbonate, sodium acetate, sodium biphosphate and the like; tonicity agents, such as, *e.g.*, dextran 40, dextran 70, dextrose, glycerin, potassium chloride, propylene glycol, sodium chloride; antioxidants and stabilizers, such as, *e.g.*, sodium bisulfite, sodium metabisulfite, sodium thiosulfite, thiourea and the like; nonionic wetting or clarifying agents, such as, *e.g.*, polysorbate 80, polysorbate 20, poloxamer 282 and tyloxapol; viscosity modifying agents, such as, *e.g.*, dextran 40, dextran 70, gelatin, glycerin, hydroxyethylcellulose, hydroxymethylpropylcellulose, lanolin, methylcellulose, petrolatum, polyethylene glycol, polyvinyl alcohol, polyvinylpyrrolidone, carboxymethylcellulose; and the like.

In one embodiment, the compositions according to the present invention are vaccine compositions.

As used herein, the term **"vaccine composition"** refers to a composition which comprises at least one antigen or immunogen and optionally an adjuvant, in a pharmaceutically acceptable excipient, and which is useful for inducing an immune response in a subject upon administration.

As used herein, the terms **"antigen"** or **"immunogen"** refer to any substance that induces a state of sensitivity and/or immune responsiveness after any latent period (normally, days to weeks in humans) and that reacts in a demonstrable way with antibodies and/or immune cells of the sensitized subject *in vivo* or *in vitro.*

As used herein, the term **"adjuvant"** refers to a substance that enhances, augments and/or potentiates an immune response to an antigen in a subject upon administration.

Examples of adjuvants include, but are not limited to, helper peptide, aluminum salts (such as, *e.g.*, aluminum hydroxide gel (also named alum), aluminum phosphate, and the like), Freund's incomplete adjuvant, Freund's complete adjuvant, saponin, Merck adjuvant 65, Smith-Kline Beecham adjuvant AS-2, Aquilla adjuvant QS-21, MPE™ immunostimulant, 3d-MPL, LEIF, calcium salts, iron salts, zinc salts, acylated tyrosine, acylated sugars, cationically-derivatized polysaccharides, anionically-derivatized polysaccharides, polyphosphazenes, biodegradable microspheres, monophosphoryl lipid A, muramyl tripeptide phosphatidyl ethanolarnine, cytokines (such as, *e.g.*, interleukin-2, interleukin-12, interleukin-4, interleukin-7 and the like), CpG-containing oligonucleotide, and combinations thereof.

In one embodiment, the vaccine composition according to the present invention comprises at least one LPS molecular species as described hereinabove, acting both as primary antigen and primary adjuvant. Indeed, a LPS molecular species comprising a core oligosaccharide domain, optionally substituted with an antigen domain, such as an O-antigen domain and/or a capping antigen, exhibits antigenic and immunogenic properties against bacterial species from which said LPS molecular species is derived.

In one embodiment, the vaccine composition according to the present invention comprises at least one LPS molecular species as described hereinabove acting as primary antigen, and at least one adjuvant which is not a LPS molecular species as described hereinabove - the LPS molecular species acting as a secondary adjuvant.

In one embodiment, the vaccine composition according to the present invention comprises at least one LPS molecular species as described hereinabove acting as a primary adjuvant, and at least one antigen which is not a LPS molecular species as described hereinabove - the LPS molecular species acting as a secondary antigen. In this embodiment, the at least one antigen may be an antigen derived from the same bacterial species as the LPS molecular species, for a combined antigenic effect against said bacterial species. Alternatively, the at least one antigen may be an antigen which is not derived from the same bacterial species as the LPS molecular species (*i.e.,* an antigen derived from another bacterial species or any other antigen such as a pathogen-related antigen, a self-antigen, an allergen-related antigen, a neoantigen, or else).

In one embodiment, the vaccine composition is a subunit vaccine. In one embodiment, the vaccine composition is a protein vaccine. In one embodiment, the vaccine composition is a cellular-based vaccine.

Suitable examples of antigens include, but are not limited to, pathogen-related antigens (such as, *e.g.*, antigens of viruses, fungi or bacteria, or immunogenic molecules derived from them), self-antigens (such as, *e.g.,* cellular antigens including cells containing normal transplantation antigens and/or tumor-related antigens, RR-Rh antigens, and antigens characteristic of, or specific to particular cells or tissues or body fluids), allergen-related antigens (such as, *e.g.,* those associated with environmental allergens, including grasses, pollens, molds, dust, insects, dander, venoms, and the like; occupational allergens, including latex, dander, urethanes, epoxy resins, and the like; food, including shellfish, peanuts, eggs, milk products, and the like; and drugs, including antibiotics, anesthetics, and the like), neoantigens, and vaccines.

Suitable examples of pathogen-related antigens include, but are not limited to, antigens derived from vaccinia, avipox virus, turkey influenza virus, bovine leukemia virus, feline leukemia virus, avian influenza, chicken pneumovirosis virus, canine parvovirus, equine influenza, FHV, Newcastle Disease Virus (NDV), Chicken/Pennsylvania/1/83 influenza virus, infectious bronchitis virus, Dengue virus, measles virus, Rubella virus, pseudorabies, Epstein-Barr Virus, HIV, SIV, EHV, BHV, HCMV, Hantaan, C. tetani, mumps, Morbillivirus, Herpes Simplex Virus type 1, Herpes Simplex Virus type 2, Human cytomegalovirus, Hepatitis A Virus, Hepatitis B Virus, Hepatitis C Virus, Hepatitis E Virus, Respiratory Syncytial Virus, Human Papilloma Virus, Influenza Virus, *Bordetella, Vitreoscilla, Salmonella, Neisseria, Borrelia, Chlamydia, Plasmodium, Toxoplasma, Cryptococcus, Streptococcus, Staphylococcus, Haemophilus, Diptheria, Tetanus, Escherichia, Candida, Aspergillus, Actinobacilus, Burkholderia, Moraxella, Pseudomonas, Vibrio, Entamoeba, Giardia,* and *Trypanasoma.*

Suitable examples of self-antigens include, but are not limited to, lupus autoantigen, Smith, Ro, La, U1-RNP, fibrillin, nuclear antigens, histones, glycoprotein gp70, ribosomal proteins, pyruvate dehydrogenase, dehydrolipoamide acetyltransferase (PCD-E2), hair follicle antigens, human tropomyosin isoform 5 (hTM5), proinsulin, insulin, IA2, GAD65, collagen type II, human cartilage gp 39 (HCgp39), gp130-RAPS, dnaJp1, citrullinated proteins and peptides (including citrullinated type II collagen, citrullinated vimentin and citrullinated fibrinogen), myelin basic protein, proteolipid protein (PLP), myelin oligodendrocyte glycoprotein (MOG), thyroid stimulating factor receptor (TSH-R), acetylcholine receptor (AchR), gliadin, PLP, glucose-6-phosphate isomerase, thyroglobulin, various tRNA synthetases, proteinase-3, and myeloperoxidase, and the like, including fragments thereof.

Suitable examples of tumor-related antigens include, but are not limited to, MART-1/Melan-A, gplOO, dipeptidyl peptidase IV (DPPIV), adenosine deaminase-binding protein (ADAbp), cyclophilin b, colorectal associated antigen (CRC)-C017-1A/GA733, carcinoembryonic antigen (CEA) and its immunogenic epitopes CAP-1 and CAP-2, etv6, amll, prostate specific antigen (PSA) and its immunogenic epitopes PSA-1, PSA-2, and PSA-3, prostate-specific membrane antigen (PSMA), T-cell receptor/CD3-zeta chain, MAGE-family of tumor antigens (*e.g.*, MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5), GAGE-family of tumor antigens (*e.g.*, GAGE-1, GAGE-2, GAGE-3, GAGE-4, GAGE- 5, GAGE-6, GAGE-7, GAGE-8, GAGE-9), BAGE, RAGE, LAGE-1, NAG, GnT-V, MUM-1, CDK4, tyrosinase, p53, MUC family (*e.g.* MUC1, MUC16, etc.), HER2/neu, p21ras, RCASl, alpha-fetoprotein, E-cadherin, alpha-catenin, beta-catenin and gamma-catenin, p120ctn, gp100.sup.Pmelll7, PRAME, NY-ESO-1, cdc27, adenomatous polyposis coli protein (APC), fodrin, connexin 37, Ig-idiotype, pl5, gp75, GM2 and GD2 gangliosides, Smad family of cancer antigens brain glycogen phosphorylase, SSX-1, SSX-2 (HOM-MEL-40), SSX-1, SSX-4, SSX-5, SCP-1 and CT-7, and c-erbB-2 and viral antigens such as the HPV-16 and HPV-18 E6 and E7 antigens and the EBV-encoded nuclear antigen (EBNA)-1, and the like, including fragments thereof. Further examples of tumor-related antigens are described in, *e.g.,* Li et al., 2004. Cancer Immunol Immunother. 53(3):139-43; Novellino et al., 2005. Cancer Immunol Immunother. 54(3):187-20; which are herein incorporated by reference in their entirety.

In one embodiment, the antigen may be conjugated to the LPS molecular species, such as, *e.g.,* at positions Z, $R_1$ and/or $R_2$ of the modified LPS molecular species of **Formula A** or **Formula B,** as described hereinabove.

In one embodiment, the compositions according to the present invention are medicaments.

As used herein, the term **"medicament"** is to be understood to refer to a variety of types of pharmacological materials intended for application or delivery to any of a variety of patients, either human or otherwise, through a variety of delivery mechanisms.

In one embodiment, the composition, pharmaceutical composition, medicament or vaccine composition according to the present invention is formulated for administration to a subject.

In one embodiment, the composition, pharmaceutical composition, medicament or vaccine composition according to the present invention is formulated for systemic or local administration to a subject.

In one embodiment, the composition, pharmaceutical composition, medicament or vaccine composition according to the present invention is formulated for administration by injection, oral administration, topical administration, nasal administration, buccal administration, rectal administration, vaginal administration, intratracheal administration, administration by endoscopy, transmucosal administration, percutaneous administration, or intratumoral administration.

In one embodiment, the composition, pharmaceutical composition, medicament or vaccine composition according to the present invention is formulated for administration by injection, preferably by systemic injection.

Examples of formulations adapted for injection include, but are not limited to, solutions, such as, *e.g.*, sterile aqueous solutions, gels, dispersions, emulsions, suspensions, solid forms suitable for using to prepare solutions or suspensions upon the addition of a liquid prior to use, such as, for example, powder, liposomal forms, outer membrane vesicles (OMVs), and the like.

Examples of systemic injections include, but are not limited to, intravenous (iv), subcutaneous (sq), intradermal (id), intramuscular (im), intraarterial, intraparenteral, intranodal, intralymphatic, intraperitoneal (ip), intracranial, intracardiac, intralesional, intraprostatic, intravaginal, intrarectal, intrathecal, intranasal, intratumoral (it), intravesicular, and perfusion.

In one embodiment, when injected, the composition, pharmaceutical composition, medicament or vaccine composition according to the present invention is sterile. Methods for obtaining a sterile composition include, but are not limited to, GMP synthesis (where GMP stands for "Good manufacturing practice").

Sterile injectable forms of a composition may be aqueous or oleaginous. These suspensions may be formulated according to techniques known in the art using suitable dispersing or wetting agents and suspending agents. The sterile injectable preparation may also be a sterile injectable solution or suspension in a non-toxic parenterally acceptable diluent or solvent. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans, biosurfactants, and other emulsifying agents or bioavailability enhancers which are commonly used in the manufacture of pharmaceutically acceptable solid, liquid, or other dosage forms may also be used for the purposes of formulation.

It will be understood that other suitable routes of administration are also contemplated in the present invention, and the administration mode will ultimately be decided by the attending physician within the scope of sound medical judgment.

In one embodiment, the composition, pharmaceutical composition, medicament or vaccine composition according to the present invention is formulated with at least one additional therapeutic agent, either unconjugated or conjugated to the LPS molecular species.

In one embodiment, the composition, pharmaceutical composition, medicament or vaccine composition according to the present invention is to be administered to a subject in need thereof before, concomitantly with, or after administration of at least one additional therapeutic agent.

Suitable examples of therapeutic agents include, but are not limited to, chemotherapeutic agents, targeted therapy agents, cytotoxic agents, antibiotics, antivirals, cell cycle-synchronizing agents, ligands for cellular receptor(s), immunomodulatory agents, pro-apoptotic agents, anti-angiogenic agents, cytokines, growth factors, antibodies or

antigen-binding fragments thereof, cell therapy, antigens, and the like.

Suitable examples of cell therapies include, but are not limited to, adoptive cell therapy, CAR-T cell therapy, allogeneic cell therapy, embryonic stem cell therapy, neural stem cell therapy, mesenchymal stem cell therapy, and hematopoietic stem cell transplantation.

Other detailed examples of these therapeutic agents have been described hereinabove as suitable agents for conjugation at positions Z, $R_1$ and/or $R_2$ of **Formula A** and **Formula B.** However, the one skilled in the art will readily understand that these therapeutic agents may additionally or alternatively be provided as unconjugated to the composition, pharmaceutical composition, medicament or vaccine composition according to the present invention.

In one embodiment, the additional therapeutic agent is a targeted therapy agent as defined hereinabove, preferably said additional therapeutic agent is an anti-CD20 monoclonal antibody or an antigen-binding fragment thereof. In one embodiment, said anti-CD20 monoclonal antibody is selected from the group comprising or consisting of ibritumomab, obinutuzumab, ocrelizumab, ofatumumab, rituximab, tositumomab, or an antigen-binding fragment thereof; preferably said anti-CD20 monoclonal antibody is rituximab or an antigen-binding fragment thereof.

In one embodiment, the additional therapeutic agent is an immunostimulatory agent as defined hereinabove, preferably said additional therapeutic agent is an immune checkpoint inhibitor. In one embodiment, said immune checkpoint inhibitor is selected from the group comprising or consisting of inhibitors of CTLA4, PD-1, PD-L1, LAG-3, B7-H3, B7-H4, TIM3, A2AR, GITR, CD47, TNFR2, CD19, and/or IDO. In one embodiment, said immune checkpoint inhibitor is selected from the group comprising or consisting of nivolumab, pembrolizumab, cemiplimab, pidilizumab, AMP-224, AMP-514, MPDL3280A, MDX-1105, MEDI-4736, arelumab, ipilimumab, tremelimumab, pidilizumab, IMP321, MGA271, BMS-986016, lirilumab, urelumab, PF-05082566, IPH2101, MEDI-6469, CP-870,893, mogamulizumab, varlilumab, avelumab, galiximab, AUNP 12, indoximod, NLG-919, and INCB024360. In one embodiment, said immune checkpoint inhibitor is an inhibitor of PD-1 or PD-L1. Examples of PD-1 inhibitors include, but are not limited to, nivolumab, pembrolizumab, cemiplimab, spartalizumab, camrelizumab, sintilimab, tislelizumab, toripalimab, AMP-224, and AMP-514. Examples of PD-L1 inhibitors include, but are not limited to, atezolizumab, avelumab, durvalumab, KN035, CK-301, AUNP12, CA-170, and BMS-986189.

As will be further detailed hereafter, the modified LPS molecular species, as well as the composition, pharmaceutical composition, medicament or vaccine composition according to the present invention, optionally formulated with at least one additional therapeutic agent, either unconjugated and/or conjugated to the LPS molecular species, are useful for a wide range of therapeutic, vaccinal and immunostimulatory purposes.

The present invention relates to a modified LPS molecular species, a composition, a pharmaceutical composition, a medicament or a vaccine composition according to the present invention, for use as a drug.

In particular, the present invention relates to a modified LPS molecular species, a composition, a pharmaceutical composition, a medicament or a vaccine composition according to the present invention, for use in treating and/or preventing cancer, an inflammatory disease, or an infectious disease, or for stimulating an immune response or vaccinating a subject in need thereof.

The present invention also relates to a method of treating and/or preventing cancer in a subject in need thereof, comprising or consisting of administering to said subject a modified LPS molecular species, a composition, a pharmaceutical composition, a medicament or a vaccine composition according to the present invention.

The present invention also relates to a modified LPS molecular species, a composition, a pharmaceutical composition, a medicament or a vaccine composition according to the present invention, for use in treating and/or preventing cancer in a subject in need thereof.

Examples of cancers include those listed in the $10^{th}$ revision of the International Statistical Classification of Diseases and Related Health Problems (ICD), under chapter II, blocks C00 to D48.

Further examples of cancers include, but are not limited to, recurrent, metastatic or multi-drug resistant cancer.

Further examples of cancers include, but are not limited to, adenofibroma, adenoma, agnogenic myeloid metaplasia, AIDS-related malignancies, ameloblastoma, anal cancer, angiofollicular mediastinal lymph node hyperplasia, angiokeratoma, angiolymphoid hyperplasia with eosinophilia, angiomatosis, anhidrotic ectodermal dysplasia, anterofacial dysplasia, apocrine metaplasia, apudoma, asphyxiating thoracic dysplasia, astrocytoma (including, *e.g.*, cerebellar astrocytoma and cerebral astrocytoma), atriodigital dysplasia, atypical melanocytic hyperplasia, atypical metaplasia, autoparenchymatous metaplasia, basal cell hyperplasia, benign giant lymph node hyperplasia, bile duct cancer (including, *e.g.*, extrahepatic bile duct cancer), bladder cancer, bone cancer, brain tumor (including, *e.g.*, brain stem glioma, cerebellar astrocytoma glioma, malignant glioma, supratentorial primitive neuroectodermal tumors, visual pathway and hypothalamic glioma, ependymoma, medulloblastoma, gestational trophoblastic tumor glioma, and paraganglioma), branchionia, female breast cancer, male breast cancer, bronchial adenomas/carcinoids, bronchopulmonary dysplasia, cancer growths of epithelial cells, pre-cancerous growths of epithelial cells, metastatic growths of epithelial cells, carcinoid heart disease, carcinoid tumor (including, *e.g.,* gastrointestinal carcinoid tumor), carcinoma (including, *e.g.,* carcinoma of unknown primary origin, adrenocortical carcinoma, islet cells carcinoma, adeno carcinoma, adeoncortical carcinoma, basal cell carcinoma, basosquamous carcinoma, bronchiolar carcinoma, Brown-Pearce carcinoma,

cystadenocarcinoma, ductal carcinoma, hepatocarcinoma, Krebs carcinoma, papillary carcinoma, oat cell carcinoma, small cell lung carcinoma, non-small cell lung carcinoma, squamous cell carcinoma, transitional cell carcinoma, Walker carcinoma, Merkel cell carcinoma, and skin carcinoma), cementoma, cementum hyperplasia, cerebral dysplasia, cervical cancer, cervical dysplasia, cholangioma, cholesteatoma, chondroblastoma, chondroectodermal dysplasia, chordoma, choristoma, chrondroma, cleidocranial dysplasia, colon cancer, colorectal cancer, local metastasized colorectal cancer, congenital adrenal hyperplasia, congenital ectodermal dysplasia, congenital sebaceous hyperplasia, connective tissue metaplasia, craniocarpotarsal dysplasia, craniodiaphysial dysplasia, craniometaphysial dysplasia, craniopharyngioma, cylindroma, cystadenoma, cystic hyperplasia (including, *e.g.*, cystic hyperplasia of the breast), cystosarcoma phyllodes, dentin dysplasia, denture hyperplasia, diaphysial dysplasia, ductal hyperplasia, dysgenninoma, dysplasia epiphysialis hemimelia, dysplasia epiphysialis multiplex, dysplasia epiphysialis punctate, ectodermal dysplasia, Ehrlich tumor, enamel dysplasia, encephaloophthalmic dysplasia, endometrial cancer (including, *e.g.*, ependymoma and endometrial hyperplasia), ependymoma, epithelial cancer, epithelial dysplasia, epithelial metaplasia, esophageal cancer, Ewing's family of tumors (including, *e.g.*, Ewing's sarcoma), extrahepatic bile duct cancer, eye cancer (including, *e.g.*, intraocular melanoma and retinoblastoma), faciodigitogenital dysplasia, familial fibrous dysplasia of jaws, familial white folded dysplasia, fibroma, fibromuscular dysplasia, fibromuscular hyperplasia, fibrous dysplasia of bone, florid osseous dysplasia, focal epithelial hyperplasia, gall bladder cancer, ganglioneuroma, gastric cancer (including, *e.g.*, stomach cancer), gastrointestinal carcinoid tumor, gastrointestinal tract cancer, gastrointestinal tumors, Gaucher's disease, germ cell tumors (including, *e.g.*, extracranial germ cell tumors, extragonadal germ cell tumors, and ovarian germ cell tumors), giant cell tumor, gingival hyperplasia, glioblastoma, glomangioma, granulosa cell tumor, gynandroblastoma, hamartoma, head and neck cancer, hemangioendothelioma, hemangioma, hemangiopericytoma, hepatocellular cancer, hepatoma, hereditary renal-retinal dysplasia, hidrotic ectodermal dysplasia, histiocytonia, histiocytosis, hypergammaglobulinemia, hypohidrotic ectodermal dysplasia, hypopharyngeal cancer, inflammatory fibrous hyperplasia, inflammatory papillary hyperplasia, intestinal cancers, intestinal metaplasia, intestinal polyps, intraocular melanoma, intravascular papillary endothelial hyperplasia, kidney cancer, laryngeal cancer, leiomyoma, leukemia (including, *e.g.*, acute lymphoblastic leukemia, acute lymphocytic leukemia, acute myeloid leukemia, acute myelogenous leukemia, acute hairy cell leukemia, acute B-cell leukemia, acute T-cell leukemia, acute HTLV leukemia, chronic lymphoblastic leukemia, chronic lymphocytic leukemia, chronic myeloid leukemia, chronic myelogenous leukemia, chronic hairy cell leukemia, chronic B-cell leukemia, chronic T-cell leukemia, and chronic HTLV leukemia), Leydig cell tumor, lip and oral cavity cancer, lipoma, liver cancer, lung cancer (including, *e.g.*, small cell lung cancer and non-small cell lung cancer), lymphangiomyoma, lymphaugioma, lymphoma (including, *e.g.*, AIDS-related lymphoma, central nervous system lymphoma, primary central nervous system lymphoma, Hodgkin's lymphoma, non-Hodgkin's lymphoma, Hodgkin's lymphoma during pregnancy, non-Hodgkin's lymphoma during pregnancy, mast cell lymphoma, B-cell lymphoma, adenolymphoma, Burkitt's lymphoma, cutaneous T-cell lymphoma, large cell lymphoma, and small cell lymphoma), lymphopenic thymic dysplasia, lymphoproliferative disorders, macroglobulinemia (including, *e.g.*, Waldenstrom's macroglobulinemia), malignant carcinoid syndrome, malignant mesothelioma, malignant thymoma, mammary dysplasia, mandibulofacial dysplasia, medulloblastoma, meningioma, mesenchymoma, mesonephroma, mesothelioma (including, *e.g.*, malignant mesothelioma), metaphysial dysplasia, metaplastic anemia, metaplastic ossification, metaplastic polyps, metastatic squamous neck cancer (including, *e.g.*, metastatic squamous neck cancer with occult primary), Mondini dysplasia, monostotic fibrous dysplasia, mucoepithelial dysplasia, multiple endocrine neoplasia syndrome, multiple epiphysial dysplasia, multiple myeloma/plasma cell neoplasm, mycosis fungoides, myelodysplastic syndrome, myeloid metaplasia, myeloproliferative disorders, chronic myeloproliferative disorders, myoblastoma, myoma, myxoma, nasal cavity and paranasal sinus cancer, nasopharyngeal cancer, prostatic neoplasm, colon neoplasm, abdomen neoplasm, bone neoplasm, breast neoplasm, digestive system neoplasm, liver neoplasm, pancreas neoplasm, peritoneum neoplasm, endocrine glands neoplasm (including, *e.g.*, adrenal neoplasm, parathyroid neoplasm, pituitary neoplasm, testicles neoplasm, ovary neoplasm, thymus neoplasm, and thyroid neoplasm), eye neoplasm, head and neck neoplasm, nervous system neoplasm (including, *e.g.*, central nervous system neoplasm and peripheral nervous system neoplasm), lymphatic system neoplasm, pelvic neoplasm, skin neoplasm, soft tissue neoplasm, spleen neoplasm, thoracic neoplasm, urogenital tract neoplasm, neurilemmoma, neuroblastoma, neuroepithelioma, neurofibroma, neurofibromatosis, neuroma, nodular hyperplasia of prostate, nodular regenerative hyperplasia, oculoauriculovertebral dysplasia, oculodentodigital dysplasia, oculovertebral dysplasia, odontogenic dysplasia, odontoma, opthalmomandibulomelic dysplasia, oropharyngeal cancer, osteoma, ovarian cancer (including, *e.g.*, ovarian epithelial cancer and ovarian low malignant potential tumor), pancreatic cancer (including, *e. g.*, islet cell pancreatic cancer and exocrine pancreatic cancer), papilloma, paraganglioma, nonchromaffin paraganglioma, paranasal sinus and nasal cavity cancer, paraproteinemias, parathyroid cancer, periapical cemental dysplasia, pheochromocytoma (including, *e.g.*, penile cancer), pineal and supratentorial primitive neuroectodermal tumors, pinealoma, pituitary tumor, plasma cell neoplasm/multiple myeloma, plasmacytoma, pleuropulmonary blastoma, polyostotic fibrous dysplasia, polyps, pregnancy cancer, pre-neoplastic disorders (including, *e.g.*, benign dysproliferative disorders such as benign tumors, fibrocystic conditions, tissue hypertrophy, intestinal polyps, colon polyps, esophageal dysplasia, leukoplakia, keratoses, Bowen's disease, Farmer's skin, solar cheilitis, and solar keratosis), primary hepatocellular

cancer, primary liver cancer, primary myeloid metaplasia, prostate cancer, pseudoachondroplastic spondyloepiphysial dysplasia, pseudoepitheliomatous hyperplasia, purpura, rectal cancer, renal cancer (including, *e.g.*, kidney cancer, renal pelvis, ureter cancer, transitional cell cancer of the renal pelvis and ureter), reticuloendotheliosis, retinal dysplasia, retinoblastoma, salivary gland cancer, sarcomas (including, *e.g.*, uterine sarcoma, soft tissue sarcoma, carcinosarcoma, chondrosarcoma, fibrosarcoma, hemangiosarcoma, Kaposi's sarcoma, leiomyosarcoma, liposarcoma, lymphangiosarcoma, myosarcoma, myxosarcoma, rhabdosarcoma, sarcoidosis sarcoma, osteosarcoma, Ewing sarcoma, malignant fibrous histiocytoma of bone, and clear cell sarcoma of tendon sheaths), sclerosing angioma, secondary myeloid metaplasia, senile sebaceous hyperplasia, septooptic dysplasia, Sertoli cell tumor, Sezary syndrome, skin cancer (including, *e.g.*, melanoma skin cancer and non-melanoma skin cancer), small intestine cancer, spondyloepiphysial dysplasia, squamous metaplasia (including, *e.g.*, squamous metaplasia of amnion), stomach cancer, supratentorial primitive neuroectodermal and pineal tumors, supratentorial primitive neuroectodermal tumors, symptomatic myeloid metaplasia, teratoma, testicular cancer, theca cell tumor, thymoma (including, *e.g.*, malignant thymoma), thyroid cancer, trophoblastic tumors (including, *e.g.*, gestational trophoblastic tumors), ureter cancer, urethral cancer, uterine cancer, vaginal cancer, ventriculoradial dysplasia, verrucous hyperplasia, vulvar cancer, Waldenstrom's macroglobulinemia, and Wilms' tumor.

In one embodiment, the cancer is selected from the group comprising or consisting of osteosarcoma, colorectal cancer, lymphoma (such as B-cell lymphoma), chondrosarcoma, bladder cancer, and breast cancer.

In one embodiment, the cancer is selected from the group comprising or consisting of osteosarcoma, chondrosarcoma, lymphoma (such as B-cell lymphoma), bladder cancer, and breast cancer.

In one embodiment, the cancer is selected from the group comprising or consisting of osteosarcoma, colorectal cancer, and lymphoma (such as B-cell lymphoma).

In one embodiment, treating cancer in a subject in need thereof further comprises administering to said subject, either before, concomitantly, or after, at least one additional therapeutic agent as defined hereinabove.

In one embodiment, the additional therapeutic agent is a targeted therapy agent as defined hereinabove, preferably said additional therapeutic agent is an anti-CD20 monoclonal antibody or an antigen-binding fragment thereof. In one embodiment, said anti-CD20 monoclonal antibody is selected from the group comprising or consisting of ibritumomab, obinutuzumab, ocrelizumab, ofatumumab, rituximab, tositumomab, or an antigen-binding fragment thereof; preferably said anti-CD20 monoclonal antibody is rituximab.

In one embodiment, the additional therapeutic agent is an immunostimulatory agent as defined hereinabove, preferably said additional therapeutic agent is an immune checkpoint inhibitor. In one embodiment, said immune checkpoint inhibitor is selected from the group comprising or consisting of inhibitors of CTLA4, PD-1, PD-L1, LAG-3, B7-H3, B7-H4, TIM3, A2AR, GITR, CD47, TNFR2, CD19, and/or IDO. In one embodiment, said immune checkpoint inhibitor is selected from the group comprising or consisting of nivolumab, pembrolizumab, cemiplimab, pidilizumab, AMP-224, AMP-514, MPDL3280A, MDX-1105, MEDI-4736, arelumab, ipilimumab, tremelimumab, pidilizumab, IMP321, MGA271, BMS-986016, lirilumab, urelumab, PF-05082566, IPH2101, MEDI-6469, CP-870,893, mogamulizumab, varlilumab, avelumab, galiximab, AUNP 12, indoximod, NLG-919, and INCB024360. In one embodiment, said immune checkpoint inhibitor is an inhibitor of PD-1 or PD-L1. Examples of PD-1 inhibitors include, but are not limited to, nivolumab, pembrolizumab, cemiplimab, spartalizumab, camrelizumab, sintilimab, tislelizumab, toripalimab, AMP-224, and AMP-514. Examples of PD-L1 inhibitors include, but are not limited to, atezolizumab, avelumab, durvalumab, KN035, CK-301, AUNP12, CA-170, and BMS-986189.

The present invention also relates to a method of treating and/or preventing inflammatory diseases in a subject in need thereof, comprising or consisting of administering to said subject a modified LPS molecular species, a composition, a pharmaceutical composition, a medicament or a vaccine composition according to the present invention.

The present invention also relates to a modified LPS molecular species, a composition, a pharmaceutical composition, a medicament or a vaccine composition according to the present invention, for use in treating and/or preventing inflammatory diseases in a subject in need thereof.

As used herein, the term **"inflammatory disease"** is used to define any disease caused by, or leading to, inflammation in a subject. The term may include, but is not limited to, (1) inflammatory and/or allergic diseases, (2) autoimmune diseases, (3) graft rejection, and (4) other diseases in which undesired inflammatory responses are to be inhibited.

Examples of inflammatory disorders include, but are not limited to, abdominal aortic aneurysm (AAA), acne, acute disseminated encephalomyelitis, acute leukocyte-mediated lung injury, Addison's disease, adult respiratory distress syndrome, AIDS dementia, allergic asthma, allergic conjunctivitis, allergic contact dermatitis, allergic rhinitis, allergic sinusitis, allergy, allograft rejection, alopecia areata, Alzheimer's disease, anaphylaxis, angioedema, ankylosing spondylitis, antiphospholipid antibody syndrome, asthma, atherosclerosis, atopic dermatitis, autoimmune hemolytic anemia, autoimmune hepatitis, autoimmune inner ear disease, Behcet's syndrome, blepharitis, bronchitis, bullous pemphigoid, chronic inflammatory diseases, chronic obstructive pulmonary disease, cirrhosis, closed-head injuries, coagulative necrosis, coeliac disease, collagenous colitis, conjunctivitis, contact dermatitis, coronary heart disease,

cutaneous necrotizing venulitis, cystic fibrosis, dermatitis, dermatomyositis, dermatopolymyositis, diabetes mellitus type 1, diabetes mellitus type 2, distal proctitis, diversion colitis, drug allergies, dry eye, eczema, encephalitis, endometriosis, endotoxin shock, epilepsy, erythema multiforme, erythema nodosum, fibrinoid necrosis, fibromyalgia, giant-cell arteritis (also named Horton's disease), glomerulonephritis, goodpasture's syndrome, gout, gouty arthritis, graft-versus-host disease (such as, *e.g.*, acute graft-versus-host disease, chronic graft-versus-host disease, and the like), Graves' disease, Guillain-Barre syndrome, Hashimoto's disease, hay fever, hyperacute transplant rejection, hyperlipidemia, hypersensitivity lung diseases, idiopathic thrombocytopenic purpura, indeterminate colitis, inflammatory bowel disease (IBD) (such as, *e.g.*, Crohn's disease, ulcerative colitis, colitis, pouchitis, ileitis, enteritis, and the like), inflammatory liver disorder, insect bite skin inflammation, insect sting allergies, interstitial cystitis, iritis, ischemic colitis, lichen planus, liquefactive necrosis, lupus, lupus erythematosus, lymphocytic colitis, meningitis, metabolic syndrome, multiple sclerosis, multitrauma, myasthenia gravis, myocarditis, myositis, narcolepsy, nephritis, obesity, osteoarthritis, osteoporosis, otitis, pancreatitis, Parkinson's disease, pemphigus vulgaris, periodontal gingivitis, periodontitis, pernicious anemia, polymyalgia rheumatica, polymyositis, postmenopausal-induced metabolic syndrome, primary biliary cirrhosis, psoriasis, psoriatic arthritis, radiation poisoning, radiation syndrome, relapsing polychondritis, retinitis, rheumatoid arthritis, rheumatoid spondylitis, rhinoconjunctivitis, sarcoidosis, scleroderma, septic shock, shingles, sinusitis, Sjögren syndrome, smooth muscle proliferation disorders, solar dermatitis, spondyloarthropathy, steatosis, stroke, systemic lupus erythematosus, urticaria, uveitis, vaginitis, vasculitis, vitiligo, and Wegener's granulomatosis.

In one embodiment, treating inflammatory diseases in a subject in need thereof further comprises administering to said subject, either before, concomitantly, or after, at least one additional therapeutic agent as defined hereinabove.

The present invention also relates to a method of treating and/or preventing infectious diseases in a subject in need thereof, comprising or consisting of administering to said subject a modified LPS molecular species, a composition, a pharmaceutical composition, a medicament or a vaccine composition according to the present invention.

The present invention also relates to a modified LPS molecular species, a composition, a pharmaceutical composition, a medicament or a vaccine composition according to the present invention, for use in treating and/or preventing infectious diseases in a subject in need thereof.

As used herein, the term **"infectious disease"** is used to define any disease caused by the invasion of a subject by infectious agents (or pathogens), their multiplication, and the reaction of the subject's tissues to these infectious agents and the toxins they produce. The term may include, but is not limited to, (1) bacterial infections, (2) viral infections, (3) fungal infections, and (4) parasite infections.

Examples of infectious diseases include, but are not limited to, *Acinetobacter* infections, actinomycosis, African sleeping sickness (also named African trypanosomiasis), AIDS (acquired immunodeficiency syndrome), amoebiasis, anaplasmosis, angiostrongyliasis, anisakiasis, anthrax, *Arcanobacterium haemolyticum* infection, Argentine hemorrhagic fever, ascariasis, aspergillosis, *Astrovirus* infection, babesiosis, *Bacillus cereus* infection, bacterial meningitis, bacterial pneumonia, bacterial vaginosis, *Bacteroides* infection, balantidiasis, bartonellosis, *Baylisascaris* infection, BK virus infection, Black Piedra, blastocystosis, blastomycosis, Bolivian hemorrhagic fever, botulism, Brazilian hemorrhagic fever, brucellosis, bubonic plague, *Burkholderia* infection, Buruli ulcer, *Calicivirus* infection, campylobacteriosis, candidiasis, capillariasis, Carrion's disease, cat-scratch disease, cellulitis, Chagas disease (also named American trypanosomiasis), chancroid, chickenpox, chikungunya, chlamydia, *Chlamydophila pneumoniae* infection, cholera, chromoblastomycosis, chytridiomycosis, clonorchiasis, *Clostridium difficile* colitis, coccidioidomycosis, Colorado tick fever, common cold (also named acute viral rhinopharyngitis or acute coryza), Creutzfeldt-Jakob disease, Crimean-Congo hemorrhagic fever, cryptococcosis, cryptosporidiosis, cutaneous larva migrans, cyclosporiasis, cysticercosis, cytomegalovirus infection, dengue fever, desmodesmus infection, dientamoebiasis, diphtheria, diphyllobothriasis, dracunculiasis, Ebola hemorrhagic fever, echinococcosis, ehrlichiosis, enterobiasis, *enterococcus* infection, enterovirus infection, epidemic typhus, erythema infectiosum, exanthem subitem, fasciolasis, fasciolopsiasis, fatal familial insomnia, filariasis, *Clostridium perfringens* poisoning, free-living amebic infection, *Fusobacterium* infection, gas gangrene, geotrichosis, Gerstmann-Straussler-Scheinker syndrome, giardiasis, glanders, gnathostomiasis, gonorrhea, granuloma inguinale, group A streptococcal infection, group B streptococcal infection, *Haemophilus influenzae* infection, hand foot and mouth disease, hantavirus pulmonary syndrome, Heartland virus disease, *Helicobacter pylori* infection, hemolytic-uremic syndrome, hemorrhagic fever with renal syndrome, hendra virus infection, hepatitis A, hepatitis B, hepatitis C, hepatitis D, hepatitis E, herpes simplex, histoplasmosis, hookworm infection, human bocavirus infection, human ewingii ehrlichiosis, human granulocytic anaplasmosis, human metapneumovirus infection, human monocytic ehrlichiosis, human papillomavirus infection, human parainfluenza virus infection, hymenolepiasis, infant botulism, infectious mononucleosis, influenza (also named flu), isosporiasis, Kawasaki disease, keratitis, *Kingella kingae* infection, kuru, lassa fever, legionellosis, leishmaniasis, leprosy, leptospirosis, listeriosis, lyme disease, lymphatic filariasis, lymphocytic choriomeningitis, malaria, Marburg hemorrhagic fever, measles, middle East respiratory syndrome (also named MERS), melioidosis (also named Whitmore's disease), meningitis, meningococcal disease, metagonimiasis, microsporidiosis, molluscum contagiosum, monkeypox, mumps, murine typhus, mycoplasma pneumonia, mycoplasma genitalium infection, mycetoma, myiasis, neonatal conjunctivitis, nipah virus infection, nocardiosis, onchocerciasis (also named river

blindness), opisthorchiasis, paracoccidioidomycosis ( also named South American blastomycosis), paragonimiasis, pasteurellosis, pediculosis capitis, pediculosis corporis, pediculosis pubis, pelvic inflammatory disease, pertussis (also named whooping cough), plague, pneumococcal infection, pneumocystis pneumonia, pneumonia, poliomyelitis, *Prevotella* infection, primary amoebic meningoencephalitis, progressive multifocal leukoencephalopathy, psittacosis, Q fever, rabies, relapsing fever, respiratory syncytial virus infection, rhinosporidiosis, rhinovirus infection, rickettsial infection, rickettsialpox, Rift Valley fever, Rocky Mountain spotted fever, rotavirus infection, rubella, salmonellosis, scabies, scarlet fever, schistosomiasis, sepsis, severe acute respiratory syndrome (also named SARS, including SARS-CoV-2), shigellosis, shingles, smallpox (also named variola), sporotrichosis, staphylococcal food poisoning, staphylococcal infection, strongyloidiasis, subacute sclerosing panencephalitis, syphilis, taeniasis, tetanus, tinea barbae, tinea blanca, tinea capitis, tinea corporis, tinea cruris, tinea manum, tinea nigra, tinea pedis, tinea unguium, tinea versicolor, toxocariasis, toxoplasmosis, trachoma, trichinosis, trichomoniasis, trichuriasis, tuberculosis, tularemia, typhoid fever, typhus fever, *Ureaplasma urealyticum* infection, Valley fever, Venezuelan equine encephalitis, Venezuelan hemorrhagic fever, *Vibrio vulnificus* infection, *Vibrio parahaemolyticus* enteritis, viral pneumonia, West Nile fever, *Yersinia pseudotuberculosis* infection, yersiniosis, yellow fever, zeaspora, zika fever, and zygomycosis.

In one embodiment, treating infectious diseases in a subject in need thereof further comprises administering to said subject, either before, concomitantly, or after, at least one additional therapeutic agent as defined hereinabove.

The present invention also relates to a method of stimulating an immune response in a subject in need thereof, comprising or consisting of administering to said subject a modified LPS molecular species, a composition, a pharmaceutical composition, a medicament or a vaccine composition according to the present invention.

The present invention also relates to a modified LPS molecular species, a composition, a pharmaceutical composition, a medicament or a vaccine composition according to the present invention, for use in stimulating an immune response in a subject in need thereof.

The present invention also relates to a use of a modified LPS molecular species, a composition, a pharmaceutical composition, a medicament or a vaccine composition according to the present invention as an immunostimulant.

In one embodiment, stimulating an immune response in a subject in need thereof further comprises administering to said subject, either before, concomitantly, or after, at least one additional therapeutic agent as defined hereinabove. In a preferred embodiment, the at least one additional therapeutic agent is an antigen, as defined hereinabove.

In one embodiment where the antigen is a self-antigen (such as, *e.g.,* a cellular antigen including cells containing normal transplantation antigens and/or tumor-related antigens, a RR-Rh antigen, or an antigen characteristic of, or specific to, particular cells or tissues or body fluids), stimulating an immune response in a subject in need thereof does not comprise administering to said subject at least one additional antigen. Alternatively, where the antigen is a self-antigen, stimulating an immune response in a subject in need thereof may comprise administering to said subject, either before, concomitantly, or after, at least one additional antigen, being either the same self-antigen or another antigen.

The present invention also relates to a method of vaccinating a subject in need thereof, comprising or consisting of administering to said subject a modified LPS molecular species, a composition, a pharmaceutical composition, a medicament or a vaccine composition according to the present invention.

The present invention also relates to a modified LPS molecular species, a composition, a pharmaceutical composition, a medicament or a vaccine composition according to the present invention, for use in vaccinating a subject in need thereof.

In one embodiment, vaccinating a subject in need thereof further comprises administering to said subject at least one additional therapeutic agent as defined hereinabove. In a preferred embodiment, the at least one additional therapeutic agent is an antigen, as defined hereinabove.

In one embodiment where the antigen is a self-antigen (such as, *e.g.,* a cellular antigen including cells containing normal transplantation antigens and/or tumor-related antigens, a RR-Rh antigen, or an antigen characteristic of, or specific to, particular cells or tissues or body fluids, including neoantigens), vaccinating a subject in need thereof does not comprise administering to said subject at least one additional antigen. Alternatively, where the antigen is a self-antigen, vaccinating a subject in need thereof may comprise administering to said subject, either before, concomitantly, or after, at least one additional antigen, being either the same self-antigen or another antigen.

It is readily understood that the therapeutic, vaccinal and immunostimulatory purposes disclosed hereinabove apply *mutatis mutandis* to subjects in need thereof being animals other than humans. Hence, the modified LPS molecular species, as well as the composition, pharmaceutical composition, medicament or vaccine composition according to the present invention, optionally formulated with at least one additional therapeutic agent, either unconjugated and/or conjugated to the LPS molecular species, are useful for a wide range of therapeutic, vaccinal and immunostimulatory purposes in the veterinary field.

As explained hereinabove, the one skilled in the art will readily recognize that the LPS molecular species should be purify to different degrees of purity depending on the intended use, and it is conceivable, in the case of veterinary applications, that the LPS molecular species be purified to a lesser extent, or even that the LPS molecular species be not purified. In this case, naturally-occurring LPS molecular species and/or modified LPS molecular species can be directly

formulated from a bacterial pellet, a culture concentrate, a culture supernatant, an enriched membrane fraction, and/or an outer membrane vesicle (OMV) fraction, with no or only few purification steps.

In one embodiment, the compositions comprising at least one LPS molecular species, useful for veterinary applications, may be the same as described hereinabove; or alternatively may comprise a mix of modified LPS molecular species selected from the group comprising or consisting of the LPS molecular species of Formula (XII)(1), and a combination thereof.

In one embodiment, the subject in need thereof is an animal. Examples of animals include, but are not limited to, mammals, birds, reptiles, amphibians, fishes, insects and mollusks.

In one embodiment, the subject in need thereof is a non-human animal, including, but not limited to, a farm animal - or an animal of agricultural value (such as, *e.g.,* cattle, cows, bison, pigs, swine, sheep, goats, horses, donkeys, alpacas, llamas, deer, elks, moose, ostriches, emus, ducks, geese, chickens, partridges, quails, pheasants, minks, salmons, codfishes, catfishes, herrings, trout, basses, perches, flounders, sharks, tuna fishes, cancers, lobsters, crayfishes, snails, clams, oysters, and the like), a companion animal (such as, *e.g.,* dog, cats, rabbits, rodents, fishes, snakes and the like), and a non-human primate (such as, *e.g.,* great apes including chimpanzees, gorillas, and orangutans; lesser apes, including gibbons; Old World monkeys; New World monkeys; and prosimians, including tarsiers, lemurs, and lorises).

In one embodiment, the subject in need thereof is a human.

In one embodiment, the subject in need thereof is an adult (*e.g.,* a subject above the age of 18 in human years or a subject after reproductive capacity has been attained). In one embodiment, the subject in need thereof is a child (*e.g.,* a subject below the age of 18 in human years or a subject before reproductive capacity has been attained).

In one embodiment, the subject in need thereof is a male. In one embodiment, the subject in need thereof is a female.

In one embodiment, the subject in need thereof is/was diagnosed with a cancer, an inflammatory disease, and/or an infectious disease. In one embodiment, the subject in need thereof is at risk of developing a cancer, an inflammatory disease, and/or an infectious disease.

The present invention relates to a kit-of-parts, comprising:

i. a modified LPS molecular species, a composition, a pharmaceutical composition, a medicament or a vaccine composition according to the present invention; and
ii. an additional therapeutic agent.

The term **"kit-of-parts",** also termed **"package", "commercial package",** or **"pharmaceutical package",** shall encompass an entity of physically separated components, which are intended for individual storage and/or use, but in functional relation to each other. In particular, the term **"kit-of-parts"** encompasses an entity of physically separated components (such as, *e.g.,* a modified LPS molecular species, a composition, a pharmaceutical composition, a medicament or a vaccine composition according to the present invention on the one hand; and an additional therapeutic agent on the other hand) which are to be used or administered separately, in any order, in a given time interval, typically but not necessarily ranging from hours to days, weeks or more; or concomitantly, either as an extemporaneous formulation or individually, in any order, within a time interval ranging from seconds to minutes at most.

Suitable examples of additional therapeutic agents include, but are not limited to, chemotherapeutic agents, targeted therapy agents, cytotoxic agents, antibiotics, antivirals, cell cycle-synchronizing agents, ligands for cellular receptor(s), immunomodulatory agents, pro-apoptotic agents, anti-angiogenic agents, cytokines, growth factors, antibodies or antigen-binding fragments thereof, cell therapy, antigens, and the like.

Detailed examples of these additional therapeutic agents have been described hereinabove as suitable agents for conjugation at positions Z, $R_1$ and/or $R_2$ of **Formula A** and **Formula B.** However, the one skilled in the art will readily understand that these additional therapeutic agents may additionally or alternatively be provided as unconjugated to the kit-of-parts according to the present invention.

In one embodiment, the additional therapeutic agent is a targeted therapy agent as defined hereinabove, preferably said additional therapeutic agent is an anti-CD20 monoclonal antibody or an antigen-binding fragment thereof. In one embodiment, said anti-CD20 monoclonal antibody is selected from the group comprising or consisting of ibritumomab, obinutuzumab, ocrelizumab, ofatumumab, rituximab, tositumomab, or an antigen-binding fragment thereof; preferably said anti-CD20 monoclonal antibody is rituximab.

In one embodiment, the additional therapeutic agent is an immunostimulatory agent as defined hereinabove, preferably said additional therapeutic agent is an immune checkpoint inhibitor. In one embodiment, said immune checkpoint inhibitor is selected from the group comprising or consisting of inhibitors of CTLA4, PD-1, PD-L1, LAG-3, B7-H3, B7-H4, TIM3, A2AR, GITR, CD47, TNFR2, CD19, and/or IDO. In one embodiment, said immune checkpoint inhibitor is selected from the group comprising or consisting of nivolumab, pembrolizumab, cemiplimab, pidilizumab, AMP-224, AMP-514, MPDL3280A, MDX-1105, MEDI-4736, arelumab, ipilimumab, tremelimumab, pidilizumab, IMP321, MGA271, BMS-986016, lirilumab, urelumab, PF-05082566, IPH2101, MEDI-6469, CP-870,893, mogamulizumab,

varlilumab, avelumab, galiximab, AUNP 12, indoximod, NLG-919, and INCB024360. In one embodiment, said immune checkpoint inhibitor is an inhibitor of PD-1 or PD-L1. Examples of PD-1 inhibitors include, but are not limited to, nivolumab, pembrolizumab, cemiplimab, spartalizumab, camrelizumab, sintilimab, tislelizumab, toripalimab, AMP-224, and AMP-514. Examples of PD-L1 inhibitors include, but are not limited to, atezolizumab, avelumab, durvalumab, KN035, CK-301, AUNP12, CA-170, and BMS-986189.

In one embodiment, the additional therapeutic agent is an antigen, as described hereinabove.

Disclosed herein is a modified lipopolysaccharide (LPS) molecular species, wherein said LPS molecular species:

- is devoid of phosphate group at position C 1 of the reducing end of its lipid A domain and/or at position C4' of the non-reducing end of its lipid A domain; and
- is substituted at position C6' of the non-reducing end of its lipid A domain by a hydrophilic moiety, with the proviso that said hydrophilic moiety is not a hydroxyl group; preferably is substituted at position C6' of the non-reducing end of its lipid A domain by a mono-, oligo- or poly-saccharide moiety.

In one embodiment, said modified LPS molecular species is of general Formula B

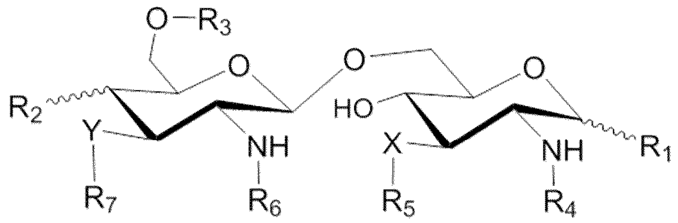

**Formula B**

wherein:

- X and Y are, independently from each other, -O- or -NH-;
- R1 is a hydroxyl group (-OH), optionally substituted, with the proviso that, if substituted, the substitution does not consist of a phosphate group (-OP03H2); and R2 is a phosphate group (-OPO3H2) or a hydroxyl group (-OH); optionally substituted; or
  R1 is a phosphate group (-OPO3H2) or a hydroxyl group (-OH); optionally substituted; and R2 is a hydroxyl group (-OH), optionally substituted, with the proviso that, if substituted, the substitution does not consist of a phosphate group (-OPO3H2);
- R3 is a mono-, oligo- or poly-saccharide moiety; optionally substituted; preferably R3 is a core oligosaccharide optionally substituted with an antigen domain, preferably an O-antigen domain and/or a capping antigen;
- R4 and R6 are, independently from each other, a fatty acid residue; optionally substituted; and
- R5 and R7 are, independently from each other, a hydrogen or a fatty acid residue; optionally substituted.

In one embodiment, said modified LPS molecular species is selected from the group comprising LPS molecular species of Formulas (I)(1)(a), (I)(1)(b), (I)(1)(c), (I)(1)(d), (I)(1)(e), (I)(1)(f), (I)(2)(a), (I)(2)(b), (I)(2)(c), (I)(3)(a), (I)(3)(b), (I)(3)(c), (II)(2)(a), (II)(2)(b), (II)(2)(c), (II)(2)(d), (III)(1)(a), (III)(1)(b), (III)(1)(c), (III)(1)(d), (IV)(1)(a), (IV)(1)(b), (IV)(1)(c), (V)(1)(a), (V)(1)(b), (V)(1)(c), (V)(1)(d), (V)(1)(e), (V)(1)(f), (V)(1)(g), (V)(1)(h), (V)(1)(i), (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), (VI)(2)(a), (VI)(2)(b), (VI)(2)(c), (VII)(1)(a), (VII)(1)(b), (VII)(1)(c), (VIII)(1)(a), (VIII)(1)(b), (VIII)(1)(c), (VIII)(1)(d), (IX)(1)(a), (IX)(1)(b), (IX)(1)(c), (IX)(1)(d), (X)(1)(a), (X)(1)(b), (X)(1)(c), (X)(1)(d), (XI)(1)(a), (XI)(1)(b), (XI)(1)(c), (XI)(1)(d), (XII)(1) and a combination thereof as shown in Figures 2-4, 7-17 and 27, wherein R at position C6' is a mono-, oligo- or polysaccharidic moiety comprising a core oligosaccharide domain and optionally, an antigen domain, preferably an O-antigen domain and/or a capping antigen.

Also disclosed herein is an enriched population of modified LPS molecular species, wherein said enriched population of LPS molecular species comprises at least 10 % of modified LPS molecular species according to the present invention; preferably at least 50% of modified LPS molecular species according to the present invention.

In one embodiment, said enriched population of modified LPS molecular species forms particles having an average size smaller than about 200 nm, preferably smaller than about 150 nm in diameter in aqueous solutions, as can be assessed by dynamic light scattering (DLS) at 25°C.

Also disclosed herein is a composition comprising at least one modified LPS molecular species according to the present invention; or an enriched population of modified LPS molecular species according to the present invention.

In one embodiment, said composition comprises:

- a mix of at least two modified LPS molecular species of Formulas (I)(1)(a), (I)(1)(b), (I)(1)(c), (I)(1)(d), (I)(1)(e) and (1)(1)(f); preferably a mix of the six modified LPS molecular species; or
- a mix of at least two modified LPS molecular species of Formulas (I)(2)(a), (I)(2)(b) and (I)(2)(c); preferably a mix of the three modified LPS molecular species; or
- a mix of at least two modified LPS molecular species of Formulas (I)(3)(a), (I)(3)(b) and (I)(3)(c); preferably a mix of the three modified LPS molecular species; or
- a mix of at least two modified LPS molecular species of Formulas (II)(2)(a), (II)(2)(b), (II)(2)(c), (II)(2)(d); preferably a mix of the four modified LPS molecular species; or
- a mix of at least two modified LPS molecular species of Formulas (III)(1)(a), (III)(1)(b), (III)(1)(c), (III)(1)(d); preferably a mix of the four modified LPS molecular species; or
- a mix of at least two modified LPS molecular species of Formulas (IV)(1)(a), (IV)(1)(b), (IV)(1)(c); preferably a mix of the three modified LPS molecular species; or
- a mix of at least two modified LPS molecular species of Formulas (V)(1)(a), (V)(1)(b), (V)(1)(c), (V)(1)(d), (V)(1)(e), (V)(1)(f), (V)(1)(g), (V)(1)(h), (V)(1)(i); preferably a mix of the nine modified LPS molecular species; or
- a mix of at least two modified LPS molecular species of Formulas (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j); preferably a mix of the ten modified LPS molecular species; or
- a mix of at least two modified LPS molecular species of Formulas (VI)(2)(a), (VI)(2)(b), (VI)(2)(c); preferably a mix of the three modified LPS molecular species; or
- a mix of at least two modified LPS molecular species of Formulas (VII)(1)(a), (VII)(1)(b), (VII)(1)(c); preferably a mix of the three modified LPS molecular species; or
- a mix of at least two modified LPS molecular species of Formulas (VIII)(1)(a), (VIII)(1)(b), (VIII)(1)(c), (VIII)(1)(d); preferably a mix of the four modified LPS molecular species; or
- a mix of at least two modified LPS molecular species of Formulas (IX)(1)(a), (IX)(1)(b), (IX)(1)(c), (IX)(1)(d); preferably a mix of the four modified LPS molecular species; or
- a mix of at least two modified LPS molecular species of Formulas (X)(1)(a), (X)(1)(b), (X)(1)(c), (X)(1)(d); preferably a mix of the four modified LPS molecular species;
- a mix of at least two modified LPS molecular species of Formulas (XI)(1)(a), (XI)(1)(b), (XI)(1)(c), (XI)(1)(d); preferably a mix of the four modified LPS molecular species; or
- a mix of modified LPS molecular species of Formula (XII)(1).

In one embodiment, the composition is a pharmaceutical composition or a vaccine composition and further comprises at least one pharmaceutically acceptable excipient.

Also disclosed herein is a naturally-occurring non-toxic LPS molecular species and/or a modified LPS molecular species, or a composition comprising the same:

- for use in treating and/or preventing cancer in a subject in need thereof,
- for use in treating and/or preventing an inflammatory disease in a subject in need thereof,
- for use in treating and/or preventing an infectious disease in a subject in need thereof,
- for use in stimulating an immune response in a subject in need thereof, and/or
- for use in vaccinating a subject in need thereof,

wherein said naturally-occurring non-toxic LPS molecular species and/or modified LPS molecular species, or composition comprising the same is selected from:

- the modified LPS molecular species according to the present invention; the enriched population of modified LPS molecular species according to the present invention; or the composition according to the present invention; or
- a LPS molecular species which does not comprise two secondary $C_{12}$ and/or $C_{14}$ fatty acid chains; and/or which does not comprise a phosphate group at both position C1 of the reducing end of its lipid A domain and position C4' of the non-reducing end of its lipid A domain.

In one embodiment, said LPS molecular species or composition is selected from:

- a LPS molecular species selected from the group comprising LPS molecular species of Formulas (I)(0)(a), (I)(0)(b), (II)(0), (II)(1)(a), (II)(1)(b), (II)(1)(c), (II)(1)(d) as shown in Figures 1 to 6;
- a mix of LPS molecular species of Formulas (I)(0)(a) and (I)(0)(b) as shown in Figure 1;
- a mix of at least two LPS molecular species of Formulas (II)(0), as shown in Figure 5;

- a mix of at least two LPS molecular species of Formulas (II)(1)(a), (II)(1)(b), (II)(1)(c), (II)(1)(d), as shown in Figure 6;
- the composition according to the present invention comprising a mix of modified LPS molecular species of Formulas (I)(1)(a), (I)(1)(b), (I)(1)(c), (I)(1)(d), (I)(1)(e) and (I)(1)(f);
- the composition according to the present invention comprising a mix of modified LPS molecular species of Formulas (I)(2)(a), (I)(2)(b) and (I)(2)(c); or
- the composition according to the present invention comprising a mix of modified LPS molecular species of Formulas (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j).

In one embodiment, treating and/or preventing cancer, an inflammatory disease or an infectious disease further comprises administering to said subject at least one additional therapeutic agent, preferably selected from the group comprising chemotherapeutic agents, targeted therapy agents, cytotoxic agents, antibiotics, antivirals, cell cycle-synchronizing agents, ligands for cellular receptor(s), immunomodulatory agents, pro-apoptotic agents, anti-angiogenic agents, cytokines, growth factors, antibodies or antigen-binding fragments thereof, cell therapy, and antigens.
In one embodiment, the at least one additional therapeutic agent is a targeted therapy agent, preferably an anti-CD20 antibody or an antigen-binding fragment thereof.
In one embodiment, the at least one additional therapeutic agent is an immunostimulatory agent, preferably an immune checkpoint inhibitor, more preferably selected from the group consisting of CTLA4, PD-1, PD-L1, LAG-3, B7-H3, B7-H4, TIM3, A2AR, GITR, CD47, TNFR2, CD19, and IDO inhibitors; most preferably the at least one additional therapeutic agent is a PD-1 or PD-L1 inhibitor.
In one embodiment, vaccinating a subject in need thereof further comprises administering to said subject at least one antigen, preferably selected from the group consisting of a pathogen-related antigen, a self-antigen, an allergen-related antigen and a neoantigen.
In one embodiment, the subject in need thereof is an animal other than a human and said naturally-occurring non-toxic LPS molecular species and/or modified LPS molecular species or composition comprising the same is selected from a LPS molecular species of Formula (XII)(1) as shown in Figure 27.
Also disclosed herein is a kit-of-parts comprising:

- a modified LPS molecular species according to the present invention; an enriched population of modified LPS molecular species according to the present invention; a composition according to the present invention; or a LPS molecular species which does not comprise two secondary $C_{12}$ and/or $C_{14}$ fatty acid chains and/or which does not comprise a phosphate group at both position C1 of the reducing end of its lipid A domain and position C4' of the non-reducing end of its lipid A domain; and
- an additional therapeutic agent, preferably selected from the group comprising chemotherapeutic agents, targeted therapy agents, cytotoxic agents, antibiotics, antivirals, cell cycle-synchronizing agents, ligands for cellular receptor(s), immunomodulatory agents, pro-apoptotic agents, anti-angiogenic agents, cytokines, growth factors, antibodies or antigen-binding fragments thereof, cell therapy, and antigens.

## BRIEF DESCRIPTION OF THE DRAWINGS

**Figure 1** shows the structure of two naturally-occurring LPS molecular species **"LPS $B_2$ 0.0"** from *Bordetella pertussis* with Formulas (I)(0)(a) and (I)(0)(b). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen.

**Figure 2** shows the structure of six chemically-modified LPS molecular species **"LPS $B_2$ 0.1"** from *Bordetella pertussis* with Formulas (I)(1)(a) to (I)(1)(f). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen.

**Figure 3** shows the structure of three chemically-modified LPS molecular species **"LPS $B_2$ 1.1"** from *Bordetella pertussis* with Formulas (I)(2)(a) to (I)(2)(c). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen.

**Figure 4** shows the structure of three chemically-modified LPS molecular species **"LPS $B_2$ 1.2"** from *Bordetella pertussis* with Formulas (I)(3)(a) to (I)(3)(c). The disaccharide of the lipid A domain is substituted at position C6' of the

non-reducing end by R, R being a mono-, oligo- or polysaccharidic moiety comprising a core oligosaccharide domain and optionally, an antigen domain, such as an O-antigen domain and/or a capping antigen.

**Figure 5** shows the structure of a naturally-occurring LPS molecular species **"LPS $W_1$ 0.0"** from *Vitreoscilla filiformis* with Formula (II)(0). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. Phosphoryl ethanolamine (PEA) residues shown in parenthesis at positions C1 of the reducing end and C4' of the non-reducing end of the lipid A domain are either present or absent.

**Figure 6** shows the structure of four chemically-modified LPS molecular species **"LPS $W_1$ 1.0"** from *Vitreoscilla filiformis* with Formulas (II)(1)(a) to (II)(1)(d). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen.

**Figure 7** shows the structure of four chemically-modified LPS molecular species **"LPS $W_1$ 1.2"** from *Vitreoscilla filiformis* with Formulas (II)(2)(a) to (II)(2)(d). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen.

**Figure 8** shows the structure of four chemically-modified LPS molecular species **"LPS $SM_1$ 1.2"** from *Salmonella enterica enterica* serovar *Minnesota* R595 with Formulas (III)(1)(a) to (III)(1)(d). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The phosphate group shown in parenthesis at position C4' of the non-reducing end of the lipid A domain is either present or absent.

**Figure 9** shows the structure of three chemically-modified LPS molecular species **"LPS $ST_1$ 1.2"** from *Salmonella enterica enterica* serovar *Typhimurium* Ra or **"LPS $E_2$ 1.2"** from *Escherichia coli* K12 or **"LPS $V_1$ 1.2"** from *Vibrio fischeri* (all three have identical lipid A domains) with Formulas (IV)(1)(a) to (IV)(1)(c). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen.

**Figure 10** shows the structure of nine chemically-modified LPS molecular species **"LPS $E_1$ 0.1"** from *Escherichia coli* J5 with Formulas (V)(1)(a) to (V)(1)(i). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen.

**Figure 11** shows the structure of ten chemically-modified LPS molecular species **"LPS $A_1$ 0.1"** from *Actinobacillus pleuropneumoniae* with Formulas (VI)(1)(a) to (VI)(1)(j). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen.

**Figure 12** shows the structure of three chemically-modified LPS molecular species **"LPS $A_1$ 1.2"** from *Actinobacillus pleuropneumoniae* with Formulas (VI)(2)(a) to (VI)(2)(c). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen.

**Figure** 13 shows the structure of three chemically-modified LPS molecular species **"LPS $K_1$ 1.2"** from *Burkholderia pseudomallei* with Formulas (VII)(1)(a) to (VII)(1)(c). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The phosphate group shown in parenthesis at position C4' of the non-reducing end of the lipid A domain is either present or absent.

**Figure 14** shows the structure of four chemically-modified LPS molecular species **"LPS $M_1$ 1.2"** from *Moraxella catarrhalis* with Formulas (VIII)(1)(a) to (VIII)(1)(d). The disaccharide of the lipid A domain is substituted at position C6' of the non-reducing end by R, R being a mono-, oligo- or polysaccharidic moiety comprising a core oligosaccharide

domain and optionally, an antigen domain, such as an O-antigen domain and/or a capping antigen.

**Figure 15** shows the structure of four chemically-modified LPS molecular species **"LPS $N_1$ 1.2"** from *Neisseria sicca* with Formulas (IX)(1)(a) to (IX)(1)(d). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen.

**Figure 16** shows the structure of four chemically-modified LPS molecular species **"LPS $P_1$ 1.2"** from *Pseudomonas aeruginosa* or **"LPS $P_2$ 1.2"** from *Pseudomonas fluorescens* (both have identical lipid A domains) with Formulas (X)(1)(a) to (X)(1)(d). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The phosphate group shown in parenthesis at position C4' of the non-reducing end of the lipid A domain, as well as the hydroxyl group at C2 of the secondary fatty acid are either present or absent.

**Figure 17** shows the structure of four chemically-modified LPS molecular species **"LPS $I_1$ 1.2"** from *Bordetella hinzii* or **"LPS $T_1$ 1.2"** from *Bordetella trematum* (both have identical lipid A domains) with Formulas (XI)(1)(a) to (XI)(1)(d). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The phosphate group shown in parenthesis at position C4' of the non-reducing end of the lipid A domain, as well as the hydroxyl group at C2 of the secondary fatty acid are either present or absent.

**Figures 18-A-D** are a set of four graphs showing DLS measurements of LPS $B_2$ 0.0, LPS $B_2$ 0.1, LPS $B_2$ 1.1 and of synthetic GLA (PHAD®, from Avanti® Polar Lipids, Inc.) in water [$H_2O$]. Triplicate measurements are shown for each suspension. Mean particle size (in nm) is indicated for each measurement, and the average value for the three mean particle size value is indicated in brackets.

**Figure 18A:** triplicate DLS measurement of LPS $B_2$ 0.0 in water.
**Figure 18B:** triplicate DLS measurement of LPS $B_2$ 0.1 in water.
**Figure 18C:** triplicate DLS measurement of LPS $B_2$ 1.1 in water.
**Figure 18D:** triplicate DLS measurement of GLA in water.

**Figures 19A**-D are a set of four graphs showing DLS measurements of LPS $B_2$ 0.0, LPS $B_2$ 0.1, LPS $B_2$ 1.1 and of synthetic GLA (PHAD®, from Avanti® Polar Lipids, Inc.) in PBS [PBS] suspensions. Triplicate measurements are shown for each suspension. Mean particle size (in nm) is indicated for each measurement, and the average value for the three mean particle size value is indicated in brackets.

**Figure 19A:** triplicate DLS measurement of LPS $B_2$ 0.0 in PBS suspension.
**Figure 19B:** triplicate DLS measurement of LPS $B_2$ 0.1 in PBS suspension.
**Figure 19C:** triplicate DLS measurement of LPS $B_2$ 1.1 in PBS suspension.
**Figure 19D:** triplicate DLS measurement of GLA in PBS suspension.

**Figure 20** is a graph showing the average particle size (in nm) of LPS $B_2$ 0.0, LPS $B_2$ 0.1, LPS $B_2$ 1.1 and of synthetic GLA (PHAD®, from Avanti® Polar Lipids, Inc.) in water [$H_2O$] and PBS [PBS] suspensions.

**Figure 21** is a set of four MALDI spectra of MPL and the *Salmonella enterica* and *Escherichia coli* candidates (LPS $SM_1$ 1.2, LPS $ST_1$ 1.2 and LPS $E_1$ 1.2), presenting the signal intensity on the *y*-axis *versus* mass-to-charge ratio (m/z) on the x-axix. MPL (monophosphoryl lipid A) is a natural LPS derivative from *Salmonella enterica enterica* serovar *Minnesota* R595, from which the phosphate group at position C1 of the reducing end of its lipid A domain has been cleaved by acidic hydrolysis together with the core part of the LPS molecular species. Unlike the modified LPS molecular species of the present invention, it is therefore also devoid of core oligosaccharide domain at position C6' of the non-reducing end of its lipid A domain. This sample is giving peaks in the lower mass region of the mass spectrum with molecular species representing only the lipid A moiety.

**Figure 22** is a set of four MALDI spectra of the *Bordetella pertussis* candidates (LPS $B_2$ 0.0, LPS $B_2$ 0.1, LPS $B_2$ 1.1 and LPS $B_2$ 1.2), presenting the signal intensity on the *y*-axis *versus* mass-to-charge ratio (m/z) on the x-axis.

**Figure 23** is a set of three MALDI spectra of the *Vitreoscilla filiformis* candidates (LPS $W_1$ 0.0, LPS $W_1$ 1.0 and LPS $W_1$ 1.2), presenting the signal intensity on the *y*-axis *versus* mass-to-charge ratio (m/z) on the x-axis.

**Figures 24A**-B are a set of two graphs showing the therapeutic efficacy of native (LPS $B_2$ 0.0 and LPS $W_1$ 0.0) LPS molecular species, alone or in combination with rituximab, in CB17-SCID mice engrafted with an established human B-lymphoma tumor model (RL cells) and subsequently immunized.

**Figure 24A:** follow-up of tumor volume in cohorts of CB17-SCID mice engrafted with RL cells and subsequently immunized with native (LPS $B_2$ 0.0 or LPS $W_1$ 0.0) LPS, alone or in combination with rituximab ["+ Ritux."]. Groups of mice treated with GLA administered intravenously (not shown) or intraperitoneally, alone [GLA] or in combination with rituximab [GLA + Ritux.], were included for comparison. Tumor bearing mice injected with rituximab [Rituximab] or PBS alone [Control] were used as controls. Data shown are median ± SEM (n = 6 mice/group) tumor volumes ($mm^3$) over time for all mice within a cohort of one experiment. * $P < 0.05$; ** $P < 0.01$; ns: not significant.

**Figure 24B:** follow-up of tumor volume in each group of mice, expressed as the percentage of complete tumor regression at D54 post-tumor challenge.

**Figures 25A-B** are a set of two graphs showing the therapeutic efficacy of chemically-modified (LPS $B_2$ 0.1 and LPS $B_2$ 1.1) LPS, alone or in combination with rituximab, in CB17-SCID mice engrafted with an established human B-lymphoma tumor model (RL cells) and subsequently immunized. PBS is used as negative control.

**Figure 25A:** follow-up of tumor volume in cohorts of CB17-SCID mice engrafted with RL cells and subsequently immunized with chemically-modified (LPS $B_2$ 0.1 or LPS $B_2$ 1.1) LPS, alone or in combination with rituximab ["+ Ritux."]. Groups of mice treated with GLA administered intravenously (not shown) or intraperitoneally, alone [GLA] or in combination with rituximab [GLA + Ritux.], were included for comparison. Tumor bearing mice injected with rituximab [Rituximab] or PBS alone [Control] were used as controls. Data shown are median +/- SEM (n = 6 mice/group) tumor volumes ($mm^3$) over time for all mice within a cohort of one experiment. * $P < 0.05$; ** $P < 0.01$; ns: not significant.

**Figure 25B:** follow-up of tumor volume in each group of mice, expressed as the percentage of complete tumor regression at D54 post-tumor challenge.

**Figures 26A-B** illustrates the capacity of the LPS $A_1$ 0.1 and LPS $B_2$ 0.1 to boost the humoral immune response to OVA in a preclinical mouse vaccination model.

**Figure 26A:** Mean (± SEM) of serum anti-OVA IgG antibody levels (μg/mL) at days 28 (black) and 39 (dark grey) in groups of mice (n = 5) immunized with OVA (10 μg/dose) formulated without adjuvant [No Adjuvant], or with either of Alum [Alum], MPLA [MPLA] or LPS $A_1$ 0.1 [LPS A0.1]. Statistical significance compared to the negative control [No Adjuvant] was assessed using unpaired Student's t-test with CI = 95%; *(Black/Grey) $P < 0.05$; ** (Black/Grey) $P < 0.01$; ***(Black) $P < 0.001$.

**Figure 26B:** Mean (± SEM) of serum anti-OVA IgG antibody levels (μg/mL) at days 28 (black) and 39 (dark grey) in groups of mice (n = 5) immunized with OVA (2 μg/dose) formulated without adjuvant [No Adjuvant], or with LPS $B_2$ 0.1 [LPS B0.1]. Statistical significance compared to the negative control [No Adjuvant] was assessed using unpaired Student's t-test with CI = 95%; *(Black) $P < 0.05$; **(Black) $P < 0.01$; ***(Black) $P < 0.001$.

**Figure 27** shows the structure of two LPS molecular species from *Salmonella enterica enterica* serovar *Minnesota* R595 (Re chemotype). Left: naturally-occurring LPS molecular species **"LPS $SM_1$ 0.0"** with Formula (XII)(0); right: chemically-modified LPS molecular species **"LPS $SM_1$ V1".** The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The 4-amino-4-deoxy-L-arabinose group (Ara4pN) or phosphate group shown in parenthesis at position C4' of the non-reducing end of the lipid A domain is either present or absent.

**Figure 28** shows the structure of three chemically-modified LPS molecular species **"LPS $B_2$ 1.0"** from *Bordetella pertussis* with Formulas (I)(4)(a) to (I)(4)(c). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The phosphate group at position C4' of the non-reducing end in parenthesis indicates absence or presence.

**Figure 29** shows the structure of two chemically-modified LPS molecular species **"LPS $B_2$ 0.2"** from *Bordetella pertussis* with Formulas (I)(5)(a) and (I)(5)(b). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen.

**Figure 30** shows the structure of two chemically-modified LPS molecular species **"LPS $B_2$ 0.3"** from *Bordetella pertussis* with Formulas (I)(6)(a) and (I)(6)(b). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The hydroxyl group at position C1 of the reducing end is substituted by X, X being an ethyl moiety.

**Figure 31** shows the structure of four chemically-modified LPS molecular species **"LPS $B_2$ 0.4"** from *Bordetella pertussis* with Formulas (I)(7)(a) to (I)(7)(d). The disaccharide (or the single glucosamine) of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The phosphate group at position C4' of the non-reducing end in parenthesis indicates absence or presence.

**Figure 32** shows the structure of three chemically-modified LPS molecular species **"LPS $B_2$ 1.3"** from *Bordetella pertussis* with Formulas (I)(8)(a) to (I)(8)(c). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The hydroxyl group at position C1 of the reducing end is substituted by X, X being an ethyl moiety. The phosphate group at position C4' of the non-reducing end in parenthesis indicates absence or presence.

**Figure 33** shows the structure of six chemically-modified LPS molecular species **"LPS $B_2$ 1.4"** from *Bordetella pertussis* with Formulas (I)(9)(a) to (I)(9)(f). The disaccharide (or the single glucosamine) of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The phosphate group at position C4' of the non-reducing end in parenthesis indicates absence or presence.

**Figure 34** shows the structure of two naturally-occurring LPS molecular species **"LPS X 0.0"** from various Enterobacteria with Formulas (XIII)(0)(a) and (XIII)(0)(b). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The glucosamine in parenthesis, substituting the phosphate group at position C4' of the non-reducing end, indicates absence or presence.

**Figure 35** shows the structure of six chemically-modified LPS molecular species **"LPS X 1.0"** from various Enterobacteria with Formulas (XIII)(1)(a) to (XIII)(1)(f). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The phosphate group at position C4' of the non-reducing end in parenthesis indicates absence or presence.

**Figure 36** shows the structure of two chemically-modified LPS molecular species **"LPS X 0.1"** from various Enterobacteria with Formulas (XIII)(2)(a) and (XIII)(2)(b). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen.

**Figure 37** shows the structure of two chemically-modified LPS molecular species **"LPS X 0.2"** from various Enterobacteria with Formulas (XIII)(3)(a) and (XIII)(3)(b). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen.

**Figure 38** shows the structure of two chemically-modified LPS molecular species **"LPS X 0.3"** from various Enterobacteria with Formulas (XIII)(4)(a) and (XIII)(4)(b). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The hydroxyl group at position C1 of the reducing end is substituted by X, X being an ethyl moiety.

**Figure 39** shows the structure of four chemically-modified LPS molecular species **"LPS X 0.4"** from various Enterobacteria with Formulas (XIII)(5)(a) to (XIII)(5)(d). The disaccharide (or the single glucosamine) of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The phosphate group at position C4' of the non-reducing end in parenthesis indicates absence or presence.

**Figure 40** shows the structure of six chemically-modified LPS molecular species **"LPS X 1.1"** from various Enterobacteria with Formulas (XIII)(6)(a) to (XIII)(6)(f). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The phosphate group at position C4' of the non-reducing end in parenthesis indicates absence or presence.

**Figure 41** shows the structure of six chemically-modified LPS molecular species **"LPS X 1.2"** from various Enterobacteria with Formulas (XIII)(7)(a) to (XIII)(7)(f). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The phosphate group at position C4' of the non-reducing end in parenthesis indicates absence or presence.

**Figure 42** shows the structure of six chemically-modified LPS molecular species **"LPS X 1.3"** from various Enterobacteria with Formulas (XIII)(8)(a) to (XIII)(8)(f). The disaccharide of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The hydroxyl group at position C1 of the reducing end is substituted by X, X being an ethyl moiety. The phosphate group at position C4' of the non-reducing end in parenthesis indicates absence or presence.

**Figure 43** shows the structure of ten chemically-modified LPS molecular species **"LPS X 1.4"** from various Enterobacteria with Formulas (XIII)(9)(a) to (XIII)(9)(j). The disaccharide (or the single glucosamine) of the lipid A domain is substituted at position C6' of the non-reducing end by R, R being a mono-, oligo- or polysaccharidic moiety comprising a core oligosaccharide domain and optionally, an antigen domain, such as an O-antigen domain and/or a capping antigen. The phosphate group at position C4' of the non-reducing end in parenthesis indicates absence or presence.

**Figure 44** is a set of four MALDI spectra of the *Bordetella pertussis* candidates (LPS $B_2$ 1.2, LPS $B_2$ 1.3, LPS $B_2$ 0.4 and LPS $B_2$ 1.4), presenting the signal intensity on the *y*-axis *versus* mass-to-charge ratio (m/z) on the x-axix.

**Figures 45A-B** illustrate the therapeutic efficacy of modified LPS $B_2$ 0.1, LPS $B_2$ 1.1 and LPS $B_2$ 1.3 as monotherapy against a syngeneic mouse model of colorectal carcinoma (MC38).

**Figure 45A:** mean tumor volume among groups of mice treated with modified LPS $B_2$ 0.1 [B0.1], LPS $B_2$ 1.1 [B1.1] and LPS $B_2$ 1.3 [B1.3], or administered with PBS [Control]. Data are presented as mean tumor volume ($mm^3$) ± SEM (n = 7 mice/group) over time. * $P < 0.05$; ** $P < 0.001$ (ANOVA test).
**Figure 45B:** comparison of tumor volumes on days 8, 11 and 13 post-tumor challenge. Data are presented as mean tumor volume ($mm^3$) ± SEM. Control vs LPS: * $P < 0.05$; ** $P < 0.01$; *** $P < 0.005$ (ANOVA test).

**Figures 46A-E** illustrate the therapeutic efficacy of a modified LPS $B_2$ 1.3 as monotherapy against an orthotopic rat model of osteosarcoma (Osa).

**Figure 46A:** tumor volume among groups of mice treated with modified LPS $B_2$ 1.3 [B1.3] or doxorubicin [Doxo] or administered with PBS [Control]. Data are presented as median tumor volume ($mm^3$) ± SEM (n = 3-8 mice/group) over time. * $P < 0.05$ (ANOVA test).
**Figure 46B:** tumor volume growth curves for individual mice in the control group.
**Figure 46C:** tumor volume growth curves for individual mice in the doxorubicin group
**Figure 46D:** tumor volume growth curves for individual mice in the LPS $B_2$ 1.3 group.
**Figure 46E:** percentages of survival among the group of rats treated with Doxorubicin [Doxo] or LPS $B_2$ 1.3 [B1.3] or administered with PBS [Control].

**Figure 47A-F** illustrate the synergistic antitumor effect of a modified LPS in combination with an immune checkpoint

inhibitor (ICI) on a syngeneic mouse model of colorectal carcinoma (MC38).

**Figure 47A:** tumor volume among groups of mice treated with LPS $B_2$ 1.3 [B1.3], anti-PD-1 mAb alone [αPD-1], a combination of both LPS $B_2$ 1.3 and anti-PD-1 mAb [B1.3 + αPD-1], or PBS [Control]. Data are presented as median tumor volume ($mm^3$) ± SEM (n = 7 mice/group) over time. * $P < 0.05$ (ANOVA test).
**Figure 47B:** tumor volume growth curves for individual mice in control group.
**Figure 47B:** tumor volume growth curves for individual mice in B1.3 group
**Figure 47B:** tumor volume growth curves for individual mice in αPD-1 group.
**Figure 47B:** tumor volume growth curves for individual mice in B1.3 + αPD-1.
**Figure 47F:** Kaplan-Meier survival curves for each group (same legend as **Figure 47A).**

**Figures 48A-B** illustrate the capacity of a modified LPS to boost the induction of memory immune responses in combination with an immune checkpoint inhibitor (ICI) on a syngeneic mouse model of colorectal carcinoma (MC38).

**Figure 48A:** mean tumor growth among anti-PD-1 mAb-treated mice [αPD-1], in combination or not with modified LPS $B_2$ 1.3 [B1.3] (n = 2 mice/group), rechallenged with MC38 cells (2nd challenge). Naive mice (n = 3) were used as control. Data are presented as means tumor volume ($mm^3$) ± SEM over the time.
**Figure 48B:** percentage survival on day 71.

**Figure 49A-D** illustrate the adjuvant activity of the modified LPS $B_2$ 0.1 in a preclinical mouse vaccination model against a SARS-CoV-2 (COVID-19 virus) spike receptor-binding domain.

**Figure 49A:** serum levels of total anti-RBD IgG antibody on days 18, 25 and 39 in the groups of mice immunized with a recombinant RBD protein alone or formulated with either LPS $B_2$ 0.1 or MPLA. Data are presented as mean total anti-RBD IgG antibody levels (absorbance at 450 nm) ± SEM.
**Figure 49B:** serum levels of anti-RBD IgG1 antibody on day 39. Data are presented as mean anti-RBD IgG1 antibody levels (absorbance at 450 nm) ± SEM.
**Figure 49C:** serum levels of anti-RBD IgG2a antibody on day 39. Data are presented as mean anti-RBD IgG1 antibody levels (absorbance at 450 nm) ± SEM.
**Figure 49D:** serum anti-RBD IgG2a/IgG1 ratios on day 39. Data are presented as mean anti-RBD IgG1 antibody ratios ± SEM. * $P < 0.05$, ** $P < 0.01$, *** $P < 0.001$ (ANOVA test).

## EXAMPLES

The present invention is further illustrated by the following examples.

## Example 1

### Production of chemically-modified LPS molecular species

In the following, the figures **"x.y"** following the LPS name refer to:

i. first figure **("x"):** where 0 means no alkaline treatment, and 1 means one alkaline treatment;
ii. second figure **("y"):** where 0 means no acidic hydrolysis treatment, 1 means one acidic hydrolysis treatment with a strong acid (*i.e.*, with $pK_a < 0$) in aqueous solution, 2 means one acidic hydrolysis treatment with a strong acid (*i.e.*, with $pK_a < 0$) in organic solution; 3 means one acidic hydrolysis treatment with a strong acid (*i.e.*, with $pK_a < 0$) in organic solution in presence of ethanol; and 4 means one acidic hydrolysis treatment with a weak acid (*i.e.*, with $pK_a > 0$) in aqueous solution.

### *Structures*

**"LPS $B_2$ 0.0",** as used herein, refers to a mix of the two naturally-occurring LPS molecular species from *Bordetella pertussis* with Formulas (I)(0)(a) and (I)(0)(b), as shown in **Fig. 1.**
**"LPS $W_1$ 0.0",** as used herein, refers to the naturally-occurring LPS molecular species from *Vitreoscilla filiformis* with Formula (II)(0), as shown in **Fig. 5.**
**"LPS $SM_1$ 0.0",** as used herein, refers to the naturally-occurring LPS molecular species from *Salmonella enterica enterica* serovar *Minnesota* R595 with Formula (XII)(0), as shown in **Fig. 27**.

***Production* & *Purification***

LPS $B_2$ 0.0, LPS $W_1$ 0.0 and LPS $SM_1$ 0.0 were produced and purified using conventional methods, in particular the method described in US patent No. 8,137,935.

Other naturally-occurring LPS molecular species have been produced and purified similarly (structures not shown), including:

i. **"LPS $ST_1$ 0.0",** from *Salmonella enterica enterica* serovar *Typhimurium* Ra;
ii. **"LPS $E_2$ 0.0",** from *Escherichia coli* K12;
iii. **"LPS $E_1$ 0.0",** from *Escherichia coli* J5;
iv. **"LPS $A_1$ 0.0",** from *Actinobacillus pleuropneumoniae;*
v. **"LPS $K_1$ 0.0",** from *Burkholderia pseudomallei;*
vi. **"LPS $M_1$ 0.0",** from *Moraxella catarrhalis;*
vii. **"LPS $N_1$ 0.0",** from *Neisseria sicca;*
viii. **"LPS $P_1$ 0.0",** from *Pseudomonas aeruginosa;*
ix. **"LPS $P_2$ 0.0",** from *Pseudomonas fluorescens;*
x. **"LPS $I_1$ 0.0",** from *Bordetella hinzii;*
xi. **"LPS $T_1$ 0.0",** from *Bordetella trematum;* and
xii. **"LPS $V_1$ 0.0",** from *Vibrio fischeri.*

***Chemical modification***

After purification, the LPS molecular species were chemically treated by acidic hydrolysis at a temperature below 85°C; and/or by alkaline hydrolysis, as indicated by the figures **"x.y"** following the LPS name. If both, alkaline hydrolysis was carried out preferably before acidic hydrolysis.

The innovative process is that acidic hydrolysis at a temperature below 85°C targets dephosphorylation of the lipid A glycosidic phosphate, which is removed *in situ,* in the complete LPS molecular species, *i.e.,* without splitting it in its lipid A and polysaccharide moieties.

It follows from these treatments several LPS molecular species as defined in **Table 1** below:

**Table 1**

| Natural LPS | Formulas | Figure | Modified LPS | Formulas | Figure | Modified LPS | Formulas | Figure | Modified LPS | Formulas | Figure |
|---|---|---|---|---|---|---|---|---|---|---|---|
| $B_2$ 0.0 | (I)(0)(a)<br>(I)(0)(b) | **Fig. 1** | $B_2$ 0.1 | (I)(1)(a)<br>(I)(1)(b)<br>(I)(1)(c)<br>(I)(1)(d)<br>(I)(1)(e)<br>(I)(1)(f) | **Fig. 2** | $B_2$ 1.1 | (I)(2)(a)<br>(I)(2)(b)<br>(I)(2)(c) | **Fig. 3** | $B_2$ 1.2 | (I)(3)(a)<br>(I)(3)(b)<br>(I)(3)(c) | **Fig. 4** |
| $W_1$ 0.0 | (II)(0) | **Fig. 5** | $W_1$ 1.0 | (II)(1)(a)<br>(II)(1)(b)<br>(II)(1)(c)<br>(II)(1)(d) | **Fig. 6** | $W_1$ 1.2 | (II)(2)(a)<br>(II)(2)(b)<br>(II)(2)(c)<br>(II)(2)(d) | Fig. 7 | | | |
| $SM_1$ 0.0 | (XII)(0) | **Fig. 27** | $SM_1$ 1.2 | (III)(1)(a)<br>(III)(1)(b)<br>(III)(1)(c)<br>(III)(1)(d) | **Fig. 8** | | | | | | |
| $ST_1$ 0.0 | *n/a* | *n/a* | $ST_1$ 1.2 | (IV)(1)(a) | **Fig. 9** | | | | | | |
| $E_2$ 0.0 | *n/a* | *n/a* | $E_2$ 1.2 | (IV)(1)(b) | | | | | | | |
| $V_1$ 0.0 | *n/a* | *n/a* | $V_1$ 1.2 | (IV)(1)(c) | | | | | | | |
| $E_1$ 0.0 | *n/a* | *n/a* | $E_1$ 0.1 | (V)(1)(a) | **Fig. 10** | | | | | | |
| | | | | (V)(1)(b)<br>(V)(1)(c)<br>(V)(1)(d)<br>(V)(1)(e)<br>(V)(1)(f)<br>(V)(1)(g)<br>(V)(1)(h)<br>(V)(1)(i) | | | | | | | |

(continued)

| Natural LPS | Formulas | Figure | Modified LPS | Formulas | Figure | Modified LPS | Formulas | Figure | Modified LPS | Formulas | Figure |
|---|---|---|---|---|---|---|---|---|---|---|---|
| $A_1$ 0.0 | *n/a* | *n/a* | $A_1$ 0.1 | (VI)(1)(a)<br>(VI)(1)(b)<br>(VI)(1)(c)<br>(VI)(1)(d)<br>(VI)(1)(e)<br>(VI)(1)(f)<br>(VI)(1)(g)<br>(VI)(1)(h)<br>(VI)(1)(i)<br>(VI)(1)(j) | **Fig. 11** | $A_1$ 1.2 | (VI)(2)(a)<br>(VI)(2)(b)<br>(VI)(2)(c) | **Fig. 12** | | | |
| $K_1$ 0.0 | *n/a* | *n/a* | $K_1$ 1.2 | (VII)(1)(a)<br>(VII)(1)(b)<br>(VII)(1)(c) | **Fig. 13** | | | | | | |
| $M_1$ 0.0 | *n/a* | *n/a* | $M_1$ 1.2 | (VIII)(1)(a)<br>(VIII)(1)(b)<br>(VIII)(1)(c)<br>(VIII)(1)(d) | **Fig. 14** | | | | | | |
| $N_1$ 0.0 | *n/a* | *n/a* | $N_1$ 1.2 | (IX)(1)(a)<br>(IX)(1)(b)<br>(IX)(1)(c)<br>(IX)(1)(d) | **Fig. 15** | | | | | | |
| $P_1$ 0.0 | *n/a* | *n/a* | $P_1$ 1.2 | (X)(1)(a)<br>(X)(1)(b)<br>(X)(1)(c)<br>(X)(1)(d) | **Fig. 16** | | | | | | |
| $P_2$ 0.0 | *n/a* | *n/a* | $P_2$ 1.2 | | | | | | | | |
| $I_1$ 0.0 | *n/a* | *n/a* | $I_1$ 1.2 | (XI)(1)(a)<br>(XI)(1)(b)<br>(XI)(1)(c)<br>(XI)(1)(d) | **Fig. 17** | | | | | | |
| $T_1$ 0.0 | *n/a* | *n/a* | $T_1$ 1.2 | | | | | | | | |

## Example 2

### DLS aggregate size analysis

*Materials and methods*

Determination of aggregates sizes of LPS $B_2$ 0.0, LPS $B_2$ 0.1, LPS $B_2$ 1.1 and of synthetic GLA (PHAD®, from Avanti® Polar Lipids, Inc.) in aqueous and PBS suspensions was determined by Dynamic Light Scattering (DLS) using the Malvern Instruments Zetasizer Nano S90 with a scattering angle $\Theta$ = 90°, incident laser wavelength $\lambda$ = 633 nm and temperature at 25°C.

Samples for measurements were prepared as follows: 200 µg of dry samples in 4 mL glass vials were suspended in 1 mL of water or PBS. LONZA LAL Reagent water and Gibco PBS pH 7.4 (without $CaCl_2$ or $MgCl_2$) were used. The solvents were added progressively (250 µL plus 250 µL plus 500 µL). Three homogenization cycles were applied after each addition of a solvent, consisting in a 10-second sonication in a Fisher Scientific FB 15050 ultrasonic bath and 5 seconds vigorous vortex agitation.

The final 0.2 mg/mL suspensions were transparent for all the three LPS $B_2$ 0.0, LPS $B_2$ 0.1, LPS $B_2$ 1.1 samples, whether in water or in PBS, and slightly opaque but homogeneous for GLA.

800 µL of each suspension were transferred to a disposable Eppendorf polystyrene semi-micro cuvette and DLS measurements were performed three times.

*Results*

As seen in **Fig. 18A-18C,** all the three LPS $B_2$ 0.0, LPS $B_2$ 0.1, LPS $B_2$ 1.1 samples in water show a monodisperse profile with an average particle size of about 18.0 nm for LPS $B_2$ 0.0, 19.9 nm for LPS $B_2$ 0.1 and 8.8 nm for LPS $B_2$ 1.1.

GLA in water also shows a monodisperse profile, but unlike LPS $B_2$ 0.0, LPS $B_2$ 0.1, LPS $B_2$ 1.1, the average particle size is of about 544 nm **(Fig. 18D).**

The same observations were made in PBS, where LPS $B_2$ 0.0, LPS $B_2$ 0.1, LPS $B_2$ 1.1 have an average particle size of about 15.0 nm, 21.4 nm and 13.5 nm, respectively **(Fig. 19A-19C),** versus about 529 nm for GLA in PBS **(Fig. 19D).**

*Conclusion*

Previous studies have shown that LPS comprising a full O-antigen domain have lengths ranging from about 17 ± 10 nm to about 37 ± 9 nm. In absence of O-antigen domain, LPS have lengths ranging from about 3 ± 2 nm to about 5 ± 3 nm (Strauss et al., 2009. J Mol Recognit. 22(5):347-55).

In view of these elements, the data presented herein clearly demonstrate that LPS $B_2$ 0.0, LPS $B_2$ 0.1, LPS $B_2$ 1.1 are soluble or easily dispersible. In water or PBS, they form stable suspensions of monodispersed nano-particles (micelles) whose size is less than 30 nm. No dispersing reagents like detergents, nor special mechanical or thermal treatments (ultrasound, microfluidic or extrusion) are necessary to obtain this result. This is in contrast to synthetic GLA which precipitates (as indicated by the slightly opaque samples) and forms aggregates of > 500 nm and more. In fact, GLA aqueous formulations are produced in presence of an additional lipid using heating and microfluidic treatment. The above-mentioned properties of LPS $B_2$ 0.0, LPS $B_2$ 0.1, LPS $B_2$ 1.1 and other similar products make them fairly easy to formulate as drug products. They can also be easily filtered at 200 nm and thus fulfill the major characteristics required for injectable drugs to avoid, e.g., bacterial contamination. These data are summarized in **Fig. 20.**

## Example 3

### Biophysical characterization

*Materials and methods*

Thin Layer Chromatography (TLC)

TLC was carried out on glass HPTLC silica gel plates (Merck). 20 µg of LPS were deposited at the origin of the HPTLC plate, and chromatographed in a solvent mixture of isobutyric acid and 1 M ammonium hydroxide (5:3 v:v) as previously described (Caroff & Karibian, 1990. Appl Environ Microbiol. 56(6):1957-9). Products were visualized by charring (in an oven at 150°C for 5 minutes) after spraying with 10% sulfuric acid in ethanol.

SDS-polyacrylamide gel analysis of LPS

15% poly-acrylamide gels were used, and 0.2 μg of LPS were loaded onto the 4% stacking gel. LPS samples preparation, electrophoresis process and Tsai&Frash Silver Nitrate coloration were performed as previously described (Laemmli, 1970. Nature. 227(5259):680-5; Tsai & Frasch, 1982. Anal Biochem. 119(1):115-9).

Matrix-Assisted Laser Desorption/Ionization (MALDI)-Time-Of-Flight (TOF) analysis

MALDI-TOF was performed as previously described (Chafchaouni-Moussaoui et al., 2011. Rapid Commun Mass Spectrom. 25(14):2043-8), in the linear mode with delayed extraction using a Perseptive Voyager STR (PE Biosystem, France) time-of-flight (TOF) mass spectrometer and/or on a Shimadzu Axima Performance system.
A suspension of lipid A (1 mg/mL) in chloroform:methanol:water (3:1.5:0.25, v:v:v) was desalted with a few grains of Dowex 50W-X8 ($H^+$), and 1 μL was deposited on the target, mixed with 1 μL of gentisic acid (2,5-dihydroxybenzoic acid, DHB) matrix suspended at 10 μg/μL in the same solvent or in 0.1 M aqueous citric acid, and dried.
Analyte ions were desorbed from the matrix with pulses from a 337 nm nitrogen laser. Spectra were obtained in the negative-ion mode at 20 kV.
When LPS were analyzed directly, they were suspended in water and decationized in the same way, using the same matrix and conditions.

***Results***

The mass-to-charge ratios (m/z) of the different LPS molecular species described herein are reported in **Table 2.** They correspond to $[M-H]^-$ ions of LPS molecular species, and their core and lipid A moieties fragments. The molecular weight (MW) corresponding to the lipid A moieties (*i.e.,* without their R substituent) are also displayed with the corresponding structures in **Figures 1-17.**

**Table 2**

| **LPS $B_2$ 0.0** (*Bordetella pertussis)* **(Fig. 1)** | | |
|---|---|---|
| **Calculated masses $[M-H]^-$** | **Observed masses $[M-H]^-$** | **Interpretation** |
| 1332.62 | 1332.8 | Lipid A |
| 1558.98 | 1559.0 | Lipid A |
| 2293.04 | 2292.7 | PS |
| 2496.07 | 2496.9 | PS |
| 3853.02 | 3852.5 | LPS |
| 4056.05 | 4056.4 | LPS |
| **LPS $B_2$ 0.1** (*Bordetella pertussis)* **(Fig. 2)** | | |
| **Calculated masses $[M-H]^-$** | **Observed masses $[M-H]^-$** | **Interpretation** |
| 872.03 | 871.7 | Lipid A |
| 1042.28 | 1042.6 | Lipid A |
| 1098.39 | 1098.4 | Lipid A |
| 1268.64 | 1269.1 | Lipid A |
| 1308.75 | 1309.1 | Lipid A |
| 1479.00 | 1479.5 | Lipid A |
| 2373.02 | 2372.4 | PS |
| 2496.07 | 2495.5 | PS |
| 3246.05 | 3246.0 | LPS |
| 3369.10 | 3369.6 | LPS |
| 3416.3 | 3417.4 | LPS |
| 3472.41 | 3472.8 | LPS |

(continued)

| LPS $B_2$ 0.1 (*Bordetella pertussis)* (Fig. 2) | | |
|---|---|---|
| **Calculated masses [M-H]$^-$** | **Observed masses [M-H]$^-$** | **Interpretation** |
| 3595.46 | 3595.4 | LPS |
| 3642.66 | 3643.3 | LPS |
| **LPS $B_2$ 1.1** *(Bordetella pertussis)* **(Fig. 3)** | | |
| **Calculated masses [M-H]$^-$** | **Observed masses [M-H]$^-$** | **Interpretation** |
| 872.03 | 872.2 | Lipid A |
| 1098.39 | 1098.5 | Lipid A |
| 1308.75 | 1308.8 | Lipid A |
| 2180.85 | 2180.3 | PS |
| 2373.02 | 2372.4 | PS |
| 2496.07 | 2496.4 | PS |
| 3053.88 | 3053.6 | LPS |
| 3246.05 | 3246.6 | LPS |
| 3369.1 | 3370.1 | LPS |
| **LPS $B_2$ 1.2** (*Bordetella pertussis)* **(Fig. 4)** | | |
| **Calculated masses [M-H]$^-$** | **Observed masses [M-H]$^-$** | **Interpretation** |
| 872.03 | 872.3 | Lipid A |
| 1082.03 | 1082.8 | Lipid A |
| 1308.75 | 1309.2 | Lipid A |
| 2293.04 | 2292.8 | PS |
| 2373.02 | 2373.1 | PS |
| 2496.07 | 2496.4 | PS |
| 3166.07 | 3165.6 | LPS |
| 3246.05 | 3246 | LPS |
| 3369.1 | 3369.7 | LPS |
| **LPS $SM_1$ 1.2** (*Salmonella enterica enterica* serovar *Minnesota* R595) **(Fig. 8)** | | |
| **Calculated masses [M-H]$^-$** | **Observed masses [M-H]$^-$** | **Interpretation** |
| 872.03 | 872.2 | Lipid A |
| 1292.75 | 1292.9 | Lipid A |
| 1074.19 | 1073.3 | LPS |
| 1092.21 | 1092.0 | LPS |
| 1214.39 | 1213.0 | LPS |
| 1312.39 | 1311.2 | LPS |
| 1512.93 | 1512.8 | LPS |
| 1715.09 | 1714.3 | LPS |
| 1733.11 | 1733.1 | LPS |
| **LPS $ST_1$ 1.2** (*Salmonella enterica enterica* serovar *Typhimurium* Ra) **(Fig. 9)** | | |
| **Calculated masses [M-H]$^-$** | **Observed masses [M-H]$^-$** | **Interpretation** |
| 872.03 | 872.1 | Lipid A |

(continued)

| LPS $ST_1$ 1.2 (*Salmonella enterica enterica* serovar *Typhimurium* Ra) **(Fig. 9)** | | |
|---|---|---|
| **Calculated masses [M-H]⁻** | **Observed masses [M-H]⁻** | **Interpretation** |
| 1054.33 | 1054.4 | Lipid A |
| 1262.68 | 1262.7 | Lipid A |
| 1807.48 | 1807.8 | PS |
| 1930.53 | 1930.7 | PS |
| 2662.49 | 2662.2 | LPS |
| 2680.51 | 2680.4 | LPS |
| 2785.54 | 2785.4 | LPS |
| 2803.56 | 2803.0 | LPS |
| 2882.67 | 2882.9 | LPS |
| 2900.69 | 2901.5 | LPS |
| 3005.72 | 3006.0 | LPS |
| 3023.74 | 3024.2 | LPS |
| **LPS $E_2$ 1.2** (*Escherichia coli* K12) **(Fig. 9)** | | |
| **Calculated masses [M-H]⁻** | **Observed masses [M-H]⁻** | **Interpretation** |
| 872.03 | 872.0 | Lipid A |
| 1054.33 | 1054.6 | Lipid A |
| 1262.68 | 1263.0 | Lipid A |
| 1796.46 | 1796.4 | PS |
| 1919.51 | 1919.5 | PS |
| 1999.49 | 1999.5 | PS |
| 2651.47 | 2651.3 | LPS |
| 2669.49 | 2669.4 | LPS |
| 2774.52 | 2774.6 | LPS |
| 2792.54 | 2792.1 | LPS |
| 2853.63 | 2854.8 | LPS |
| 2871.65 | 2872.0 | LPS |
| 2994.7 | 2995.0 | LPS |
| 3074.68 | 3075.0 | LPS |
| **LPS Wi 0.0** (*Vitreoscilla filiformis*) | | |
| **Calculated masses [M-H]⁻** | **Observed masses [M-H]⁻** | **Interpretation** |
| 1544.91 | 1544.8 | Lipid A |
| 1667.96 | 1668.1 | Lipid A |
| 1791.00 | 1791.3 | Lipid A |
| | 3208.3 | LPS |
| | 3331.6 | LPS |
| | 3455.1 | LPS |
| | 3524.0 | LPS |

(continued)

| LPS Wi 1.0 (*Vitreoscilla filiformis*) **(Fig. 6)** | | |
|---|---|---|
| **Calculated masses [M-H]-** | **Observed masses [M-H]-** | **Interpretation** |
| 839.79 | 839.8 | Lipid A |
| 1022.10 | 1022.3 | Lipid A |
| 1204.40 | 1205.1 | Lipid A |
| | 1262.7 | PS |
| | 1855.2 | PS |
| | 2504.1 | LPS |
| | 2667.7 | LPS |
| | 2685.8 | LPS |
| | 2877.9 | LPS |
| | 3059.3 | LPS |
| **LPS Wi 1.2** (*Vitreoscilla filiformis*) **(Fig. 7)** | | |
| **Calculated masses [M-H]-** | **Observed masses [M-H]-** | **Interpretation** |
| 759.81 | 759.6 | Lipid A |
| 943.12 | 942.2 | Lipid A |
| 1125.30 | 1124.7 | Lipid A |
| | 1262.1 | PS |
| | 1854.4 | PS |
| | 2047.6 | PS |
| | 2405.8 | LPS |
| | 2423.2 | LPS |
| | 2568.8 | LPS |
| | 2587.1 | LPS |
| | 2606.2 | LPS |
| | 2798.1 | LPS |
| | 2943.4 | LPS |
| | 2961.2 | LPS |
| | 2980.3 | LPS |

MALDI-MS spectra analysis of MPL and the *Salmonella enterica* and *Escherichia coli* candidates **(Fig. 21)**

*MPL*

The MALDI-MS spectrum of MPL (monophosphoryl lipid A) is shown for comparison. MPL is a natural LPS derivative, originating from *Salmonella Minnesota* R595 Re-Type, from which the phosphate group at position C1 of the reducing end of the lipid A domain has been liberated by acidic hydrolysis, together with the core part of the LPS molecular species. It is used in different vaccine preparations by GSK.

As expected, the spectrum corresponding to MPL only shows peaks in the lower-mass region. A major peak signals at *m*/*z* 1729 and corresponds to a molecular species with six fatty acid chains, among which three 3OH-$C_{14}$, one $C_{16}$, one $C_{14}$, one $C_{12}$; and only one phosphate at position C4' of the di-GlcN disaccharide. A peak at *m*/*z* 1491 corresponds to a molecular species without $C_{16}$, and at *m*/*z* 1280 without $C_{16}$ and $C_{14}$.

In the present invention, as seen by comparison of the spectra given also in **Fig. 21,** the lipid A domains of the different LPS are still carrying the core and polysaccharide moieties of the native LPS molecular species, appearing in the

middle-mass and high-mass regions respectively.

*LPS $SM_1$ 1.2*

The spectrum corresponds to a *Salmonella enterica enterica* serovar *Minnesota* R595 LPS molecular species, modified by removal of the primary ester-linked fatty acids, followed by removal of the glycosidic lipid A phosphate group. Therefore, the main peak at *m/z* 1292 corresponds to a lipid moiety with one phosphate group and 4 fatty acids, namely two 3-OH-$C_{14}$, one $C_{16}$ and one $C_{12}$ (Formula (III)(1)(d) of **Fig. 8).** The LPS molecular species correspond to this lipid A plus one Kdo at *m/z* 1512, and two Kdo at *m/z* 1733.

*LPS $ST_1$ 1.2*

The spectrum corresponds to a *Salmonella enterica enterica* serovar *Typhimurium* Ra LPS molecular species, modified by removal of all ester-linked fatty acids for a fragmention signaling at *mlz* 872 (Formula (IV)(1)(a) of **Fig. 9),** and still bearing a $C_{12}$ at *mlz* 1054 (Formula (IV)(1)(b) of **Fig. 9),** as well as removal of the glycosidic phosphate group. The core ions obtained by fragmentation in the MS process appear at *m/z* 1763 and 1807, and with additional phosphoryl ethanolamine (PEA) residues at *m/z* 1886 and 1930 respectively. The core structure, when split, corresponds to one Kdo, three heptoses, three hexoses, one *N*-acetyl-glucosamine and two phosphate groups for a total molecular weight of 1808 u. The main molecular species corresponding to lipid A moieties at *m/z* 872 plus a core at 1808, appear at *mlz* 2680 and at *mlz* 2803 for an additional PEA and 2882.9 for diphosphoryl ethanolamine (PPEA), or at *m/z* 2901 for an additional lateral Kdo.

*LPS $E_2$ 1.2*

The spectrum corresponds to *Escherichia coli* K12 LPS molecular species, modified by removal of ester-linked fatty acids and giving signals in the lipid A region corresponding to molecular species described for $ST_1$ 1.2 at *m/z* 872 (Formula (IV)(1)(a) of **Fig. 9)** and 1054 (Formula (IV)(1)(b) of **Fig. 9),** because the native LPS from these batches had similar lipid A structures.

The core structure was slightly different from $ST_1$ LPS with one Kdo, four heptoses, four hexoses, and two phosphate groups for a molecular weight of 1797 u signaling at *m/z* 1796 for the corresponding ion. It comes at *m/z* 1876 plus phosphate or at *m/z* 1919 plus PEA and *m/z* 1999 for PPEA. With an additional lipid A moiety at *m/z* 872, the LPS molecular species appear at *m/z* 2669, *m/z* 2792 plus PEA, 2872 plus PPEA or at 2872 from 2651 (the anydro-core structure) plus Kdo and at *m/z* 2995 for additional PEA and *m/z* 3075 for an additional phosphate.

<u>MALDI-MS spectra analysis of the *Bordetella pertussis* candidates **(Fig. 22)**</u>

*LPS $B_2$ 0.0*

The negative-ion spectrum of LPS $B_2$ 0.0 shows three zones of signals:

i. in the 3000-4200 Da region, corresponding to LPS molecular ions;
ii. in the 1800-2800 Da region, corresponding to core oligosaccharide-fragment ions; and
iii. in the 1200-1700 Da region, corresponding to lipid A-fragment ions.

In the 1200-1700 Da region, the two major peaks at *m/z* 1332 and 1559 correspond respectively to tetra- and penta-acylated species of the lipid A moiety of LPS $B_2$ 0.0, with Formulas (I)(0)(a) and (I)(0)(b) respectively, as shown in **Figure 1.**

The 1800-2800 Da region corresponds to the naturally heterogeneous core oligosaccharide moieties. The peak at *m/z* 2293 corresponds to the well-characterized anhydro-dodecasaccharide described in the literature, followed by its diphosphoryl and diphosphoryl-ethanolamine forms, signaling respectively at *m/z* 2453 and 2497.

In the 3000-4200 Da region, corresponding to native LPS molecular species, the peaks at *m/z* 4056 and 3852 correspond to the penta-acylated LPS with a dodecasaccharide core, with and without PPEA, respectively.

*LPS $B_2$ 0.1*

In the 1200-1700 Da region, peaks correspond to fragment-ions monophosphoryl lipid A moieties obtained by targeted chemical dephosphorylation of the native LPS $B_2$ 0.0 molecules. Dephosphorylation was aimed precisely at the level of the glycosidic phosphate of the lipid moiety, as described above, and correspond to small amounts of mono-phosphoryl penta-acyl lipid A at *m/z* 1479 (Formula (1)(1)(f) of **Fig. 2),** a mono-phosphoryl triacyl molecular species at *m/z* 1098 (Formula (I)(1)(c) of **Fig. 2),** and a di-acyl mono-phosphoryl molecular species at *m/z* 871 (Formula (I)(1)(a) of **Fig. 2).**

As described above for the native LPS $B_2$ 0.0 molecular species, the core oligosaccharide and LPS molecular species appear respectively in the 2000-2600 Da region, and higher masses up to 3000-3700 Da respectively. Compared to the native LPS $B_2$ 0.0 molecules described above, a decrease in *m/z* ratio is observed in the higher mass regions, due to the chemical modification (corresponding to the dephosphorylation at position C1 of the reducing end of the lipid A domain, and partial de-O-acylation).

*LPS $B_2$ 1.1*

Peaks in the higher mass region show the dephosphorylation at position C1 of the reducing end of the lipid A domain and additional removal of fatty acid chains, namely a 3-OH-$C_{14}$ primary fatty acid chain, a 3-OH-$C_{10}$ primary fatty acid chain and a $C_{14}$ secondary fatty acid chain. These new species display signals at *m/z* 3053 and 3370. When the PEA group is removed from *mlz* 3370, the resulting species displays a signal at *mlz* 3246.

Peaks in the lower mass region are mainly represented by the mono-phosphoryl di-acyl lipid A at *m/z* 872 (Formula (I)(2)(a) of **Fig. 3)** and the mono-phosphoryl triacyl lipid A at *m/z* 1098 (Formula (I)(2)(b) of **Fig. 3).**

*LPS $B_2$ 1.2*

Three main molecular species appear in the higher masses' region. The one at *m/z* 3165 corresponds to a modified LPS with a lipid A moiety at *m/z* 872 from which the glycosidic phosphate group and all ester-linked fatty acids were removed, plus a dodecasaccharide at *m/z* 2292 (Formula (I)(3)(a) of **Fig. 4).** The second and third peaks, respectively at *m/z* 3246 and 3369, correspond to modified LPS with additional core phosphate and ethanolamine. Like for the preceding spectra, molecular ions generated by fragmentation in the mass spectrometer appear in the lipid A and core oligosaccharide-mass regions.

<u>MALDI-MS spectra analysis of the *Vitreoscilla filiformis* candidates **(Fig. 23**)</u>

*LPS $W_1$ 0.0*

Two major peaks are observed in the lower mass region of the spectrum, corresponding to fragment-ions generated during the mass spectrometry process. One is observed at *m/z* 1544, corresponding to a lipid A molecular species containing two glucosamines, two phosphates, four primary hydroxydecanoic acids (3-OH-$C_{10}$) and two secondary dodecanoic acids ($C_{12}$) (Formula (II)(0) of **Fig. 5).** Another major molecular ion observed at *m/z* 1668 corresponds to the molecular ion carrying one additional PEA residue (+123 u).

Peaks appearing in the higher mass region correspond to natural heterogeneity, they signal between *m/z* 3208 and 3577 corresponding to a core polysaccharide of undescribed structure at about 1800 Da, linked at position C6' of the non-reducing end of the lipid A domain.

*LPS $W_1$ 1.0*

Peaks appearing in the lower mass region of the spectrum correspond to fragment-ions of the *Vitreoscilla filiformis* lipid A, from which were removed two ester-linked 3-OH-$C_{10}$ and signaling at *m/z* 1205 (Formula (II)(1)(d) of **Fig. 6).** Further loss of a secondary-ester-linked $C_{12}$ leads to a molecular ion at *m/z* 1023 (Formulas (II)(1)(b) and (II)(1)(c) of **Fig. 6).**

*LPS $W_1$ 1.2*

When the alkaline treatment is followed by the release of the glycosidic phosphate group by acidic treatment, peaks appear at *m/z* 1124 (Formula (II)(2)(d) of **Fig. 7)** from the molecular species at 1205, and at *m/z* 942 (Formula (II)(2)(b) and (II)(2)(c) of **Fig. 7)** from 1023, corresponding to a tetra-acyl and triacyl lipid A with two 3-OH-$C_{10}$, one of which being esterified with a secondary $C_{12}$ fatty acid chain. A small peak at *m/z* 759 corresponds to the mono-phosphoryl di-acyl lipid A (Formula (II)(2)(a) of **Fig. 7).** In the middle-masses region, peaks corresponding to core fragments appear, when added to the de-O-acylated lipid A moieties they correspond to the LPS molecular species signaling in the higher masses' region.

***Conclusion***

LPS $B_2$ 0.0 corresponds to a native candidate (i.e., before chemical treatment by acidic hydrolysis and optionally

by alkaline hydrolysis) displaying some heterogeneity due to the natural heterogeneity of the lipid A domain made of two main molecular species: a tetra- and a penta-acylated molecular species. Other bacterial strains and/or growth conditions can lead to either homogeneous tetra- or penta-acylated LPS molecular species. Heterogeneity at the level of the *Bordetella pertussis* oligosaccharide core is mainly due to the presence of nona- and dodeca-saccharide cores. Some heterogeneity is also observed in the oligosaccharide core region due to a partial substitution with a PEA group.

LPS $B_2$ 0.1 was obtained after chemical treatment by acidic hydrolysis of LPS $B_2$ 0.0, thus removing the glycosidic phosphate at position C1 of the reducing end of its lipid A domain, and partial removal of some ester-linked fatty acids.

LPS $B_2$ 1.1 was obtained after chemical treatment by acidic hydrolysis of LPS $B_2$ 0.0 and further by alkaline hydrolysis, which removed some fatty acids.

LPS $B_2$ 1.2 was obtained after de-O-acylation by alkaline treatment and acidic hydrolysis of LPS $B_2$ 0.0, thereby removing the glycosidic phosphate at position C1 of the reducing end of the de-O-acylated lipid A domain.

LPS Wi 0.0, isolated from *Vitreoscilla filiformis,* contains a hexa-acylated lipid A moiety with mainly short chain fatty acid (four 3-OH-$C_{10}$ and two $C_{12}$). It is also characterized by the presence of phosphoryl-ethanolamine substituents on both phosphate groups, leading to some heterogeneity in addition to LPS classical heterogeneity due to fatty acid chains. This original structure is expected to react differently from the classical *Escherichia coli* or *Salmonella enterica* LPS molecules.

LPS Wi 1.0 was obtained by alkaline treatment of native LPS Wi 0.0, releasing two 3-OH-$C_{10}$ and one $C_{12}$. The resulting molecular species correspond to a major tri-acyl molecular LPS and a minor tetra-acyl one.

LPS Wi 1.2 was obtained by targeted dephosphorylation of the lipid A glycosidic phosphate from the LPS Wi 1.0 de-*O*-acylated LPS.

These examples of different LPS structures from different bacterial genera illustrate the capacities of the innovative chemical modifications applied to native LOS and LPS structures, to obtain new *in situ* dephosphorylated lipid A molecules remaining linked to their core oligosaccharide moieties.

It should be noted that dephosphorylation at position C1 of the reducing end of the lipid A domain was achieved with a very high specificity and efficacy, with more than 90% and even up to 99% of LPS molecular species being dephosphorylated at position C1 while retaining their core oligosaccharide moiety at position C6' of the non-reducing end of their lipid A domain.

The new dephosphorylation process can now be applied to detoxify all LPS structures described so far, and leads to safe and powerful immunostimulant compounds. This process has been applied to a wide panel of different structures from Enterobacterial and non-Enterobacterial LPS, among which those described in **Example 1** above.

## Example 4

### *In vitro* pyrogenic activity of LPS $B_2$ 0.0, LPS $B_2$ 0.1, LPS $B_2$ 1.1 and LPS Wi 0.0

The pyrogenic activity of naturally-occurring LPS $B_2$ 0.0 and LPS Wi 0.0, and of modified LPS $B_2$ 0.1 and LPS $B_2$ 1.1 was evaluated *in vitro* on human cells in monocytes activation tests (MAT - EEU/pg). The objectives were to demonstrate the relevance of selected LPS, and to position the naturally-occurring LPS $B_2$ 0.0 and LPS Wi 0.0, and modified LPS $B_2$ 0.1 and LPS $B_2$ 1.1 relative to benchmark natural or synthetic TLR4 agonists used in human vaccines (MPL/GSK) or currently in clinical development (GLA/Merck), in terms of endotoxin activity.

### *Materials and methods*

Monocyte Activation tests (MAT)

Standardized Monocyte Activation tests (MAT) based on human cryo-blood and with IL-6 readout (HaemoMAT Kit - Haemochrom Diagnostica GmbH) were performed on a wide range of concentrations (from 400 to 0.04 ng/mL) of the naturally-occurring LPS $B_2$ 0.0 and LPS Wi 0.0 and modified LPS $B_2$ 0.1 and LPS $B_2$ 1.1, following the European Pharmacopeia (Ph. Eur. 9.8 section 2.6.30).

Briefly, human PBMC (CryoBlood) were cultured overnight in presence of different concentrations (400 to 0.04 ng/mL) of the tested molecules. The concentration of IL-6 (a pro-inflammatory cytokine) was then quantified in the supernatant by ELISA.

Equivalent endotoxin level (EEU/μg) was then estimated for each molecule. Samples were tested with a positive control (PPC) of 0.5 EU/mL (interference test). The solution was considered free of interfering factors (i.e., valid) if under the conditions of the test, the measured concentration of the endotoxin added to the test solution was within 50 % to 200 % of the known added endotoxin concentration, after subtraction of any endotoxin detected in the solution without added endotoxin.

Rabbit pyrogen tests (RPT)

Standardized Rabbit Pyrogen Test (RPT) were conducted according to European Pharmacopeia (Ph.Eur. 5.1.10). Product tested includes the naturally-occurring LPS $B_2$ 0.0 and LPS Wi 0.0 and modified LPS $B_2$ 0.1, LPS $B_2$ 1.1 and LPS $B_2$ 1.2, as well as a non-GMP equivalent of the GLA from Merck (Avanti Polar Lipids).

Briefly, groups of three rabbits, weighing 2.5 to 3 kg received one intravenous injection of the tested product in 1 mL/kg of body weight.

For each candidate, several dose ranges were tested from 0.1 to 1000 ng/kg. For each animal, the baseline rectal temperature was recorded. After 30 minutes, the rabbits were inoculated in the ear vein. Temperatures were recorded every 6.5 minutes for 3 hours. The rising temperature of the rabbit body temperature is that the highest body temperature measure within the 3 hours, minus the normal body temperature.

Result judgment: the dose of a given product was considered not pyrogen when the sum of the maximum temperature rises in the three individual rabbits ($\Delta$T+) does not exceed 1.15°C. Several doses of each candidates were tested to define their MNPD corresponding to the highest dose of a given product that can be administered in rabbits without inducing febrile reaction.

***Results***

Monocyte Activation tests (MAT)

**Table 3** illustrates the pyrogenic activity of the naturally-occurring LPS $B_2$ 0.0 and LPS Wi 0.0 and modified LPS $B_2$ 0.1 and LPS $B_2$ 1.1 evaluated *in vitro* on human cells in MAT assays (EEU/$\mu$g). These results are compared to the pyrogenicity of other natural or synthetic LPS as described in the literature, including:

- monophosphoryl lipid A from *Salmonella enterica enterica* serovar *Minnesota* R595 [MPLA] from InvivoGen (a non-GMP analog of MPL),
- glucopyranosyl lipid A [GLA] from Merck (a synthetic analog of MPL), and
- a natural non-detoxified LPS from *Escherichia coli* [O55:B5].

**Table 3**

| LPS | $B_2$ 0.0 | $B_2$ 0.1 | $B_2$ 1.1 | $W_1$ 0.0 | MPLA | GLA | O55:B5 |
|---|---|---|---|---|---|---|---|
| **MAT (EEU/$\mu$g)** | 995.5 ($\pm$6.4) | 952.5 ($\pm$102) | 891.5 ($\pm$26.2) | 867.0 ($\pm$45.3) | 1000[1] | 15700[2] ($\pm$0.43) | 5000-10000[3] |
| 1: Invivogen, MPLAs technical data sheet, version 19C19-MM, available at https://www.invivogen.com/sites/default/files/invivogen/products/files/mplas_tds.pdf<br>2: Misquith et al., 2014. Colloids Surf B Biointerfaces. 113:312-319<br>3: Weary et al., 1980. Appl Environ Microbiol. 40(6):1148-51 | | | | | | | |

The outcome of MAT assays demonstrated that the pyrogenic activity of the tested LPS is in the same range, but demonstrated that type $B_2$ LPS (from *Bordetella pertussis)* are slightly more pyrogen than W0.1 (from *Vitreoscilla filiformis*); and that LPS $B_2$ 1.1 (presenting higher level of detoxification) is less pyrogen than LPS $B_2$ 0.1 and LPS $B_2$ 0.0 **(Table 3).**

This allows a ranking of the naturally-occurring and modified LPS molecular species based on their estimated pyrogenic activity *in vitro:*
LPS Wi 0.0 > LPS $B_2$ 1.1 > LPS $B_2$ 0.1 > LPS $B_2$ 0.0.

Rabbit pyrogen tests (RPT)

Set of Rabbit Pyrogen Tests (RPT) led to estimate the maximum non-pyrogenic dose (MNPD) of all naturally-occurring LPS $B_2$ 0.0 and LPS Wi 0.0 and modified LPS $B_2$ 0.1, LPS $B_2$ 1.1 and LPS $B_2$ 1.2 **(Table 4).**

Table 4

| | Wi 0.0 | $B_2$ 0.0 | $B_2$ 0.1 | $B_2$ 1.1 | $B_2$ 1.2 | MPL[1] | GLA[2] | GSK1795-091[3] | LPS O55:B[4] |
|---|---|---|---|---|---|---|---|---|---|
| **MNPD (ng/Kg)** | 0.1 | 5 | 12.5 | 300 | 450 | 320 | 34 | 2.5 | 1 |

1: Carl R. Alving, Immunobiol. 1993
2: Internally generated data
3: GlaxoSmithKline. A 2-Part Randomized, Double-Blind (Sponsor-Unblinded), Placebo-Controlled, Ascending Dose and Parallel Group Study of TLR4 Agonist (GSK1795091) Administered to Healthy Subjects. 2017
4: Greisman SE et al., Proc Soc Exp Biol Med. 1969

Data demonstrated that among naturally-occurring LPS, the MNPD of the type Wi candidate is 50 times higher than the MNPD of the type $B_2$, demonstrating that the naturally-occurring LPS Wi 0.0 is significantly more pyrogen than the naturally-occurring LPS $B_2$ 0.0. Among type $B_2$ LPS, the MNPD of the naturally-occurring LPS $B_2$ 0.0 was also found to be 2.5×, 60× and 90× higher than those of the modified LPS candidates LPS $B_2$ 0.0, LPS $B_2$ 1.1 and LPS $B_2$ 1.1 respectively.

This demonstrates an inverse correlation between the detoxification level and the pyrogenic activity of the modified LPS. The MNPD of our native and modified LPS were compared to those evaluated or described in the literature for naturally-occurring (not modified) LPS and LPS derivatives (natural or synthetic) currently used in human vaccines (MPL/GSK) in clinical development (GLA/Merck, and GSK1795091/GSK).

The naturally-occurring LPS $B_2$ 0.0 was found to present a MNPD 2 to 5 times higher than a benchmark natural LPS from *Escherichia coli* (O55:B5), or the synthetic LPS from GSK (GSK179509), but 8 times and 64 times lower than a non-GMP equivalent of the GLA and the MPL respectively.

In contrast, the type $B_2$ LPS with the highest level of modification (LPS $B_2$ 1.2) was found to present a MNPD 450 times higher than a naturally-occurring LPS from *Escherichia coli* (O55:B5), 13.2 times and 180 times higher than the GLA and GSK1795091 respectively, and substantially higher (1.12 times) than MPL.

***Conclusion***

Results from *in vitro* pyrogen tests (MAT assays) highlight the relevance of our detoxification process to reduce the pyrogenic activity of native LPS. They also allow to position the naturally-occurring LPS $B_2$ 0.0 and LPS Wi 0.0 and modified LPS $B_2$ 0.1 and LPS $B_2$ 1.1 relative to commercialized natural or synthetic LPS whose pyrogenic activity, evaluated by *in vitro* pyrogen tests, have been described in the literature (Endotoxin Level - EU/pg). This includes GLA from Merck (a synthetic TLR4 agonist), MPLA from InvivoGen (an analog of MPL from GSK), and a natural, non-detoxified, LPS from *Escherichia coli* (O55:B5).

This demonstrates that in term of pyrogenicity, the naturally-occurring LPS $B_2$ 0.0 and LPS Wi 0.0 and modified LPS $B_2$ 0.1 and LPS $B_2$ 1.1 are close to MPLA and thus of MPL (GSK), currently used as adjuvant in several commercialized vaccines, but they are significantly less pyrogenic than the synthetic TLR4 agonists GLA (Merck) currently in clinical development, or natural, non-detoxified, LPS from E. Coli O55:B5.

Data from the *in vivo* pyrogen test (RPT assay) provide proof-of-concept of the capacity of our detoxification process to significantly reduce the pyrogenic activity of native LPS. They also demonstrated that the type B LPS ($B_2$ 1.2), presenting the highest level of modification, presents a pyrogenic activity significantly reduced compared to a commercialized natural LPS and benchmark LPS derivatives currently on the market (MPL - GSK) or in clinical development (GLA - Merck and GK1795091 - GSK).

## Example 5

### Pre-clinical proof-of-concept study: therapeutic efficacy of LPS $B_2$ 0.0, LPS $B_2$ 0.1, LPS $B_2$ 1.1 and LPS Wi 0.0, in combination with a monoclonal antibody

Therapeutic efficacy of naturally-occurring LPS $B_2$ 0.0 and LPS Wi 0.0, and of modified LPS $B_2$ 0.1 and LPS $B_2$ 1.1, alone or in combination with rituximab (an anti-CD20 monoclonal antibody), was evaluated in relevant tumor graft animal models. The objectives were to demonstrate the efficiency of selected LPS in a context of pre-existing tumor, to compare it with a synthetic TLR4 agonist (glucopyranosyl lipid A, GLA), and to demonstrate the relevance of their synergistic combination with monoclonal antibodies.

***Materials and methods***

The therapeutic efficacy of native (LPS $B_2$ 0.0 and LPS Wi 0.0) and modified (LPS $B_2$ 0.1 and LPS $B_2$ 1.1) LPS,

alone or in combination with rituximab, was analyzed *in vivo* against a human B-lymphoma tumor model (RL cells) using tumor rejection assays with a therapeutic setting in mice.

Cohort of CB17-SCID mice were engrafted subcutaneously in the abdominal right flank with $5\times10^6$ human B-lymphoma cells (RL cells). When the tumor volume had reached 100 $mm^3$ (at day 20), mice were randomized (n=6 mice/group), and the first treatment was administered.

Mice were treated once a week for 3 weeks (at days 20, 27 and 34) with LPS $B_2$ 0.0, LPS $B_2$ 0.1, LPS $B_2$ 1.1 or LPS Wi 0.0, used alone or in combination with rituximab (Genentech), an anti-human CD20 mAb.

Groups of mice treated with GLA (Merck/Immune Design Corp.) administered either intravenously or intraperitoneally at 0.5 mg/kg, alone or in combination with rituximab, were included for comparison.

LPS $B_2$ 0.0, LPS $B_2$ 0.1, LPS $B_2$ 1.1 or LPS Wi 0.0 were administered intravenously at a dose of 0.5 mg/kg. Rituximab was administered intraperitoneally at a dose of 30 mg/kg.

Tumor-bearing mice injected with PBS (control) or rituximab alone were used as controls.

The tumor volume was measured twice a week (length $\times$ width) with a caliper. The tumor volume was determined using the formula:

$$Tumor\ volume = \frac{4}{3} \times \pi \times r^3$$

All mice were raised in specific pathogen-free (SPF) environment with free access to standard food and water.

### *Results*

Treatment with rituximab alone was found to significantly impair the growth of established RL tumor cells compared to non-treated mice. Treatment with either of the native (LPS $B_2$ 0.0 or LPS Wi 0.0) LPS molecular species alone, the modified (LPS $B_2$ 0.1 and LPS $B_2$ 1.1) LPS molecular species alone, or GLA alone, did not show any impairment in the growth of RL tumor cells **(Fig. 24A & 25A).**

However, the combination of rituximab with either of the native (LPS $B_2$ 0.0 or LPS Wi 0.0) or the modified (LPS $B_2$ 0.1 and LPS $B_2$ 1.1) LPS molecular species was found to lead to a more significant impairment of tumor growth compared to each single modality treatments **(Fig. 24A & 25A).**

A better therapeutic efficacy was also observed in the group of mice treated with rituximab in combination with LPS $B_2$ 0.1 or LPS Wi 0.0, than with the synthetic TLR4 agonist GLA injected intraperitoneally **(Fig. 24A & 25A).**

Finally, while only 17% (1/6) of tumor regression were observed in the group mice treated with rituximab alone, 50% to 67% of complete and sustained regression of established RL tumor cells were observed in groups of mice treated with the native or modified LPS (50% for LPS $B_2$ 0.0 and LPS $B_2$ 1.1; 67% for LPS $B_2$ 0.1 and LPS $W_1$ 0.0) **(Fig. 24B & 25B).**

In contrast, no tumor regression was observed in the groups of mice treated with the native or modified LPS alone, or with PBS **(Fig. 24B & 25B).**

Complete tumor regression (50%) was also observed in the group of mice treated with rituximab in combination with GLA when injected intraperitoneally.

However, all the mice treated with GLA intravenously had to be euthanized following the first injection as they displayed a severely altered aspect and behavior: lethargy, hunched over themselves, bristle hair, and fast breathing. Altogether these symptoms were indicative of a strong and fast toxicity of the GLA when injected in the blood stream.

These altered aspects and behavior were not observed with LPS $B_2$ 0.0, LPS $B_2$ 0.1, LPS $B_2$ 1.1 or LPS Wi 0.0 administered intravenously.

### *Conclusion*

These results demonstrate the capacity of the native (LPS $B_2$ 0.0 or LPS Wi 0.0) and modified (LPS $B_2$ 0.1 and LPS $B_2$ 1.1) LPS molecular species to significantly boost the therapeutic efficacy of rituximab against an established human B-lymphoma model.

Results also demonstrate that, alone or in combination with rituximab, both the native (LPS $B_2$ 0.0 or LPS Wi 0.0) and modified (LPS $B_2$ 0.1 and LPS $B_2$ 1.1) LPS molecular species are as least as effective as GLA against a human B-lymphoma tumor model. However, as demonstrated in **Example 2,** GLA suffers drawbacks, in particular in terms of precipitation and aggregation, which is not observed with the tested LPS molecular species.

Finally, these results demonstrate that, in contrast to GLA, the systemic injection of the native (LPS $B_2$ 0.0 or LPS Wi 0.0) and modified (LPS $B_2$ 0.1 and LPS $B_2$ 1.1) LPS molecular species by intravenous route is well-tolerated in mice, tending to demonstrate that these LPS molecular species are less toxic than the synthetic TLR4 agonist GLA.

**Example 6**

**Adjuvant activity of LPS $A_1$ 0.1 and LPS $B_2$ 0.1 in a preclinical mouse vaccination model**

The capacity of the LPS $A_1$ 0.1 and LPS $B_2$ 0.1 to enhance the humoral immune response to the ovalbumin antigen (OVA) was evaluated in a preclinical mouse vaccination model.

The objectives were to demonstrate the adjuvant activity of modified LPS molecular species, and to compare it with the benchmark adjuvants Alhydrogel® (Alum) and MPLA - an analog of the TLR4 agonist MPL (GSK).

***Materials and Methods***

A cohort of BALB/c mice (n = 5 mice/group) was immunized intramuscularly with OVA antigen (Invivogen - 10 pg/dose or 2 μg/dose) formulated with either LPS $A_1$ 0.1 (25 μg/dose) or LPS $B_2$ 0.1 (25 μg/dose), and received a boost intramuscularly 14 days later (D14).

Groups of mice immunized with OVA alone (2 or 10 μg/dose) were used as negative controls.

Groups of mice immunized (prime/boost) with OVA formulated with MPLA (Invivogen - 25 μg/dose) or Alum (100 μg/dose) were included for comparison.

Serum samples were collected on days 28 and 39 from each mouse, and the levels of anti-OVA IgG antibodies were measured by enzyme-linked immunosorbent assay (ELISA).

***Results***

Serum anti-OVA IgG levels, both at days 28 and 39, were significantly higher in mice treated with LPS $A_1$ 0.1 and LPS $B_2$ 0.1 compared to mice treated with OVA without adjuvant (LPS $A_1$ 0.1 *vs* No Adjuvant: P = 0.008 and P = 0.0025; LPS $B_2$ 0.1 *vs* No Adjuvant: P = $6.44 \times 10^{-7}$ and P = 0.006) **(Fig. 26A & 26B).**

Serum anti-OVA IgG levels, both at days 28 and 39, were also significantly higher in mice treated with LPS $A_1$ 0.1 compared to mice treated with Alum or MPLA (LPS $A_1$ 0.1 *vs* Alum: P = 0.018 and P = 0.008; LPS $A_1$ 0.1 *vs* MPLA: P = 0.036 and P = 0.017) **(Fig. 26A).** In addition, although serum anti-OVA IgG levels were slightly increased between days 28 and 39 in mice treated with Alum (P = 0.016), no such difference was observed in the other groups.

***Conclusion***

Results demonstrate the capacity of the LPS $A_1$ 0.1 and LPS $B_2$ 0.1 to boost the humoral immune response to OVA in a preclinical mouse vaccination model, and thus demonstrate their adjuvant activity.

Results also demonstrate that the LPS $A_1$ 0.1 presents an adjuvant activity higher than Alum and MPLA in this preclinical vaccination model.

**Example 7**

**Veterinary applications**

LPS $SM_1$ 0.0, a naturally-occurring LPS molecular species from *Salmonella enterica enterica* serovar *Minnesota* R595 (Re chemotype) with Formula (XII)(0), as shown in **Fig. 27,** was produced using conventional methods for analysis purposes.

In view of an intended veterinary use, LPS $SM_1$ 0.0 was extracted but not purified upon production. The bacterial lysate, comprising LPS $SM_1$ 0.0 and other co-extracted bacterial components (e.g., proteins and the like), was treated to obtain detoxified LPS $SM_1$ V1 with Formula (XII)(1), as shown in **Fig. 27.**

LPS $SM_1$ V1 has lost several fatty chains, as well as its 4-amino-4-deoxy-L-arabinose group (Ara4pN) at position C4' of the non-reducing end of its lipid A domain. A majority (≈ 80-90%) of LPS $SM_1$ V1 maintained its phosphate group at position C4' of the non-reducing end of its lipid A domain, while the remaining 10-20% lost this phosphate group. LPS $SM_1$ V1 thus comprises a mix of LPS molecular species with and without phosphate group at position C4'.

The bacterial lysate comprising LPS $SM_1$ V1 was used for DLS aggregate size analysis, biophysical characterization, pyrogenic activity assays, and preliminary *in vivo* tolerance tests.

LPS $SM_1$ V1 was diluted in PBS pH 7.5 at 50 nmol/mL, and filtered through a 200-nm filter to remove large debris and aggregates. No nucleic acid contaminants and 2-4 μg/mL of protein contaminants were measured in the sample.

DLS measurements (carried out as described in Example 2) showed that the sample was soluble or easily dispersible, forming stable suspensions of monodispersed nano-particles (micelles) whose size ranged from about 6 nm to about 18 nm.

LC-MS/MS and MALDI-TOF analysis was carried out to confirm the structure of LPS $SM_1$ V1 as compared to the native species LPS $SM_1$ 0.0 (data not shown).

Pyrogenic activity assays (carried out as described in Example 4) have shown a MAT (EU/mL) of around $7\times10^5$; using the well-known limulus amebocyte lysate (LAL) test, a very low activity of around 50 EU/mL was measured (*i.e.,* about 0-1 EU/nmol).

100 μL of this sample were administered intravenously to mouse, and their tolerance was followed-up for several days (general condition and weight were observed). On day 1 following administration, the tested mice lost in average 13.4% of their weight, and displayed hair bristling out. Quickly on day 2 after administration, the tested mice starting to get back in shape, until day 3 when the tested mice were healthy. This demonstrates that, in contrast to GLA, the systemic injection of LPS $SM_1$ V1 by intravenous route is well-tolerated in mice.

## Example 8

### Production of chemically-modified LPS molecular species

In the following, the figures "x.y" following the LPS name refer to:

i. first figure ("x"): where 0 means no alkaline treatment, and 1 means one alkaline treatment;
ii. second figure ("y"): where 0 means no acidic hydrolysis treatment, 1 means one acidic hydrolysis treatment with a strong acid (*i.e.,* with $pK_a < 0$) in aqueous solution, 2 means one acidic hydrolysis treatment with a strong acid (*i.e.,* with $pK_a < 0$) in organic solution; 3 means one acidic hydrolysis treatment with a strong acid (*i.e.,* with $pK_a < 0$) in organic solution in presence of an alcohol; and 4 means one acidic hydrolysis treatment with a weak acid (*i.e.,* with $pK_a > 0$) in aqueous solution.

### *Structures*

**"LPS $B_2$ 0.0",** as used herein, refers to a mix of the two naturally-occurring LPS molecular species from *Bordetella pertussis* with Formulas (I)(0)(a) and (I)(0)(b), as shown in **Fig. 1.**

**"LPS X 0.0",** as used herein, refers to the naturally-occurring LPS molecular species from various Enterobacteria with Formula (XIII)(0)(a) and (XIII)(0)(b), as shown in **Fig. 34.**

### *Production & Purification*

LPS $B_2$ 0.0 and LPS X 0.0 were produced and purified using conventional methods, in particular the method described in US patent No. 8,137,935.

### *Chemical modification*

After purification, the LPS molecular species were chemically treated by acidic hydrolysis at a temperature below 85°C; and/or by alkaline hydrolysis, as indicated by the figures "x.y" following the LPS name. If both, alkaline hydrolysis was carried out preferably before acidic hydrolysis.

The innovative process is that acidic hydrolysis at a temperature below 85°C targets dephosphorylation of the lipid A glycosidic phosphate, which is removed *in situ,* in the complete LPS molecular species, *i.e.,* without splitting it in its lipid A and polysaccharide moieties.

It follows from these treatments several LPS molecular species as defined below:

From $B_2$ 0.0 (formulas (I)(0)(a) and (I)(0)(b) shown in **Fig. 1):**

- $B_2$ 1.0 (single alkali treatment): formulas (I)(4)(a), (I)(4)(b) and (I)(4)(c) shown in **Fig. 28;**
- $B_2$ 0.1 (single acid treatment): formulas (I)(1)(a), (I)(1)(b), (I)(1)(c), (I)(1)(d), (I)(1)(e) and (1)(1)(f) shown in **Fig. 2;**
- $B_2$ 0.2 (single acid treatment): formulas (I)(5)(a) and (I)(5)(b) shown in **Fig. 29;**
- $B_2$ 0.3 (single acid treatment): formulas (I)(6)(a) and (I)(6)(b) shown in **Fig. 30;**
- $B_2$ 0.4 (single acid treatment): formulas (I)(7)(a), (I)(7)(b), (I)(7)(c) and (I)(7)(d) shown in **Fig. 31;**
- $B_2$ 1.1 (double alkali/acid treatment): formulas (I)(2)(a), (I)(2)(b) and (I)(2)(c) shown in **Fig. 3;**
- $B_2$ 1.2 (double alkali/acid treatment): formulas (I)(3)(a), (I)(3)(b) and (I)(3)(c) shown in **Fig. 4;**
- $B_2$ 1.3 (double alkali/acid treatment): formulas (I)(8)(a), (I)(8)(b) and (I)(8)(c) shown in **Fig. 32;** and
- $B_2$ 1.4 (double alkali/acid treatment): formulas (I)(9)(a), (I)(9)(b), (I)(9)(c), (I)(9)(d), (I)(9)(e) and (I)(9)(f) shown in **Fig. 33.**

From X 0.0 (formulas (XIII)(0)(a) and (XIII)(0)(b) shown in **Fig. 34):**

- X 1.0 (single alkali treatment): formulas (XIII)(1)(a), (XIII)(1)(b), (XIII)(1)(c), (XIII)(1)(d), (XIII)(1)(e) and (XIII)(1)(f) shown in **Fig. 35;**
- X 0.1 (single acid treatment): formulas (XIII)(2)(a) and (XIII)(2)(b) shown in **Fig. 36;**
- X 0.2 (single acid treatment): formulas (XIII)(3)(a) and (XIII)(3)(b) shown in **Fig. 37;**
- X 0.3 (single acid treatment): formulas (XIII)(4)(a) and (XIII)(4)(b) shown in **Fig. 38;**
- X 0.4 (single acid treatment): formulas (XIII)(5)(a), (XIII)(5)(b), (XIII)(5)(c) and (XIII)(5)(d) shown in **Fig. 39;**
- X 1.1 (double alkali/acid treatment): formulas (XIII)(6)(a), (XIII)(6)(b), (XIII)(6)(c), (XIII)(6)(d), (XIII)(6)(e) and (XIII)(6)(f) shown in **Fig. 40;**
- X 1.2 (double alkali/acid treatment): formulas (XIII)(7)(a), (XIII)(7)(b), (XIII)(7)(c), (XIII)(7)(d), (XIII)(7)(e) and (XIII)(7)(f) shown in **Fig. 41;**
- X 1.3 (double alkali/acid treatment): formulas (XIII)(8)(a), (XIII)(8)(b), (XIII)(8)(c), (XIII)(8)(d), (XIII)(8)(e) and (XIII)(8)(f) shown in **Fig. 42;** and
- X 1.4 (double alkali/acid treatment): formulas (XIII)(9)(a), (XIII)(9)(b), (XIII)(9)(c), (XIII)(9)(d), (XIII)(9)(e), (XIII)(9)(f), (XIII)(9)(g), (XIII)(9)(h), (XIII)(9)(i) and (XIII)(9)(j) shown in **Fig. 43.**

## Example 9

## Biophysical characterization

### *Materials and methods*

Thin Layer Chromatography (TLC)

TLC was carried out on glass HPTLC silica gel plates (Merck). 20 μg of LPS were deposited at the origin of the HPTLC plate, and chromatographed in a solvent mixture of isobutyric acid and 1 M ammonium hydroxide (5:3 v:v) as previously described (Caroff & Karibian, 1990. Appl Environ Microbiol. 56(6):1957-9). Products were visualized by charring (in an oven at 150°C for 5 minutes) after spraying with 10% sulfuric acid in ethanol.

SPS-polyacrylamide gel analysis of LPS

15% poly-acrylamide gels were used, and 0.2 μg of LPS were loaded onto the 4% stacking gel. LPS samples preparation, electrophoresis process and Tsai&Frash Silver Nitrate coloration were performed as previously described (Laemmli, 1970. Nature. 227(5259):680-5; Tsai & Frasch, 1982. Anal Biochem. 119(1):115-9).

Matrix-Assisted Laser Desorption/Ionization (MALDI)-Time-Of-Flight (TOF) analysis

MALDI-TOF was performed as previously described (Chafchaouni-Moussaoui et al., 2011. Rapid Commun Mass Spectrom. 25(14):2043-8), in the linear mode with delayed extraction using a Perseptive Voyager STR (PE Biosystem, France) time-of-flight (TOF) mass spectrometer and/or on a Shimadzu Axima Performance system.

A suspension of lipid A (1 mg/mL) in chloroform:methanol:water (3:1.5:0.25, v:v:v) was desalted with a few grains of Dowex 50W-X8 ($H^+$), and 1 μL was deposited on the target, mixed with 1 μL of gentisic acid (2,5-dihydroxybenzoic acid, DHB) matrix suspended at 10 μg/μL in the same solvent or in 0.1 M aqueous citric acid, and dried.

Analyte ions were desorbed from the matrix with pulses from a 337 nm nitrogen laser. Spectra were obtained in the negative-ion mode at 20 kV.

When LPS were analyzed directly, they were suspended in water and decationized in the same way, using the same matrix and conditions.

### *Results*

The mass-to-charge ratios (m/z) of the different LPS molecular species described herein are reported in **Table 5.** They correspond to $[M-H]^-$ ions of LPS molecular species, and their core and lipid A moieties fragments.

**Table 5**

| **LPS $B_2$ 1.2** (*Bordetella pertussis)* **(Fig. 4)** | | |
|---|---|---|
| **Calculated masses $[M-H]^-$** | **Observed masses $[M-H]^-$** | **Interpretation** |
| 872.03 | 872.0 | Lipid A 1 |
| 1082.03 | 1082.3 | Lipid A2 |
| 1308.75 | 1308.7 | Lipid A3 |
| 2293.04 | 2292.8 | PS1 |
| 2373.02 | 2373.1 | PS2 |
| 3166.07 | 3167.5 | LPS=Lipid A1+PS1 |
| 3246.05 | 3247.3 | LPS=Lipid A1+PS2 |
| 3376.07 | 3375.8 | LPS=Lipid A2+PS1 |
| 3456.07 | 3456.2 | LPS=Lipid A2+PS2 |
| 3602.79 | 3600.7 | LPS=Lipid A3+PS1 |
| 3682.79 | 3681.4 | LPS=Lipid A3+PS2 |
| **LPS $B_2$ 1.3** (*Bordetella pertussis)* **(Fig. 32)** | | |
| **Calculated masses $[M-H]^-$** | **Observed masses $[M-H]^-$** | **Interpretation** |
| 872.03 | Non detected | Lipid A1 |
| 900.1 | Non detected | Lipid A1'=Lipid A1+Ethyl |
| 1082.03 | 1082.2 | Lipid A2 |
| 1110.4 | 1110.5 | Lipid A2'=Lipid A2+Ethyl |
| 1308.75 | 1308.8 | Lipid A3 |
| 1336.8 | 1336.7 | Lipid A3'=Lipid A3+Ethyl |
| 2293.04 | 2292.9 | PS1 |
| 2373.02 | 2373.0 | PS2 |
| 3166.07 | 3166.8 | LPS=Lipid A1+PS1 |
| 3194.2 | 3195.4 | LPS=Lipid A1'+PS1 |
| 3376.07 | 3376.5 | LPS=Lipid A2+PS1 |
| 3456.07 | 3456.2 | LPS=Lipid A2+PS2 |
| 3484.2 | 3485.2 | LPS=Lipid A2'+PS2 |
| 3630.9 | 3629.9 | LPS=Lipid A3'+PS1 |
| 3682.79 | 3681.4 | LPS=Lipid A3+PS2 |
| **LPS $B_2$ 0.4** (*Bordetella pertussis)* **(Fig. 31)** | | |
| **Calculated masses $[M-H]^-$** | **Observed masses $[M-H]^-$** | **Interpretation** |
| 1479.0 | 1479.3 | Lipid A (1P,2GlcN,5FA) |
| 2293.04 | 2292.7 | PS |
| 2908.9 | 2908.2 | LPS=LipA(0P, 1GlcN,2FA)*+PS |
| 3466.6 | 3466.6 | LPS=LipA(0P,2GlcN,4FA)*+PS |
| 3693.0 | 3692.2 | LPS=LipA(0P,2GlcN,5FA)*+PS |
| 3773.0 | 3772.8 | LPS=LipA(1P,2GlcN,5FA)*+PS |

(continued)

| LPS $B_2$ 1.4 (*Bordetella pertussis*) (Fig. 33) | | |
|---|---|---|
| **Calculated masses [M-H]$^-$** | **Observed masses [M-H]$^-$** | **Interpretation** |
| 2293.04 | 2292.7 | PS |
| 2908.9 | 2908.6 | LPS=LipA(0P, 1GlcN,2FA)**+PS |
| 3086.0 | 3085.8 | LPS=LipA(0P,2GlcN,2FA)**+PS |
| 3296.0 | 3296.1 | LPS=LipA(0P,2GlcN,3FA)**+PS |
| 3522.8 | 3522.5 | LPS=LipA(0P,2GlcN,4FA)**+PS |
| * LipA(0P,1GlcN,2FA): non-phosphorylated mono-glucosamine di-acyl lipid A<br>* LipA(0P,2GlcN,4FA): non-phosphorylated di-glucosamine tetra-acyl lipid A<br>* LipA(0P,2GlcN,5FA): non-phosphorylated di-glucosamine penta-acyl lipid A<br>* LipA(1P,2GlcN,5FA): mono-phosphoryl di-glucosamine penta-acyl lipid A<br>** LipA(0P,1GlcN,2FA): non-phosphorylated mono-glucosamine di-acyl lipid A<br>** LipA(0P,2GlcN,2FA): non-phosphorylated di-glucosamine di-acyl lipid A<br>** LipA(0P,2GlcN,3FA): non-phosphorylated di-glucosamine tri-acyl lipid A<br>** LipA(0P,2GlcN,2FA): non-phosphorylated di-glucosamine di-acyl lipid A | | |

MALDI-MS spectra analysis of LPS $B_2$ 1.2, LPS $B_2$ 1.3, LPS $B_2$ 0.4 and LPS $B_2$ 1.4 **(Fig.** 44)

*LPS $B_2$ 1.2*

Three main lipid A related fragments are observed at *m/z:* 872, 1082, 1308, corresponding to mono-phosphoryl di-, tri- and tetra-acyl lipid A molecular species. Two major PS related fragments are observed at *m/z* 2293 (PS1) and 2373 (PS2), corresponding to dodecasaccharide cores with respectively non-substituted Kdo and Kdo substituted with a phosphate group. LPS molecular ions correspond to different combinations of these lipid A and PS structures, they are observed at *m/z* 3167, 3247, 3376, 3456, 3601 and 3681.

*LPS $B_2$ 1.3*

Several peaks common with the modified LPS $B_2$ 1.2 spectrum are observed, *e.g.,* at *m/z* 1082 and 1308 for lipid A related fragments, at *m/z* 2293 (PS1) and 2373 (PS2) for PS related fragments and at *m/z* 3167, 3376, 3456, 3681 for LPS molecular ions. Additional peaks are observed corresponding to molecular species substituted with an ethyl group at position C1 of the reducing end of their lipid A domain (*i.e.,* at +28 mass units relatively to peaks of non-substituted molecular species). These new peaks are observed at *m/z* 1110 and 1336 for lipid A related fragments and at *m/z* 3195, 3485 and 3629 for LPS molecular ions.

*LPS $B_2$ 0.4*

Molecular species detected in modified LPS $B_2$ 0.4 contain either mono-phosphoryl lipid A or non-phosphorylated lipid A (see the phosphate group at position C4 of the non-reducing end of the lipid A domain in parenthesis in **Fig. 31).** Mono-phosphoryl penta-acyl lipid A molecular species is observed as a fragment ion at *m/z* 1479. Non-phosphorylated lipid A related fragments are non-detectable in the negative-ion mode. The only PS related fragment ion is observed at *m/z* 2293 and correspond to dodecasaccharide with non-substituted Kdo. LPS molecular ions are observed at *m/z* 2908, 3466, 3692 and 3773, they correspond to the non-phosphorylated core of twelve sugars attached to respectively non-phosphorylated mono-glucosamine di-acyl lipid A, non-phosphorylated di-glucosamine tetra-acyl lipid A, non-phosphorylated di-glucosamine penta-acyl lipid A and mono-phosphoryl di-glucosamine penta-acyl lipid A.

*LPS $B_2$ 1.4*

Molecular species detected in modified LPS $B_2$ 1.4 contain predominantly non-phosphorylated lipid A (see the phosphate group at position C4 of the non-reducing end of the lipid A domain in parenthesis in **Fig. 33).** Non-phosphorylated lipid A related fragments are non-detectable in the negative-ion mode. The only PS related fragment ion is observed at *m/z* 2293 and corresponds to dodecasaccharide with non-substituted Kdo. LPS molecular ions are observed at *m/z* 2908, 3085, 3296 and 3522, they correspond to the non-phosphorylated core of twelve sugars attached to

respectively non-phosphorylated mono-glucosamine di-acyl lipid A, non-phosphorylated di-glucosamine di-acyl lipid A, non-phosphorylated di-glucosamine tri-acyl lipid A and non-phosphorylated di-glucosamine tetra-acyl lipid A.

## Example 10

### *In vivo* pyrogenic activity of LPS $B_2$ 1.2 *versus* LPS $B_2$ 1.3

The pyrogenic activity of modified LPS $B_2$ 1.2 and LPS $B_2$ 1.3 was evaluated *in vivo* using the rabbit pyrogen test (RPT), as described in section 5.1.10 of the European Pharmacopeia. Both LPS were administered at the same dose of 450 ng/kg. Two experiments were performed for each modified LPS, with 3 rabbits/experiment (A1, A2 and A3).

### *Results*

**Table 6** summarizes the temperature increase (individual and sum of three animals) observed for modified LPS $B_2$ 1.2 and LPS $B_2$ 1.3 in two RPT experiments.

**Table 6**

| Modified LPS | | Temperature increase | |
|---|---|---|---|
| | | Experiment 1 | Experiment 2 |
| $B_2$ 1.2 | A1 | 0.75°C | 1.1°C |
| | A2 | 1.05°C | 0.70°C |
| | A3 | 1.10°C | 0.70°C |
| | **Sum** | **2.7°C** | **2.5°C** |
| $B_2$ 1.3 | A1 | 0.50°C | 0.45°C |
| | A2 | 0.25°C | 0.65°C |
| | A3 | 0.50°C | 1.00°C |
| | **Sum** | **1.25°C** | **2.1°C** |

It follows from these results that the additional substitution with an ethyl group at position C1 of the reducing end of the lipid A domain in LPS $B_2$ 1.3 leads to substantial decrease in pyrogenicity when compared with LPS $B_2$ 1.2 in which position C1 of the reducing end comprises an unsubstituted hydroxyl group (as seen by the reduced increase in temperature in all three rabbits, for each duplicate experiment).

## Example 11

### Therapeutic efficacy of modified LPS as monotherapy on a syngeneic mouse model of colorectal carcinoma

Therapeutic efficacy of modified LPS $B_2$ 0.1, LPS $B_2$ 1.1 and LPS $B_2$ 1.3 was evaluated on a syngeneic mouse model of colorectal carcinoma (MC38). The objectives were to demonstrate and compare the efficacy of selected modified LPS as stand-alone treatment against cancer.

### *Materials and methods*

The therapeutic efficacies of modified LPS $B_2$ 0.1, LPS $B_2$ 1.1 and LPS $B_2$ 1.3 were analyzed *in vivo* against a mouse model of colorectal carcinoma (MC38 cells) using tumor rejection assays with a therapeutic setting.
C57BL/6 mice (4- to 6-week-old) were injected subcutaneously (s.c.) into the right flank with $2\times10^6$ MC38 tumor cells. When tumor volumes reached 150 $mm^3$ (day 6), mice were randomized into LPS $B_2$ 0.1, LPS $B_2$ 1.1 and LPS $B_2$ 1.3 groups (n = 7 mice per group).
A group of mice injected with PBS was used as control.
Modified LPS were administrated intravenously into the tail vein, once a week at 0.5 mg/kg/dose.
Tumor growth was monitored twice a week by caliper measurement and determined using the formula:

$$Tumor\ volume = \frac{4}{3} \times \pi \times r^3$$

### *Results*

**Fig. 45** illustrates the therapeutic efficacy of modified LPS $B_2$ 0.1, LPS $B_2$ 1.1 and LPS $B_2$ 1.3 on a syngeneic mouse model of colorectal carcinoma (MC38).

Treatment with modified LPS $B_2$ 0.1, LPS $B_2$ 1.1 and LPS $B_2$ 1.3 was found to significantly impair the growth of established MC38 tumor cells compared to control mice **(Fig. 45A).** This was observed only 2 days post-tumor treatment and confirmed on day 11 **(Fig. 45B).** On day 13, a significant tumor growth reduction was only observed in the groups of mice treated with LPS $B_2$ 1.1 and LPS $B_2$ 1.3. No differences in tumor growth were observed between the groups of animals that received one of the modified LPS, although a slightly more marked tumor growth reduction was observed within the LPS $B_2$ 1.3 group on day 13.

### *Conclusion*

These results provide proof-of-concept of the therapeutic efficacy of the modified LPS $B_2$ 0.1, LPS $B_2$ 1.1 and LPS $B_2$ 1.3 as a monotherapy against a syngeneic mouse model of colorectal carcinoma and suggest that the modified LPS $B_2$ 1.3 is slightly more effective than LPS $B_2$ 0.1 and LPS $B_2$ 1.1.

## Example 12

### Therapeutic efficacy of modified LPS as monotherapy on an orthotopic rat model of osteosarcoma

Therapeutic efficacy of a modified LPS as monotherapy was evaluated against an orthotopic rat model of osteosarcoma (Osa) and was compared to a chemotherapeutic agent (Doxorubicin) commonly used as standard of care in this indication.

### *Materials and methods*

The therapeutic efficacy of the modified LPS $B_2$ 1.3 was analyzed *in vivo* against an orthotopic rat model of osteosarcoma (Osa) using tumor rejection assays with a therapeutic setting.

Sprague-Dawley (SD) rats were engrafted with Osa tumor cells in a paratibial position (left posterior tibia) after a periosteal abrasion. When tumors reached an average tumor volume of 150 $mm^3$, rats (n = 3 to 8/group) were randomized into LPS $B_2$ 1.3, Doxorubicin (Doxo), or PBS (Control) groups.

LPS $B_2$ 1.3 was administered intravenously into the tail vein, twice a week for 2 weeks at 0.25 mg/kg.

Doxorubicin was administered intraperitoneally twice a week for 2 weeks at 1 mg/kg.

Tumor growth and survival were monitored twice a week. Tumor volumes V were assessed according to the following formula:

$$V = 0.5 * L * C$$

with L the longest and C the shortest tumor diameter.

### *Results*

**Fig. 46** illustrates the therapeutic efficacy of the modified LPS $B_2$ 1.3 on an orthotopic rat model of Osteosarcoma (Osa).

Treatment with modified LPS $B_2$ 1.3 as monotherapy was found to significantly impair the growth of established Osa tumor cells compared to rats that received doxorubicin (P = 0.03 on day 28 post-tumor challenge) or PBS as a control (P = 0.05 on day 31 post-tumor challenge) **(Fig. 46A).**

Treatment with modified LPS $B_2$ 1.3 was also found to induce complete tumor regressions in 37.5 % of the treated rats (complete responses (CR) on day 31 - **Fig. 46D)** while no tumor regressions were observed in the groups of animals that received PBS or doxorubicin **(Figs. 46B** and **46C,** respectively).

This was associated with an increased significant improvement in the survival of animals. Approximately 90 % of animals that received LPS $B_2$ 1.3 were alive on day 31. By contrast, only 40 % of animals that received doxorubicin and 0 % of animals that received PBS as a control were alive on day 31 **(Fig. 46E).**

No significant difference in tumor growth and percentages of complete responses were observed among the groups of rats that received doxorubicin or PBS as a control, although percentage of animals alive on day 31 were higher in the doxorubicin group.

***Conclusion***

These results provide preclinical proof-of-concept of the high therapeutic efficacy of the modified LPS $B_2$ 1.3 as standalone treatment for osteosarcoma and demonstrate that the modified LPS $B_2$ 1.3 is significantly more effective than doxorubicin, a standard-of-care in this indication.

**Example 13**

**Synergistic antitumor effect of modified LPS with immune checkpoint inhibitors**

Therapeutic efficacy of the modified LPS $B_2$ 1.3 as monotherapy or in combination with an immune checkpoint inhibitor (ICI) (anti-mouse PD-1 monoclonal antibody, clone RMP1-14) was evaluated on a syngeneic mouse model of colorectal carcinoma (MC38). The objectives were to demonstrate the capacity of the modified LPS $B_2$ 1.3 to boost the therapeutic efficacy of an ICI.

***Materials and methods***

The therapeutic efficacy of the modified LPS $B_2$ 1.3, alone and in combination with an ICI, was analyzed *in vivo* against a mouse model of colorectal carcinoma (MC38 cells) using tumor rejection assays with a therapeutic setting.

C57BL/6 mice (4- to 6-week-old) were injected subcutaneously (s.c.) into the right flank with $2\times10^6$ MC38 tumor cells. When tumor volumes reached 150 $mm^3$ (day 6), mice were randomized, and the first treatment was administered.

Groups of mice (n = 7 per group) were treated once a week for 2 weeks with the modified LPS $B_2$ 1.3 (0.5 mg/kg in i.v.) alone or in combination with an anti-mouse PD-1 monoclonal antibody (clone RMP1-14 - 12.5 mg/kg in i.p.).

Groups of mice administered with the anti-mouse PD-1 monoclonal antibody alone, or with PBS, were used as controls.

Tumor growth was monitored twice a week by caliper measurement and determined using the formula:

$$Tumor\ volume = \frac{4}{3} \times \pi \times r^3$$

Mice from anti-PD-1 (n = 2) and LPS $B_2$ 1.3 + anti-PD-1 (n = 2) groups, which totally eradicated MC38 tumor cells in primary responses, were rechallenged with live $2\times10^6$ MC38 cells.

Age-matched naive mice (n = 3) were used as control.

Tumor growth was monitored twice a week by caliper measurement.

***Results***

**Fig. 47** illustrates the therapeutic efficacy of the modified LPS $B_2$ 1.3, as monotherapy or in combination with an anti-PD-1 mAb, on a syngeneic mouse model of colorectal carcinoma (MC38).

**Fig. 48** illustrates the capacity of the modified LPS $B_2$ 1.3 to boost the induction of effective anti-tumor memory responses against MC38 tumor cells with an anti-PD-1 mAb during tumor rechallenge assays.

Treatment with the modified LPS $B_2$ 1.3 and the anti-mouse PD-1 mAb as monotherapies were found to significantly impair the growth of established MC38 tumor cells as compared to control mice **(Fig. 47A).** Anti-PD-1 mAb was however significantly more effective than the modified LPS $B_2$ 1.3 in this model in terms of tumor growth reduction (P = 0.042 on day 13) and rate of complete responses (CR). While 29 % of complete tumor regressions were observed in animals that received the anti-PD1 mAb as monotherapy **(Fig. 47D),** 0 % were observed in animals that received PBS as a control **(Fig. 47B)** or the modified LPS $B_2$ 1.3 as standalone treatment **(Fig. 47C).**

The combination of LPS $B_2$ 1.3 with the anti-PD1 mAb was found to be more effective than anti-PD-1 mAb alone (P = 0.025 on day 18) and LPS $B_2$ 1.3 alone (P = 0.016 on day 13) to impair the growth of established MC38 tumor cells **(Fig. 47A).** This was associated with an increased rate of complete responses: 33.3 % for combination group on day 18 (Fig. **47E)** *vs* 29 % and 0 % for anti-PD-1 **(Fig. 47D)** and LPS $B_2$ 1.3 **(Fig. 47C)** groups, respectively. This was also associated with significant improvement in the survival of animals. Approximately 67 % of animals that received the combination of LPS $B_2$ 1.3 and anti-PD1 mAb were alive on day 27 post-tumor challenge. By contrast, only 29 % of animals that received

anti-PD1 mAb as monotherapy and 0 % of animals that received LPS $B_2$ 1.3 as monotherapy or PBS as a control were alive on day 27 **(Fig. 47F).**

All the mice from the anti-PD-1 and LPS $B_2$ 1.3 + anti-PD-1 groups, which totally eradicated MC38 tumor cells in primary responses, were used for a rechallenge study with MC38 tumor cells. Mice treated with LPS $B_2$ 1.3 in combination with anti-PD-1 were all found to be resistant to a secondary challenge with new MC38 tumor cells **(Fig. 48A),** associated with long-term survival of 100 % of animals that received the combination on day 71 post-tumor challenge **(Fig. 48B).** By contrast, only 50 % of the mice treated with the anti-PD-1 mAb alone were alive on day 71 and 0 % among the control group of naive mice.

### *Conclusion*

These results demonstrate the capacity of the modified LPS $B_2$ 1.3 to boost the therapeutic efficacy of ICI against established tumors, associated with increased rates of complete responses and survival. Results also demonstrate the capacity of the modified LPS $B_2$ 1.3 to potentiate the capacity of the anti-PD-1 mAb to induce effective anti-tumor memory responses against MC38 tumor cells for long lasting protection against relapses.

Altogether, these results provide proof-of-concept of synergistic antitumor effect of the modified LPS $B_2$ 1.3 with ICI to control the growth and eradicate established tumors *in vivo,* but also to prevent relapse or recurrence of the tumors.

## Example 14

### Adjuvant activity of LPS $B_2$ 0.1 in a preclinical mouse vaccination model

The capacity of the modified LPS $B_2$ 0.1 to enhance the humoral immune response to the SARS-CoV-2 spike receptor-binding domain (RBD) was evaluated in a preclinical mouse vaccination model. The objectives were to demonstrate the adjuvant activity of the modified LPS $B_2$ 0.1, and to compare it with a non-GMP equivalent of the MPL (GSK), a benchmark LPS derivative currently used as vaccine adjuvant in human vaccines.

### *Materials and methods*

Three cohorts of BALB/c mice (n = 5/group) were immunized intramuscularly three times at 2 weeks interval with a recombinant SARS-CoV-2 Spike RBD domain (RBD - 10 μg/dose) alone or formulated with the modified LPS $B_2$ 0.1 or MPLA (Invivogen - non-GMP equivalent of MPL).

MPLA and LPS $B_2$ 0.1 were administered at 20 μg/dose.

Anti-RBD IgG, IgG1 et IgG2a antibody levels were measured by ELISA on blood samples collected on days 18, 25 and 39.

### *Results*

**Fig. 49** illustrates the capacity of the modified LPS $B_2$ 0.1 to boost the humoral immune response against a recombinant SARS-CoV-2 spike receptor-binding domain (RBD) in a preclinical mouse vaccination model.

Higher serum levels of total anti-RBD IgG antibody were detected in the group of mice that received the recombinant SARS-CoV-2 spike RBD formulated with LPS $B_2$ 0.1 compared to control group of mice that only received the recombinant RBD antigen both on day 18 ($P = 7\times10^{-4}$), and day 25 ($P = 9\times10^{-4}$) and notably on day 39 (P = 0.001) **(Fig. 49A).**

The higher increase of total anti-RBD IgG antibody levels were observed after the second boost (day 25 *vs* day 39: $P = 3\times10^{-5}$). No significant differences were observed between the LPS $B_2$ 0.1 and MPLA groups on days 18 and 25, but significantly higher average levels of total RBD-specific IgG antibody were detected on day 39 in the LPS $B_2$ 0.1 group compared to MPLA group (P = 0.005). This was associated with the presence of higher average levels of anti-RBD IgG1 **(Fig. 49B)** and notably IgG2a **(Fig. 49C)** antibodies in the groups of mice treated with the recombinant antigen formulated with LPS $B_2$ 0.1 compared to MPLA (P = 0.009 and 0.015 respectively) and control groups (P = 0.002 and 0.05 respectively). Accordingly, higher average IgG2a/IgG1 ratio were observed in the LPS $B_2$ 0.1 group **(Fig. 49D)** compared to MPLA group (P = 0.02) demonstrating a polarization of the immune responses towards a more $Th_1$ profile.

### *Conclusion*

Results of this study demonstrate the capacity of the modified LPS $B_2$ 0.1 to potentiate the induction of specific humoral immune responses in a preclinical mouse vaccination model against a SARS-CoV2 antigen, and thus demonstrate its adjuvant activity.

Results also demonstrate that the modified LPS $B_2$ 0.1 presents a better adjuvant activity than a non GMP equivalent of the benchmark MPL, associated with a polarization of the immune responses toward a more marked $Th_1$-like phenotype.

These results thus confirmed the results of our previous study conduct in a preclinical mouse vaccination model against OVA antigen.

## Example 15

### Synergistic anti-tumor effect of modified LPS in combination of chemotherapy

The efficacy of modified LPS $B_2$ 0.1, LPS $B_2$ 1.1 and LPS $B_2$ 1.3 as monotherapy or in combination with chemotherapy was evaluated on human osteosarcoma cells in 3D-cell culture. The objective was to evaluate the synergistic antitumor effect of the modified LPS with chemotherapy.

### *Materials and methods*

3D-cultures of human osteosarcoma cells (Saos-2 cells - 2500 cells/well in very low attachment 96 well plates) were incubated (ratio target/effector: 1/3; n = 6 wells/group) with human monocyte-derived macrophages (THP-1 incubated with PMA - 150 nM/days for 48 hours), stimulated or not with one of the modified LPS $B_2$ 0.1, LPS $B_2$ 1.1 or LPS $B_2$ 1.3 (100 ng/ml for 16 hours).

Cultures were performed in presence or absence of a chemotherapeutic agent (etoposide - 5 μM final). Cultures without etoposide and stimulated macrophages are used as control.

IncuCyte® Green Caspase-3/7 (2.5 μM final) is added in each culture well to monitor tumor cell apoptosis.

After 24 hours of incubation, number of apoptotic Saos-2 tumor cells is evaluated in each 3D-cell culture by fluorescence microscopy and Incucyte® live-cell analysis.

## Claims

1. An enriched population of modified lipopolysaccharide (LPS) molecular species, comprising at least 80 % of modified LPS molecular species, wherein said modified LPS molecular species:

    - are devoid of phosphate group at position C1 of the reducing end of their lipid A domain; and
    - are substituted at position C6' of the non-reducing end of their lipid A domain by a hydrophilic moiety, with the proviso that said hydrophilic moiety is not a hydroxyl group; preferably are substituted at position C6' of the non-reducing end of their lipid A domain by a mono-, oligo- or poly-saccharide moiety.

2. The enriched population of modified LPS molecular species according to claim 1, wherein the modified LPS molecular species are of general **Formula A**

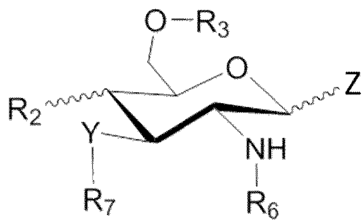

**Formula A**

wherein:

- Y is -O- or -NH-;
- $R_2$ is a phosphate group ($-OPO_3H_2$) or a hydroxyl group (-OH); optionally substituted;
- $R_3$ is a monosaccharide, an oligosaccharide or a polysaccharide; optionally substituted; preferably $R_3$ is a core oligosaccharide (i.e., an inner core, or an inner and outer core) optionally substituted with a O-antigen domain;
- $R_6$ is a fatty acid residue; optionally substituted;
- $R_7$ is a hydrogen or a fatty acid residue; optionally substituted;

- Z is a hydroxyl group (-OH), optionally substituted, with the proviso that, if substituted, the substitution does not consist of a phosphate group ($-OPO_3H_2$), or
Z is

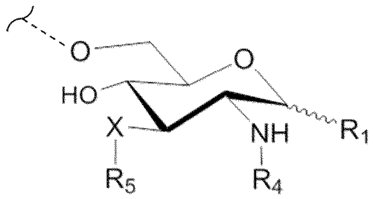

,

wherein

- X is -O- or -NH-;
- $R_1$ is a hydroxyl group (-OH), optionally substituted, with the proviso that, if substituted, the substitution does not consist of a phosphate group ($-OPO_3H_2$);
- $R_4$ is a fatty acid residue; optionally substituted; and
- $R_5$ is a hydrogen or a fatty acid residue; optionally substituted;

preferably wherein the modified LPS molecular species are of general **Formula B**

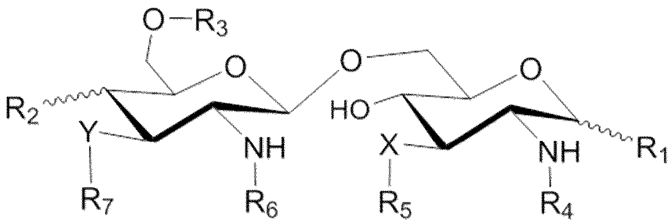

**Formula B**

wherein:

- X and Y are, independently from each other, -O- or -NH-;
- $R_1$ is a hydroxyl group (-OH), optionally substituted, with the proviso that, if substituted, the substitution does not consist of a phosphate group ($-OPO_3H_2$); and $R_2$ is a phosphate group ($-OPO_3H_2$) or a hydroxyl group (-OH), optionally substituted;
- $R_3$ is a mono-, oligo- or poly-saccharide moiety; optionally substituted; preferably $R_3$ is a core oligosaccharide optionally substituted with an antigen domain, preferably an O-antigen domain and/or a capping antigen;
- $R_4$ and $R_6$ are, independently from each other, a fatty acid residue; optionally substituted; and
- $R_5$ and $R_7$ are, independently from each other, a hydrogen or a fatty acid residue; optionally substituted.

**3.** The enriched population of modified LPS molecular species according to claim **1** or **2,** wherein the modified LPS molecular species are selected from the group comprising LPS molecular species of Formulas (I)(1)(a), (I)(1)(b), (I)(1)(c), (I)(1)(d), (I)(1)(e), (I)(1)(f), (I)(2)(a), (I)(2)(b), (I)(2)(c), (I)(3)(a), (I)(3)(b), (I)(3)(c), (I)(5)(a), (I)(5)(b), (I)(6)(a), (I)(6)(b), (I)(7)(a), (I)(7)(b), (I)(7)(c), (I)(7)(d), (I)(8)(a), (I)(8)(b), (I)(8)(c), (I)(9)(a), (I)(9)(b), (I)(9)(c), (I)(9)(d), (I)(9)(e), (I)(9)(f), (II)(2)(a), (II)(2)(b), (II)(2)(c), (II)(2)(d), (III)(1)(a), (III)(1)(b), (III)(1)(c), (III)(1)(d), (IV)(1)(a), (IV)(1)(b), (IV)(1)(c), (V)(1)(a), (V)(1)(b), (V)(1)(c), (V)(1)(d), (V)(1)(e), (V)(1)(f), (V)(1)(g), (V)(1)(h), (V)(1)(i), (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j), (VI)(2)(a), (VI)(2)(b), (VI)(2)(c), (VII)(1)(a), (VII)(1)(b), (VII)(1)(c), (VIII)(1)(a), (VIII)(1)(b), (VIII)(1)(c), (VIII)(1)(d), (IX)(1)(a), (IX)(1)(b), (IX)(1)(c), (IX)(1)(d), (X)(1)(a), (X)(1)(b), (X)(1)(c), (X)(1)(d), (XI)(1)(a), (XI)(1)(b), (XI)(1)(c), (XI)(1)(d), (XIII)(2)(a), (XIII)(2)(b), (XIII)(3)(a), (XIII)(3)(b), (XIII)(4)(a), (XIII)(4)(b), (XIII)(5)(a), (XIII)(5)(b), (XIII)(5)(c), (XIII)(5)(d), (XIII)(6)(a), (XIII)(6)(b), (XIII)(6)(c), (XIII)(6)(d), (XIII)(6)(e), (XIII)(6)(f), (XIII)(7)(a), (XIII)(7)(b), (XIII)(7)(c), (XIII)(7)(d), (XIII)(7)(e), (XIII)(7)(f), (XIII)(8)(a), (XIII)(8)(b), (XIII)(8)(c), (XIII)(8)(d), (XIII)(8)(e), (XIII)(8)(f), (XIII)(9)(a), (XIII)(9)(b), (XIII)(9)(c), (XIII)(9)(d), (XIII)(9)(e), (XIII)(9)(f), (XIII)(9)(g), (XIII)(9)(h), (XIII)(9)(i), (XIII)(9)(j), and a combination thereof as shown in **Figures 2-4, 7-17, 29-33** and **36-43,**
wherein **R** at position C6' is a mono-, oligo- or polysaccharidic moiety comprising a core oligosaccharide domain and

optionally, an antigen domain, such as an O-antigen domain and/or a capping antigen; and wherein **X** at position C1 is an ethyl moiety.

**4.** The enriched population of modified LPS molecular species according to any one of claims **1** to **3,** wherein the modified LPS molecular species form particles having an average size smaller than about 200 nm, preferably smaller than about 150 nm in diameter in aqueous solutions, as can be assessed by dynamic light scattering (DLS) at 25°C.

**5.** A composition comprising an enriched population of modified LPS molecular species according to any one of claims **1** to **4.**

**6.** The composition according to claim **5,** wherein said composition comprises:

i. an enriched population comprising a mix of at least two modified LPS molecular species of Formulas (I)(1)(a), (I)(1)(b), (I)(1)(c), (I)(1)(d), (I)(1)(e) and (1)(1)(f); preferably a mix of the six modified LPS molecular species; or
ii. an enriched population comprising a mix of at least two modified LPS molecular species of Formulas (I)(2)(a), (I)(2)(b) and (I)(2)(c); preferably a mix of the three modified LPS molecular species; or
iii. an enriched population comprising a mix of at least two modified LPS molecular species of Formulas (I)(3)(a), (I)(3)(b) and (I)(3)(c); preferably a mix of the three modified LPS molecular species; or
iv. an enriched population comprising a mix of at least two modified LPS molecular species of Formulas (I)(5)(a) and (I)(5)(b); preferably a mix of the two modified LPS molecular species; or
v. an enriched population comprising a mix of at least two modified LPS molecular species of Formulas (I)(6)(a) and (I)(6)(b); preferably a mix of the two modified LPS molecular species; or
vi. an enriched population comprising a mix of at least two modified LPS molecular species of Formulas (I)(7)(a), (I)(7)(b), (I)(7)(c) and (I)(7)(d); preferably a mix of the four modified LPS molecular species; or
vii. an enriched population comprising a mix of at least two modified LPS molecular species of Formulas (I)(8)(a), (I)(8)(b) and (I)(8)(c); preferably a mix of the three modified LPS molecular species; or
viii. an enriched population comprising a mix of at least two modified LPS molecular species of Formulas (I)(9)(a), (I)(9)(b), (I)(9)(c), (I)(9)(d), (I)(9)(e) and (1)(9)(f); preferably a mix of the six modified LPS molecular species; or
ix. an enriched population comprising a mix of at least two modified LPS molecular species of Formulas (II)(2)(a), (II)(2)(b), (II)(2)(c), (II)(2)(d); preferably a mix of the four modified LPS molecular species; or
x. an enriched population comprising a mix of at least two modified LPS molecular species of Formulas (III)(1)(a), (III)(1)(b), (III)(1)(c), (III)(1)(d); preferably a mix of the four modified LPS molecular species; or
xi. an enriched population comprising a mix of at least two modified LPS molecular species of Formulas (IV)(1)(a), (IV)(1)(b), (IV)(1)(c); preferably a mix of the three modified LPS molecular species; or
xii. an enriched population comprising a mix of at least two modified LPS molecular species of Formulas (V)(1)(a), (V)(1)(b), (V)(1)(c), (V)(1)(d), (V)(1)(e), (V)(1)(f), (V)(1)(g), (V)(1)(h), (V)(1)(i); preferably a mix of the nine modified LPS molecular species; or
xiii. an enriched population comprising a mix of at least two modified LPS molecular species of Formulas (VI)(1)(a), (VI)(1)(b), (VI)(1)(c), (VI)(1)(d), (VI)(1)(e), (VI)(1)(f), (VI)(1)(g), (VI)(1)(h), (VI)(1)(i), (VI)(1)(j); preferably a mix of the ten modified LPS molecular species; or
xiv. an enriched population comprising a mix of at least two modified LPS molecular species of Formulas (VI)(2)(a), (VI)(2)(b), (VI)(2)(c); preferably a mix of the three modified LPS molecular species; or
xv. an enriched population comprising a mix of at least two modified LPS molecular species of Formulas (VII)(1)(a), (VII)(1)(b), (VII)(1)(c); preferably a mix of the three modified LPS molecular species; or
xvi. an enriched population comprising a mix of at least two modified LPS molecular species of Formulas (VIII)(1)(a), (VIII)(1)(b), (VIII)(1)(c), (VIII)(1)(d); preferably a mix of the four modified LPS molecular species; or
xvii. an enriched population comprising a mix of at least two modified LPS molecular species of Formulas (IX)(1)(a), (IX)(1)(b), (IX)(1)(c), (IX)(1)(d); preferably a mix of the four modified LPS molecular species; or
xviii. an enriched population comprising a mix of at least two modified LPS molecular species of Formulas (X)(1)(a), (X)(1)(b), (X)(1)(c), (X)(1)(d); preferably a mix of the four modified LPS molecular species; or
xix. an enriched population comprising a mix of at least two modified LPS molecular species of Formulas (XI)(1)(a), (XI)(1)(b), (XI)(1)(c), (XI)(1)(d); preferably a mix of the four modified LPS molecular species; or
xx. an enriched population comprising a mix of at least two modified LPS molecular species of Formulas (XIII)(2)(a) and (XIII)(2)(b); preferably a mix of the two modified LPS molecular species; or
xxi. an enriched population comprising a mix of at least two modified LPS molecular species of Formulas (XIII)(3)(a) and (XIII)(3)(b); preferably a mix of the two modified LPS molecular species; or
xxii. an enriched population comprising a mix of at least two modified LPS molecular species of Formulas (XIII)(4)(a) and (XIII)(4)(b); preferably a mix of the two modified LPS molecular species; or

xxiii. an enriched population comprising a mix of at least two modified LPS molecular species of Formulas (XIII)(5)(a), (XIII)(5)(b), (XIII)(5)(c) and (XIII)(5)(d); preferably a mix of the four modified LPS molecular species; or
xxiv. an enriched population comprising a mix of at least two modified LPS molecular species of Formulas (XIII)(6)(a), (XIII)(6)(b), (XIII)(6)(c), (XIII)(6)(d), (XIII)(6)(e) and (XIII)(6)(f); preferably a mix of the six modified LPS molecular species; or
xxv. an enriched population comprising a mix of at least two modified LPS molecular species of Formulas (XIII)(7)(a), (XIII)(7)(b), (XIII)(7)(c), (XIII)(7)(d), (XIII)(7)(e) and (XIII)(7)(f); preferably a mix of the six modified LPS molecular species; or
xxvi. an enriched population comprising a mix of at least two modified LPS molecular species of Formulas (XIII)(8)(a), (XIII)(8)(b), (XIII)(8)(c), (XIII)(8)(d), (XIII)(8)(e) and (XIII)(8)(f); preferably a mix of the six modified LPS molecular species; or
xxvii. an enriched population comprising a mix of at least two modified LPS molecular species of Formulas (XIII)(9)(a), (XIII)(9)(b), (XIII)(9)(c), (XIII)(9)(d), (XIII)(9)(e), (XIII)(9)(f), (XIII)(9)(g), (XIII)(9)(h), (XIII)(9)(i) and (XIII)(9)(j); preferably a mix of the ten modified LPS molecular species.

**7.** The composition according to claim **5** or **6,** being a pharmaceutical composition or a vaccine composition and further comprising at least one pharmaceutically acceptable excipient.

**8.** A naturally-occurring non-toxic LPS molecular species and/or an enriched population of modified LPS molecular species, or a composition comprising the same, for use in:

- treating and/or preventing cancer, an inflammatory disease or an infectious disease in a subject in need thereof, or
- stimulating an immune response in a subject in need thereof, or
- vaccinating a subject in need thereof,

wherein said naturally-occurring non-toxic LPS molecular species and/or enriched population of modified LPS molecular species, or composition comprising the same is selected from:

- the enriched population of modified LPS molecular species according to any one of claims **1** to **4;** or the composition according to any one of claims **5** to **7;** or
- a LPS molecular species which does not comprise two secondary $C_{12}$ and/or $C_{14}$ fatty acid chains simultaneously present at positions C2' and C3' of their lipid A domain.

**9.** The naturally-occurring non-toxic LPS molecular species and/or the enriched population of modified LPS molecular species, or the composition comprising the same for use according to claim **8,** wherein said LPS molecular species or composition is selected from:

- a LPS molecular species selected from the group comprising LPS molecular species of Formulas (I)(0)(a), (I)(0)(b), (II)(0), (II)(1)(a), (II)(1)(b), (II)(1)(c), (II)(1)(d) as shown in Figures 1, 5 and 6;
- a mix of LPS molecular species of Formulas (I)(0)(a) and (I)(0)(b) as shown in Figure 1;
- a mix of at least two LPS molecular species of Formulas (II)(0), as shown in Figure 5;
- a mix of at least two LPS molecular species of Formulas (II)(1)(a), (II)(1)(b), (II)(1)(c), (II)(1)(d), as shown in Figure 6;
- the composition according to any one of claims **5** to **7** comprising a mix of modified LPS molecular species of Formulas (I)(1)(a), (I)(1)(b), (I)(1)(c), (I)(1)(d), (I)(1)(e) and (I)(1)(f);
- the composition according to any one of claims **5** to **7** comprising a mix of modified LPS molecular species of Formulas (I)(2)(a), (I)(2)(b) and (I)(2)(c);
- the composition according to any one of claims **5** to **7** comprising a mix of modified LPS molecular species of Formulas (VI)(1)(a), (VI)(1)(b), (VI)(1)(c), (VI)(1)(d), (VI)(1)(e), (VI)(1)(f), (VI)(1)(g), (VI)(1)(h), (VI)(1)(i) and (VI)(1)(j); or
- the composition according to any one of claims **5** to **7** comprising a mix of modified LPS molecular species of Formulas (I)(8)(a), (I)(8)(b) and (I)(8)(c).

**10.** The naturally-occurring non-toxic LPS molecular species and/or the modified LPS molecular species, or the composition comprising the same for use according to claim **8** or **9,** wherein treating and/or preventing cancer, an inflammatory disease or an infectious disease further comprises administering to said subject at least one additional therapeutic agent, preferably selected from the group comprising chemotherapeutic agents, targeted therapy agents,

cytotoxic agents, antibiotics, antivirals, cell cycle-synchronizing agents, ligands for cellular receptor(s), immunomodulatory agents, pro-apoptotic agents, anti-angiogenic agents, cytokines, growth factors, antibodies or antigen-binding fragments thereof, cell therapy, and antigens.

**11.** The naturally-occurring non-toxic LPS molecular species and/or the modified LPS molecular species, or the composition comprising the same for use according to claim **10,** wherein the at least one additional therapeutic agent is a targeted therapy agent, preferably an anti-CD20 antibody or an antigen-binding fragment thereof, more preferably the at least one additional therapeutic agent is rituximab or an antigen-binding fragment thereof.

**12.** The naturally-occurring non-toxic LPS molecular species and/or the modified LPS molecular species, or the composition comprising the same for use according to claim **10,** wherein the at least one additional therapeutic agent is an immunostimulatory agent, preferably an immune checkpoint inhibitor, more preferably an immune checkpoint inhibitor selected from the group consisting of CTLA4, PD-1, PD-L1, LAG-3, B7-H3, B7-H4, TIM3, A2AR, GITR, CD47, TNFR2, CD19, and IDO inhibitors; even more preferably the at least one additional therapeutic agent is a PD-1 or PD-L1 inhibitor; most preferably the immune checkpoint inhibitor is a PD-1 inhibitor, in particular, an anti-PD-1 antibody or an antigen-binding fragment thereof.

**13.** The naturally-occurring non-toxic LPS molecular species and/or the modified LPS molecular species, or the composition comprising the same for use according to claim **8** or **9,** wherein vaccinating, or stimulating an immune response in, a subject in need thereof further comprises administering to said subject at least one antigen, preferably selected from the group consisting of a pathogen-related antigen, a self-antigen, an allergen-related antigen and a neoantigen.

**14.** The naturally-occurring non-toxic LPS molecular species and/or the modified LPS molecular species, or the composition comprising the same for use according to any one of claims **8** to **13,** wherein the subject in need thereof is an animal other than a human and said naturally-occurring non-toxic LPS molecular species and/or modified LPS molecular species or composition comprising the same is selected from a LPS molecular species of Formula (XII)(1) as shown in Figure 27.

**15.** A kit-of-parts comprising:

- an enriched population of modified LPS molecular species according to any one of claims **1** to **4;** a composition according to any one of claims **5** to **7;** or a LPS molecular species which does not comprise two secondary $C_{12}$ and/or $C_{14}$ fatty acid chains simultaneously present at positions C2' and C3' of their lipid A domain; and
- an additional therapeutic agent, preferably selected from the group comprising chemotherapeutic agents, targeted therapy agents, cytotoxic agents, antibiotics, antivirals, cell cycle-synchronizing agents, ligands for cellular receptor(s), immunomodulatory agents, pro-apoptotic agents, anti-angiogenic agents, cytokines, growth factors, antibodies or antigen-binding fragments thereof, cell therapy, and antigens.

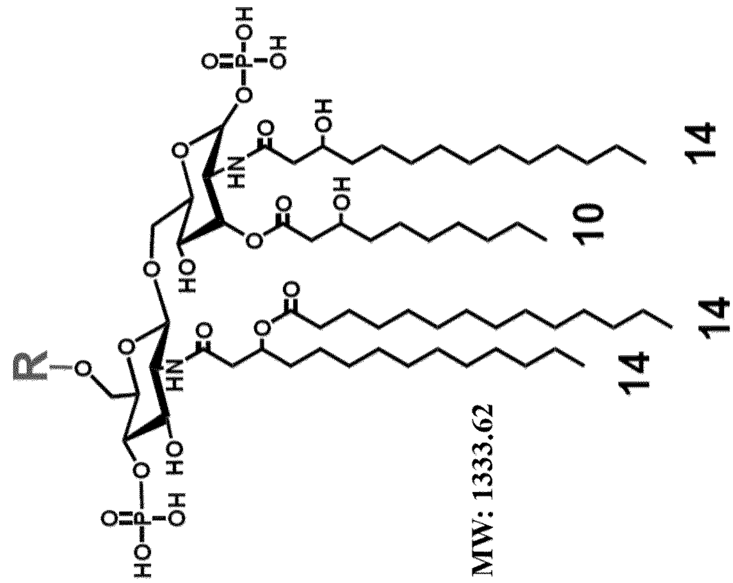

MW: 1333.62

(I)(0)(a)

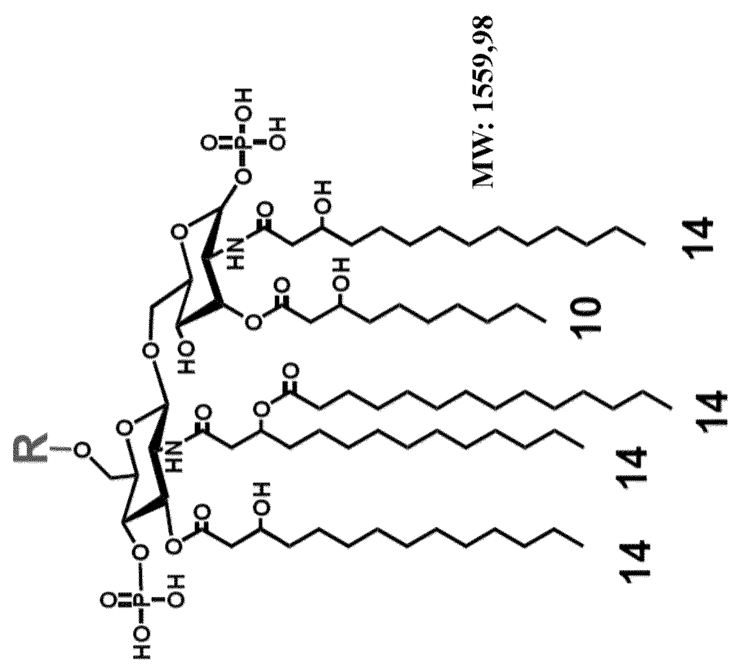

MW: 1559,98

(I)(0)(b)

FIG. 1

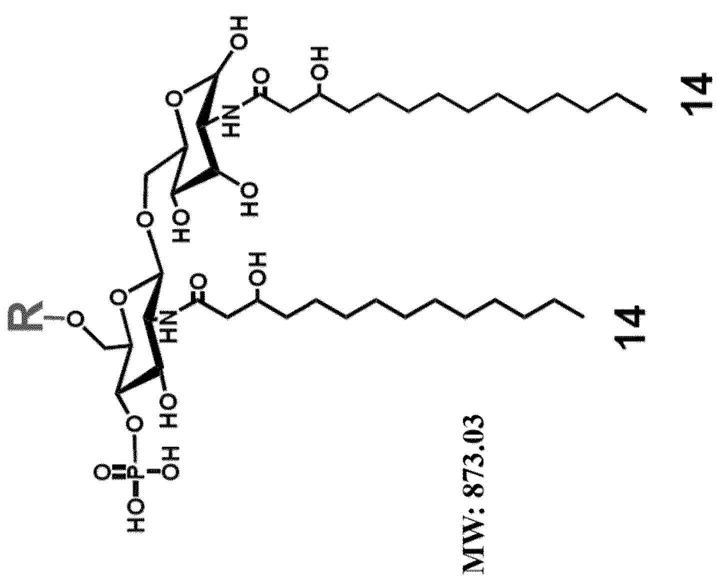

MW: 873.03

(I)(1)(a)

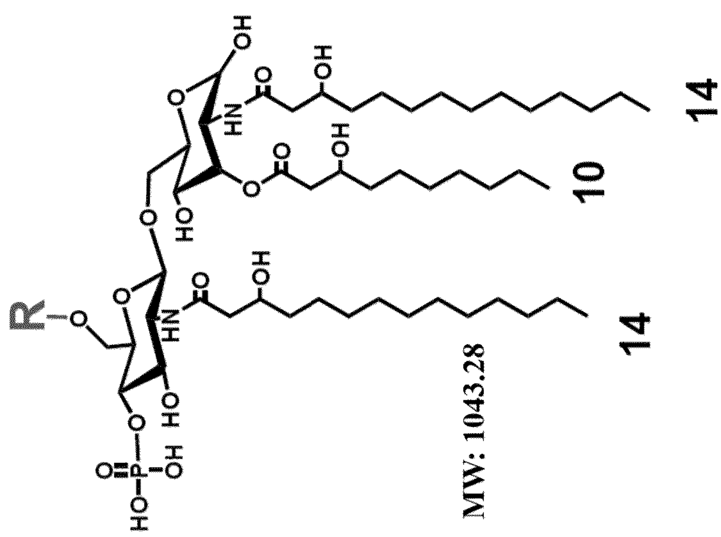

MW: 1043.28

(I)(1)(b)

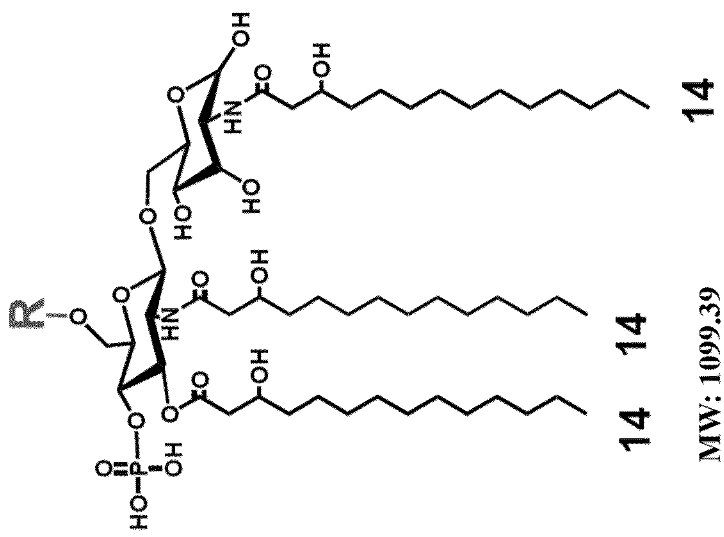

MW: 1099.39

(I)(1)(c)

FIG. 2

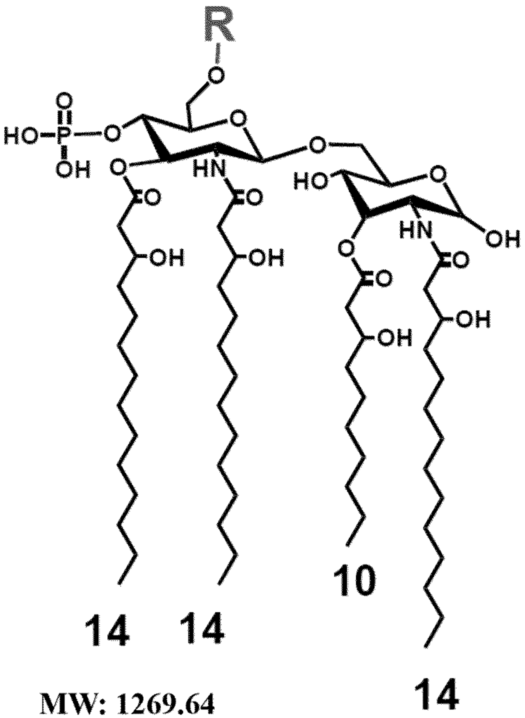

MW: 1269.64

(I)(1)(d)

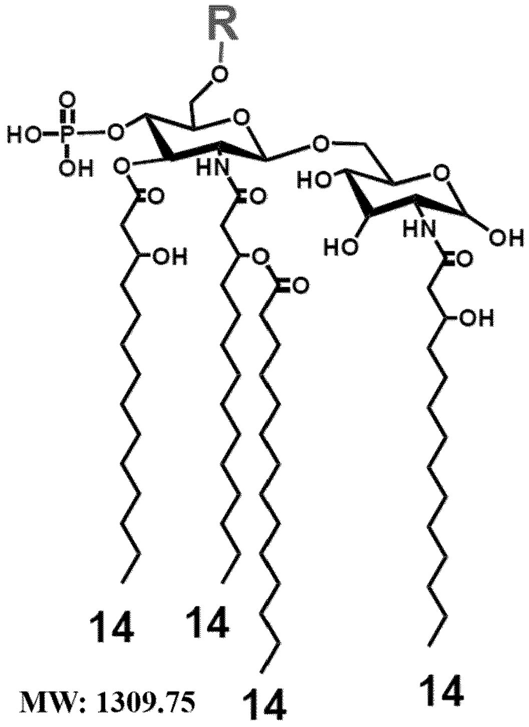

MW: 1309.75

(I)(1)(e)

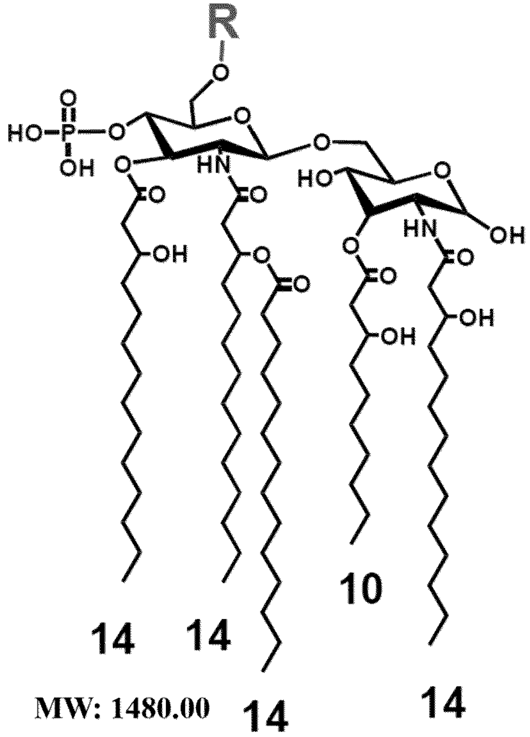

MW: 1480.00

(I)(1)(f)

FIG. 2 (cont.)

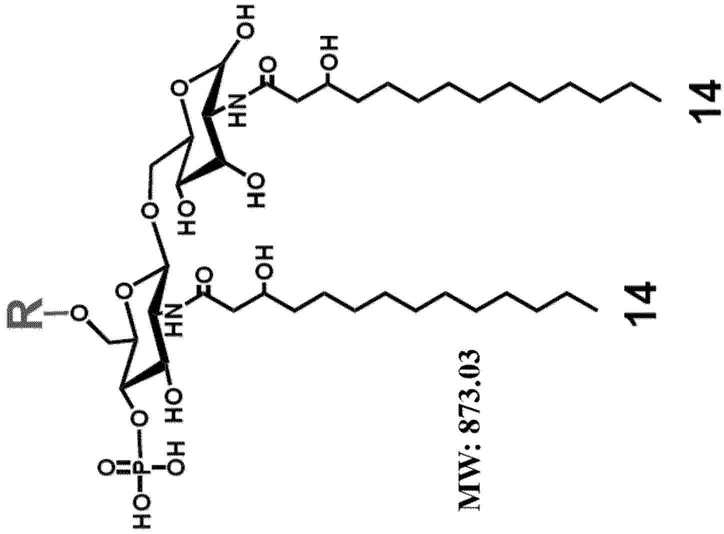

MW: 873.03

(I)(2)(a)

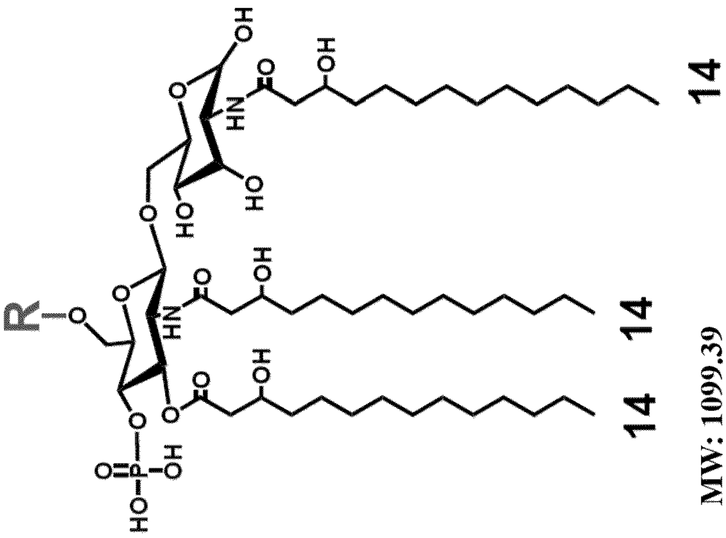

MW: 1099.39

(I)(2)(b)

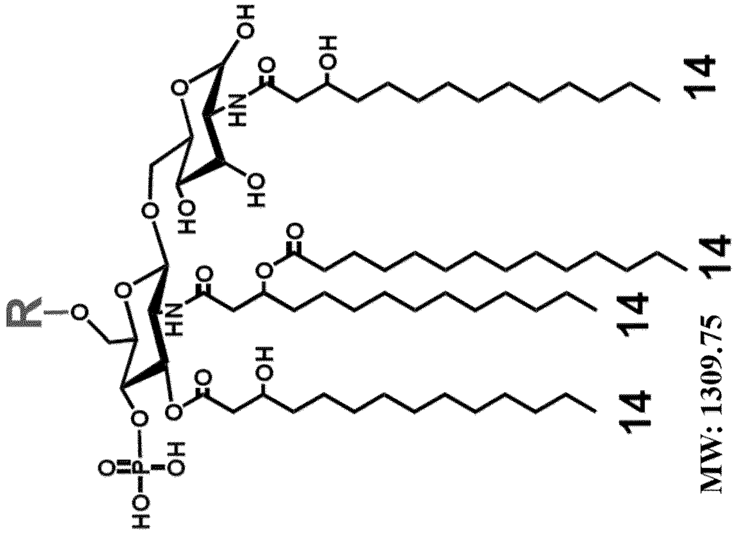

MW: 1309.75

(I)(2)(c)

FIG. 3

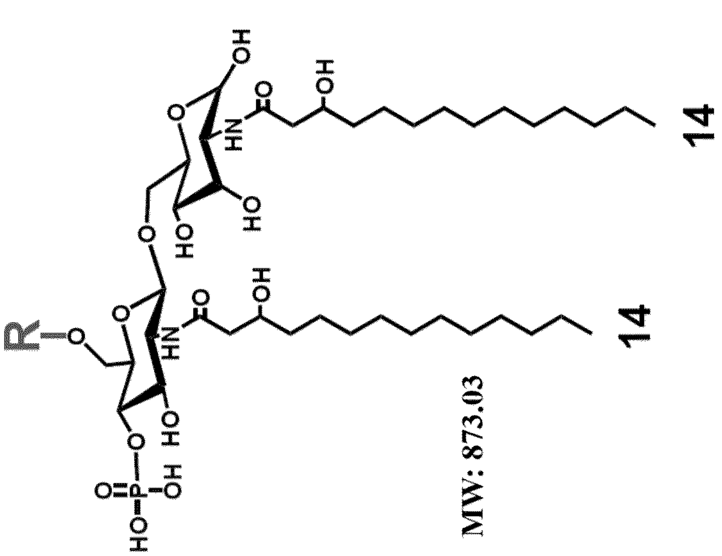

MW: 873.03

(I)(3)(a)

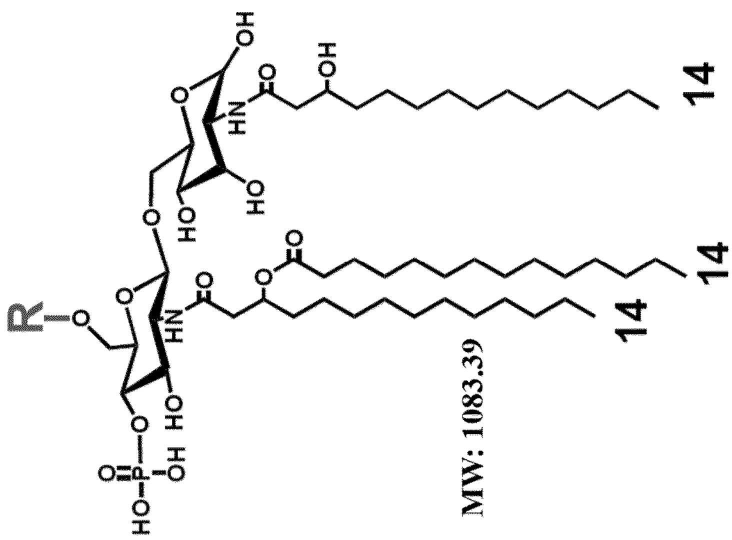

MW: 1083.39

(I)(3)(b)

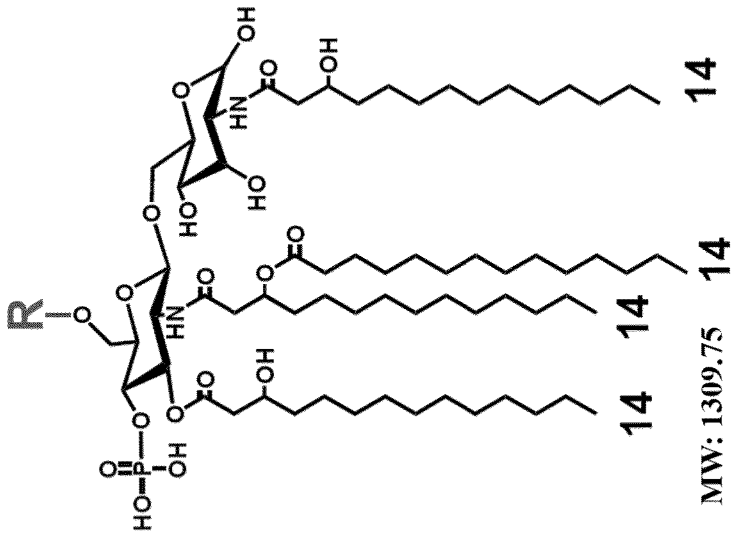

MW: 1309.75

(I)(3)(c)

FIG. 4

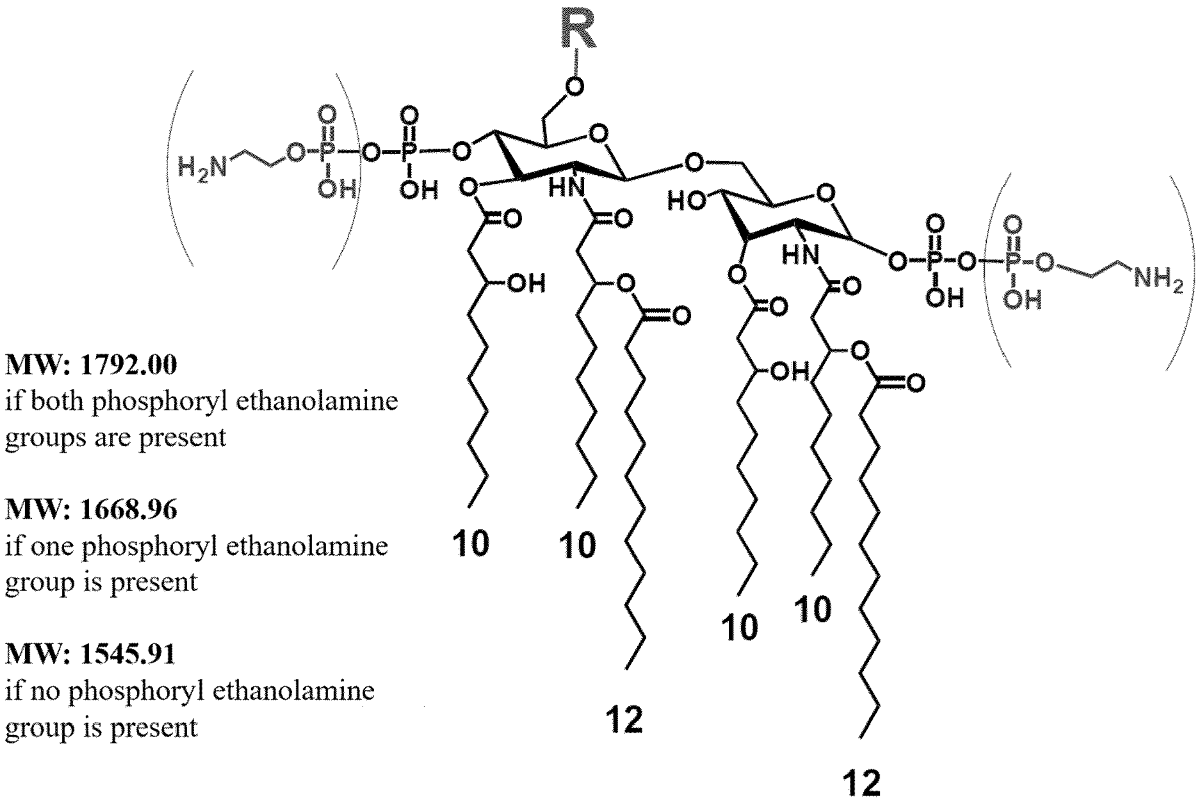

**MW: 1792.00**
if both phosphoryl ethanolamine groups are present

**MW: 1668.96**
if one phosphoryl ethanolamine group is present

**MW: 1545.91**
if no phosphoryl ethanolamine group is present

**(II)(0)**

**FIG. 5**

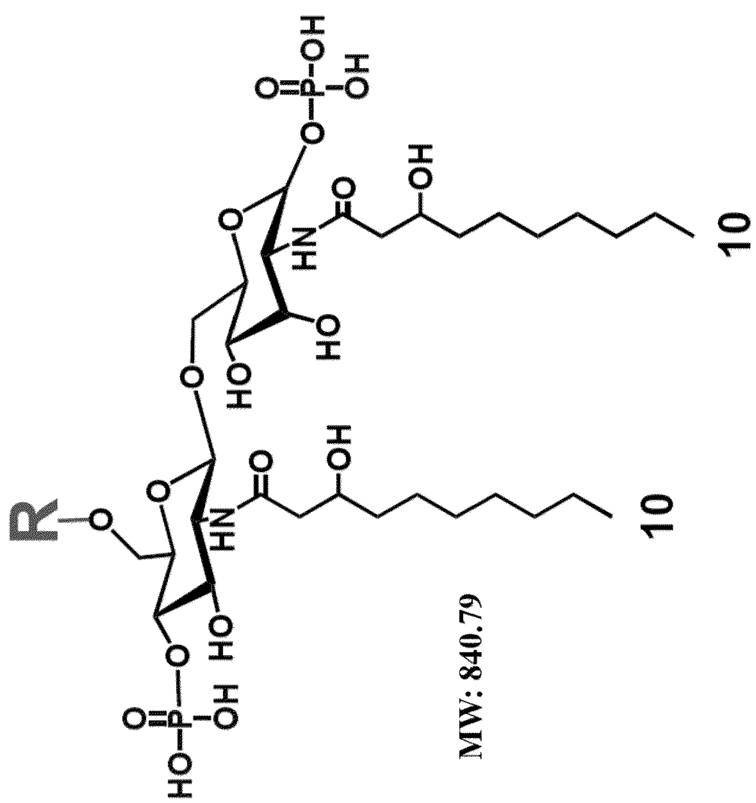

MW: 840.79

(II)(1)(a)

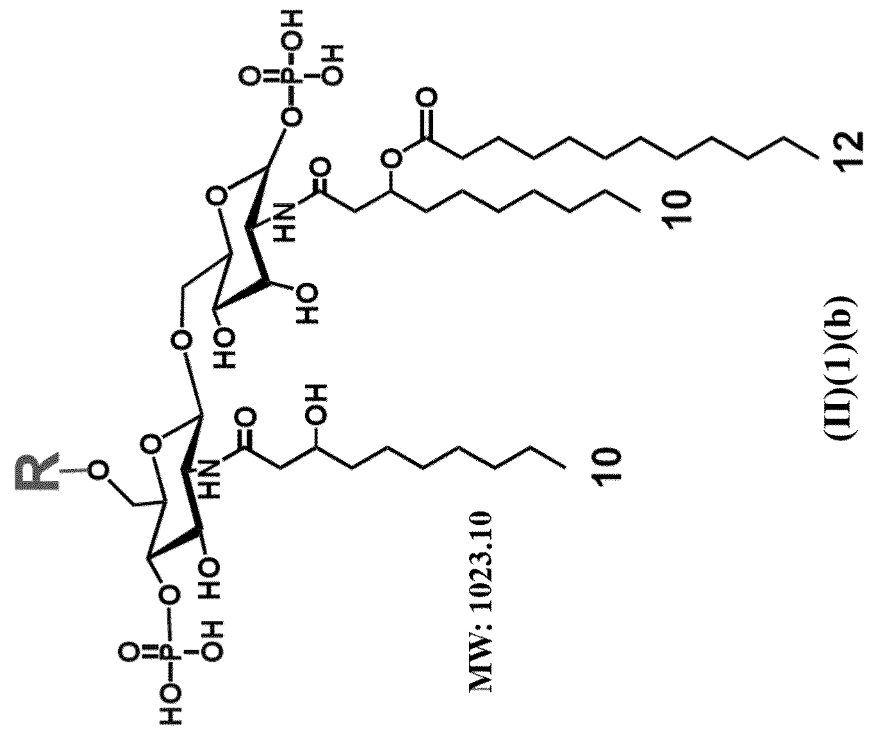

MW: 1023.10

(II)(1)(b)

FIG. 6

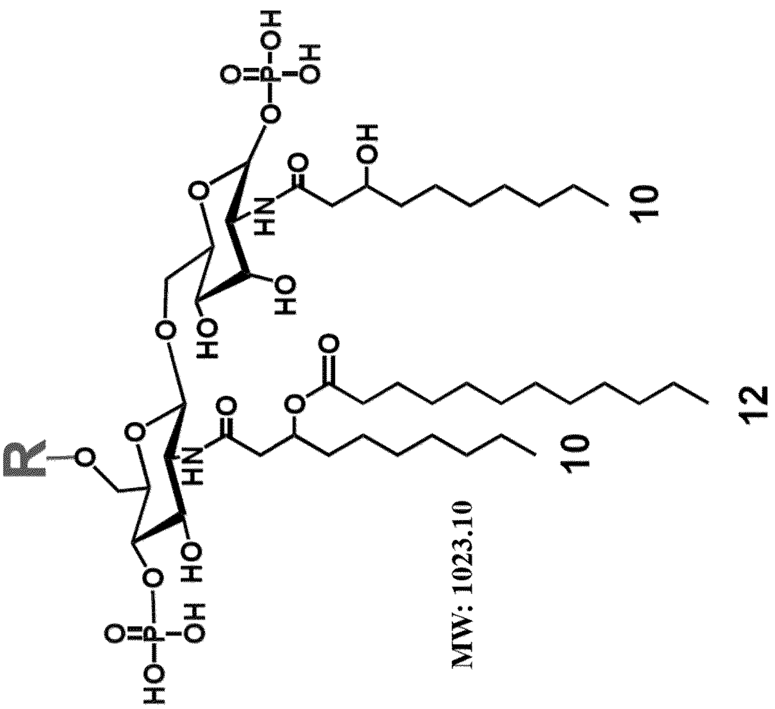

MW: 1023.10

(II)(1)(c)

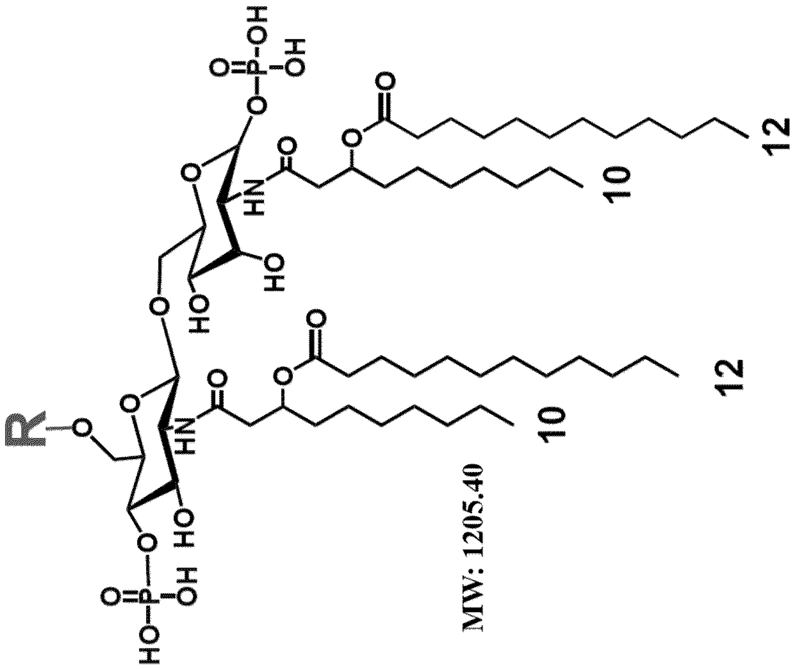

MW: 1205.40

(II)(1)(d)

FIG. 6 (cont.)

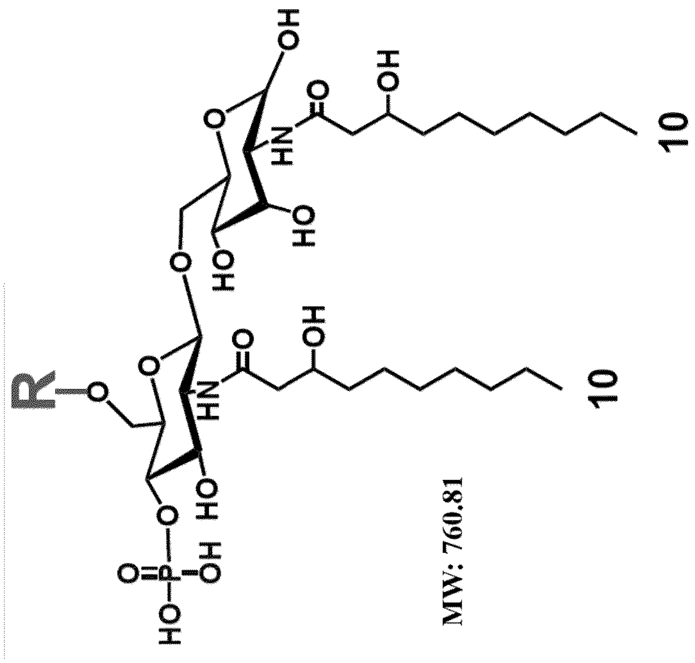

MW: 760.81

(II)(2)(a)

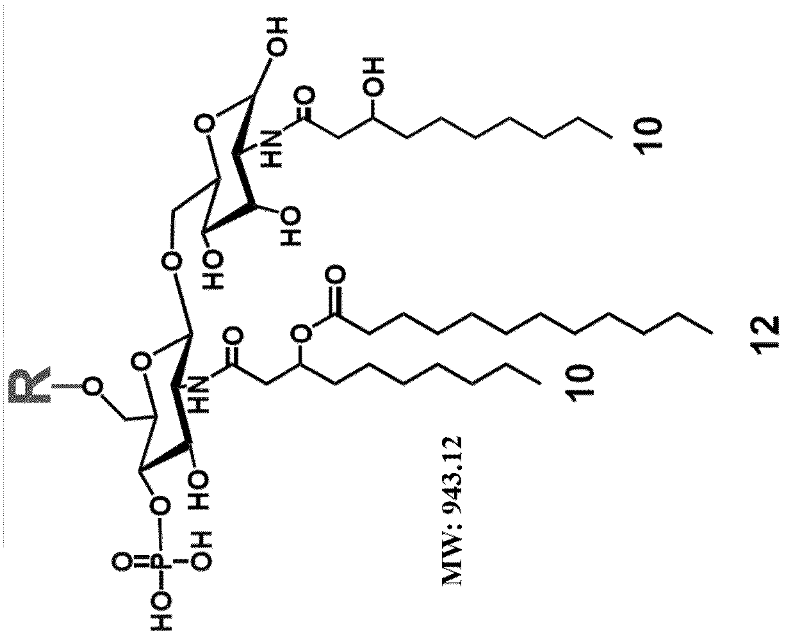

MW: 943.12

(II)(2)(b)

FIG. 7

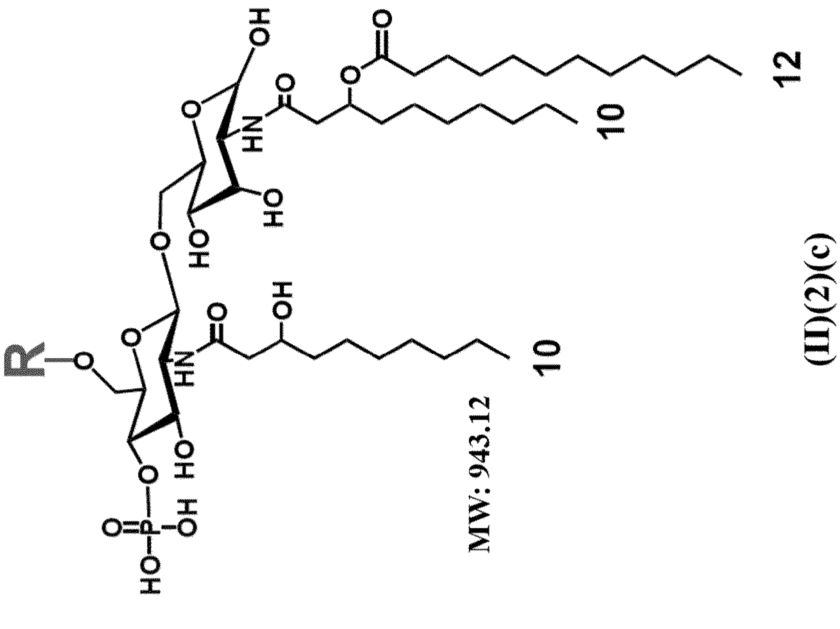

MW: 943.12

(II)(2)(c)

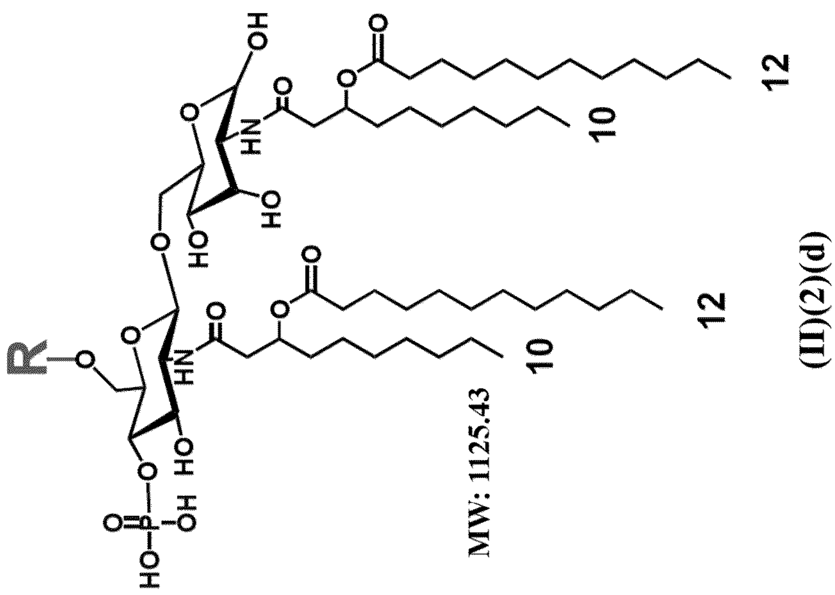

MW: 1125.43

(II)(2)(d)

FIG. 7 (cont.)

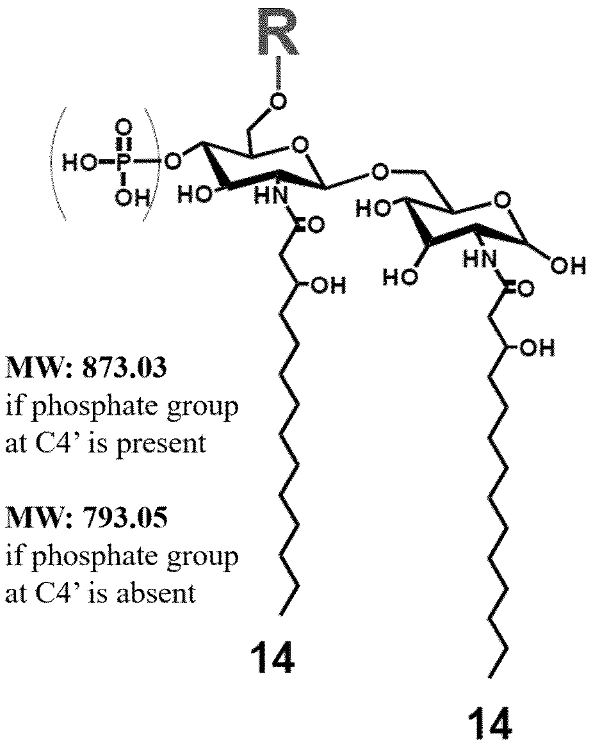

**MW: 873.03**
if phosphate group
at C4' is present

**MW: 793.05**
if phosphate group
at C4' is absent

(III)(1)(a)

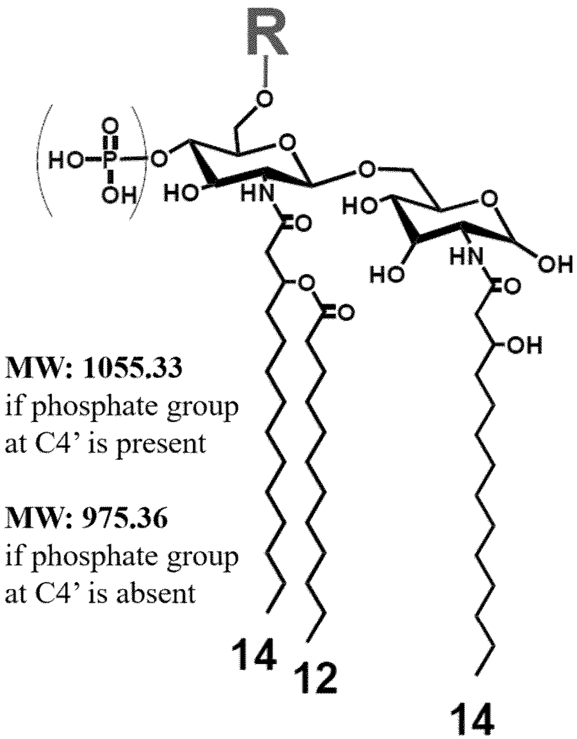

**MW: 1055.33**
if phosphate group
at C4' is present

**MW: 975.36**
if phosphate group
at C4' is absent

(III)(1)(b)

**FIG. 8**

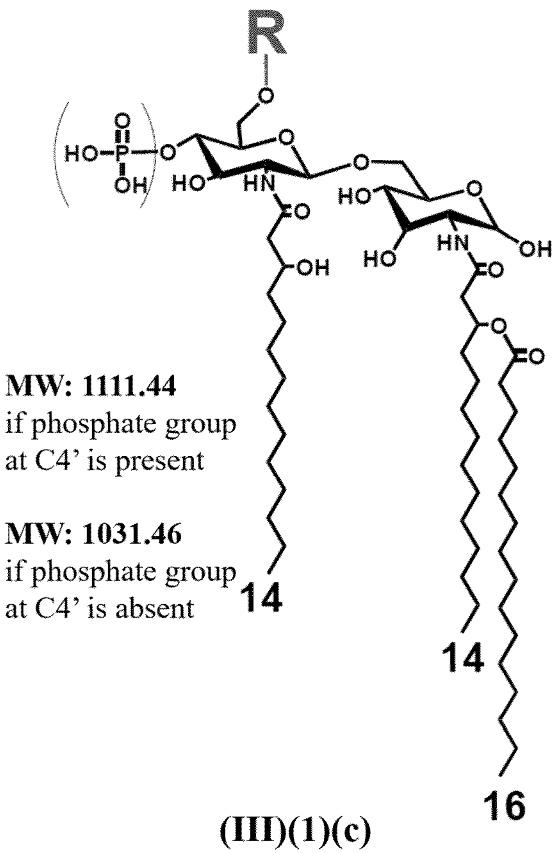

MW: 1111.44
if phosphate group
at C4' is present

MW: 1031.46
if phosphate group
at C4' is absent

(III)(1)(c)

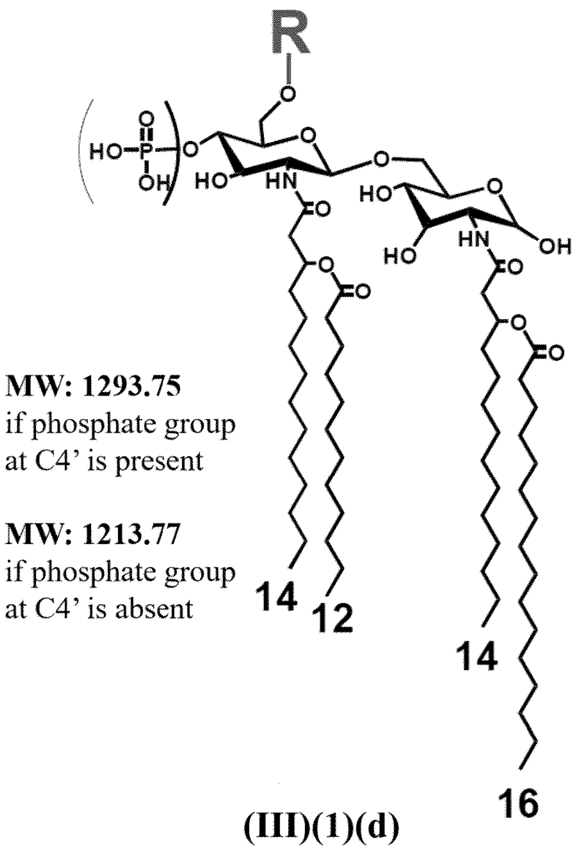

MW: 1293.75
if phosphate group
at C4' is present

MW: 1213.77
if phosphate group
at C4' is absent

(III)(1)(d)

FIG. 8 (cont.)

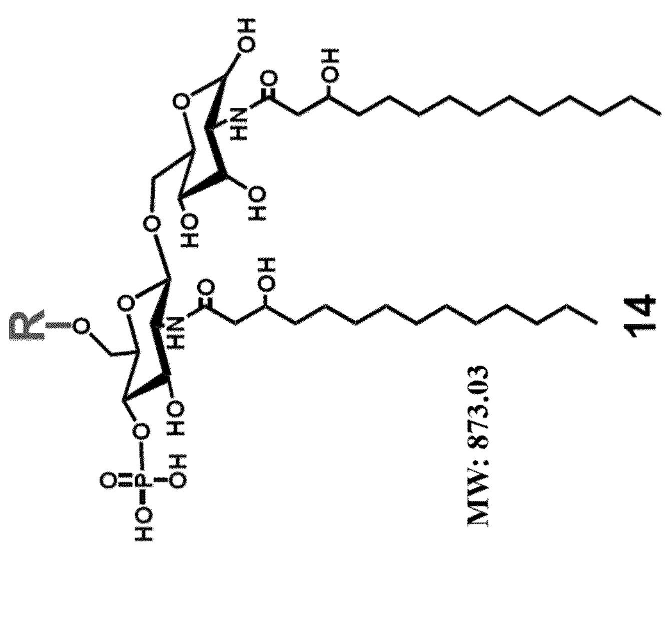

MW: 873.03

(IV)(1)(a)

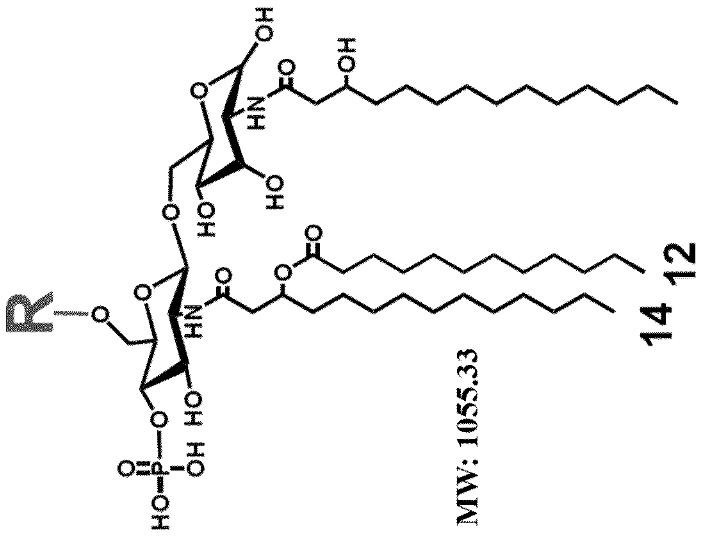

MW: 1055.33

(IV)(1)(b)

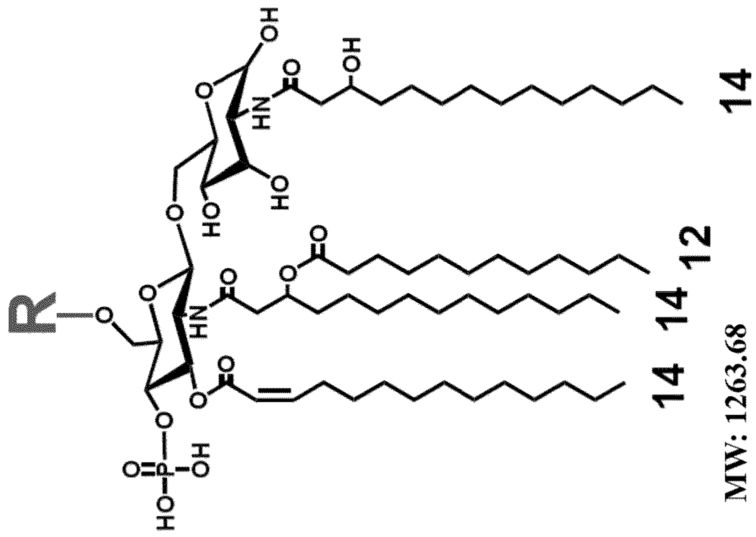

MW: 1263.68

(IV)(1)(c)

FIG. 9

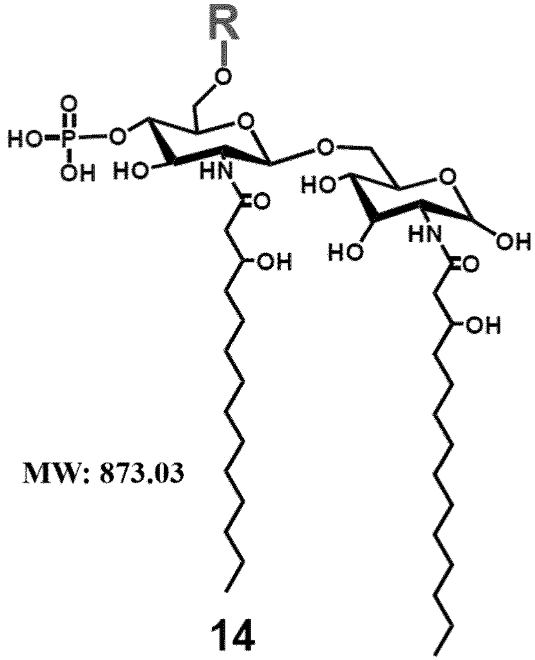

MW: 873.03

(V)(1)(a)

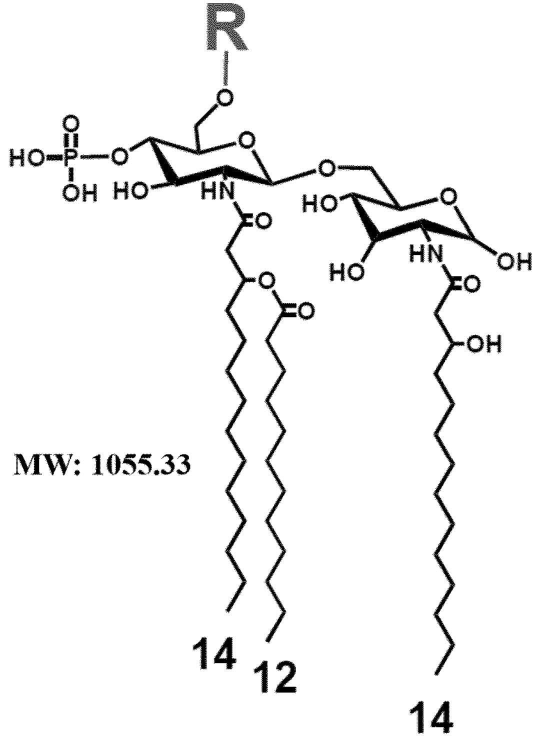

MW: 1055.33

(V)(1)(b)

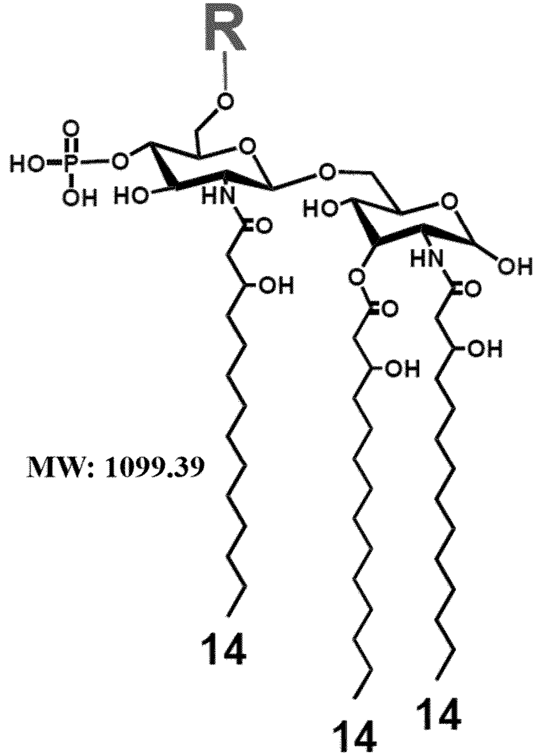

MW: 1099.39

(V)(1)(c)

FIG. 10

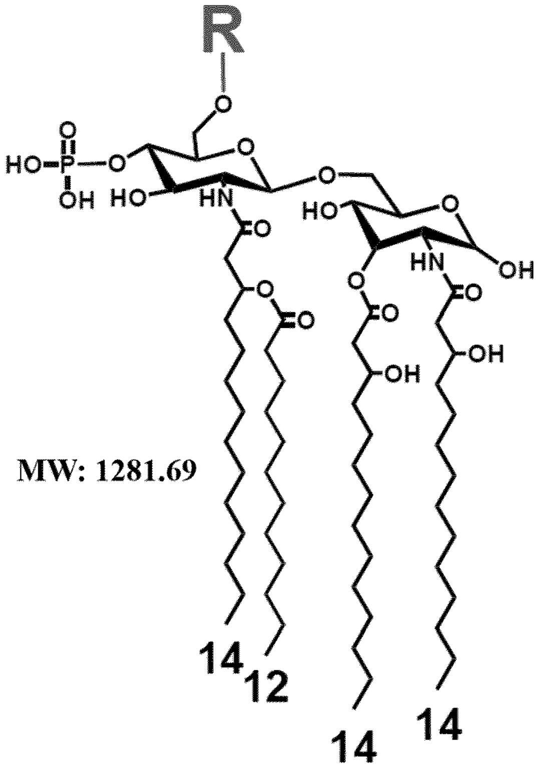

MW: 1281.69

(V)(1)(d)

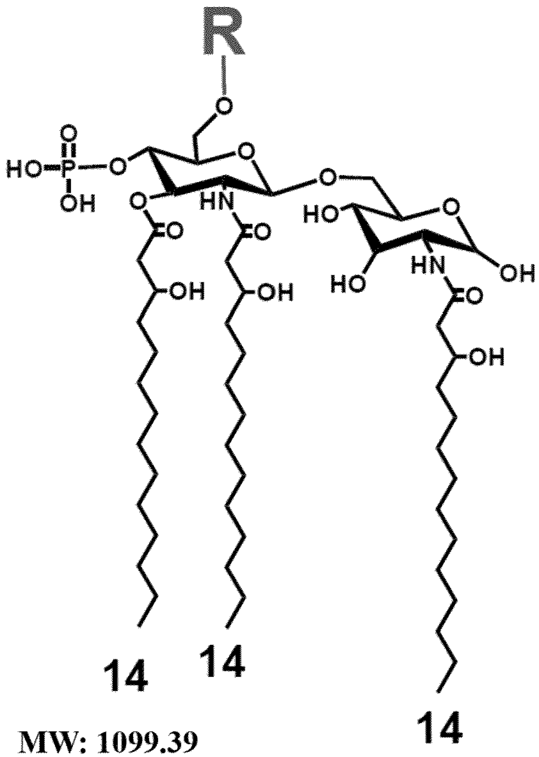

MW: 1099.39

(V)(1)(e)

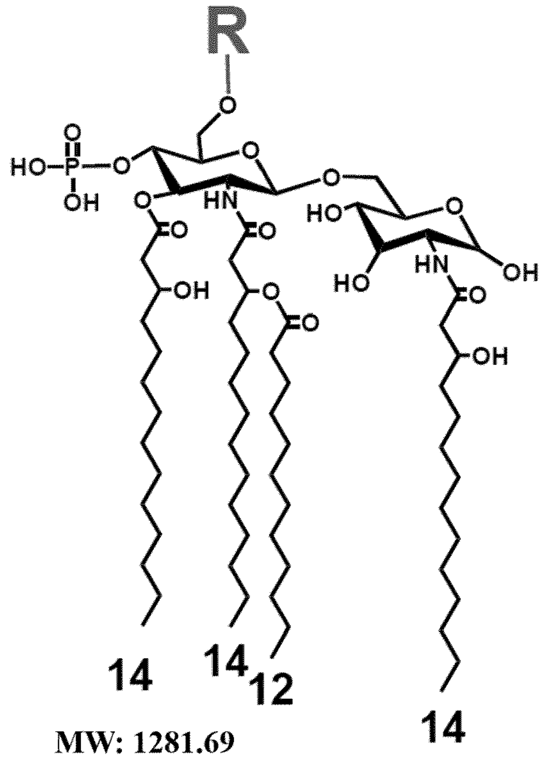

MW: 1281.69

(V)(1)(f)

FIG. 10 (cont.)

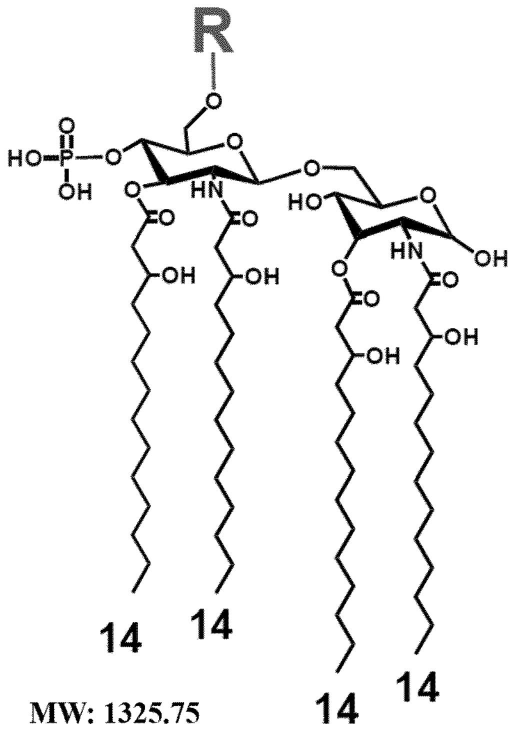

MW: 1325.75

(V)(1)(g)

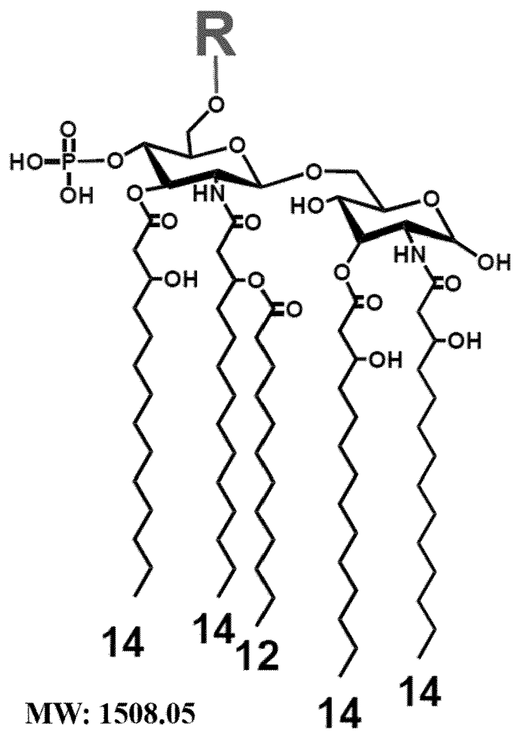

MW: 1508.05

(V)(1)(h)

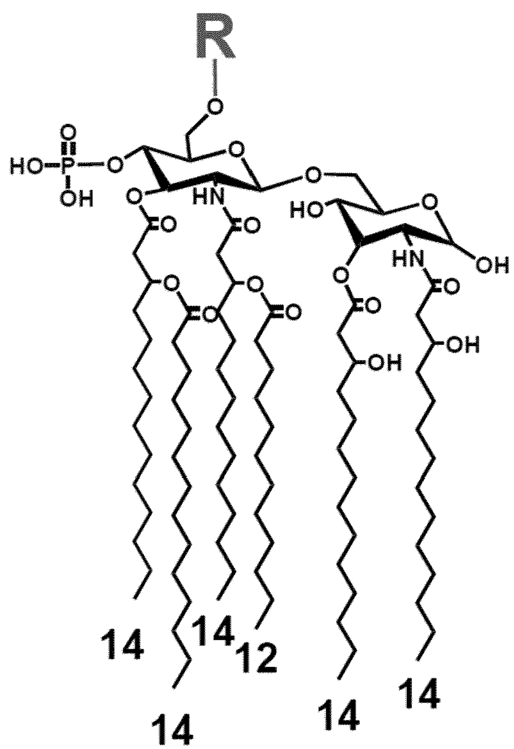

MW: 1718.42

(V)(1)(i)

FIG. 10 (cont.)

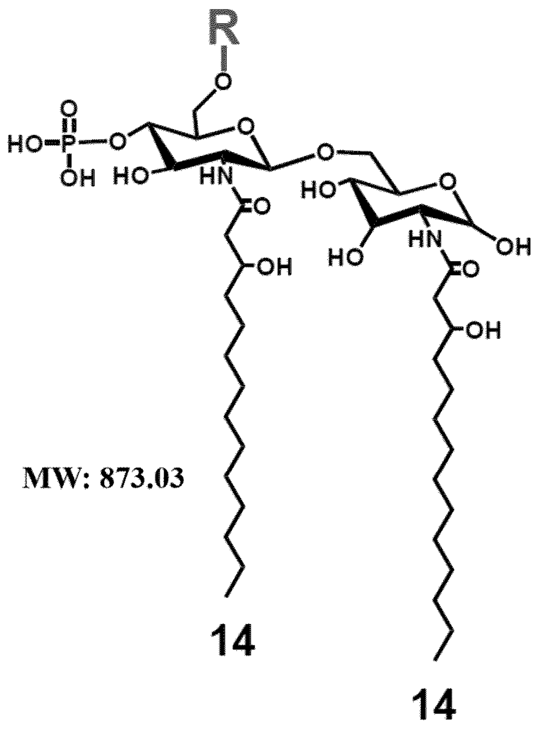

MW: 873.03

(VI)(1)(a)

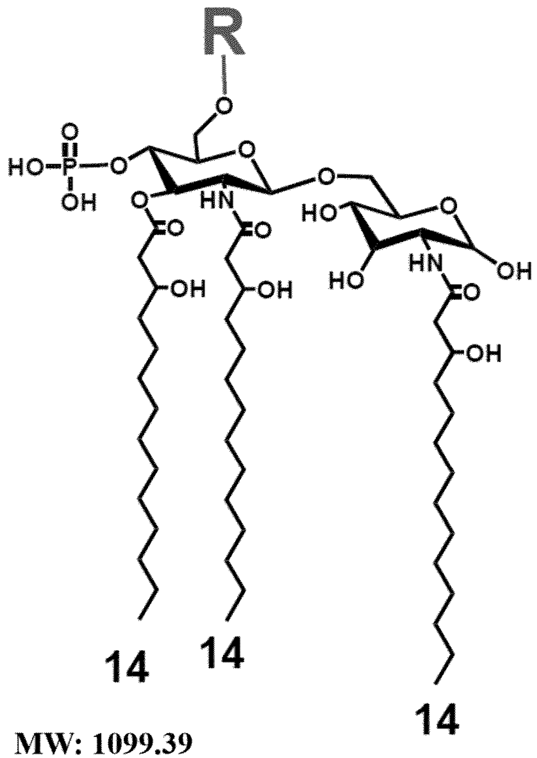

MW: 1099.39

(VI)(1)(b)

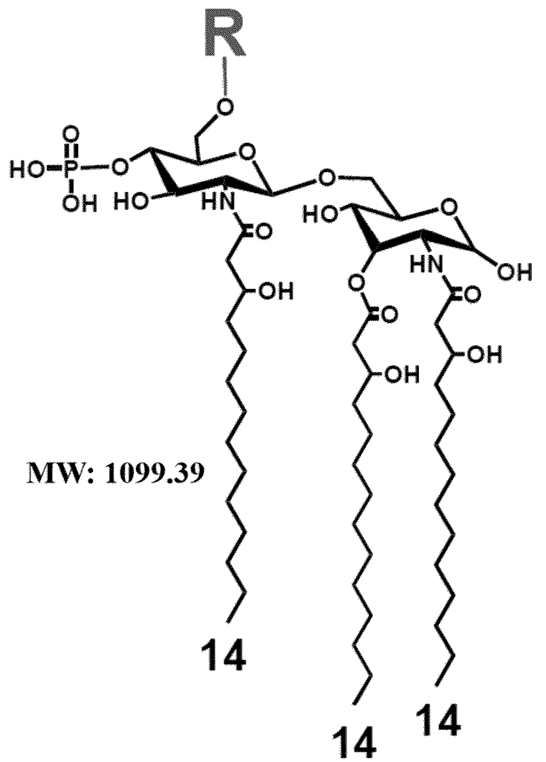

MW: 1099.39

(VI)(1)(c)

FIG. 11

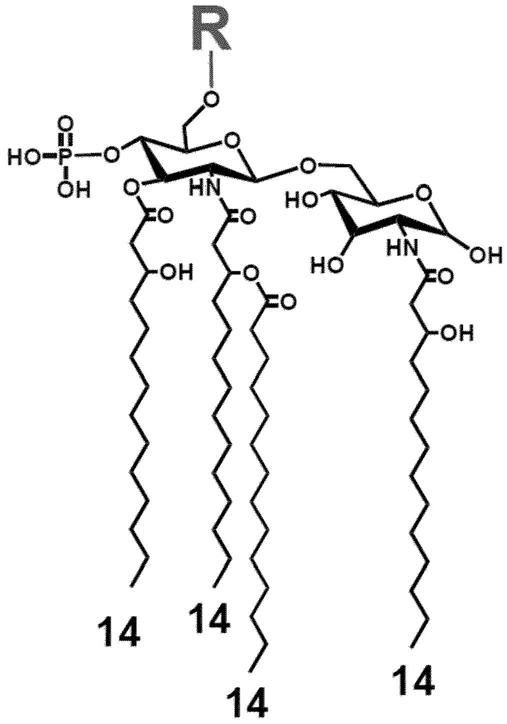

MW: 1309.75

(VI)(1)(d)

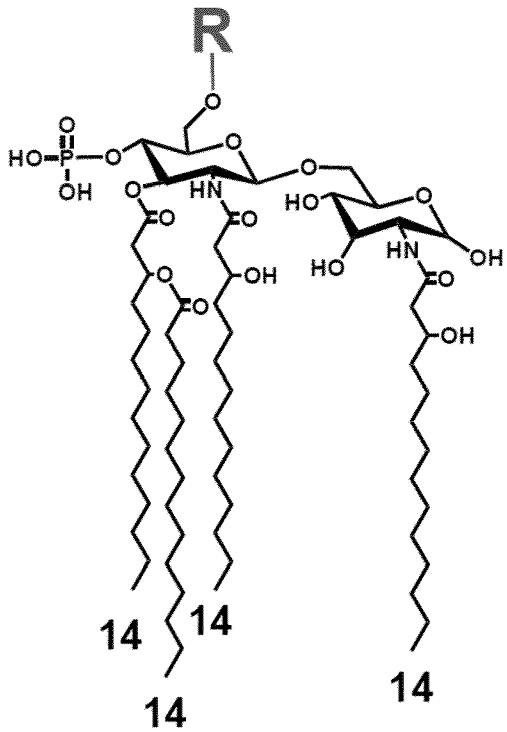

MW: 1309.75

(VI)(1)(e)

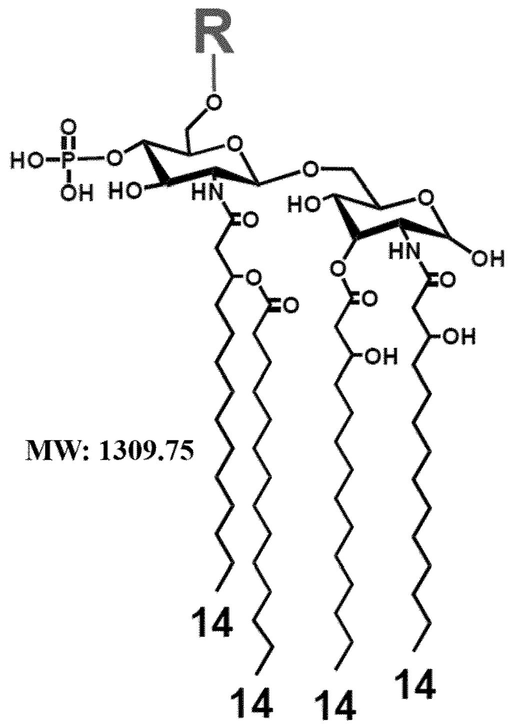

MW: 1309.75

(VI)(1)(f)

FIG. 11 (cont.)

MW: 1325.75

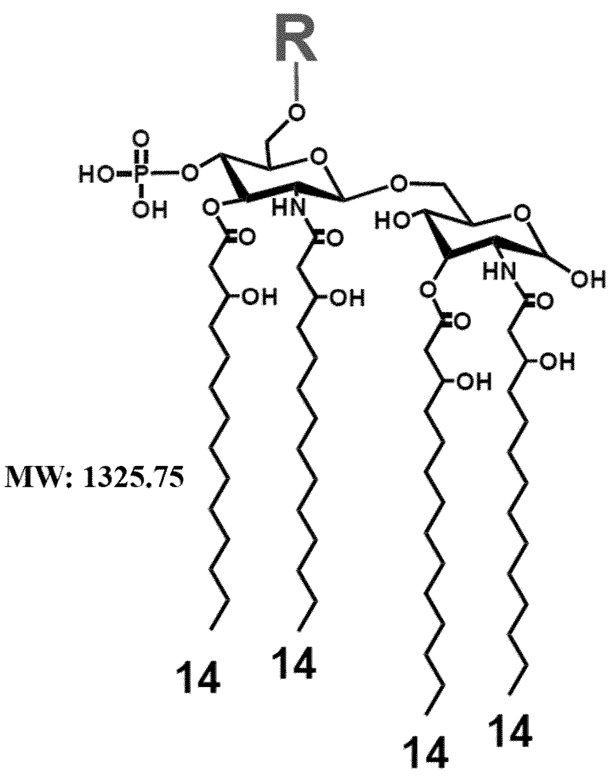

(VI)(1)(g)

MW: 1536.11

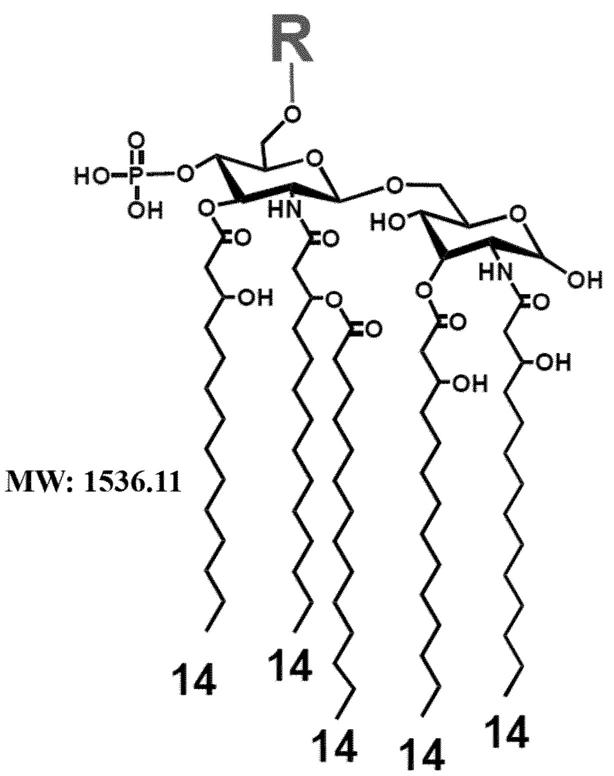

(VI)(1)(h)

FIG. 11 (cont.)

MW: 1536.11

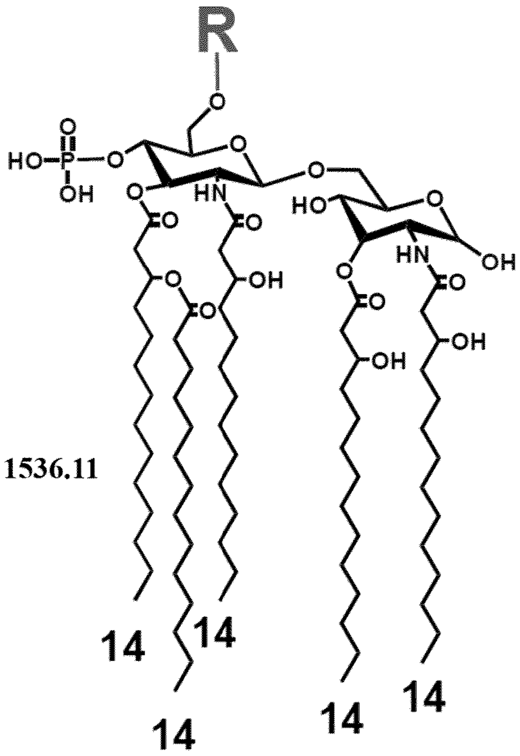

(VI)(1)(i)

MW: 1746.47

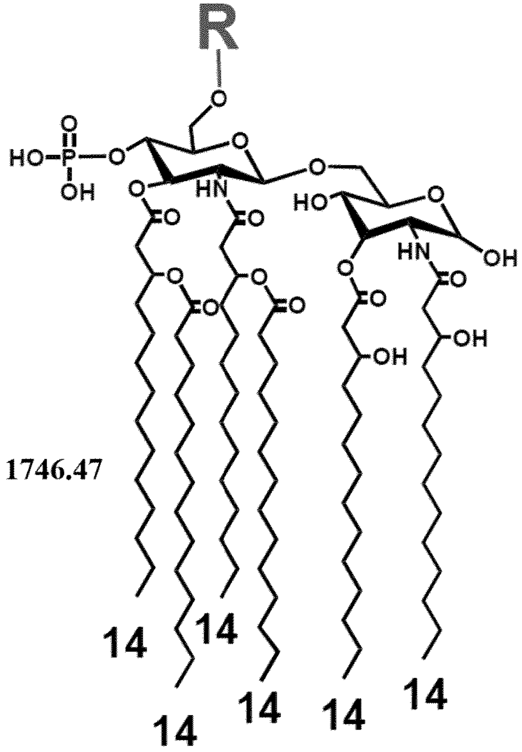

(VI)(1)(j)

FIG. 11 (cont.)

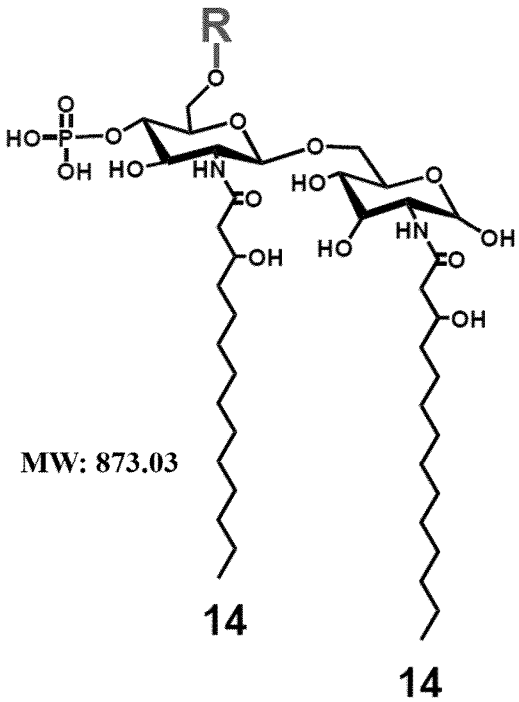

MW: 873.03

(VI)(2)(a)

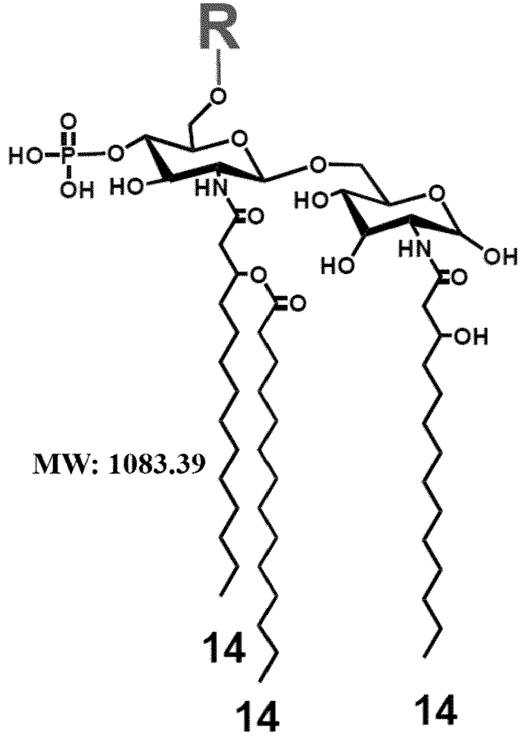

MW: 1083.39

(VI)(2)(b)

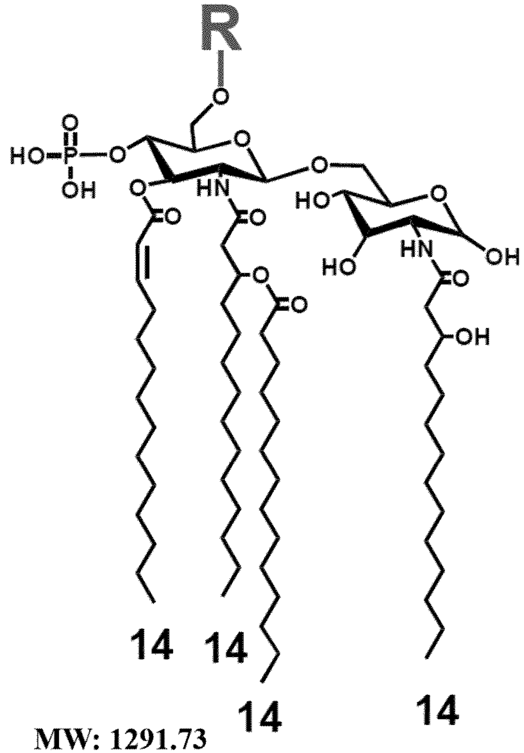

MW: 1291.73

(VI)(2)(c)

FIG. 12

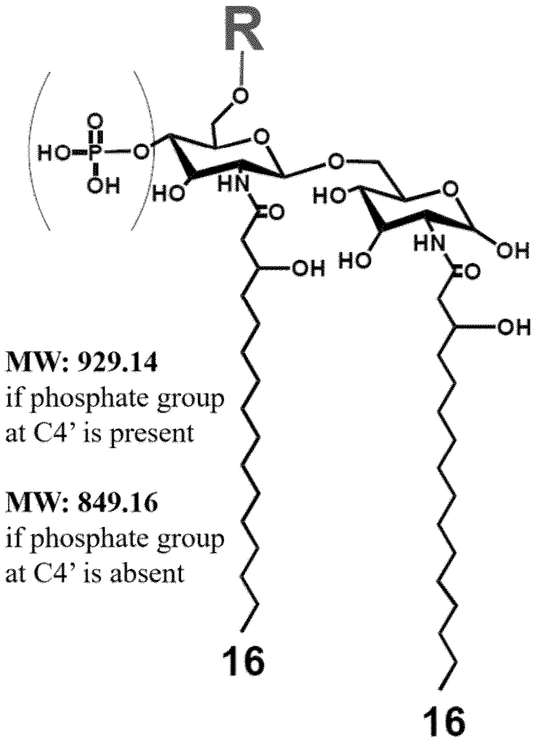

**MW: 929.14**
if phosphate group
at C4' is present

**MW: 849.16**
if phosphate group
at C4' is absent

**(VII)(1)(a)**

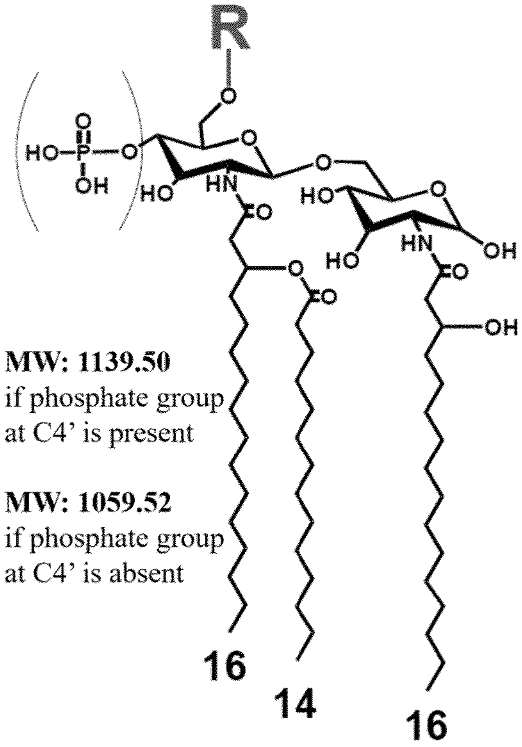

**MW: 1139.50**
if phosphate group
at C4' is present

**MW: 1059.52**
if phosphate group
at C4' is absent

**(VII)(1)(b)**

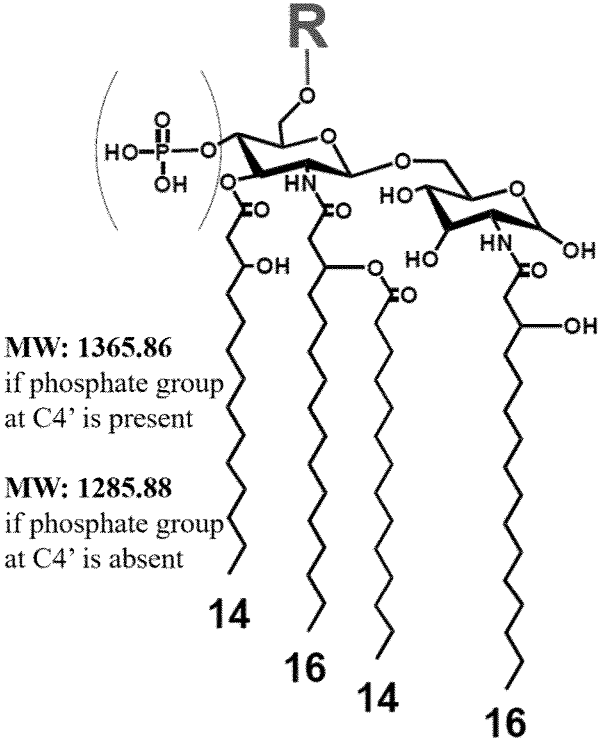

**MW: 1365.86**
if phosphate group
at C4' is present

**MW: 1285.88**
if phosphate group
at C4' is absent

**(VII)(1)(c)**

**FIG. 13**

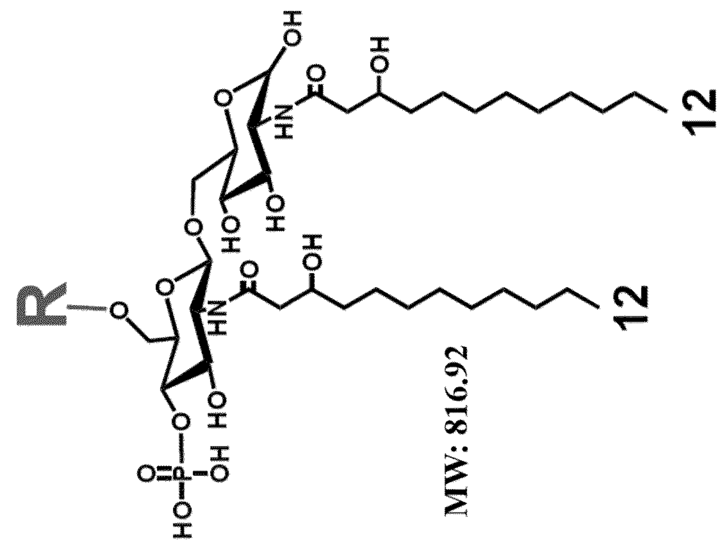

MW: 816.92

(VIII)(1)(a)

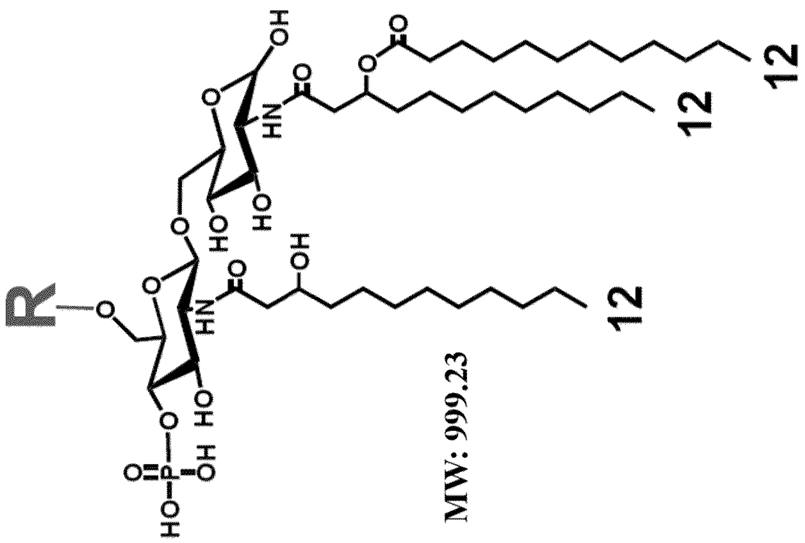

MW: 999.23

(VIII)(1)(b)

FIG. 14

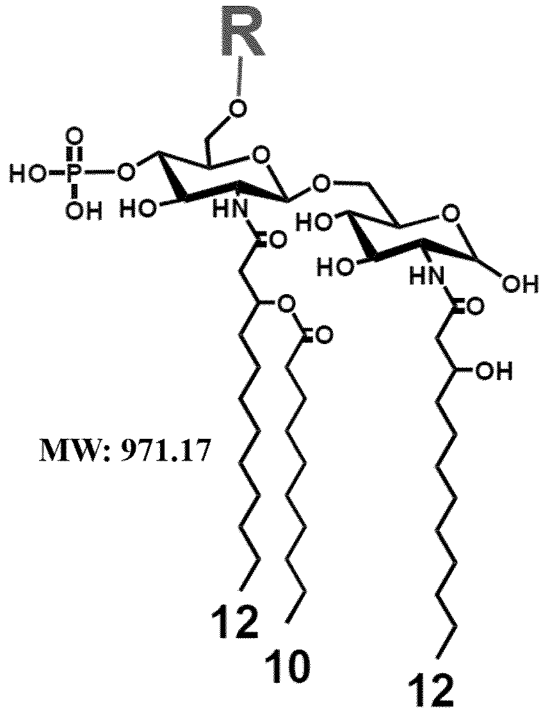

MW: 971.17

(VIII)(1)(c)

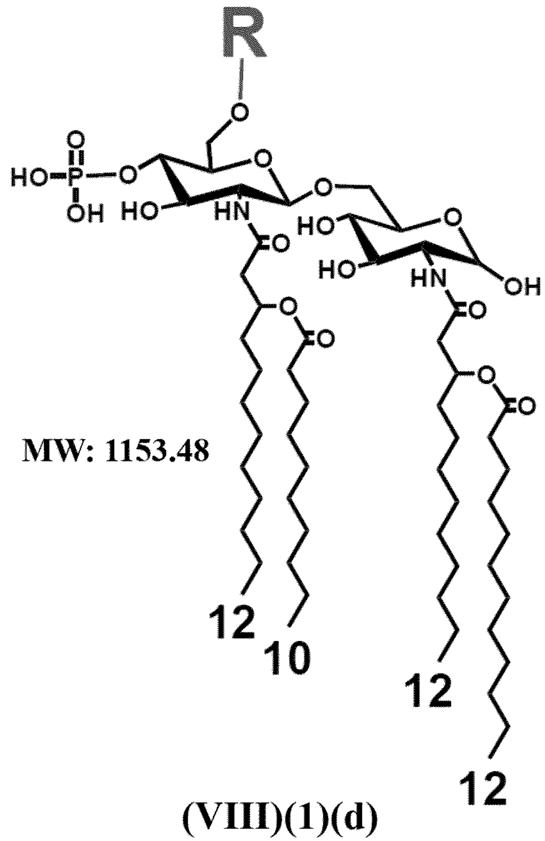

MW: 1153.48

(VIII)(1)(d)

FIG. 14 (cont.)

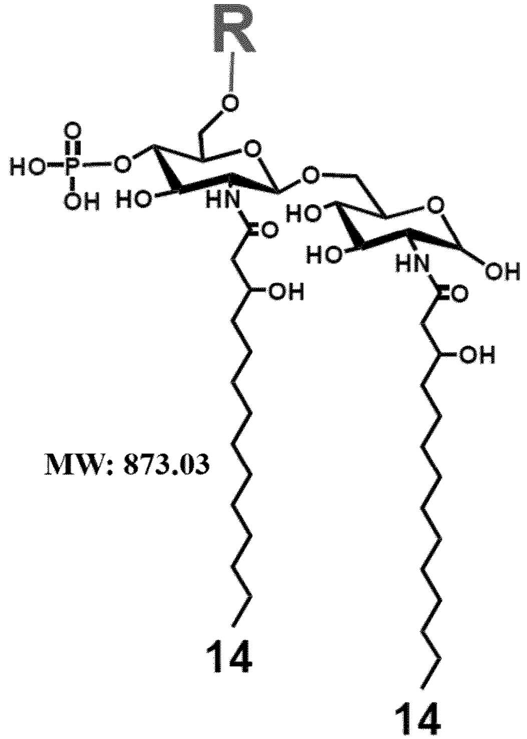

MW: 873.03

(IX)(1)(a)

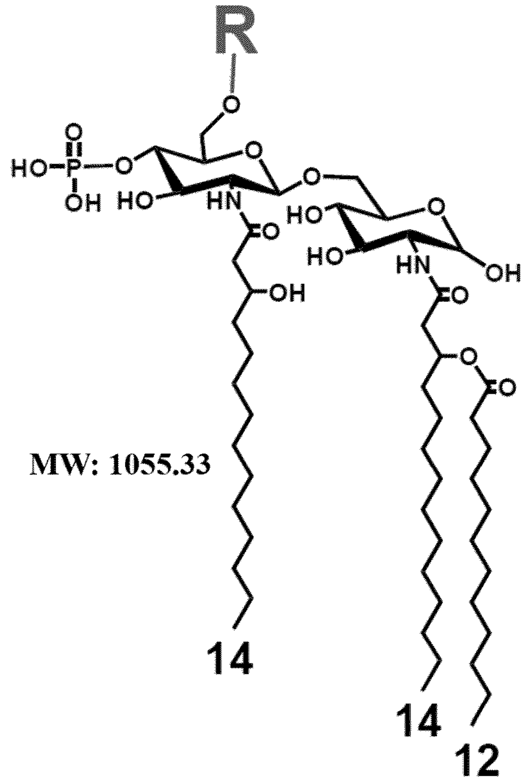

MW: 1055.33

(IX)(1)(b)

FIG. 15

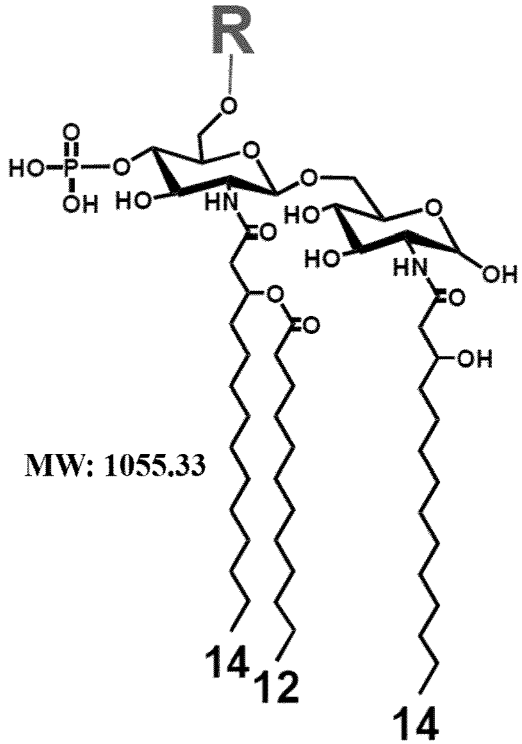

MW: 1055.33

(IX)(1)(c)

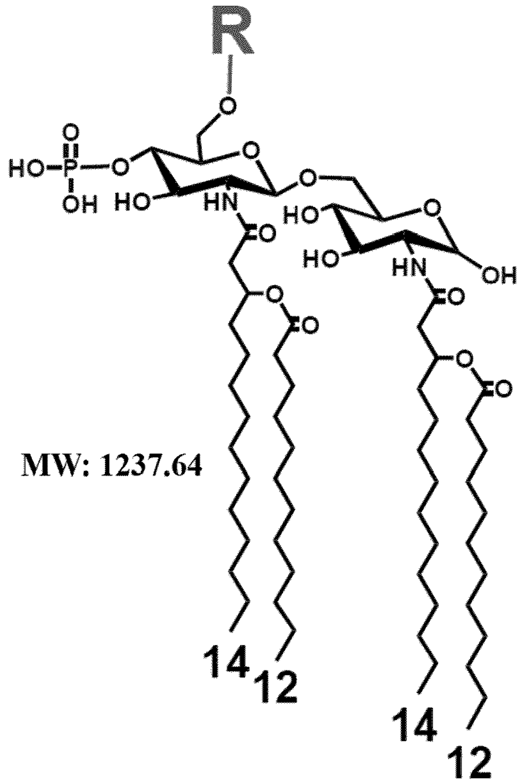

MW: 1237.64

(IX)(1)(d)

FIG. 15 (cont.)

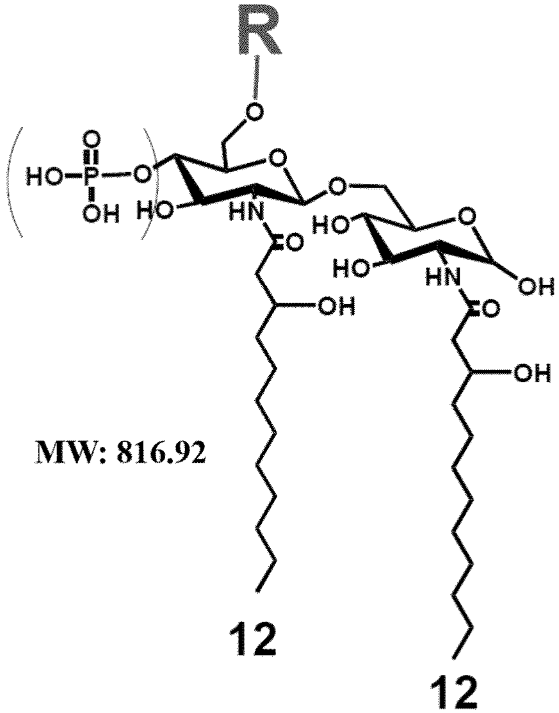

MW: 816.92

(X)(1)(a)

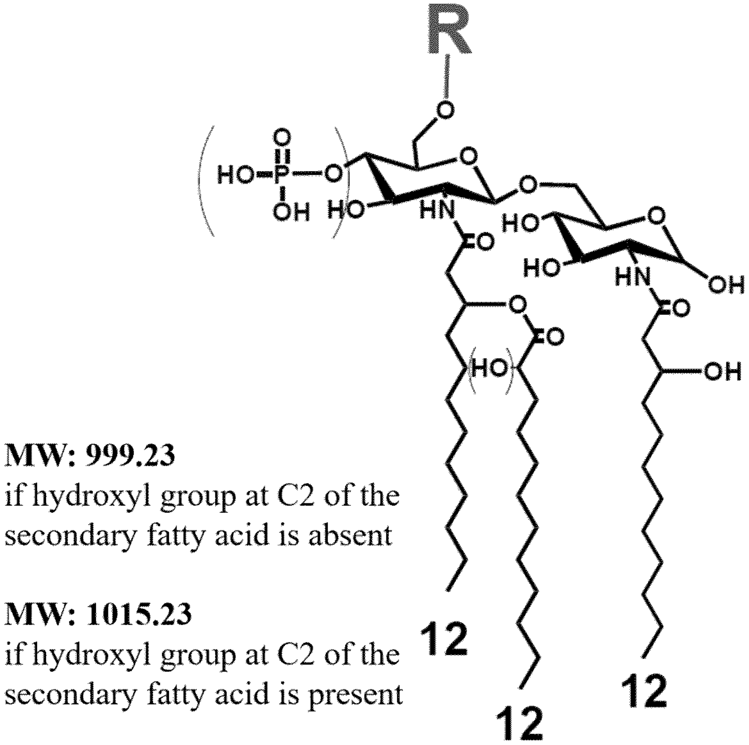

**MW: 999.23**
if hydroxyl group at C2 of the secondary fatty acid is absent

**MW: 1015.23**
if hydroxyl group at C2 of the secondary fatty acid is present

(X)(1)(b)

**FIG. 16**

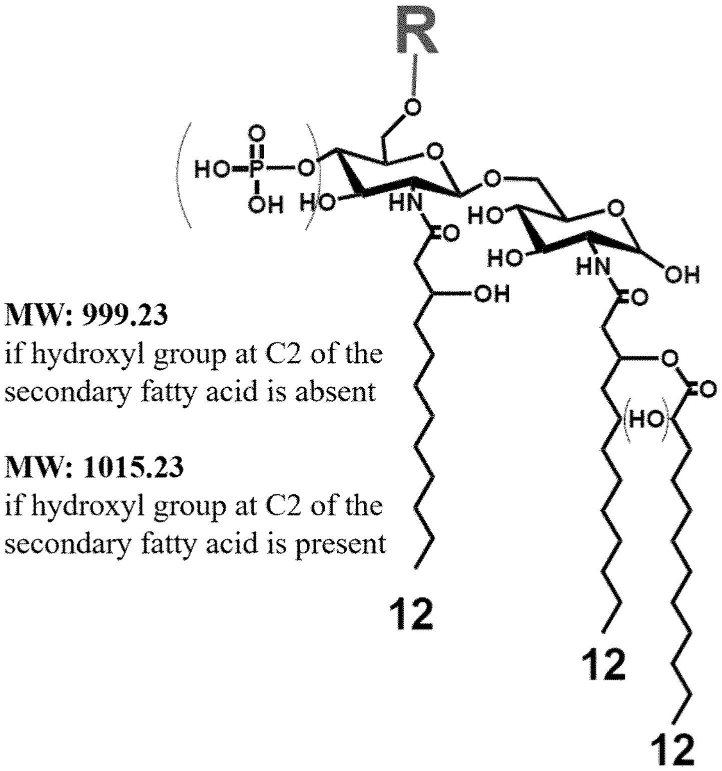

**MW: 999.23**
if hydroxyl group at C2 of the secondary fatty acid is absent

**MW: 1015.23**
if hydroxyl group at C2 of the secondary fatty acid is present

(X)(1)(c)

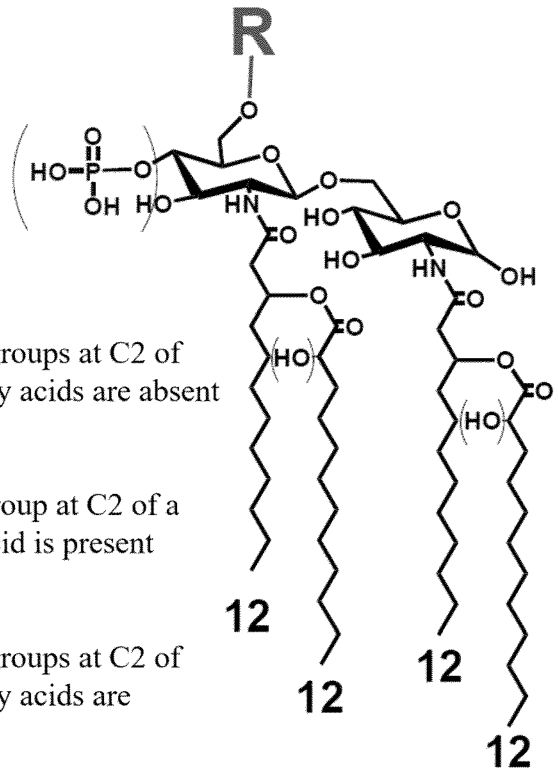

**MW: 1181.53**
if both hydroxyl groups at C2 of the secondary fatty acids are absent

**MW: 1197.53**
if one hydroxyl group at C2 of a secondary fatty acid is present

**MW: 1213,53**
if both hydroxyl groups at C2 of the secondary fatty acids are present

(X)(1)(d)

**FIG. 16 (cont.)**

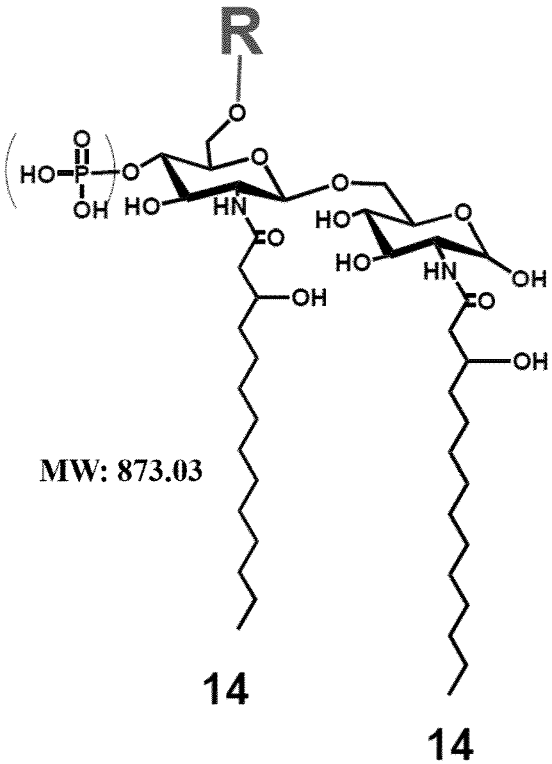

MW: 873.03

(XI)(1)(a)

MW: 1083.39
if hydroxyl group at C2 of the secondary fatty acid is absent

MW: 1099.39
if hydroxyl group at C2 of the secondary fatty acid is present

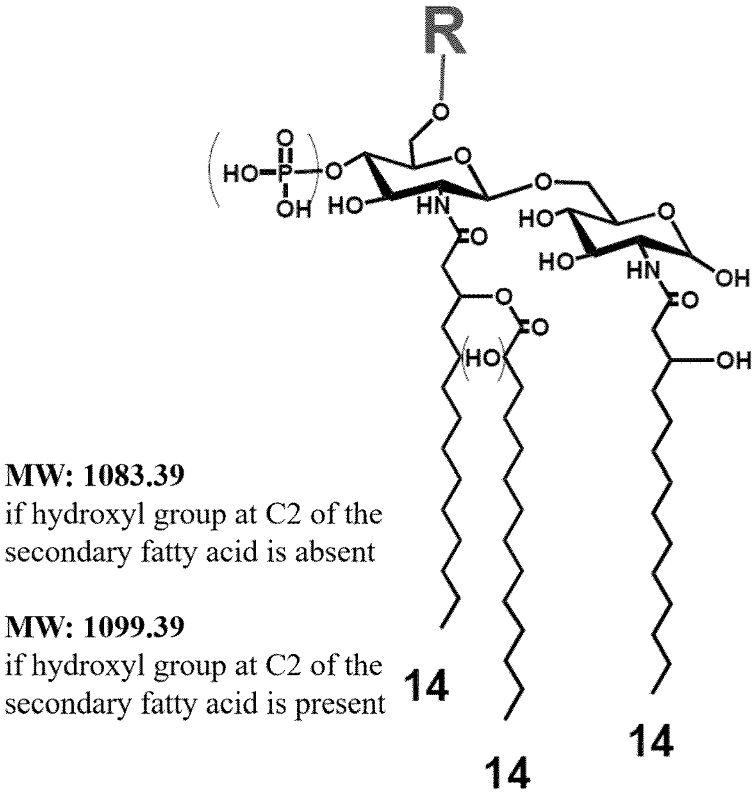

(XI)(1)(b)

FIG. 17

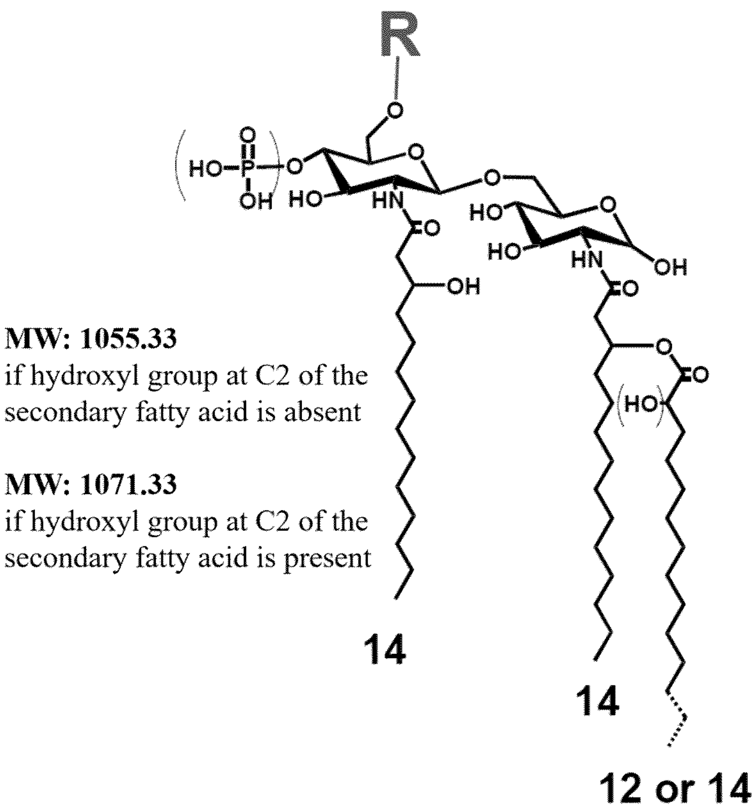

MW: 1055.33
if hydroxyl group at C2 of the secondary fatty acid is absent

MW: 1071.33
if hydroxyl group at C2 of the secondary fatty acid is present

(XI)(1)(c)

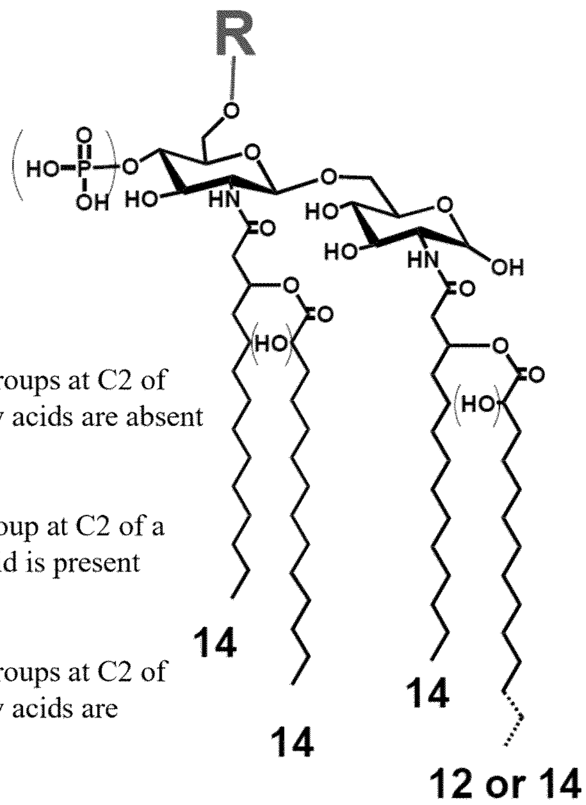

MW: 1265.70
if both hydroxyl groups at C2 of the secondary fatty acids are absent

MW: 1281.69
if one hydroxyl group at C2 of a secondary fatty acid is present

MW: 1297.69
if both hydroxyl groups at C2 of the secondary fatty acids are present

(XI)(1)(d)

FIG. 17 (cont.)

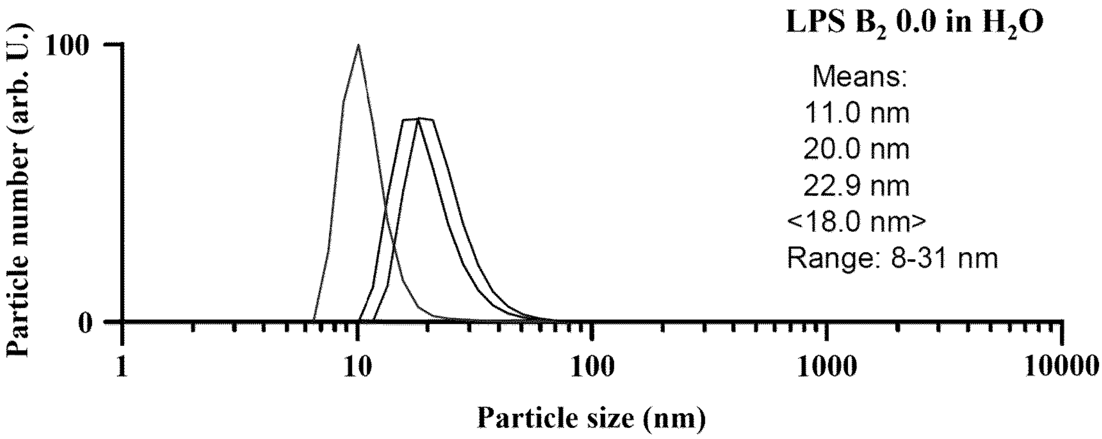


FIG. 18A

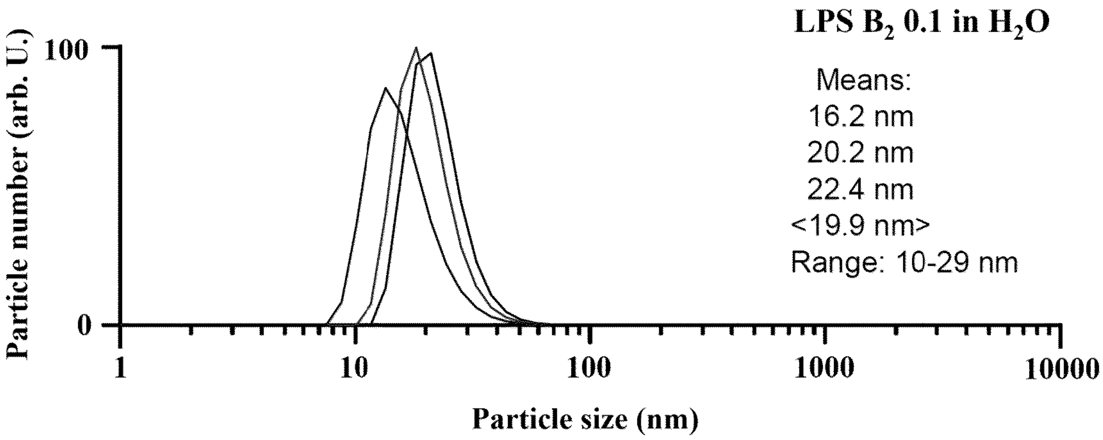


FIG. 18B

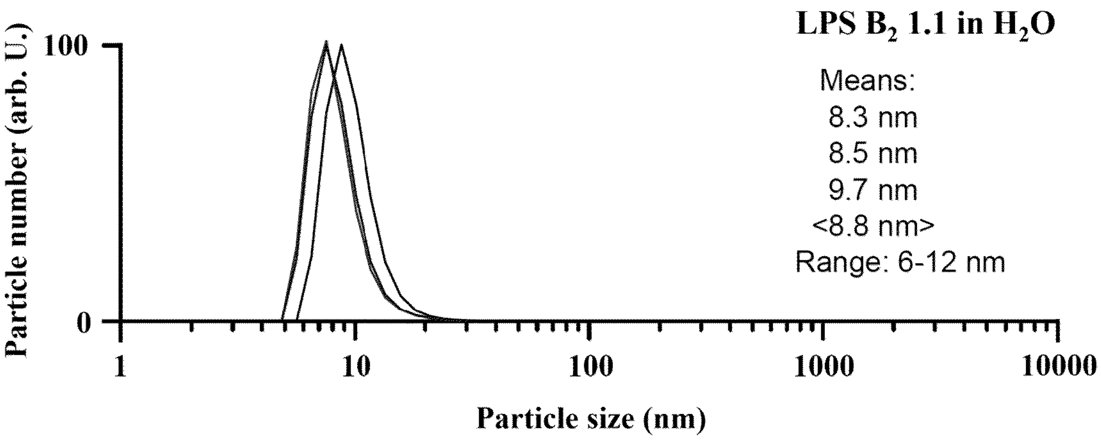


FIG. 18C

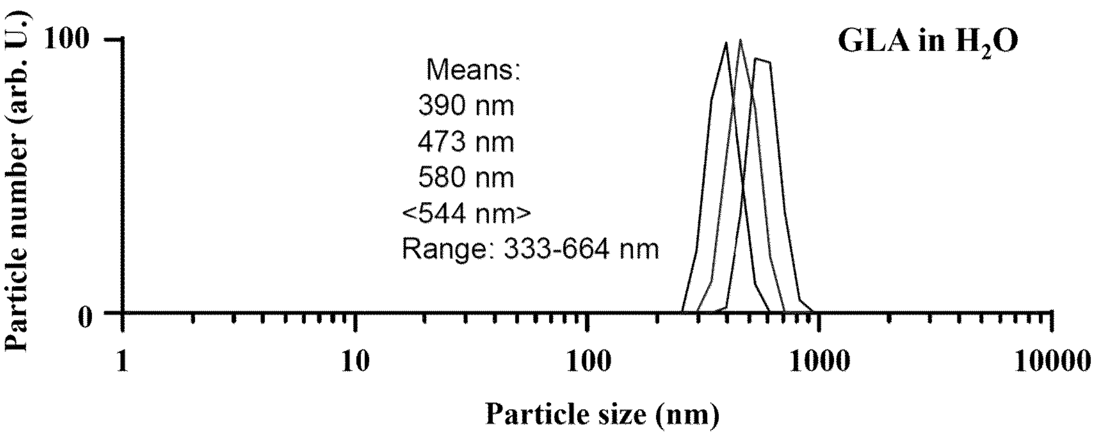


FIG. 18D

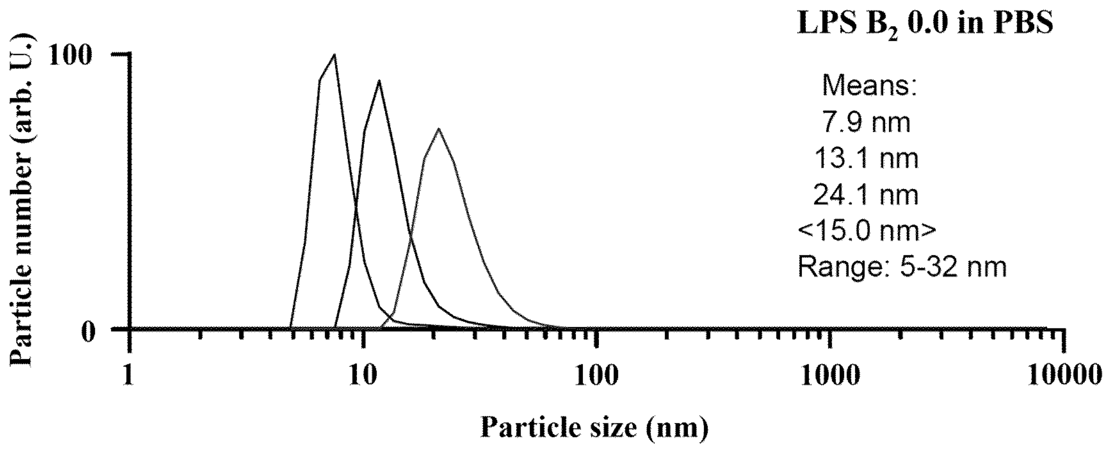


FIG. 19A

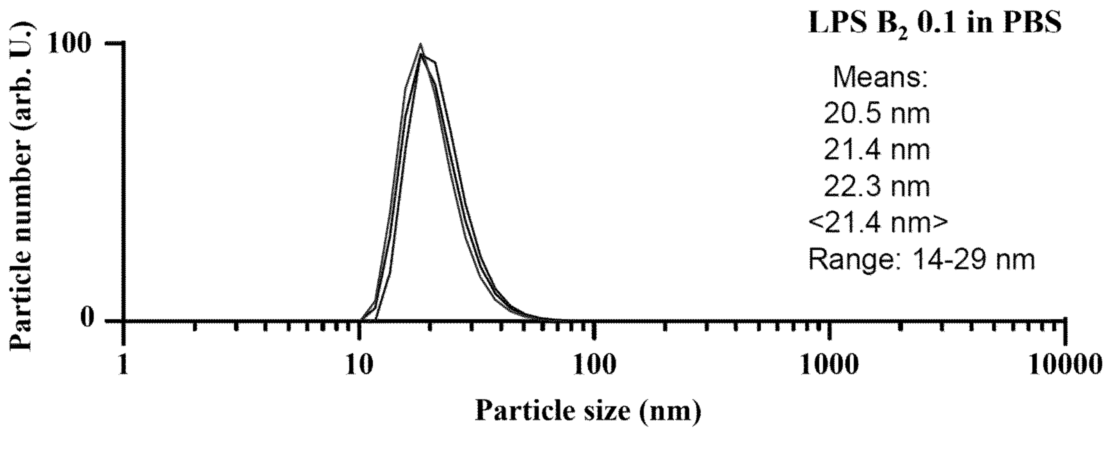


FIG. 19B

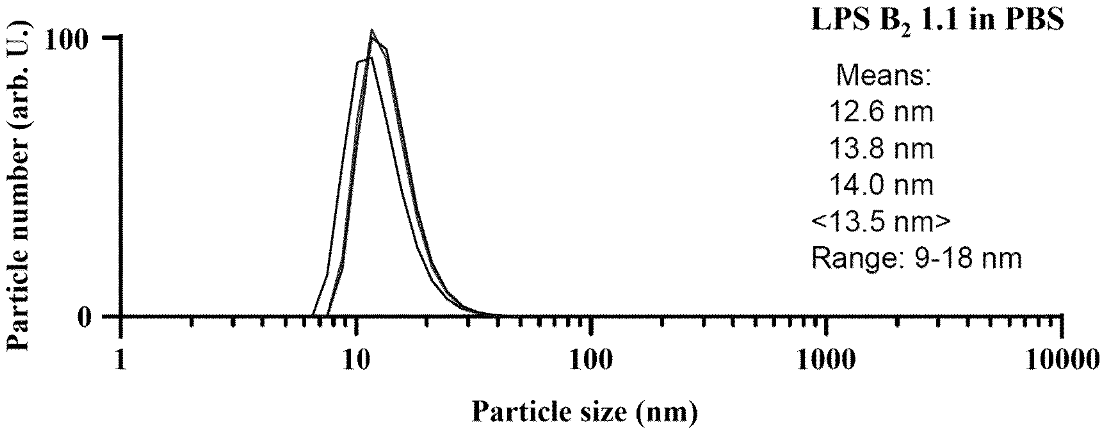


FIG. 19C

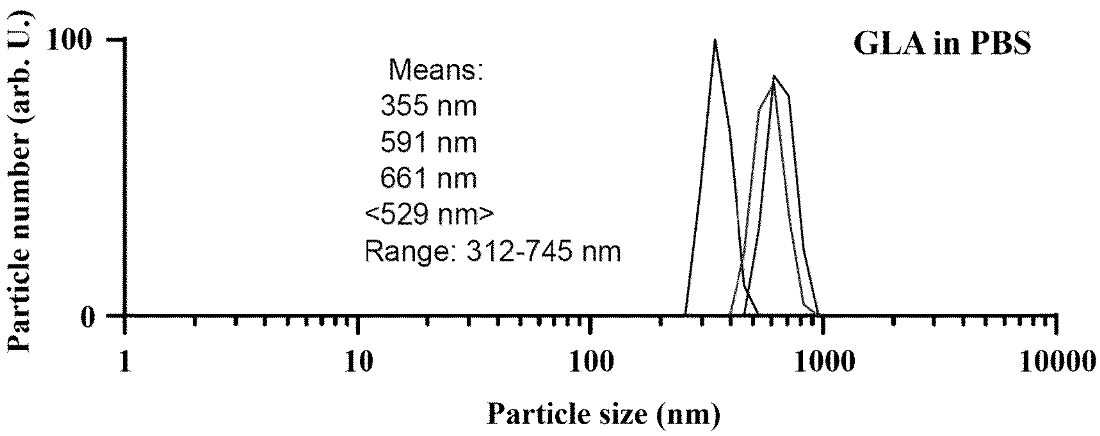


FIG. 19D

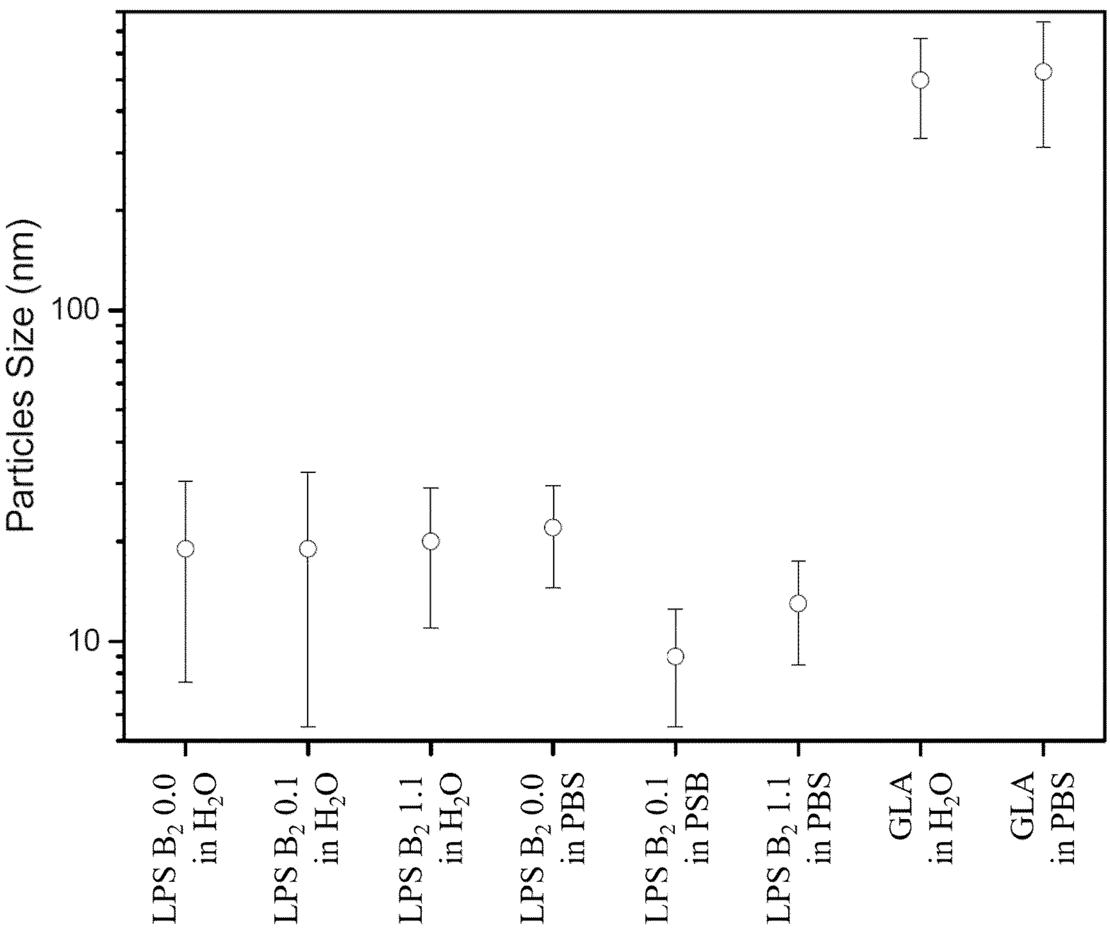
Particles Size (nm)
100
10
LPS B2 0.0 in H2O
LPS B2 0.1 in H2O
LPS B2 1.1 in H2O
LPS B2 0.0 in PBS
LPS B2 0.1 in PSB
LPS B2 1.1 in PBS
GLA in H2O
GLA in PBS

**FIG. 20**

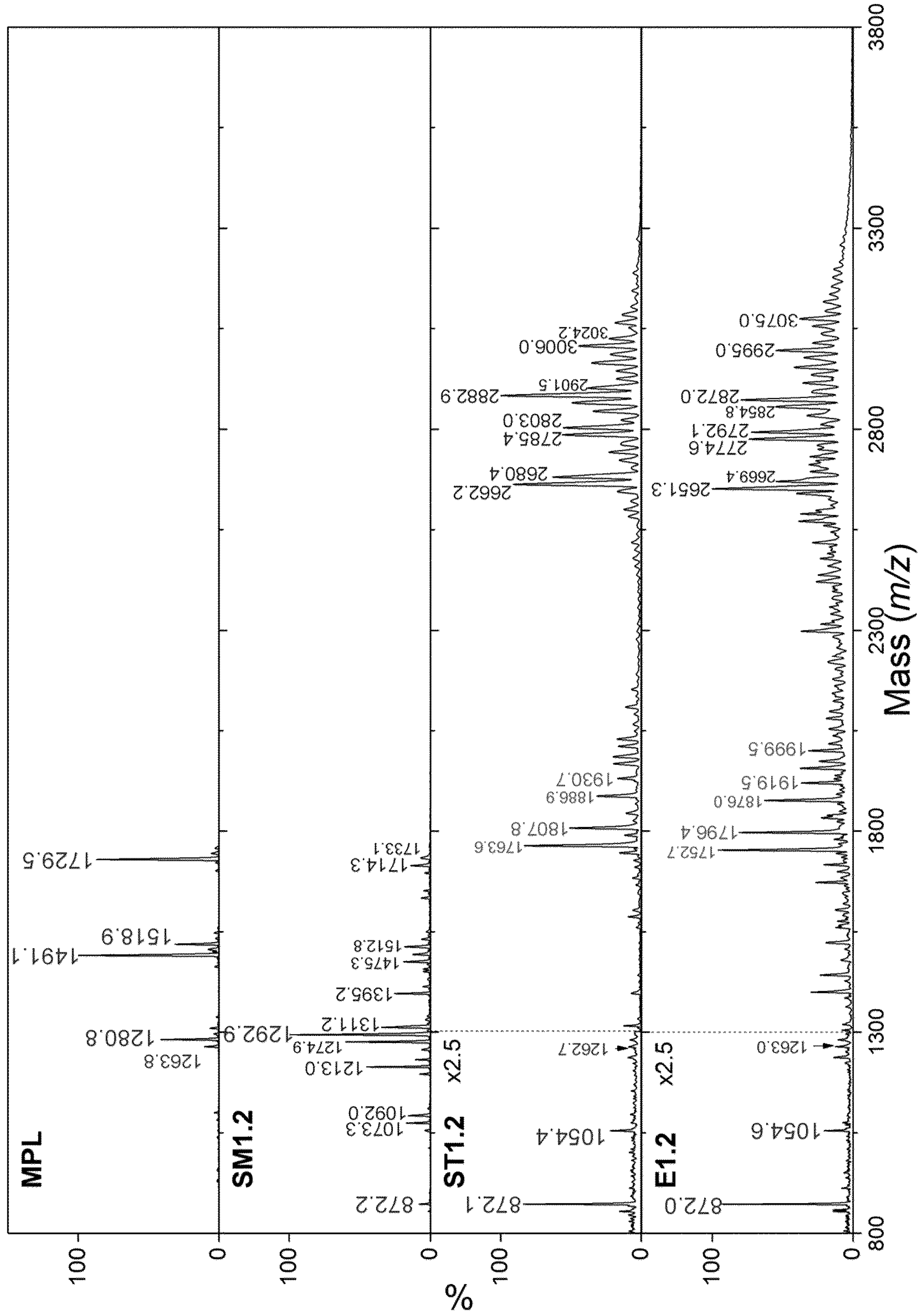
MPL
SM1.2
ST1.2
E1.2
x2.5
x2.5
%
Mass (m/z)
872.2
1263.8
1280.8
1491.1
1518.9
1729.5
1073.3
1092.0
1213.0
1274.9
1292.9
1311.2
1395.2
1475.3
1512.8
1714.3
1733.1
872.1
1054.4
1262.7
1763.6
1807.8
1886.9
1930.7
2662.2
2680.4
2785.4
2803.0
2882.9
2901.5
3006.0
3024.2
872.0
1054.6
1263.0
1752.7
1796.4
1876.0
1919.5
1999.5
2651.3
2669.4
2774.6
2792.1
2854.8
2872.0
2995.0
3075.0
800
1300
1800
2300
2800
3300
3800
0
100

FIG. 21

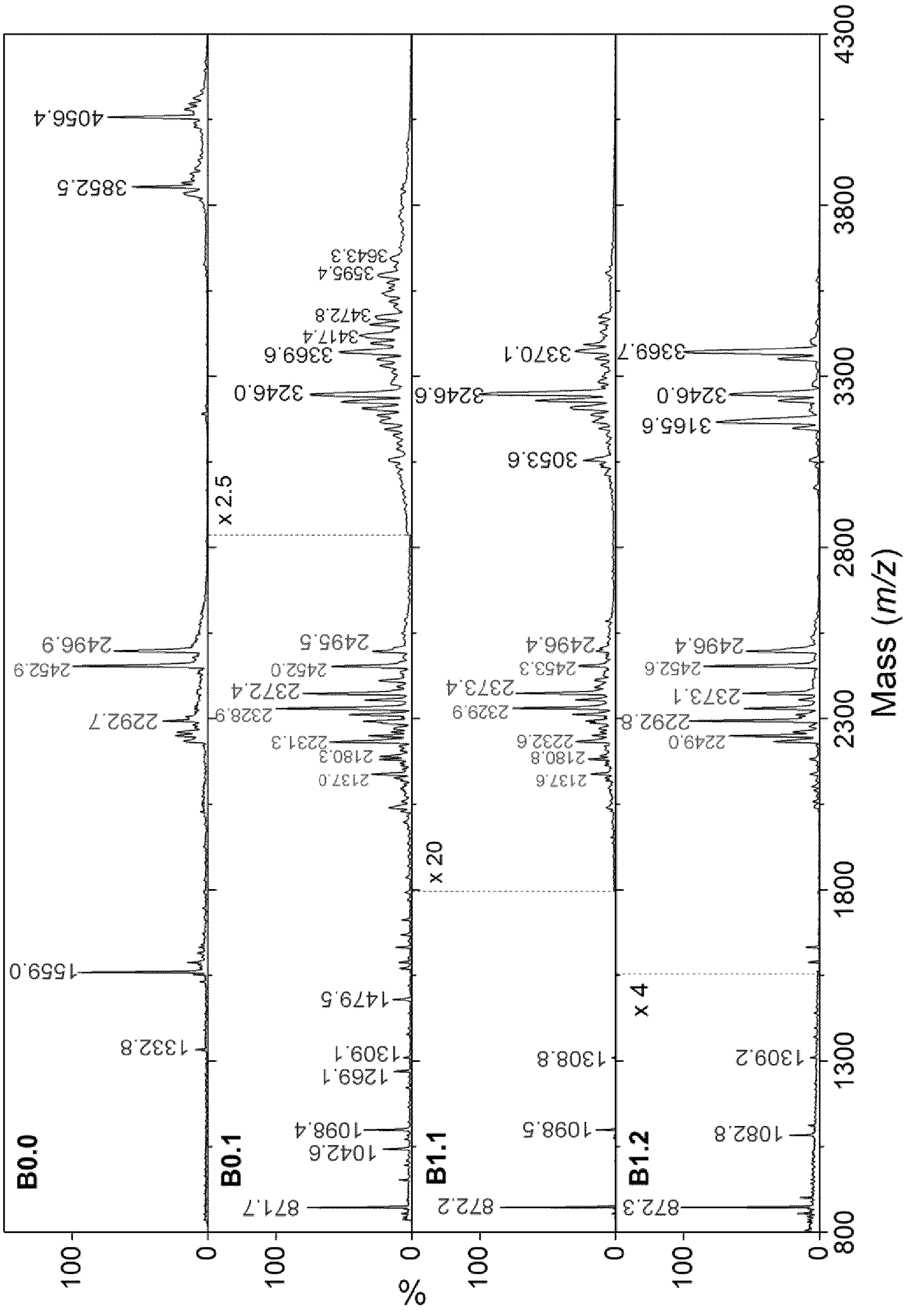
B0.0
B0.1
B1.1
B1.2
%
100
0
Mass (m/z)
800
1300
1800
2300
2800
3300
3800
4300
x 2.5
x 20
x 4
1332.8
1559.0
2292.7
2452.9
2496.9
3852.5
4056.4
871.7
1042.6
1098.4
1269.1
1309.1
1479.5
2137.0
2180.3
2231.3
2328.9
2372.4
2452.0
2495.5
3246.0
3369.6
3417.4
3472.8
3595.4
3643.3
872.2
1098.5
1308.8
2137.6
2180.8
2232.6
2329.9
2373.4
2453.3
2496.4
3053.6
3246.6
3370.1
872.3
1082.8
1309.2
2249.0
2292.8
2373.1
2452.6
2496.4
3165.6
3246.0
3369.7

FIG. 22

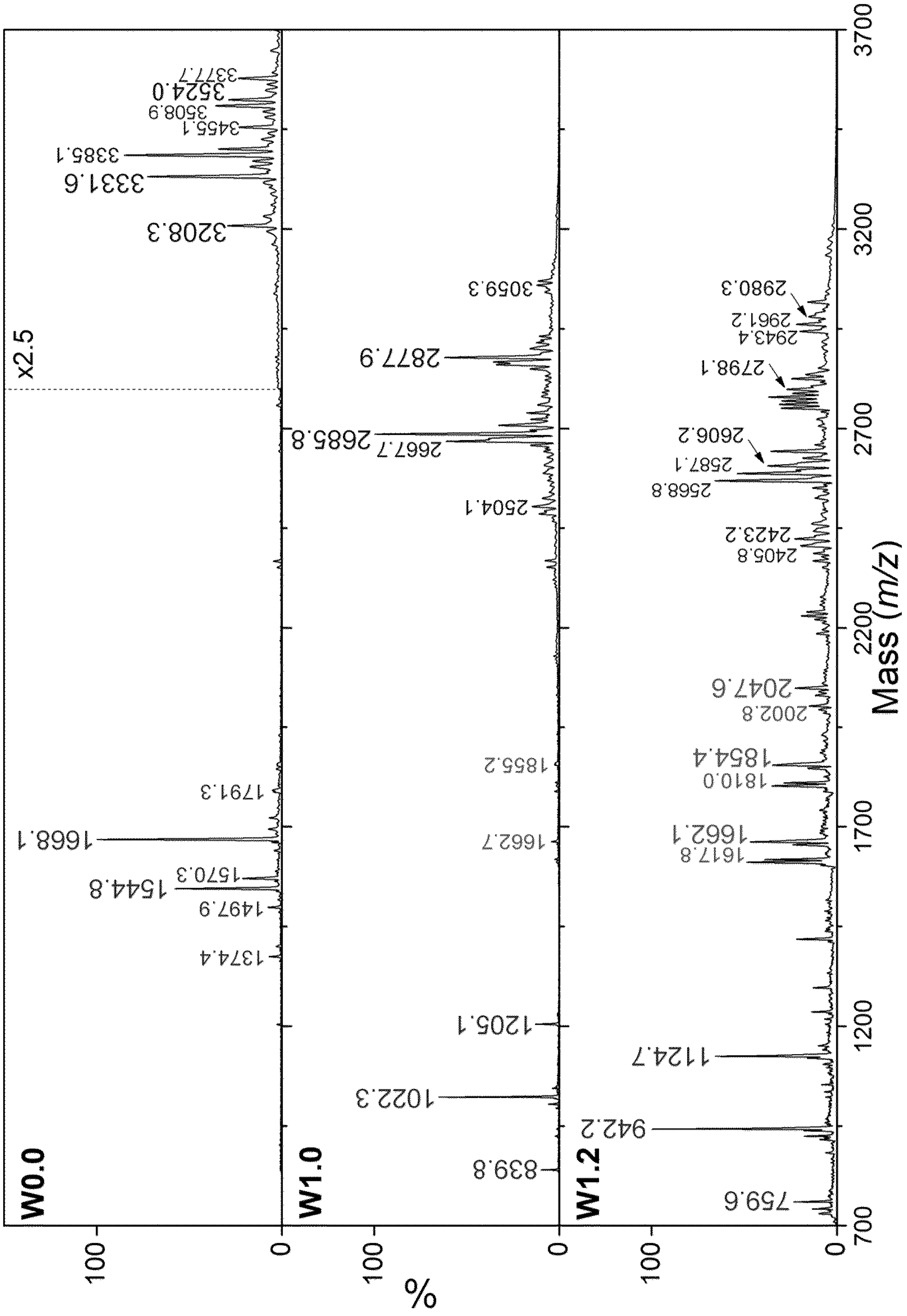
W0.0
W1.0
W1.2
x2.5
%
Mass (m/z)
100
0
700
1200
1700
2200
2700
3200
3700
1374.4
1497.9
1544.8
1570.3
1668.1
1791.3
3208.3
3331.6
3385.1
3455.1
3508.9
3524.0
3577.7
839.8
1022.3
1205.1
1662.7
1855.2
2504.1
2667.7
2685.8
2877.9
3059.3
759.6
942.2
1124.7
1617.8
1662.1
1810.0
1854.4
2002.8
2047.6
2405.8
2423.2
2568.8
2587.1
2606.2
2798.1
2943.4
2961.2
2980.3

FIG. 23

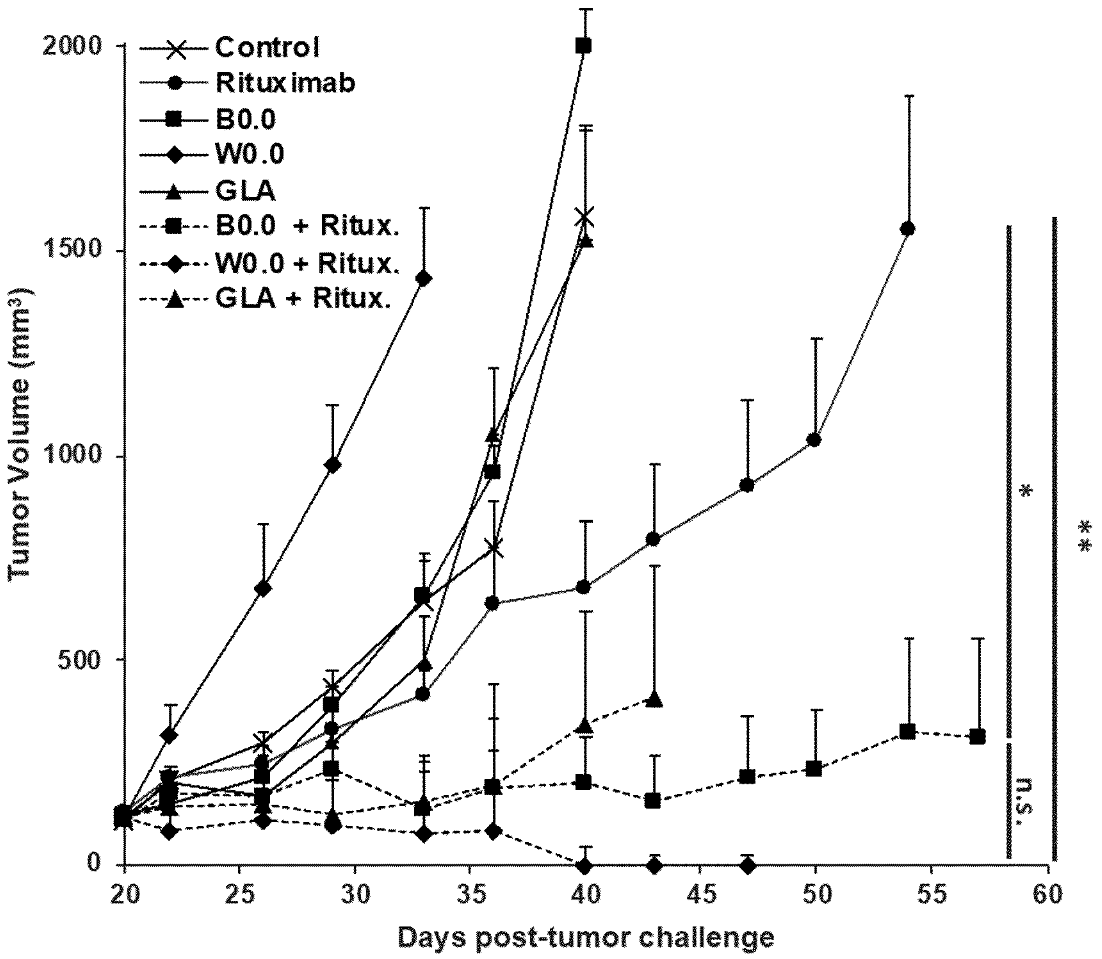
Control
Rituximab
B0.0
W0.0
GLA
B0.0 + Ritux.
W0.0 + Ritux.
GLA + Ritux.
Tumor Volume (mm³)
2000
1500
1000
500
0
20
25
30
35
40
45
50
55
60
Days post-tumor challenge
*
**
n.s.

FIG. 24A

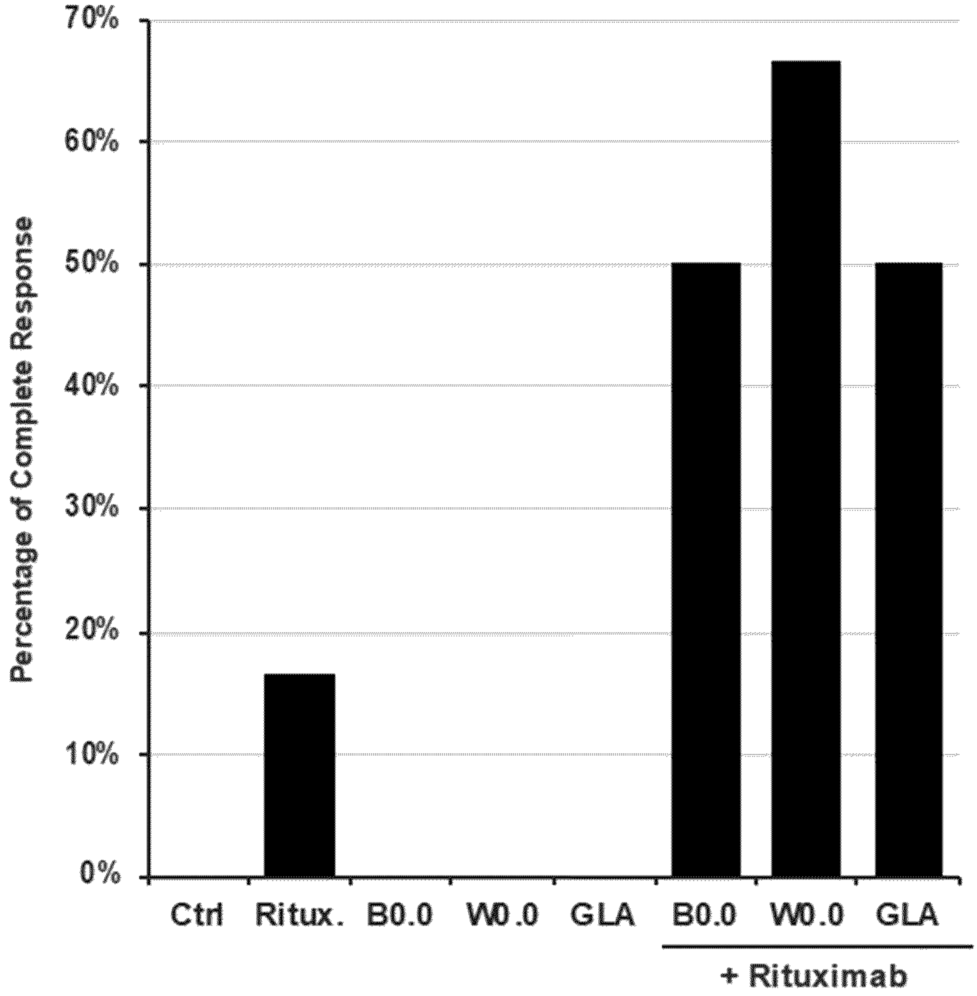
70%
60%
50%
40%
30%
20%
10%
0%
Percentage of Complete Response
Ctrl
Ritux.
B0.0
W0.0
GLA
B0.0
W0.0
GLA
+ Rituximab

FIG. 24B

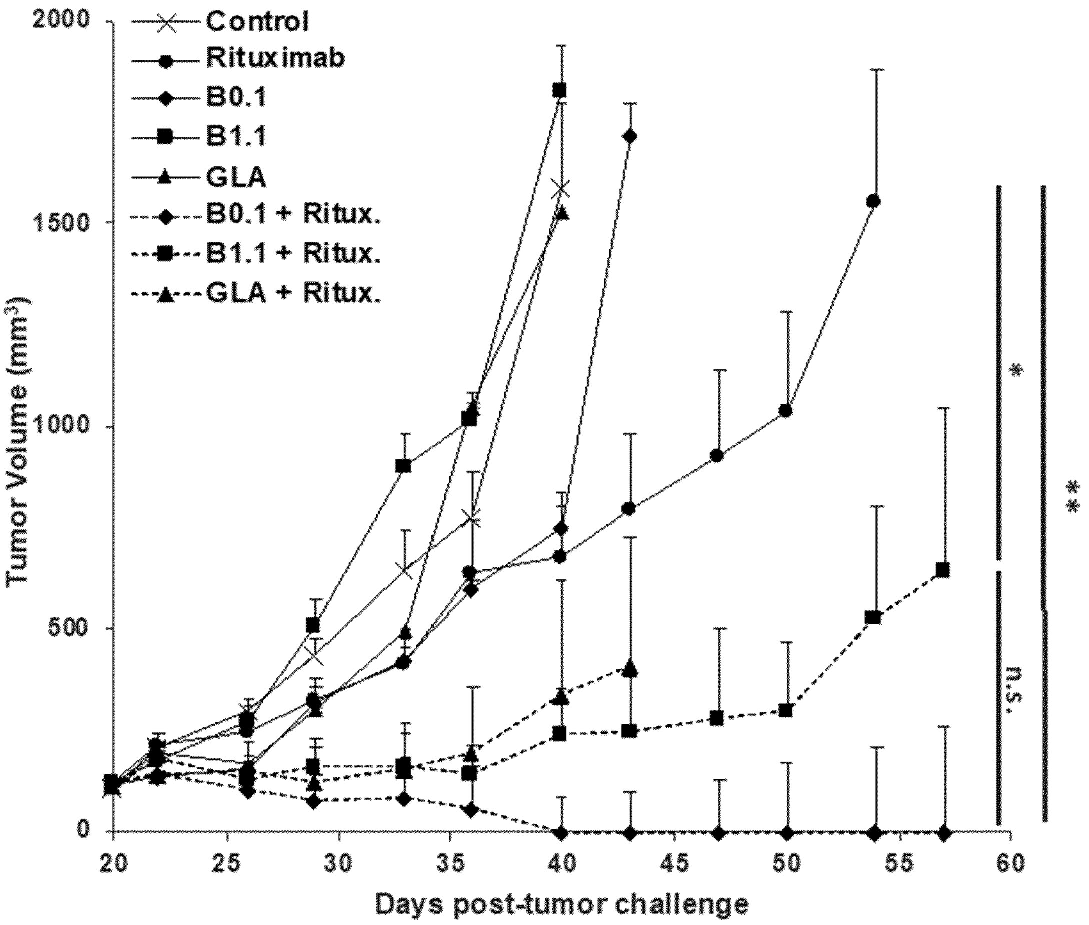
Control
Rituximab
B0.1
B1.1
GLA
B0.1 + Ritux.
B1.1 + Ritux.
GLA + Ritux.
Tumor Volume (mm3)
2000
1500
1000
500
0
20
25
30
35
40
45
50
55
60
Days post-tumor challenge
*
**
n.s.

FIG. 25A

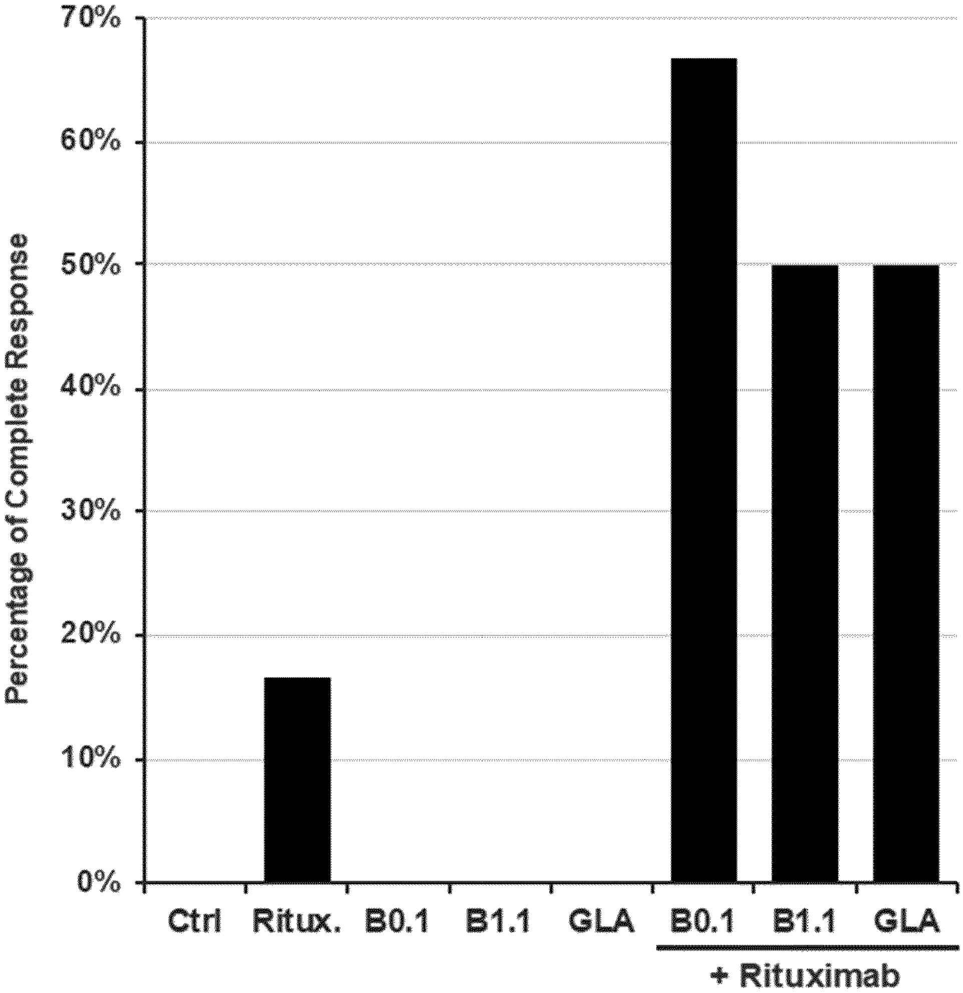
70%
60%
50%
40%
30%
20%
10%
0%
Percentage of Complete Response
Ctrl
Ritux.
B0.1
B1.1
GLA
B0.1
B1.1
GLA
+ Rituximab

FIG. 25B

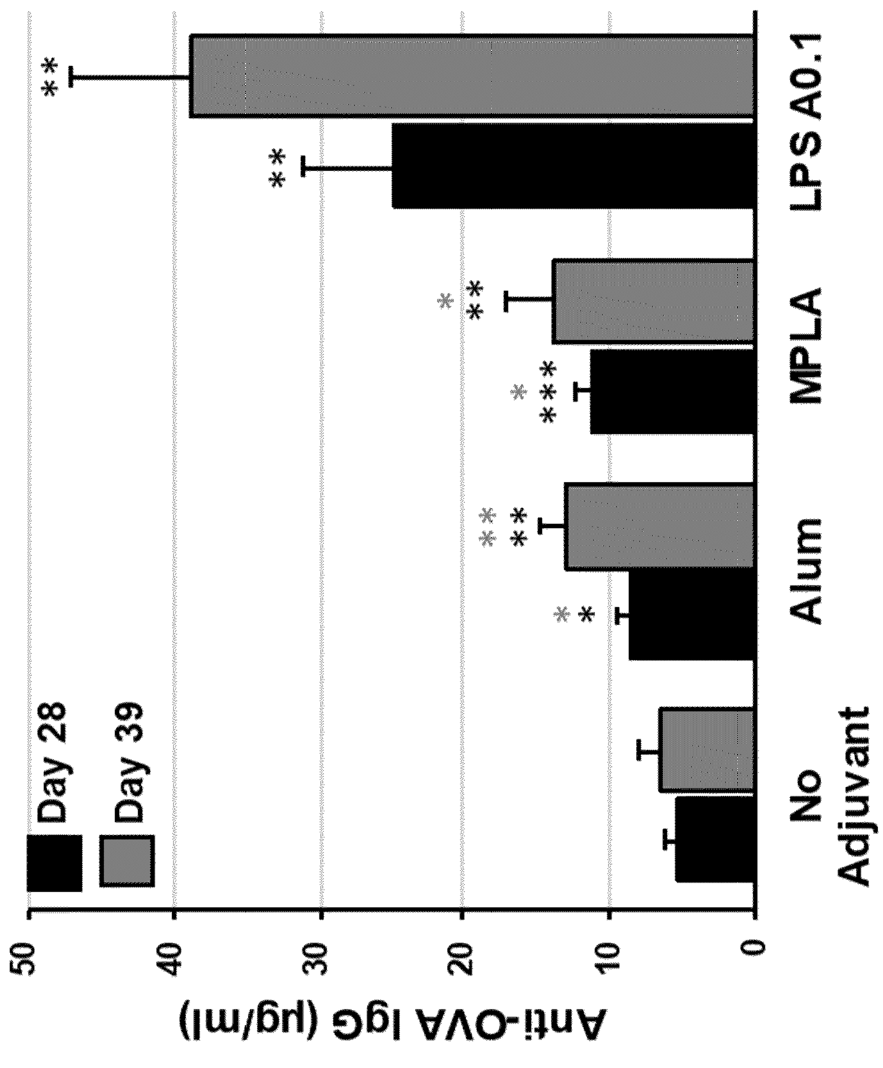


FIG. 26A

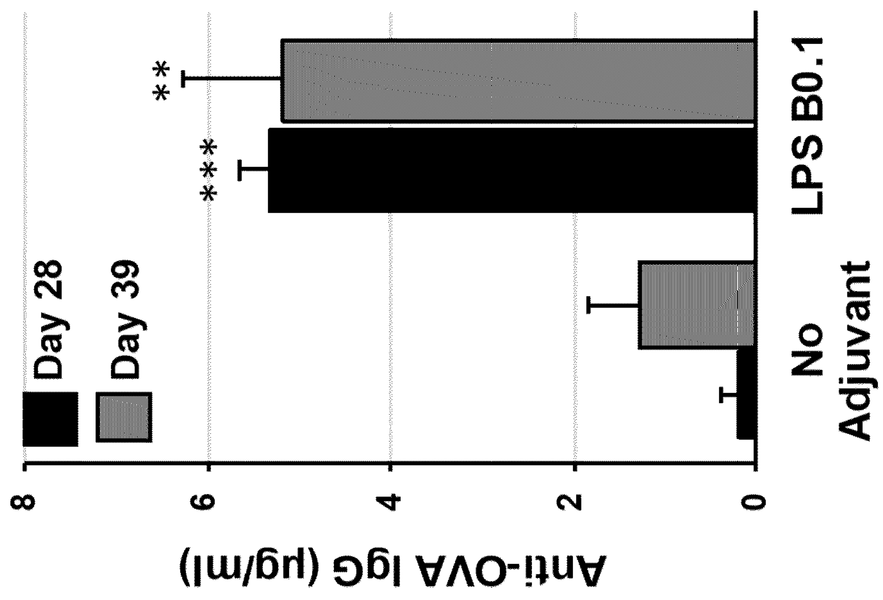


FIG. 26B

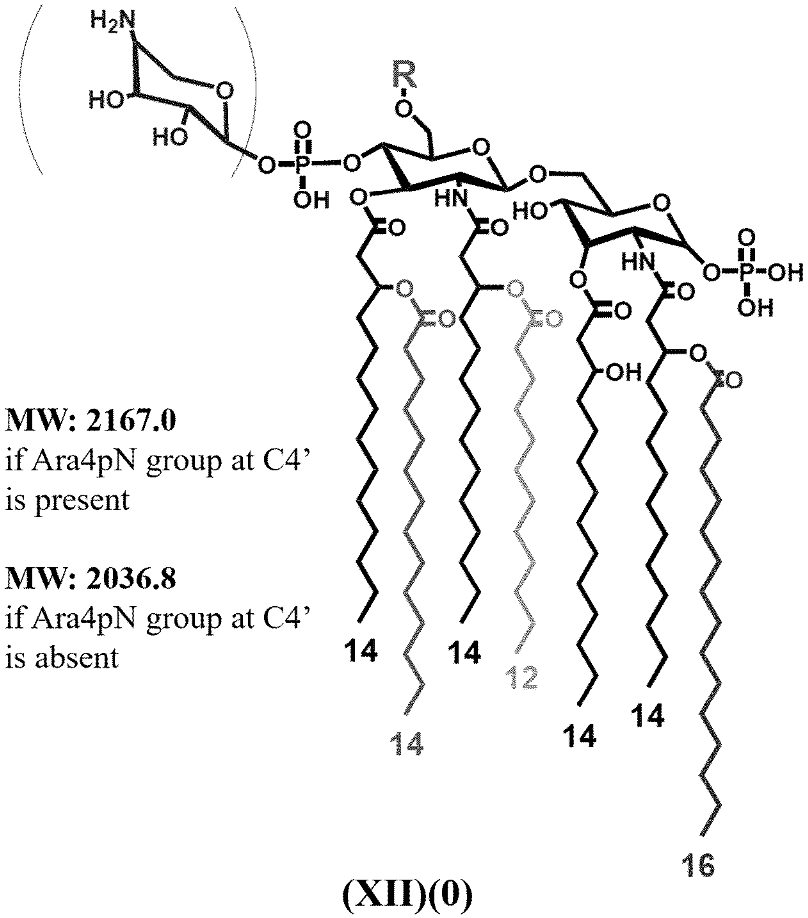

**MW: 2167.0**
if Ara4pN group at C4' is present

**MW: 2036.8**
if Ara4pN group at C4' is absent

(XII)(0)

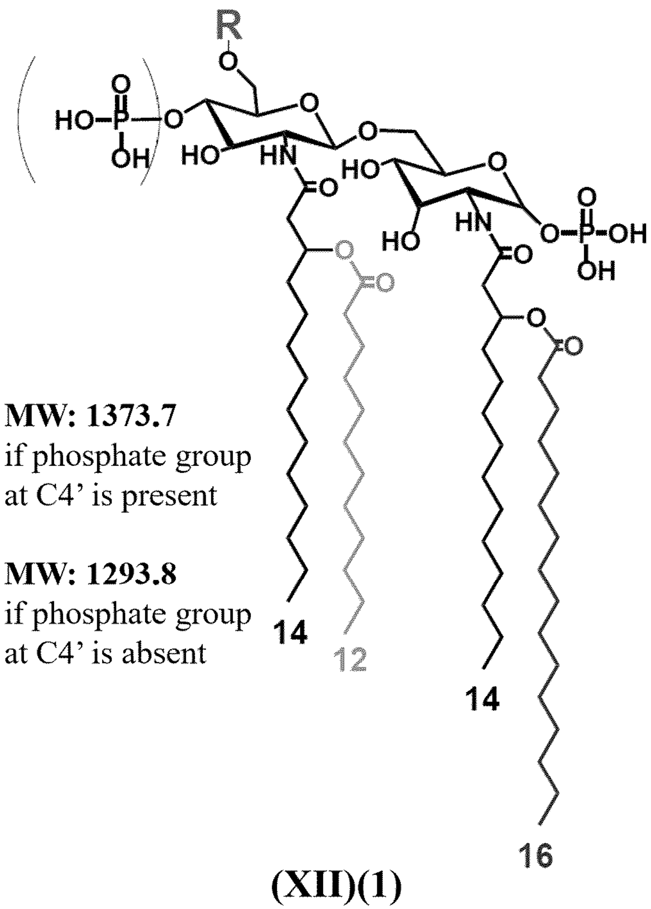

**MW: 1373.7**
if phosphate group at C4' is present

**MW: 1293.8**
if phosphate group at C4' is absent

(XII)(1)

FIG. 27

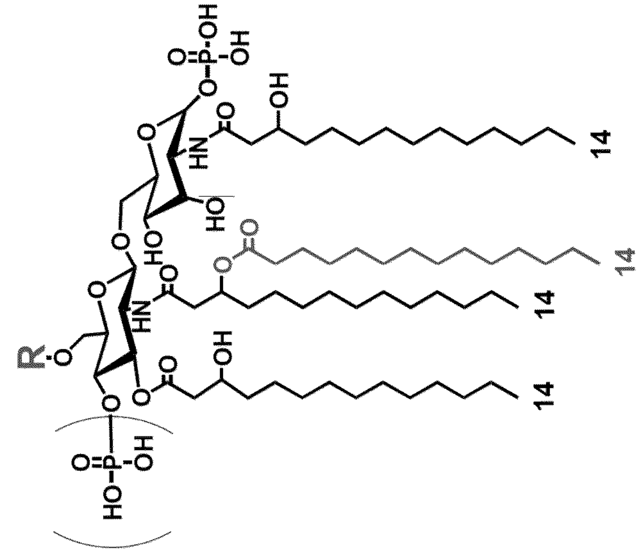

(I)(4)(a)

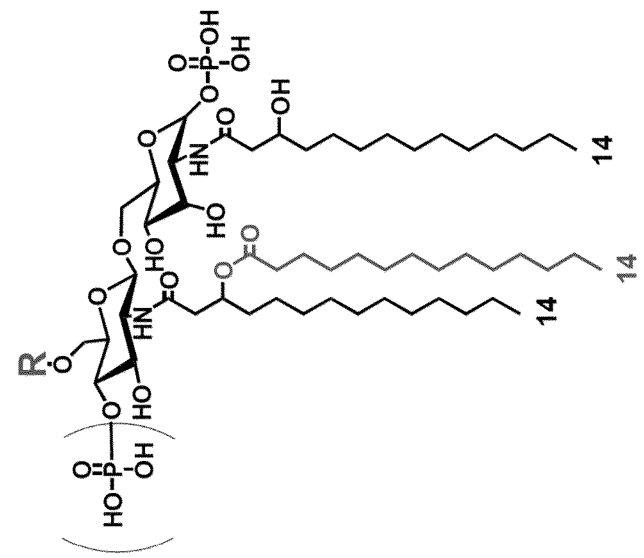

(I)(4)(b)

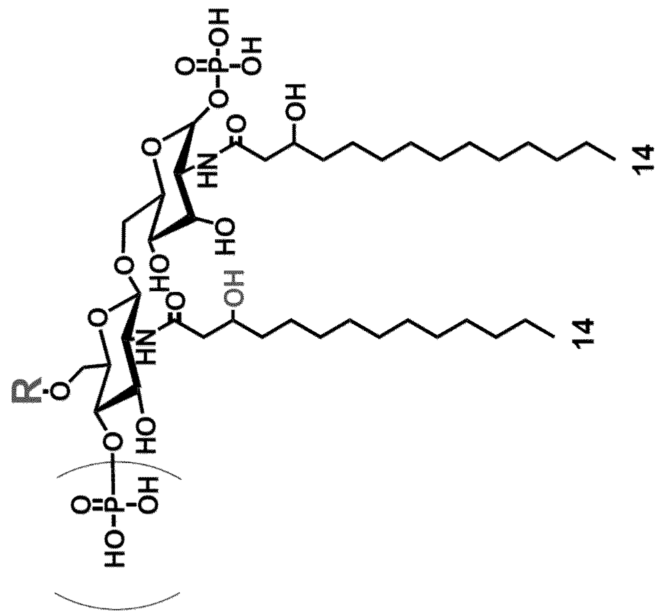

(I)(4)(c)

FIG. 28

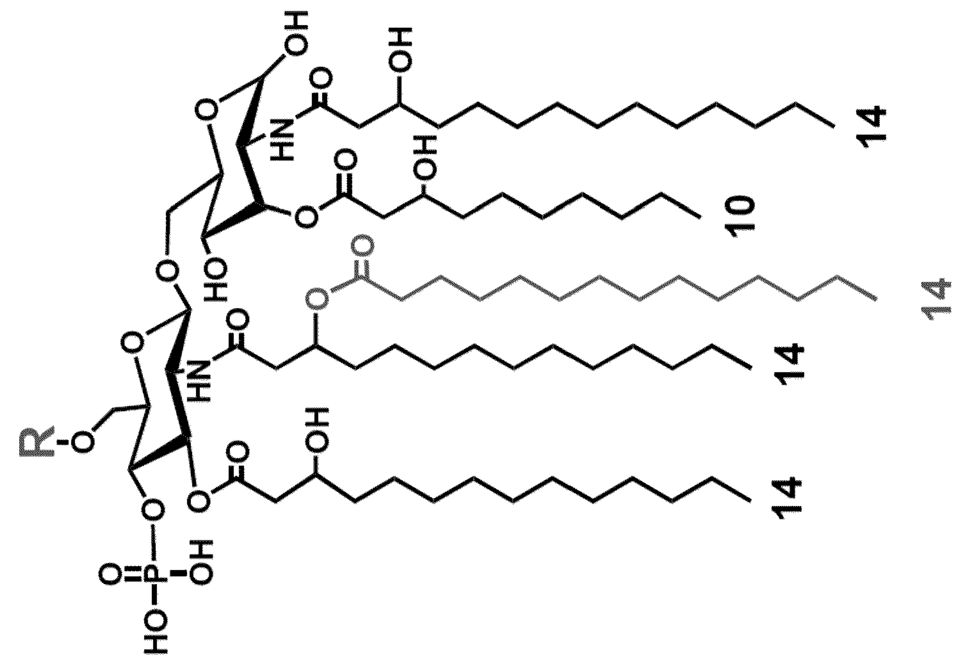

(I)(5)(a)

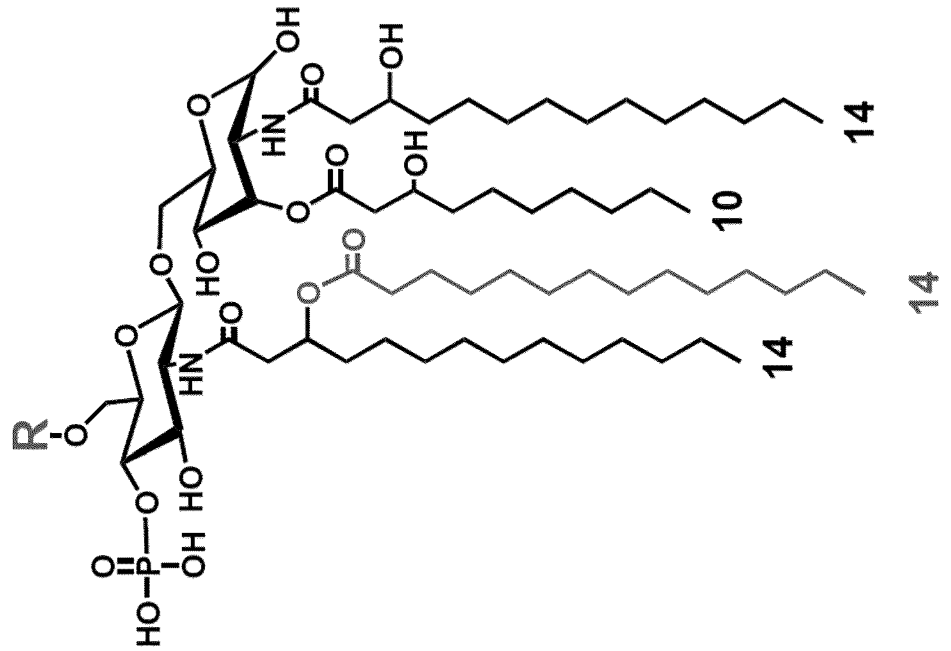

(I)(5)(b)

FIG. 29

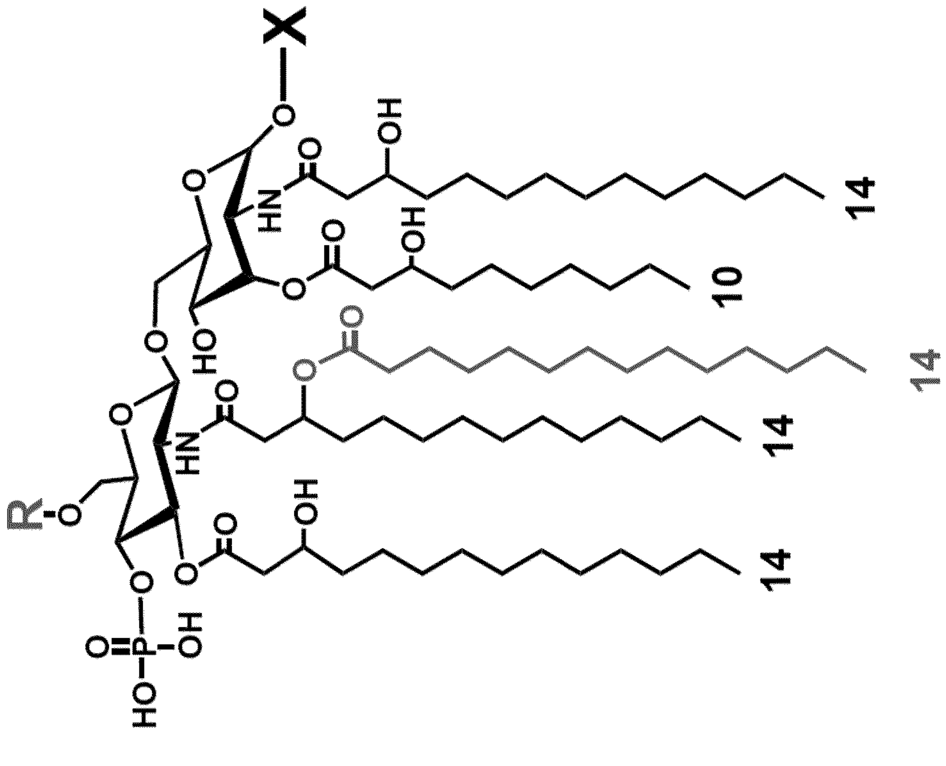

(I)(6)(a)

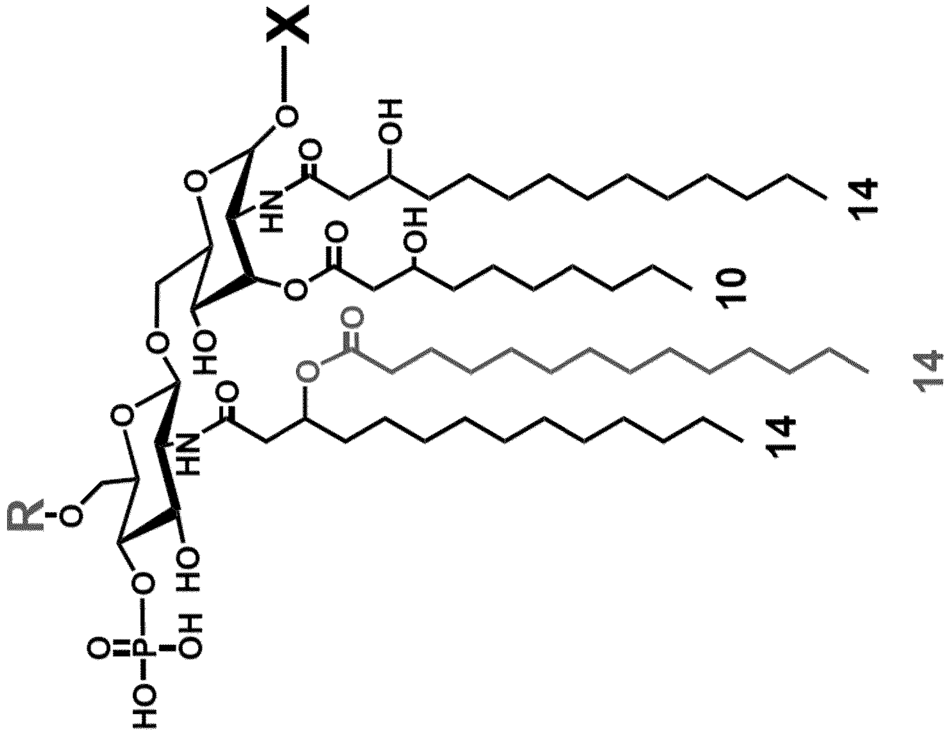

(I)(6)(b)

FIG. 30

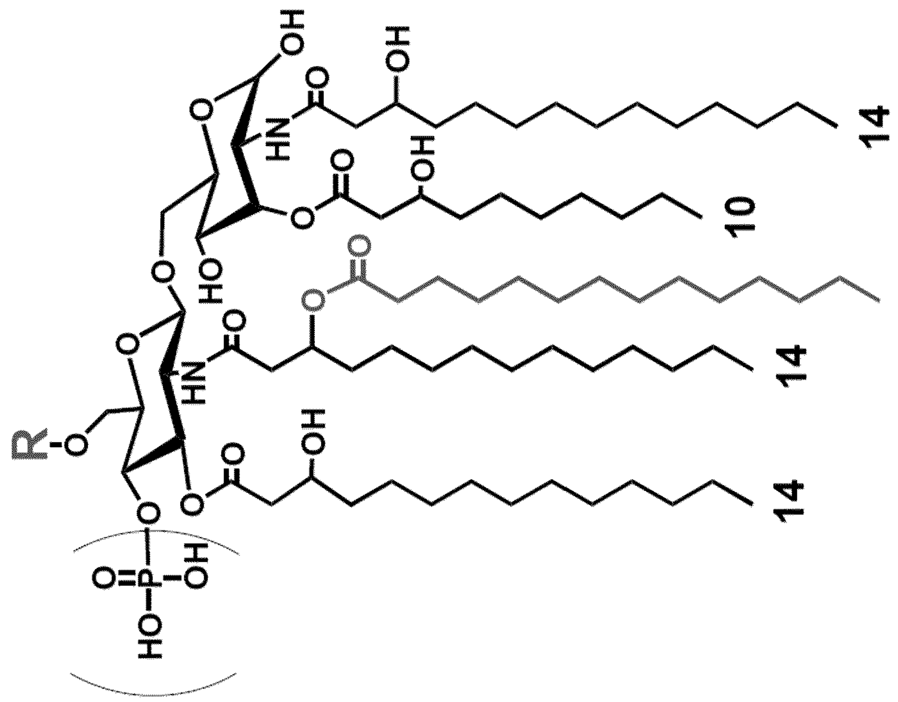

(I)(7)(a)

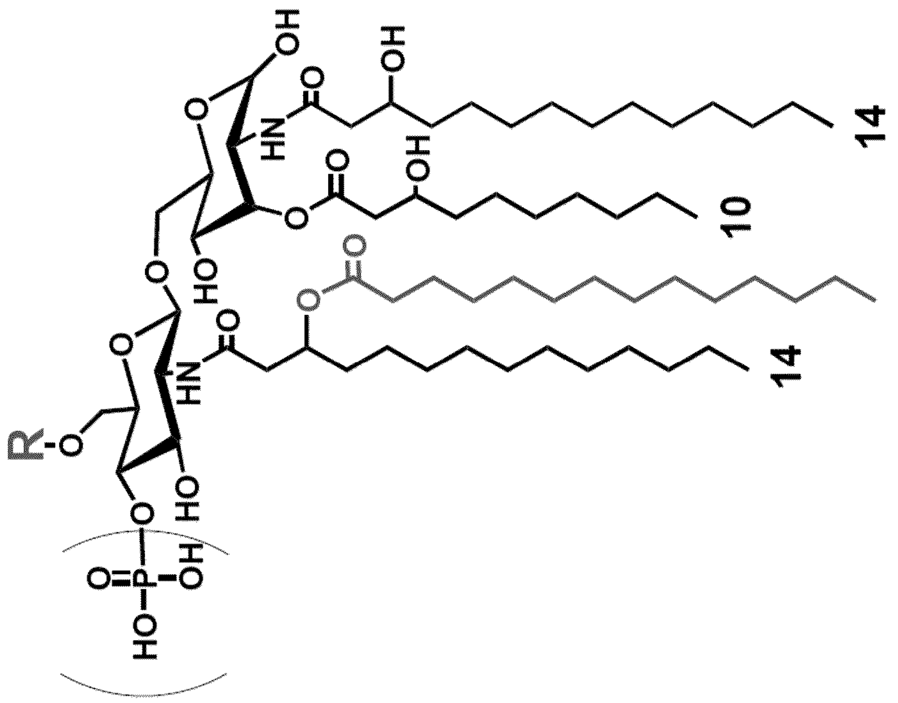

(I)(7)(b)

FIG. 31

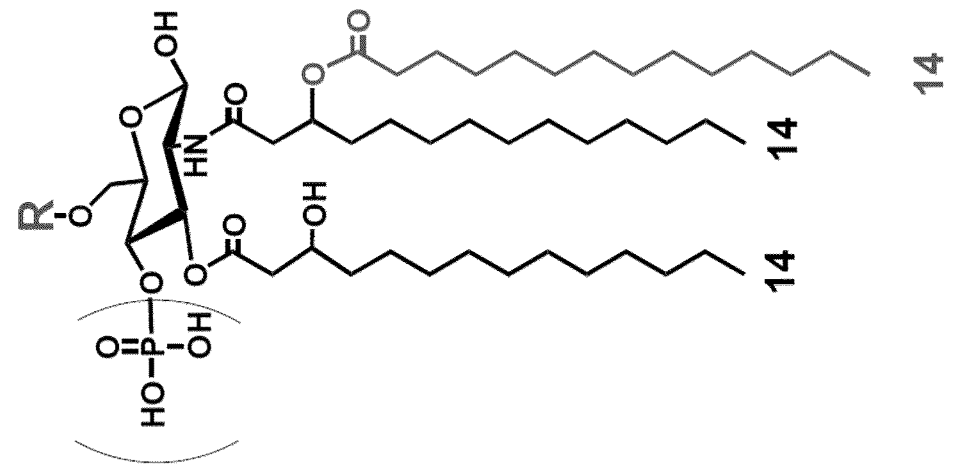

(I)(7)(c)

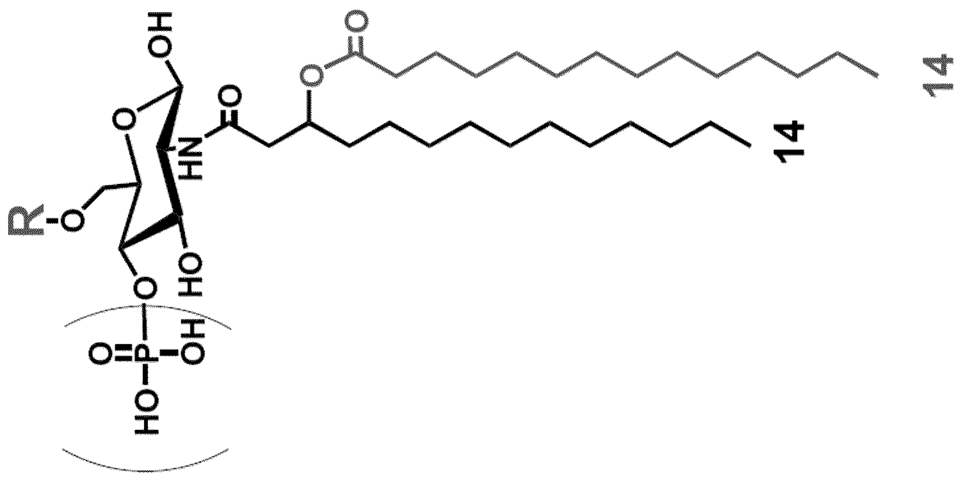

(I)(7)(d)

FIG. 31 (cont.)

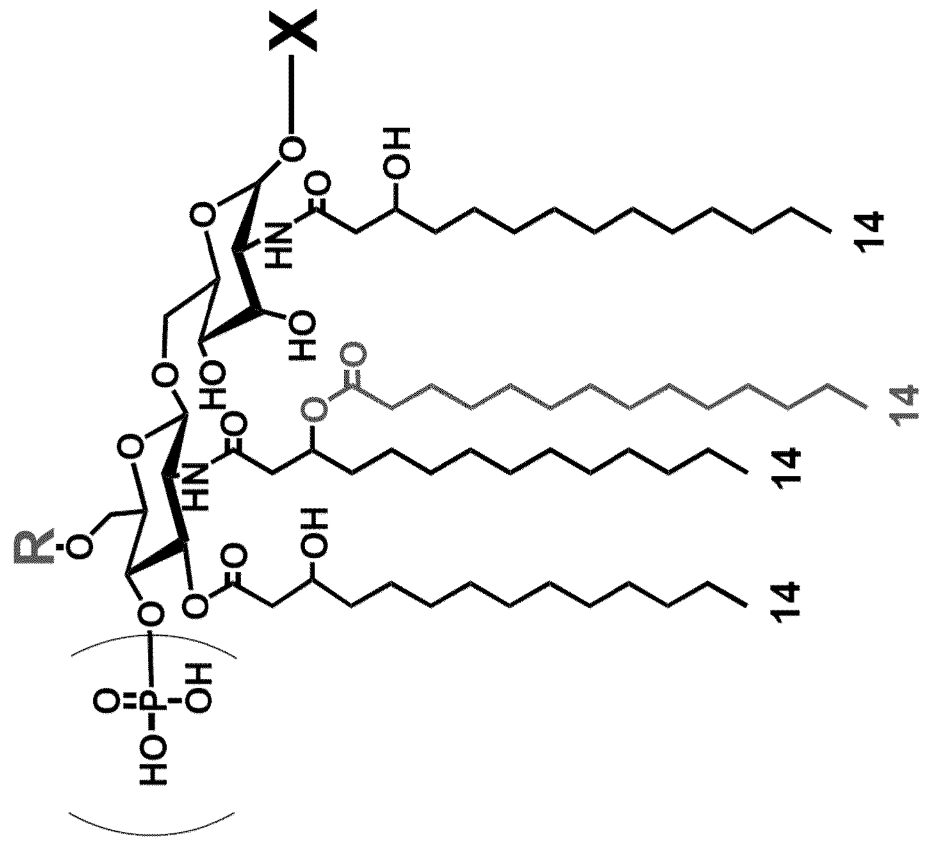

(I)(8)(a)

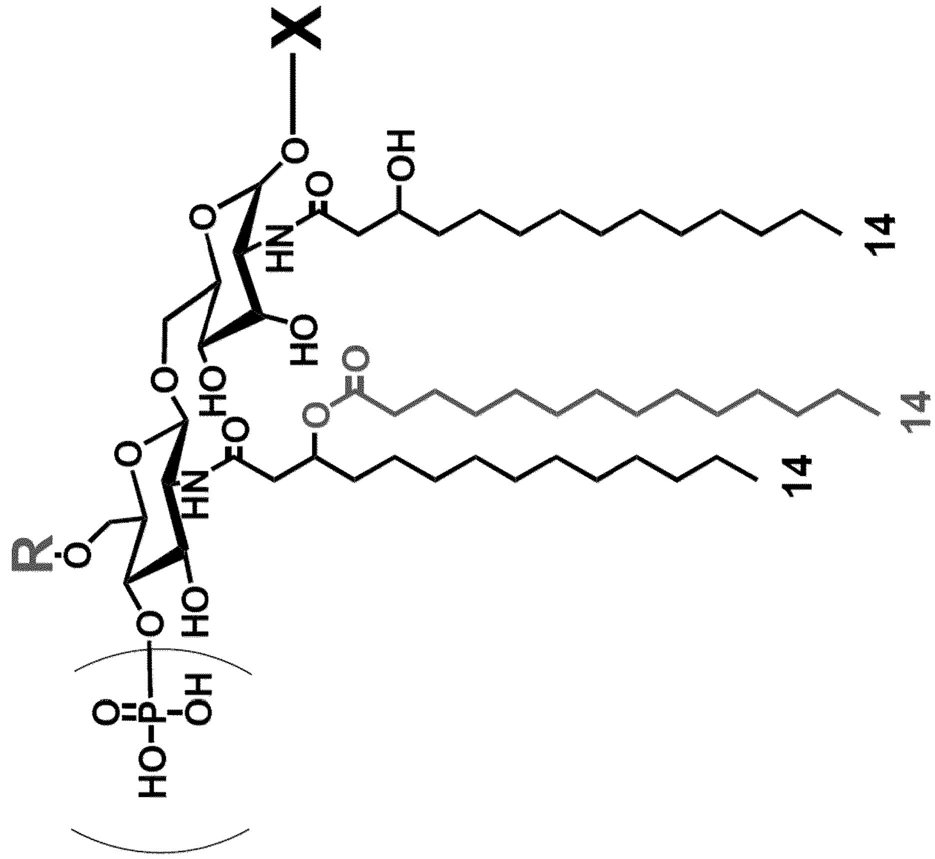

(I)(8)(b)

FIG. 32

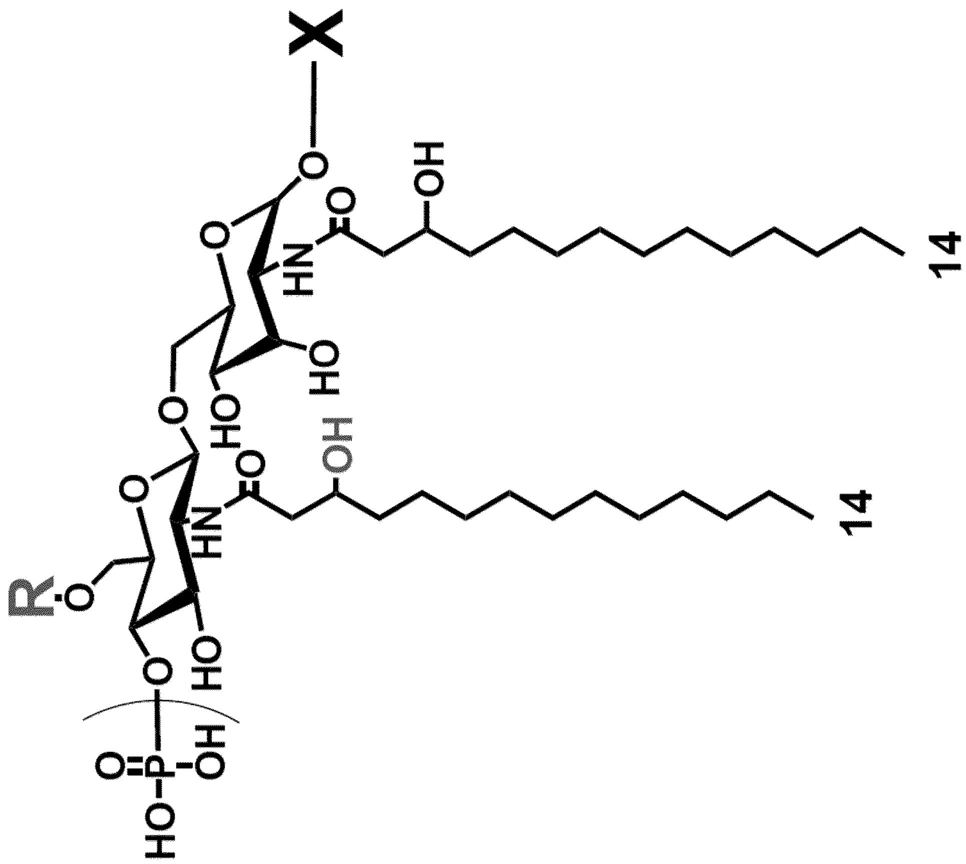

(I)(8)(c)

FIG. 32 (cont.)

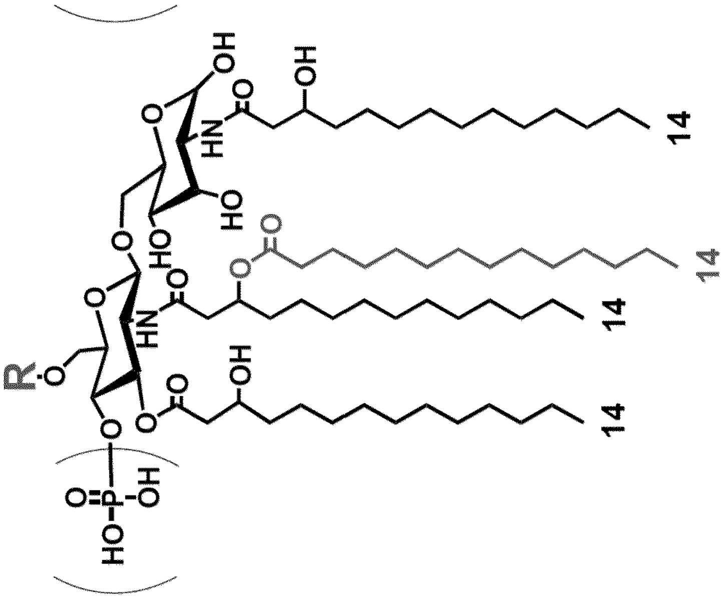

(I)(9)(a)

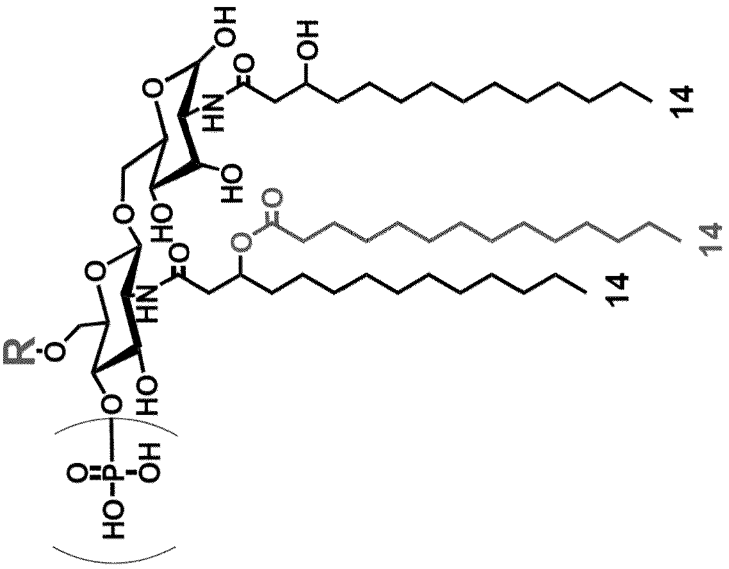

(I)(9)(b)

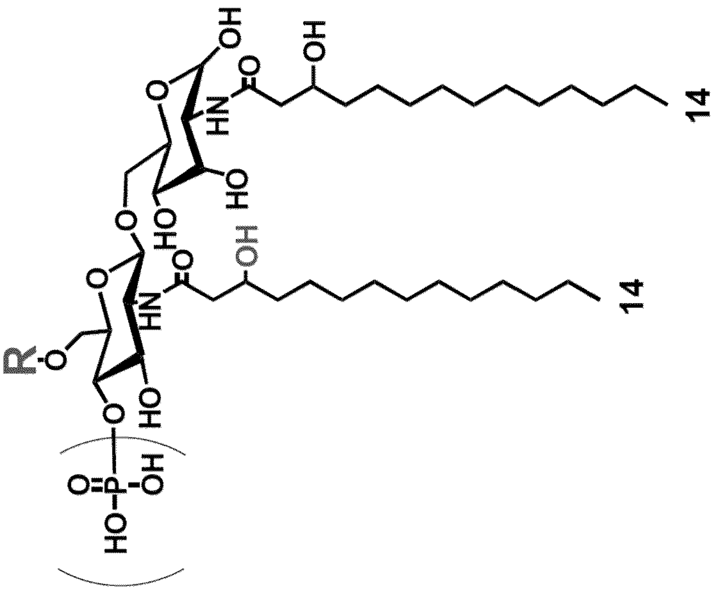

(I)(9)(c)

FIG. 33

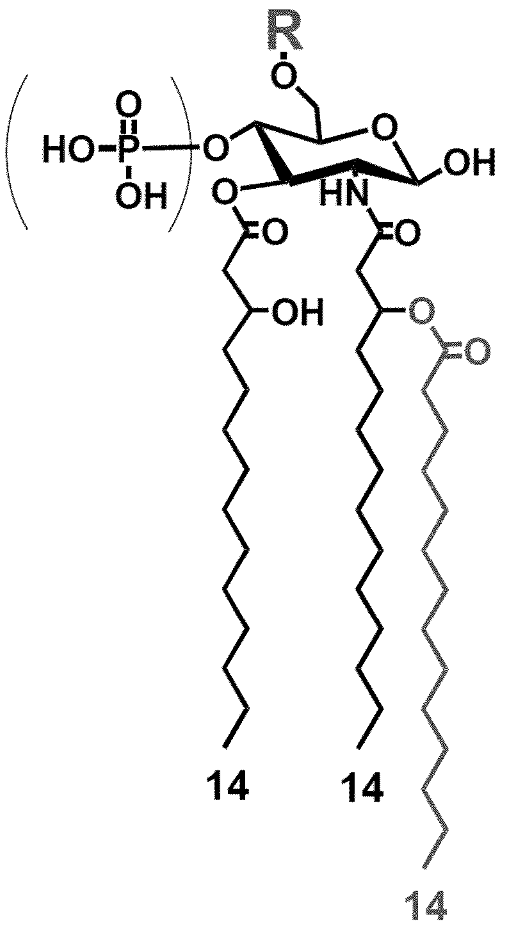

(I)(9)(d)

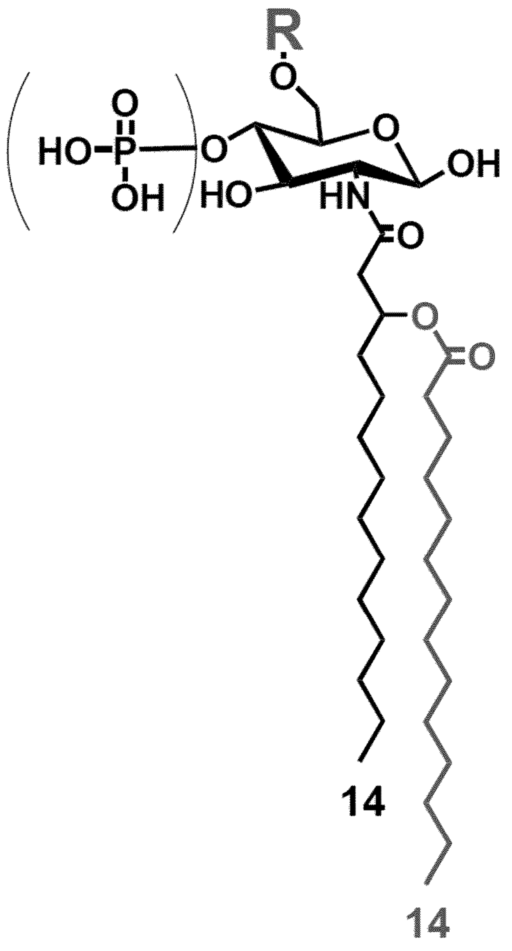

(I)(9)(e)

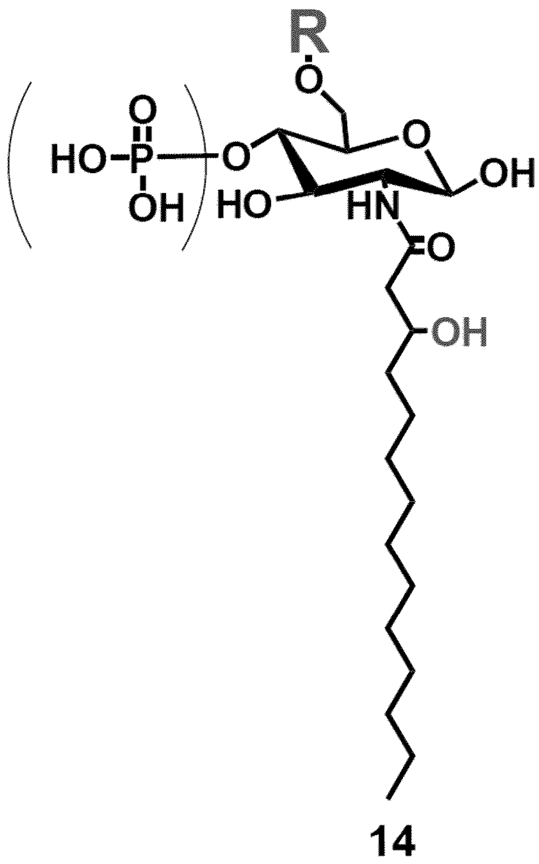

(I)(9)(f)

FIG. 33 (cont.)

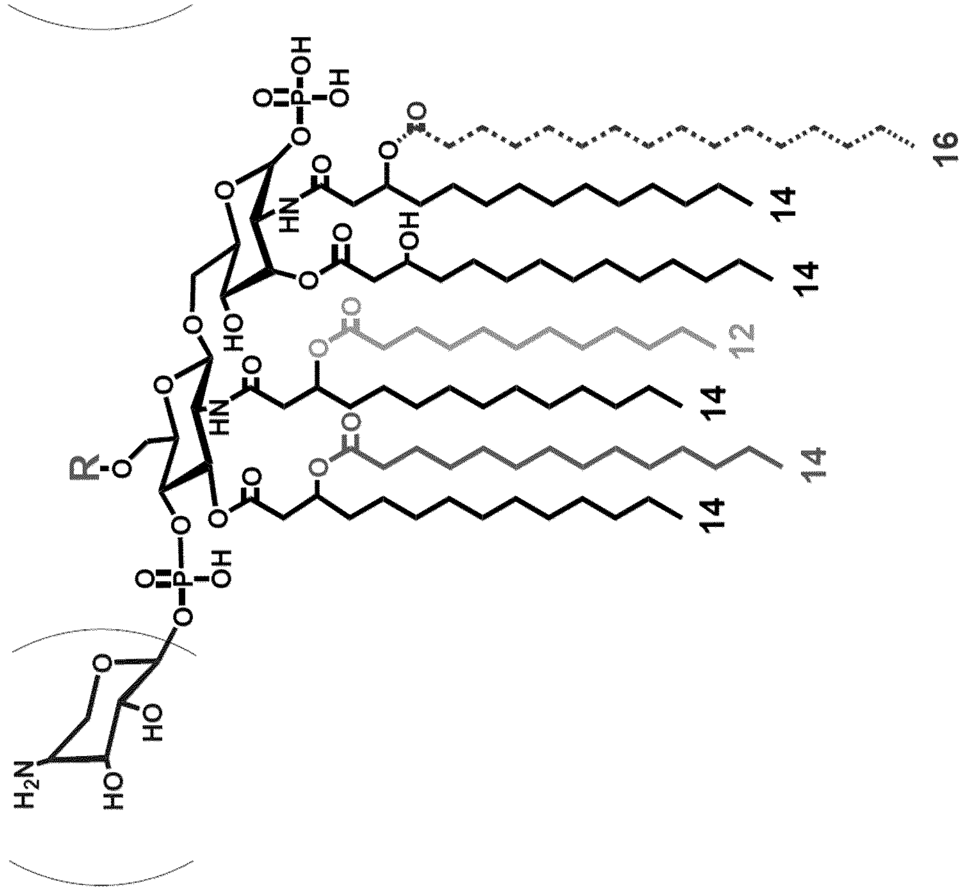

(XIII)(0)(a)

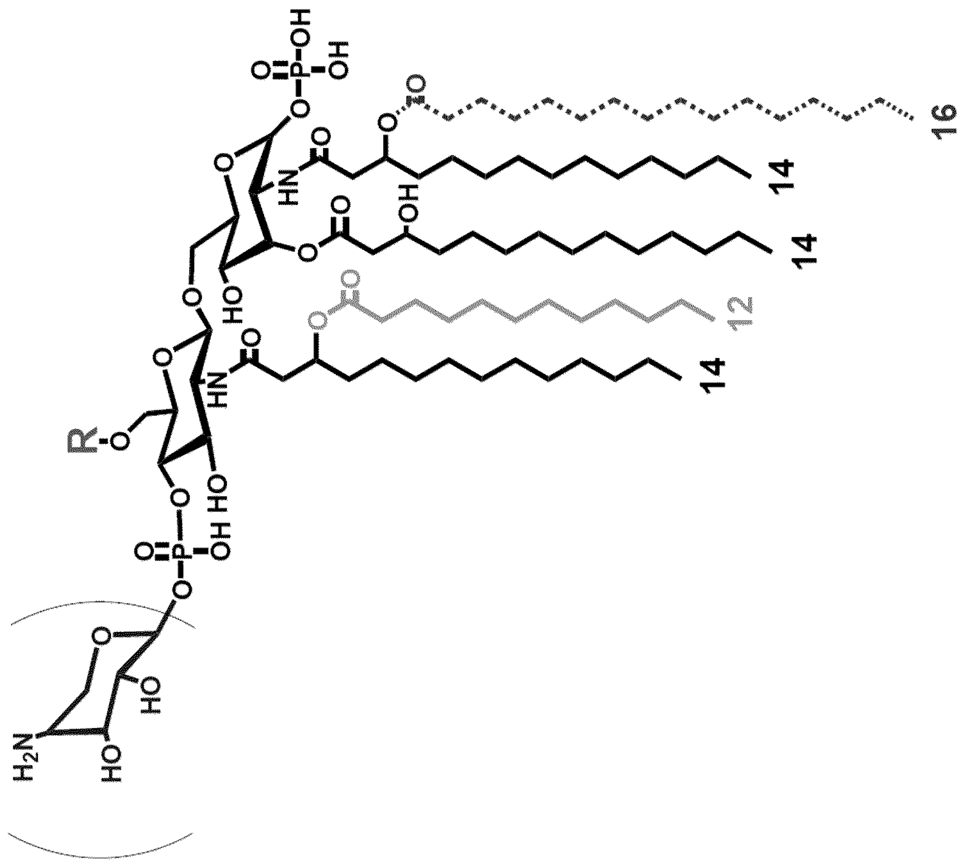

(XIII)(0)(b)

FIG. 34

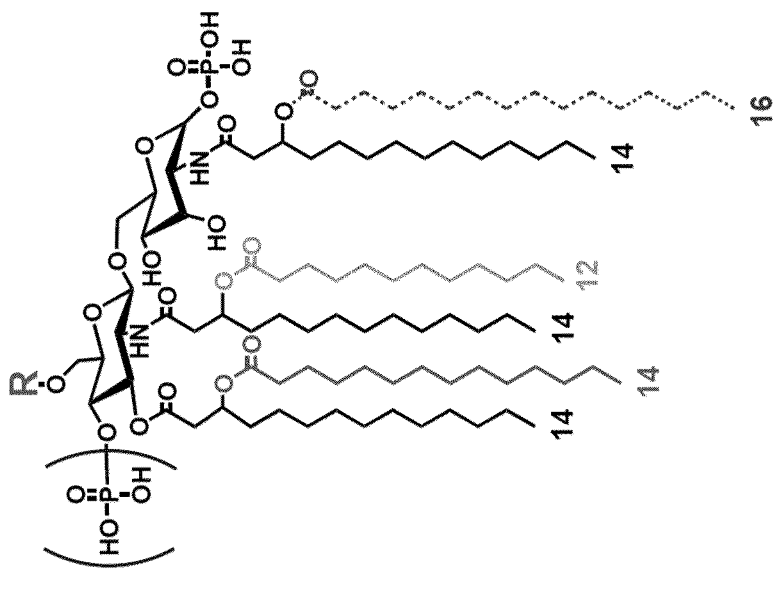

(XIII)(1)(a)

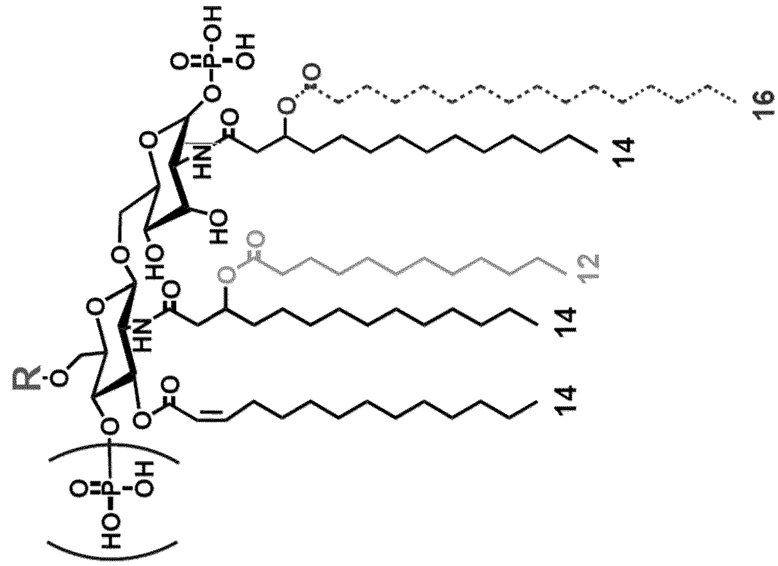

(XIII)(1)(b)

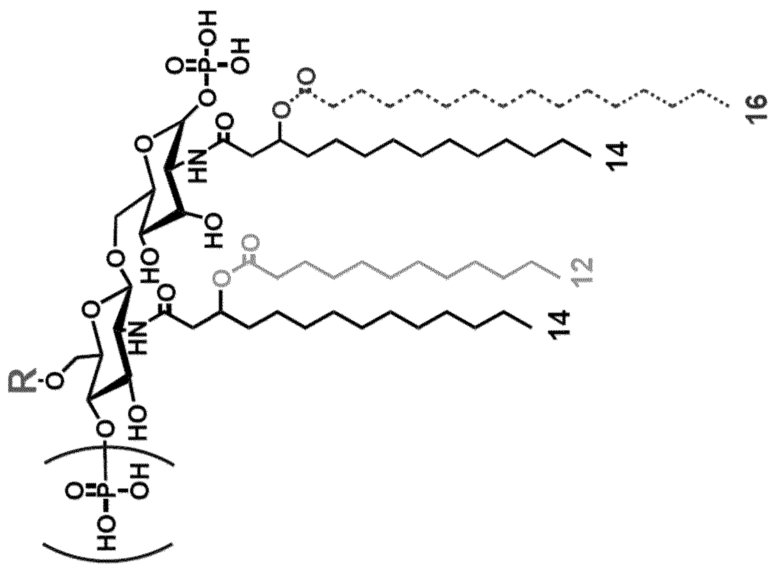

(XIII)(1)(c)

FIG. 35

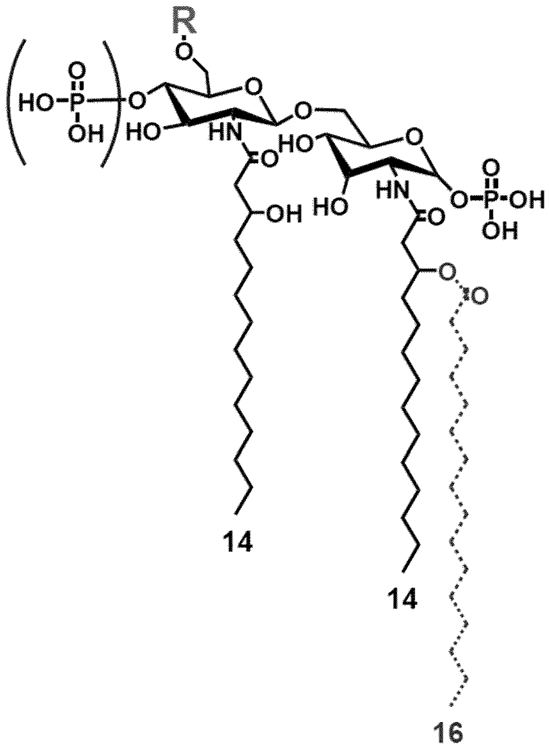

(XIII)(1)(d)

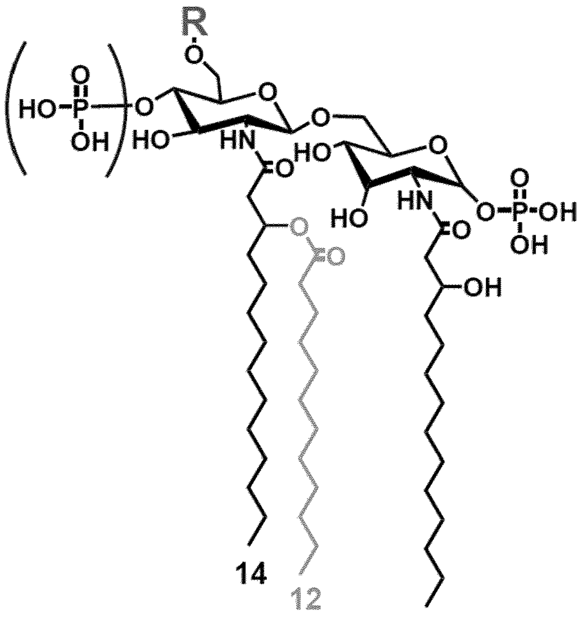

(XIII)(1)(e)

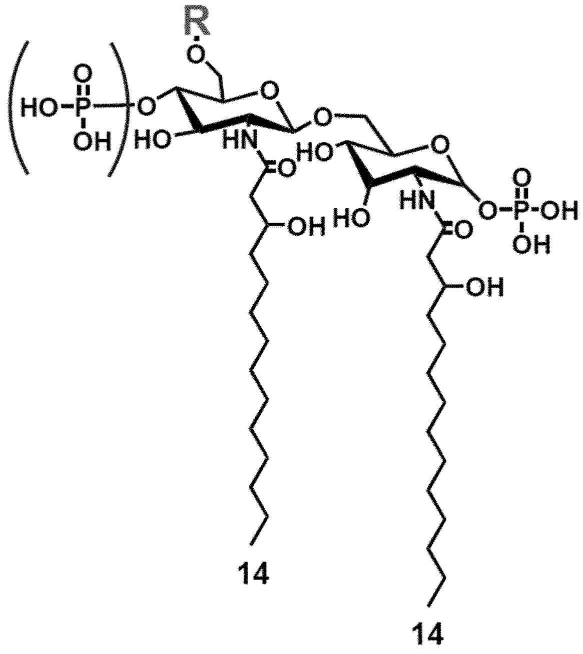

(XIII)(1)(f)

FIG. 35 (cont.)

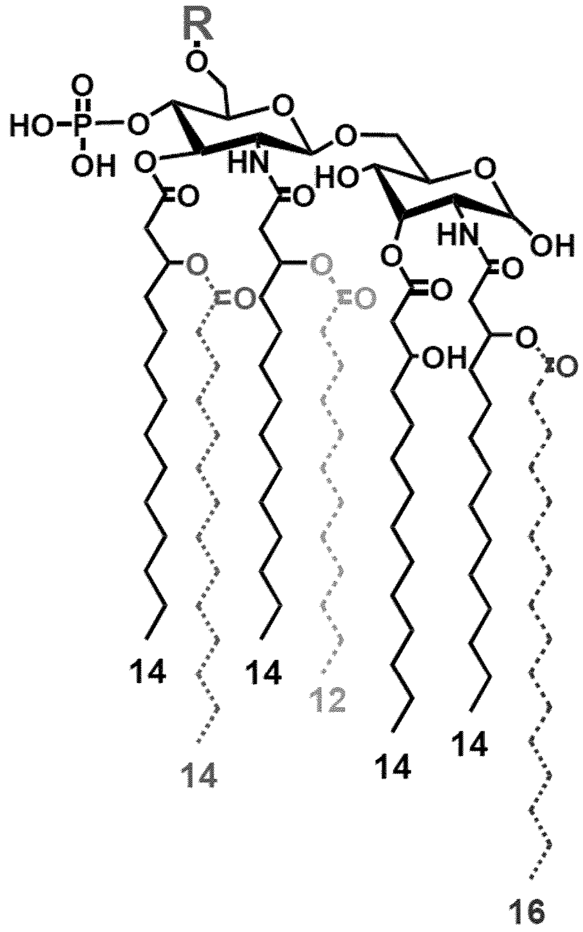

(XIII)(2)(a)

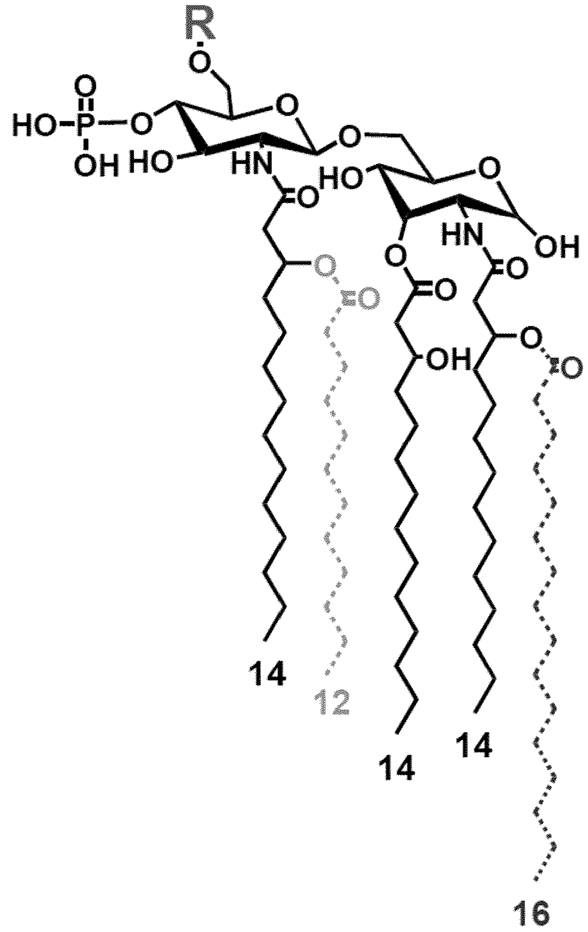

(XIII)(2)(b)

FIG. 36

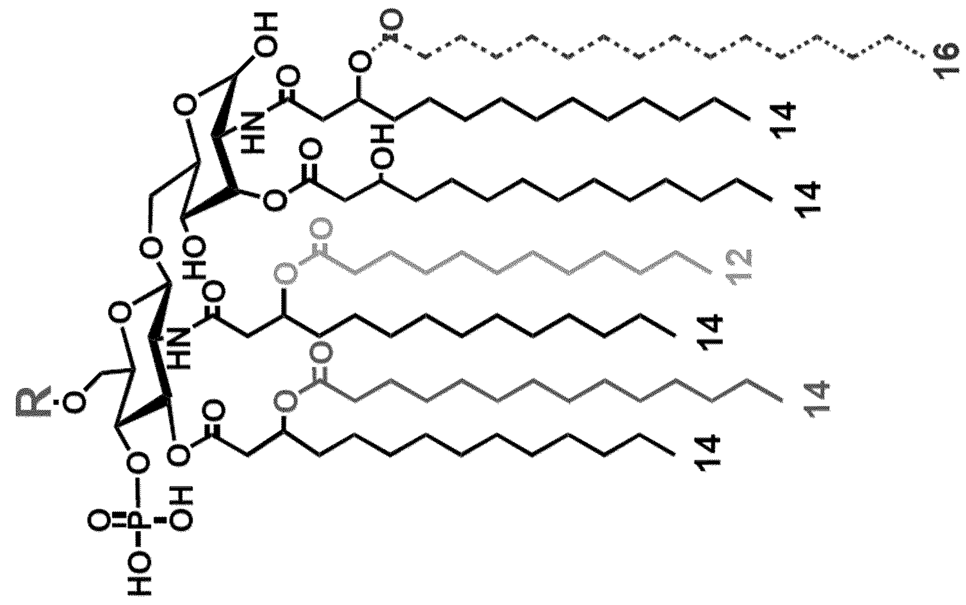

(XIII)(3)(a)

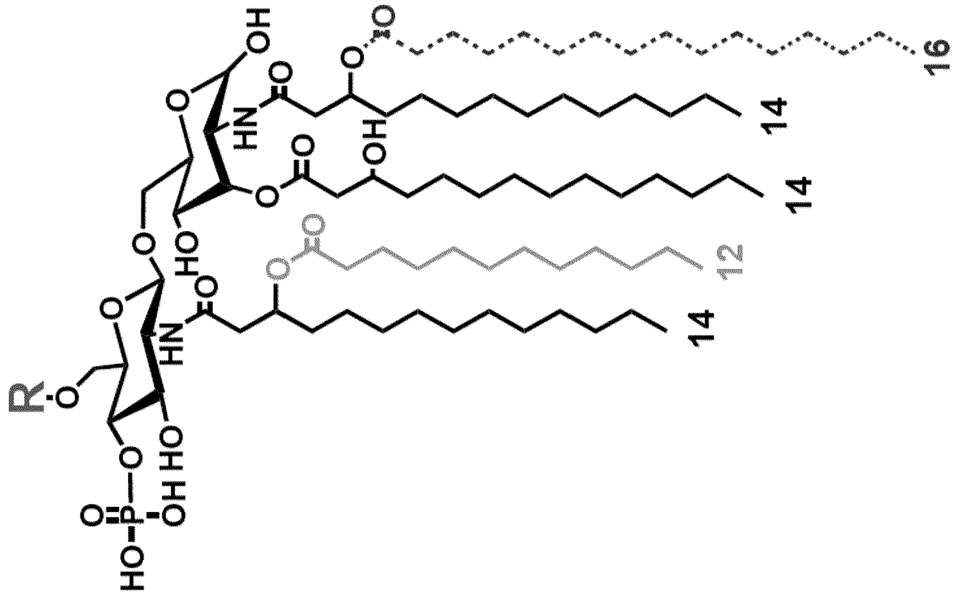

(XIII)(3)(b)

FIG. 37

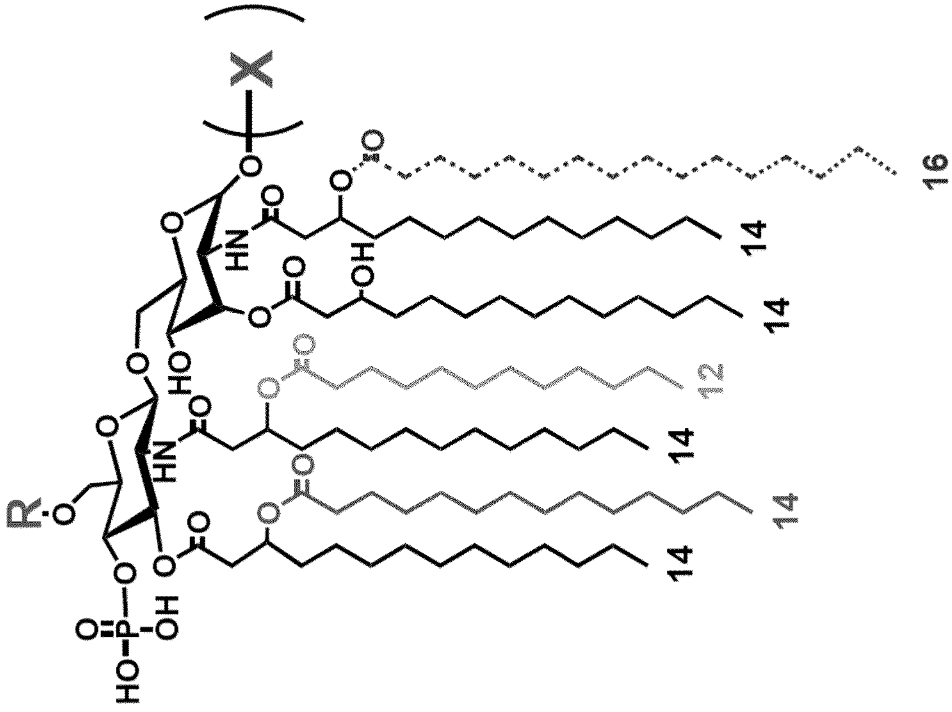

(XIII)(4)(a)

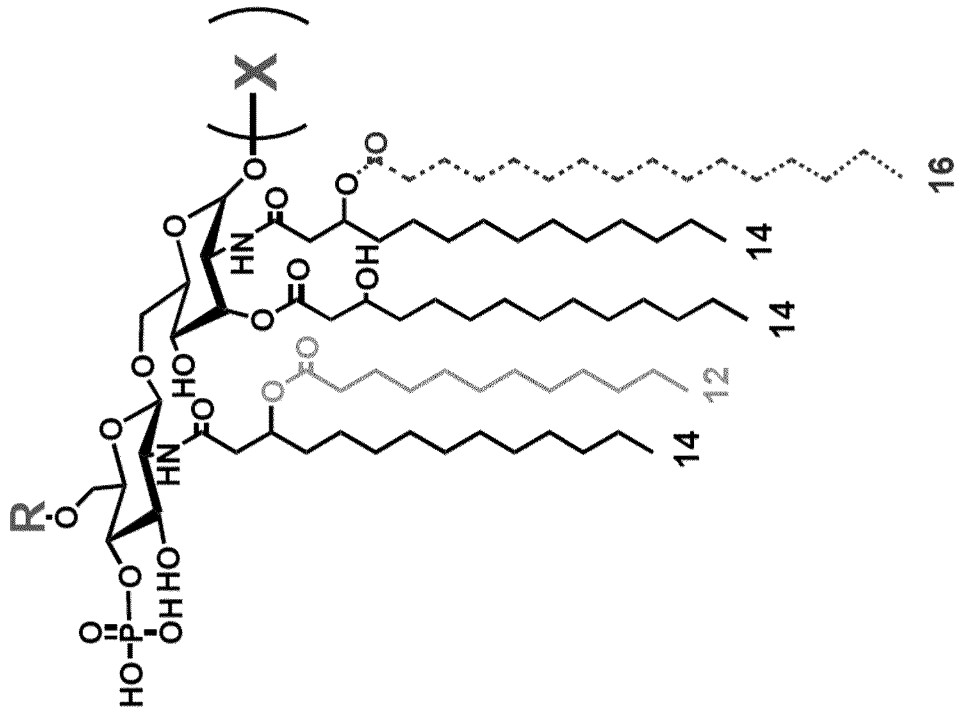

(XIII)(4)(b)

FIG. 38

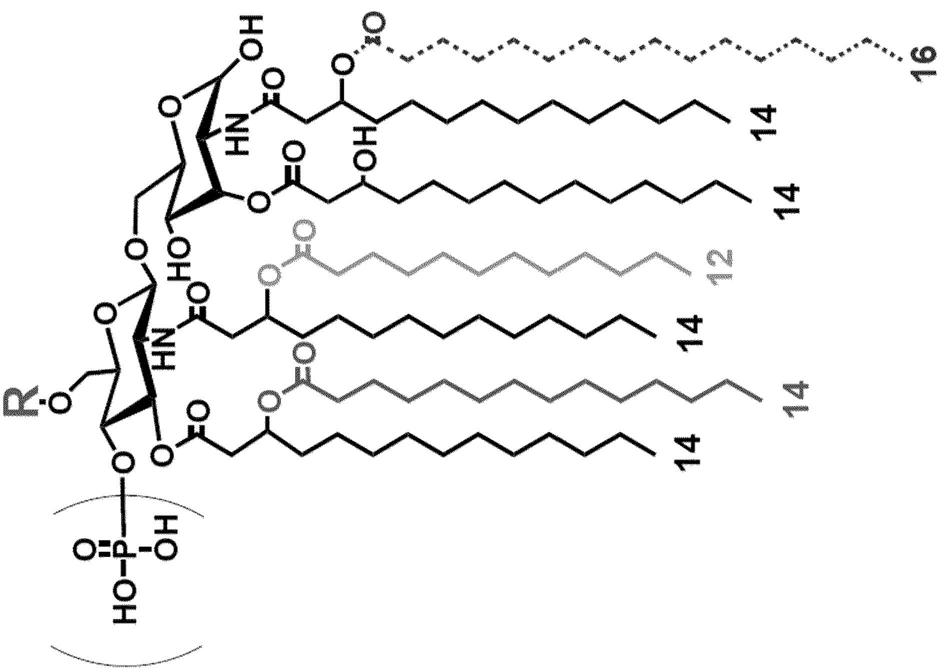

(XIII)(5)(a)

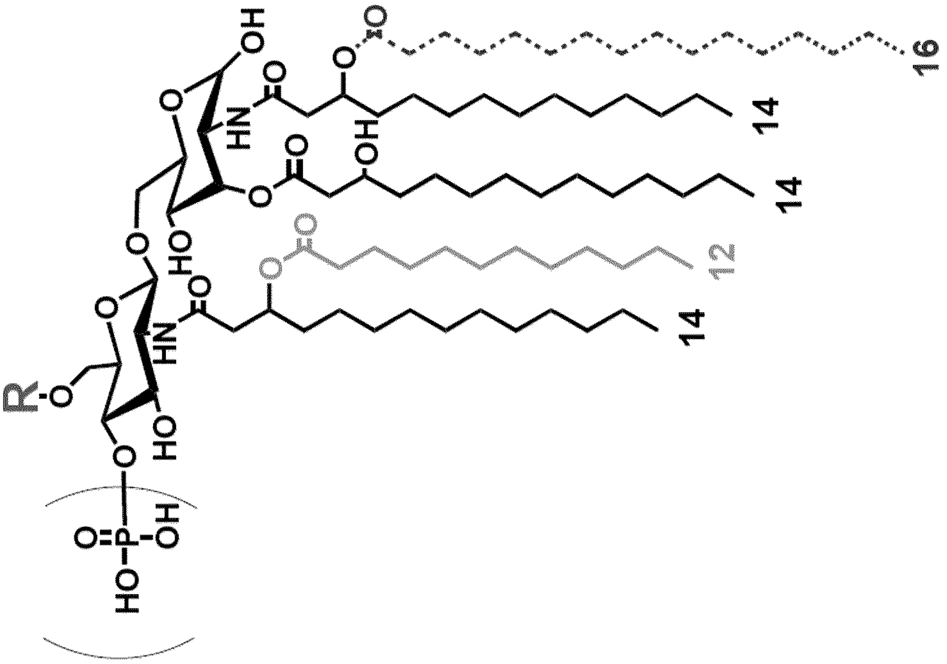

(XIII)(5)(b)

FIG. 39

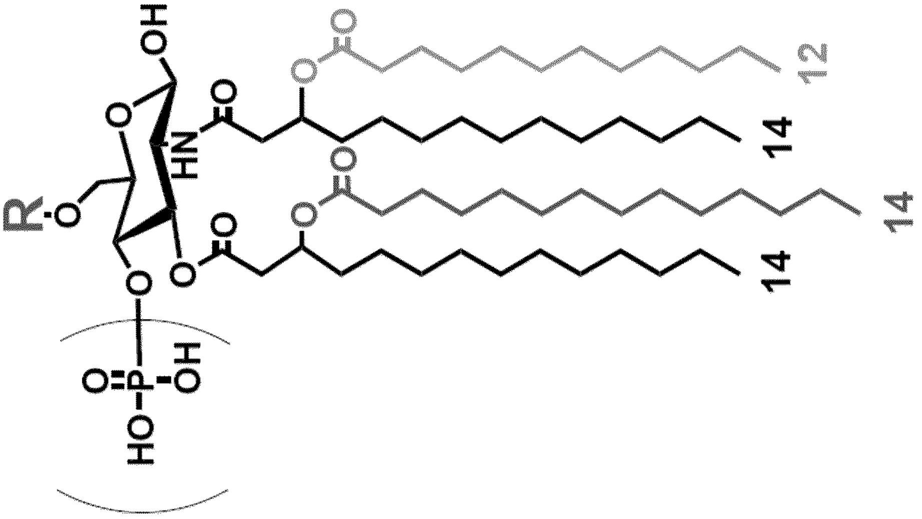

(XIII)(5)(c)

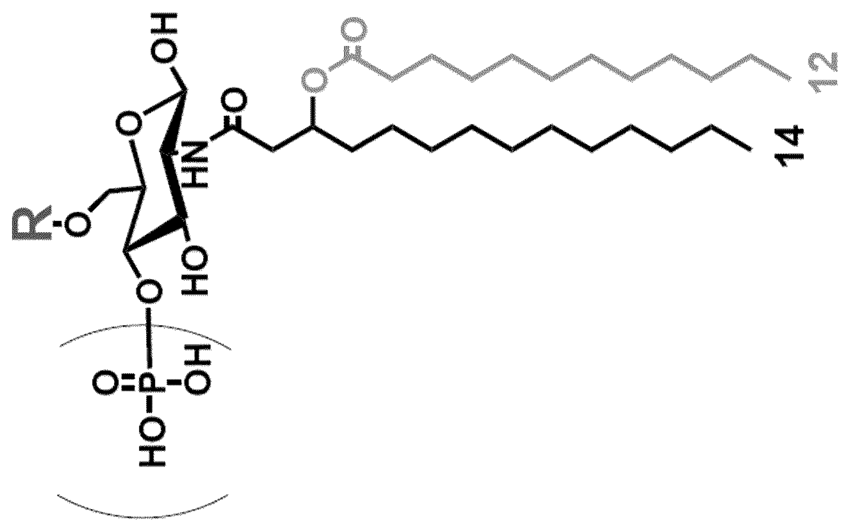

(XIII)(5)(d)

FIG. 39 (cont.)

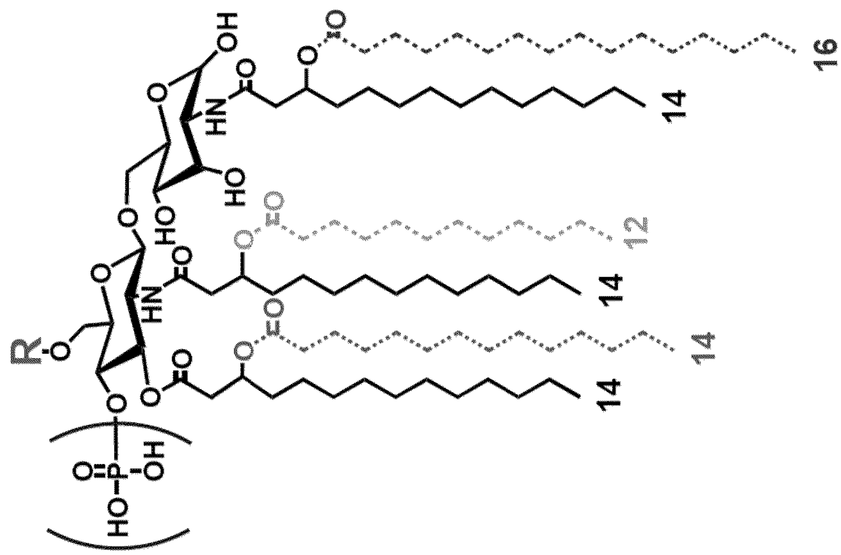

(XIII)(6)(a)

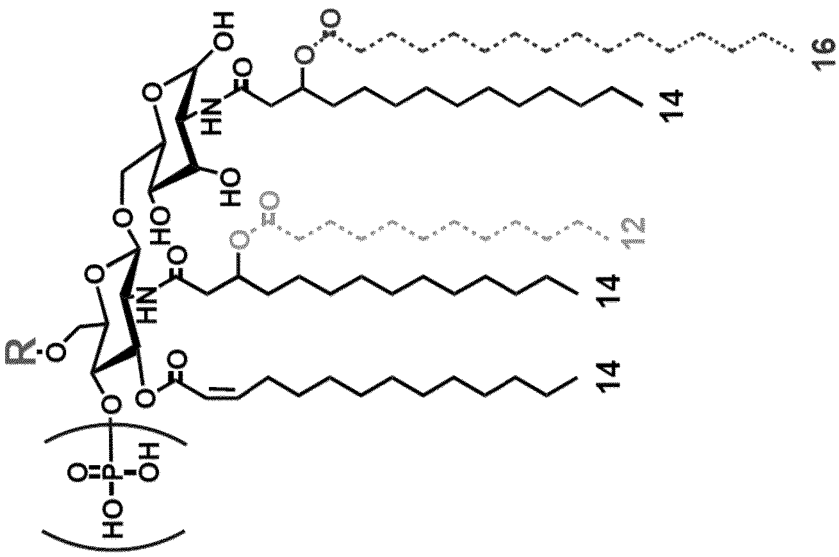

(XIII)(6)(b)

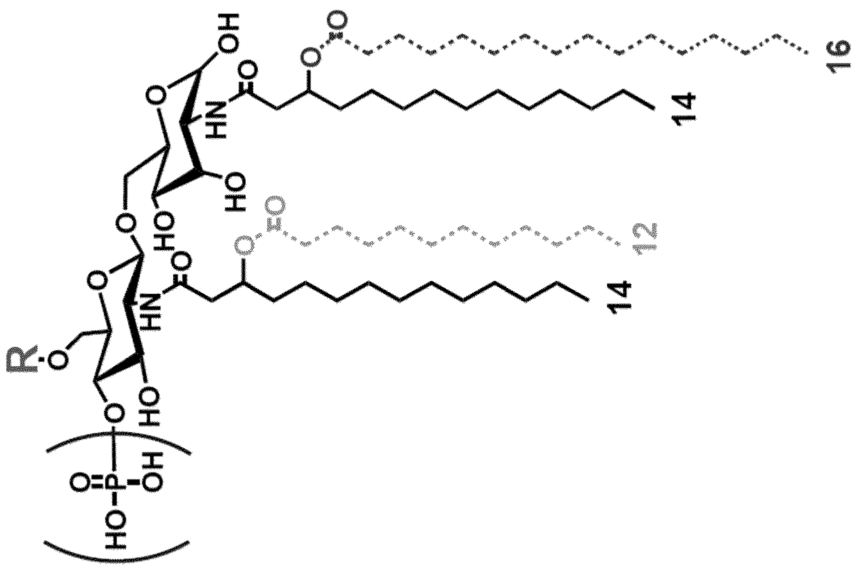

(XIII)(6)(c)

FIG. 40

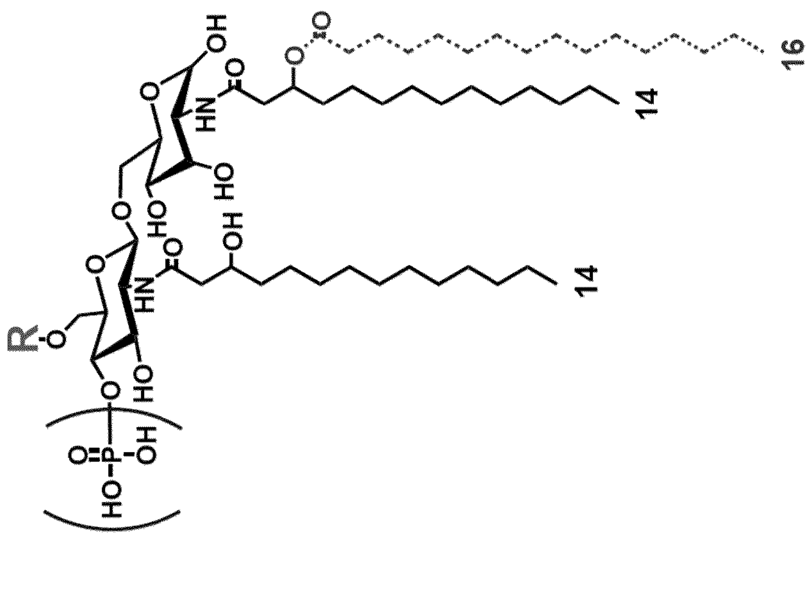

(XIII)(6)(d)

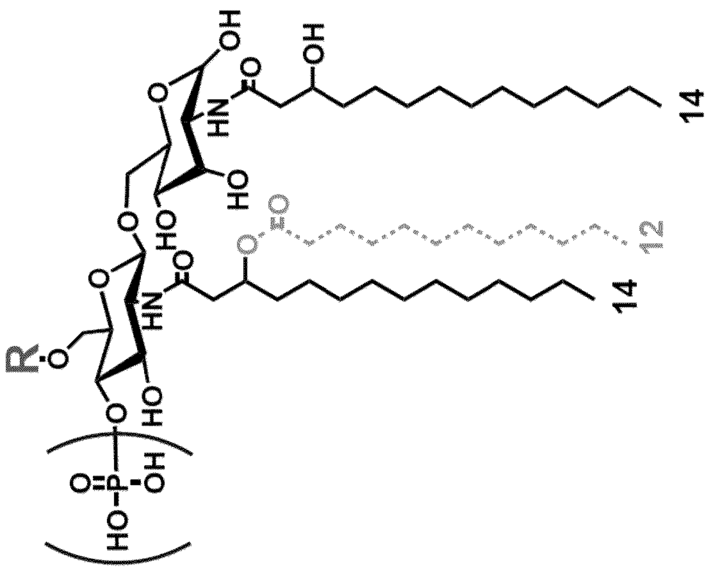

(XIII)(6)(e)

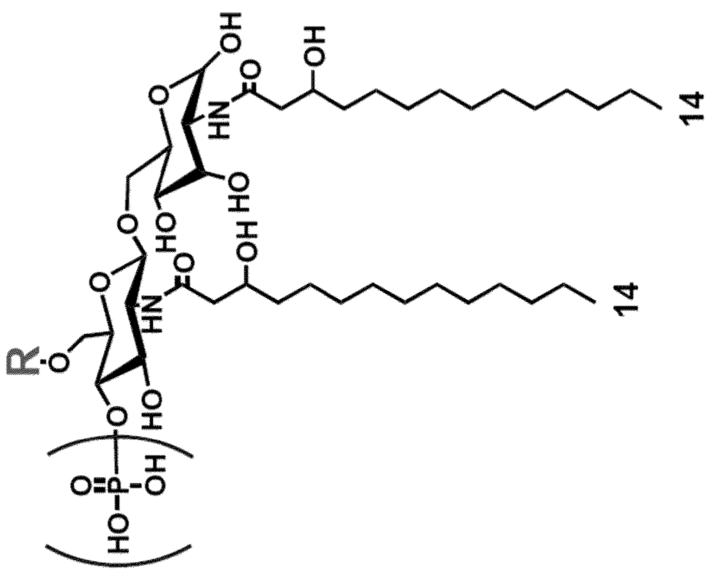

(XIII)(6)(f)

FIG. 40 (cont.)

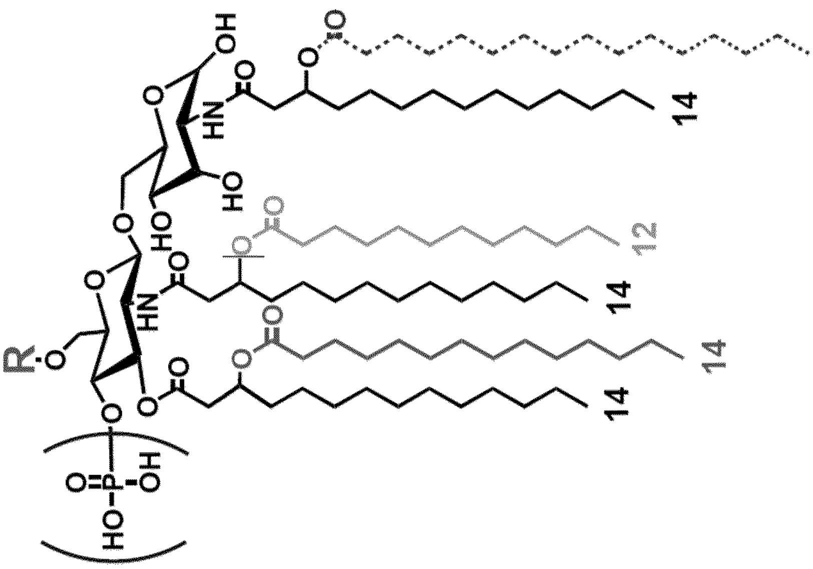

(XIII)(7)(a)

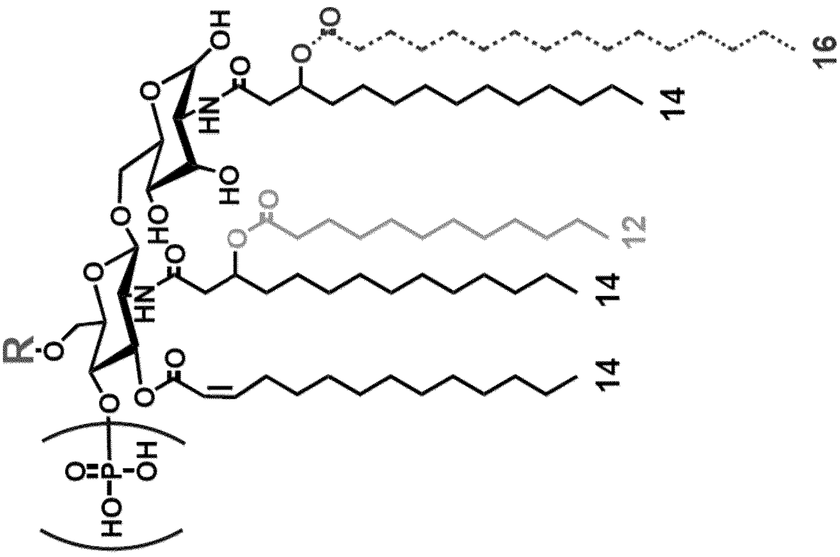

(XIII)(7)(b)

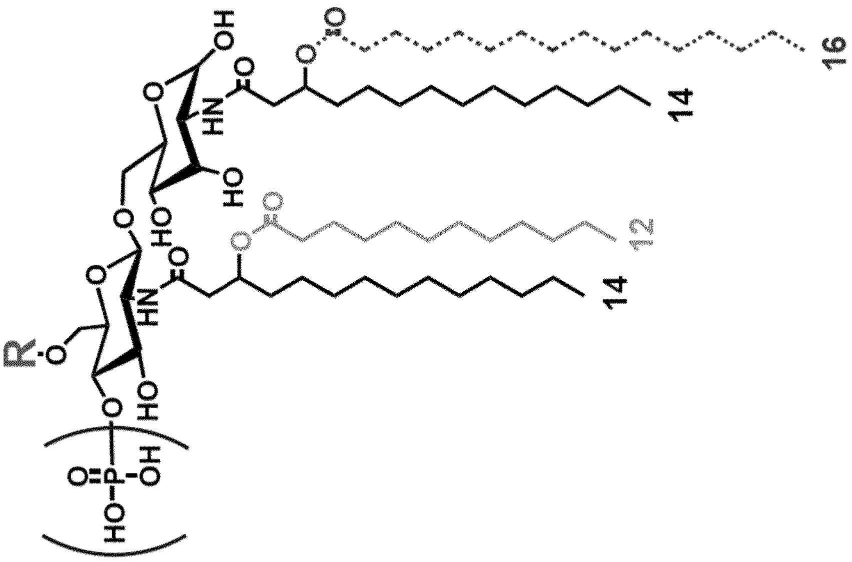

(XIII)(7)(c)

FIG. 41

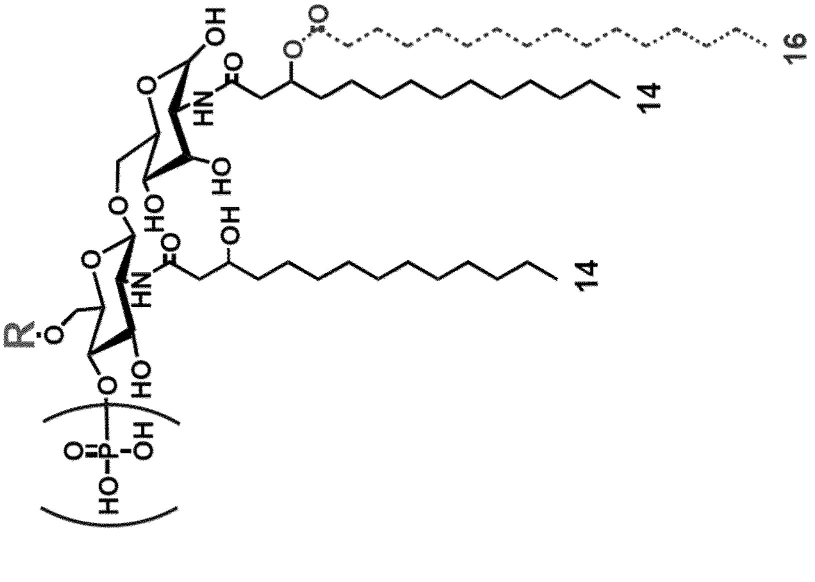

(XIII)(7)(d)

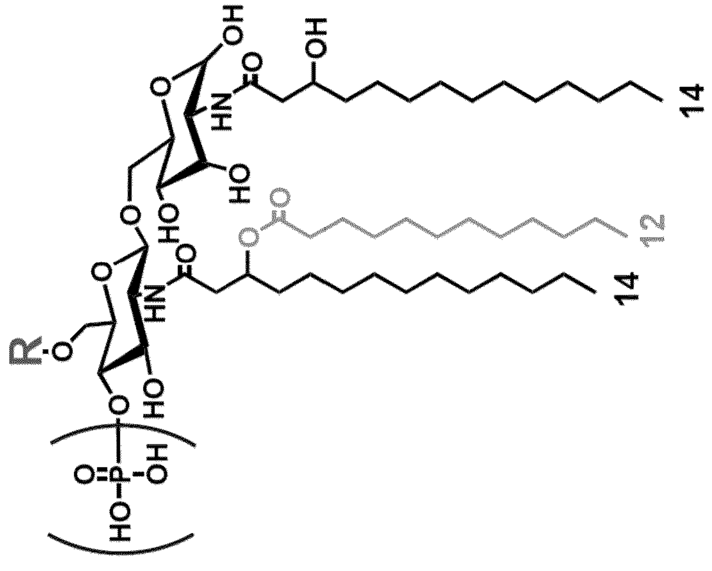

(XIII)(7)(e)

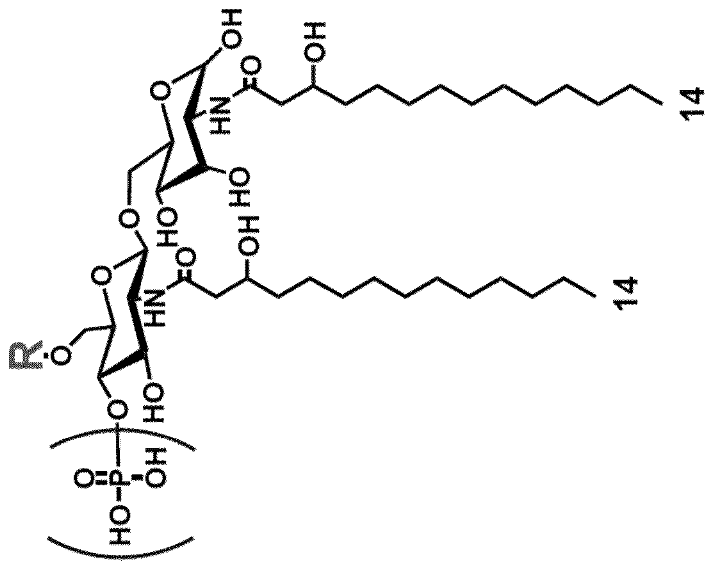

(XIII)(7)(f)

FIG. 41 (cont.)

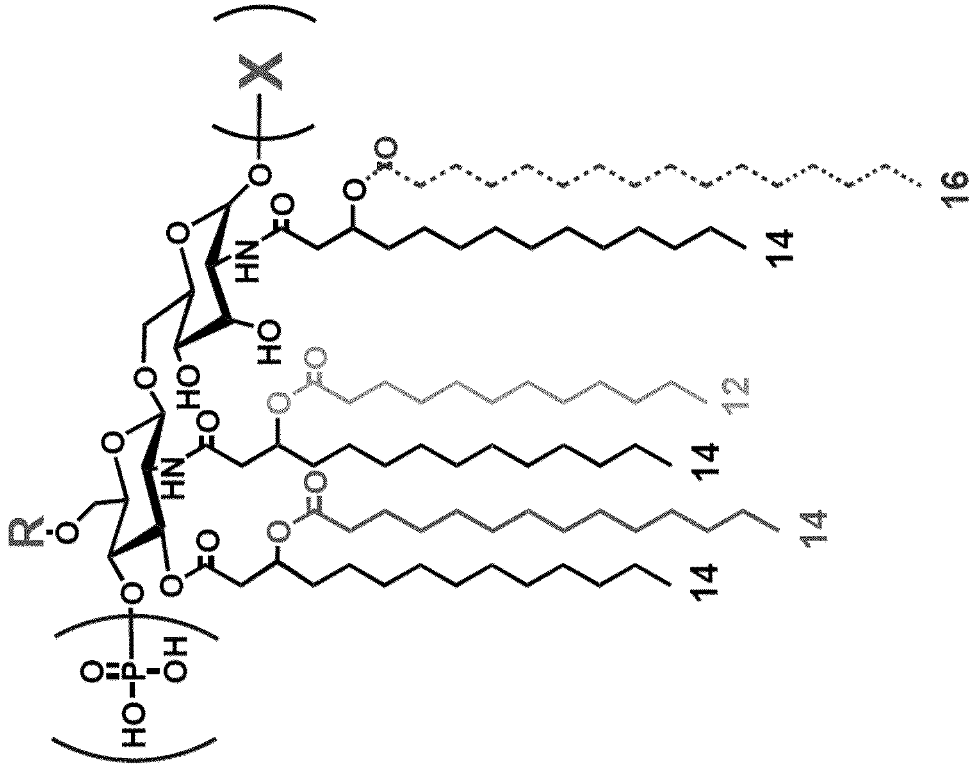

(XIII)(8)(a)

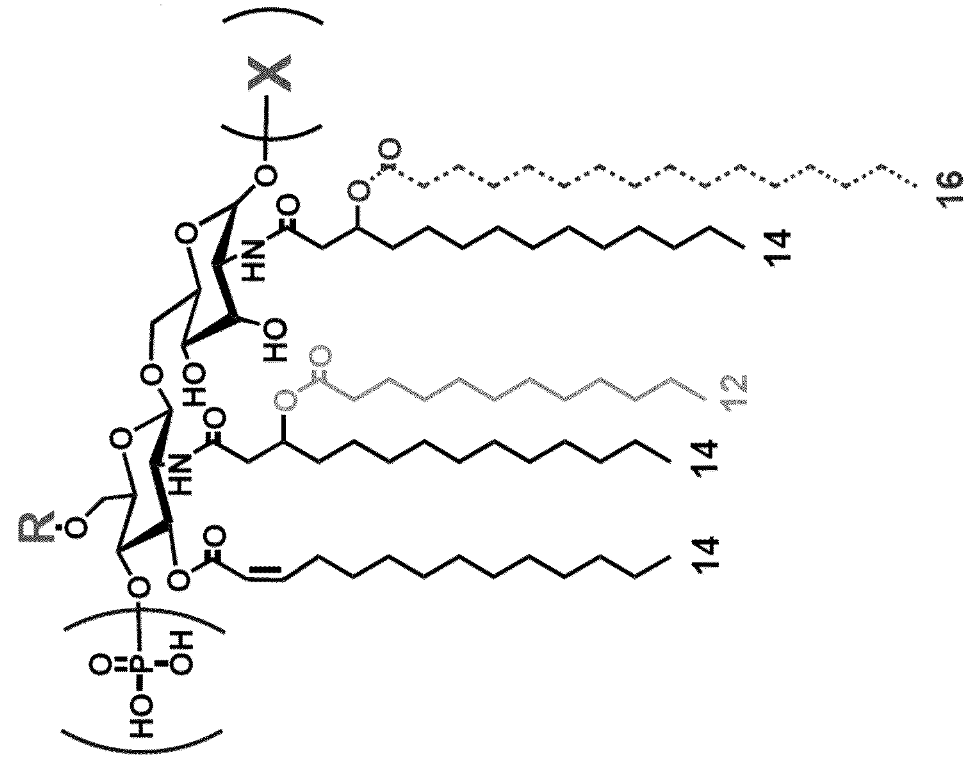

(XIII)(8)(b)

FIG. 42

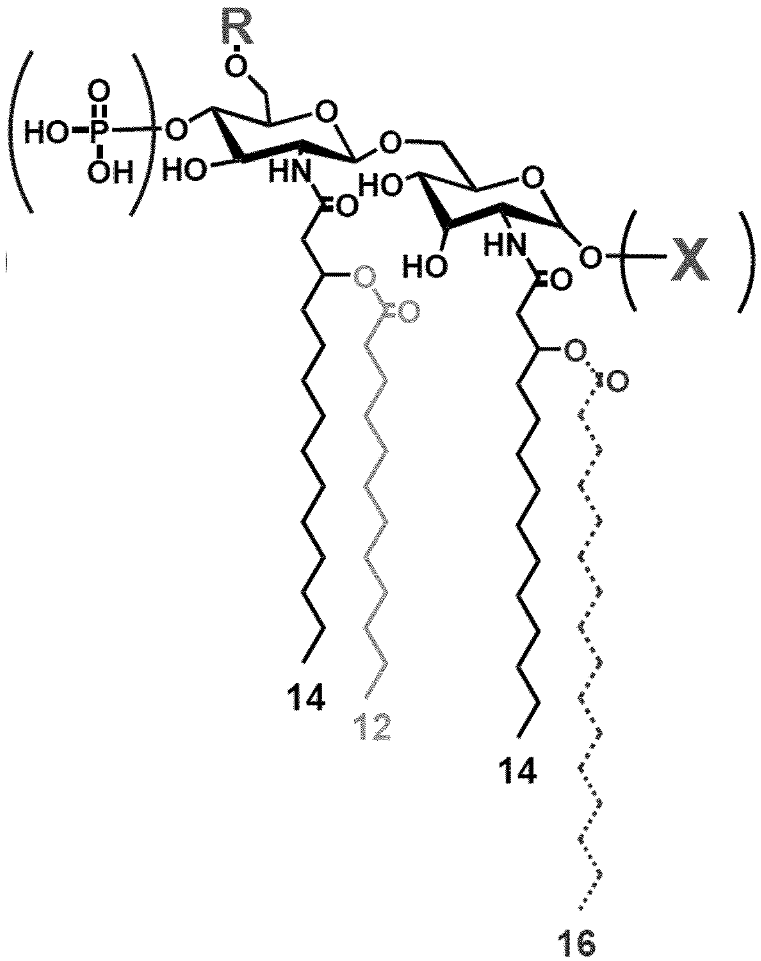

(XIII)(8)(c)

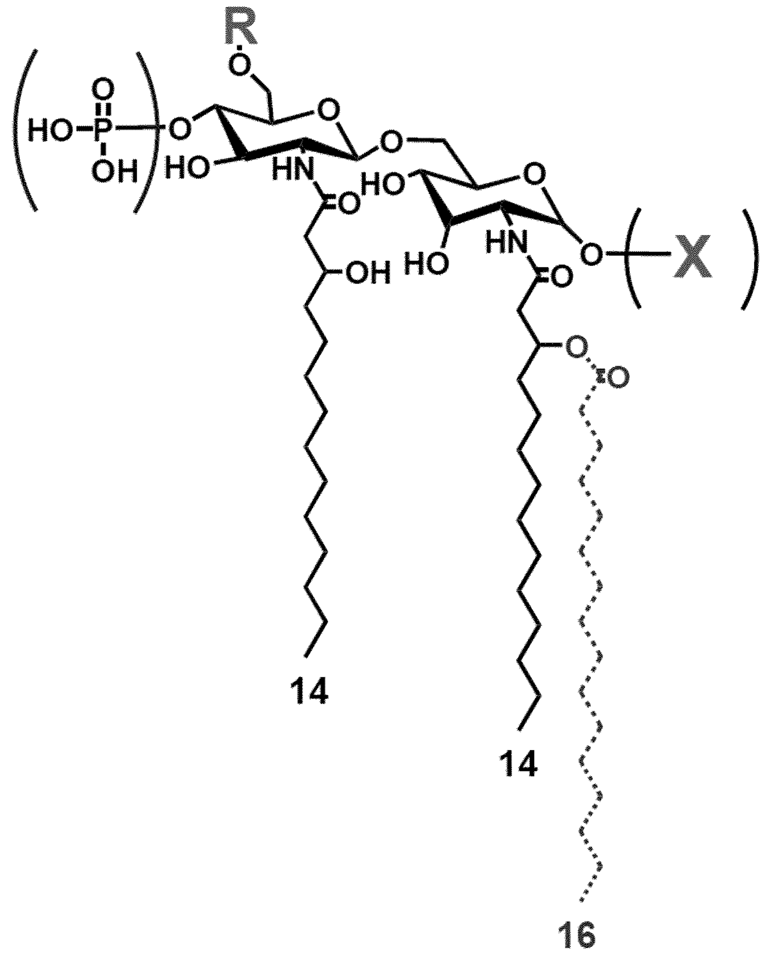

(XIII)(8)(d)

FIG. 42 (cont.)

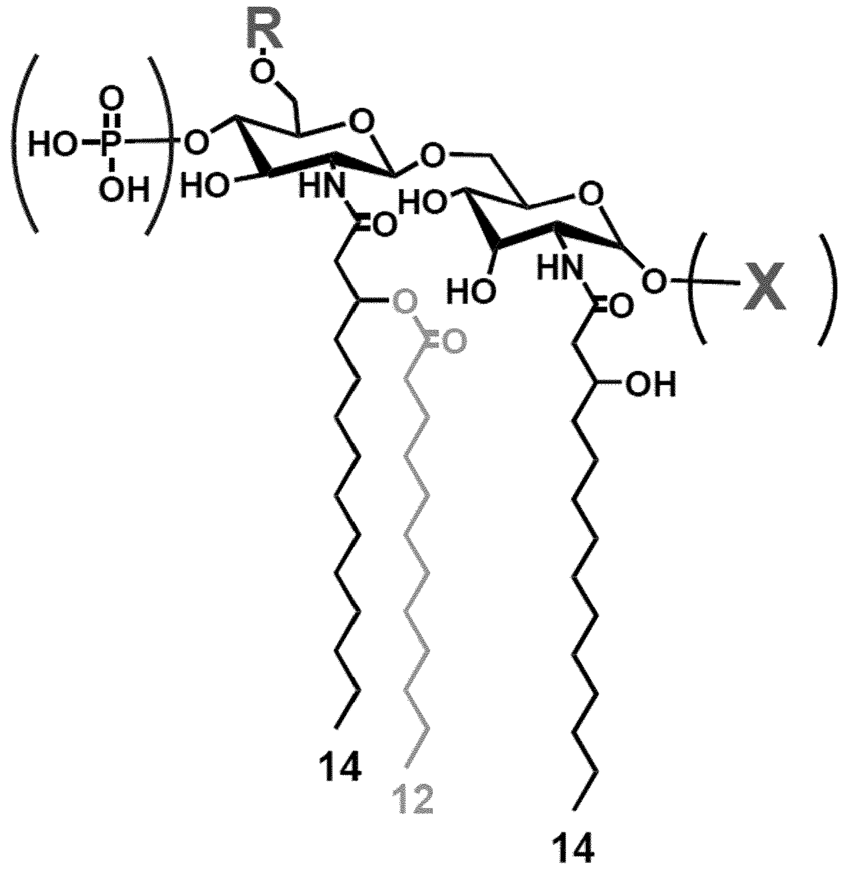

(XIII)(8)(e)

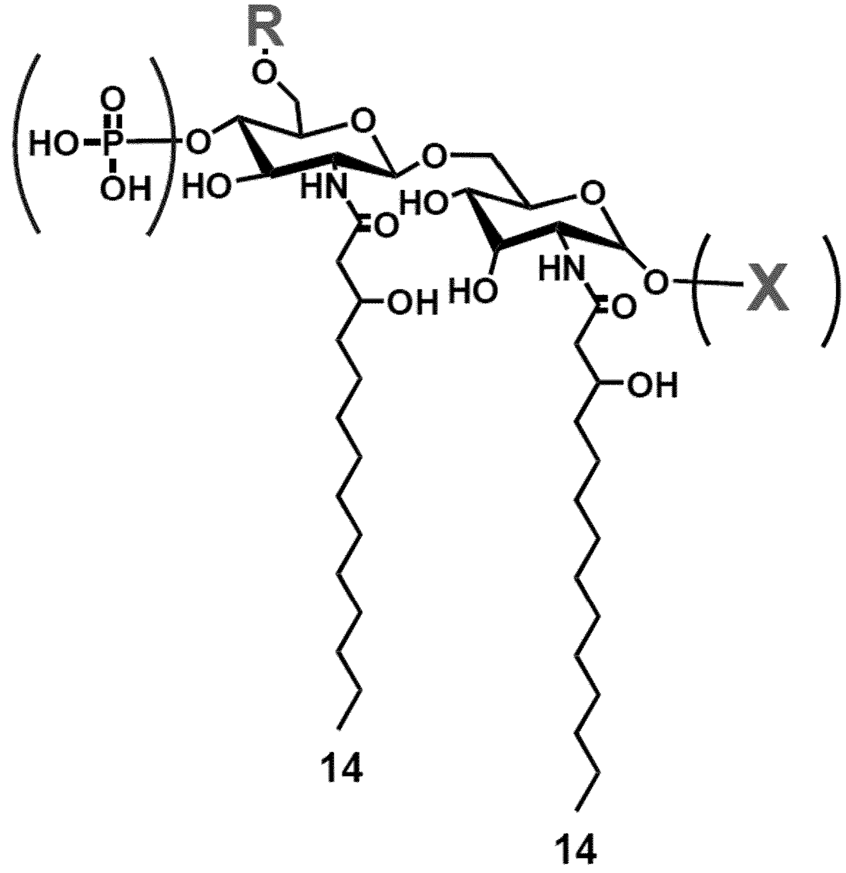

(XIII)(8)(f)

FIG. 42 (cont.)

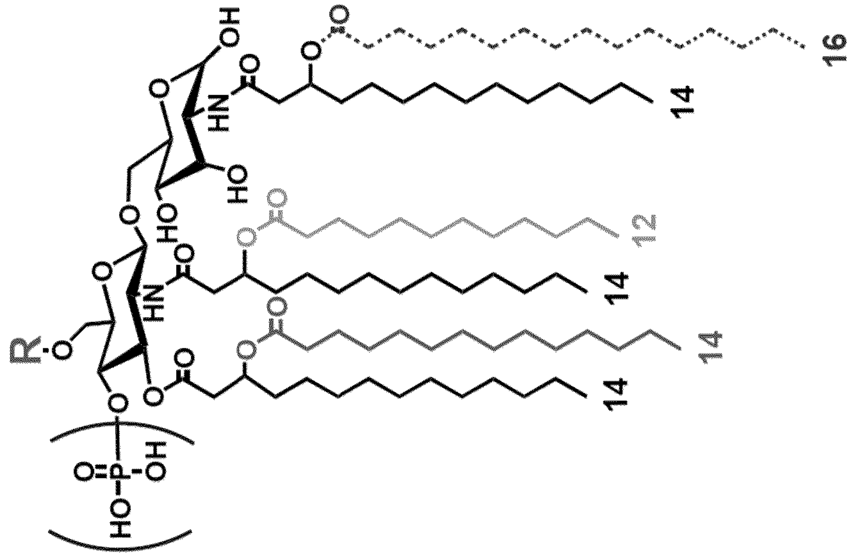

(XIII)(9)(a)

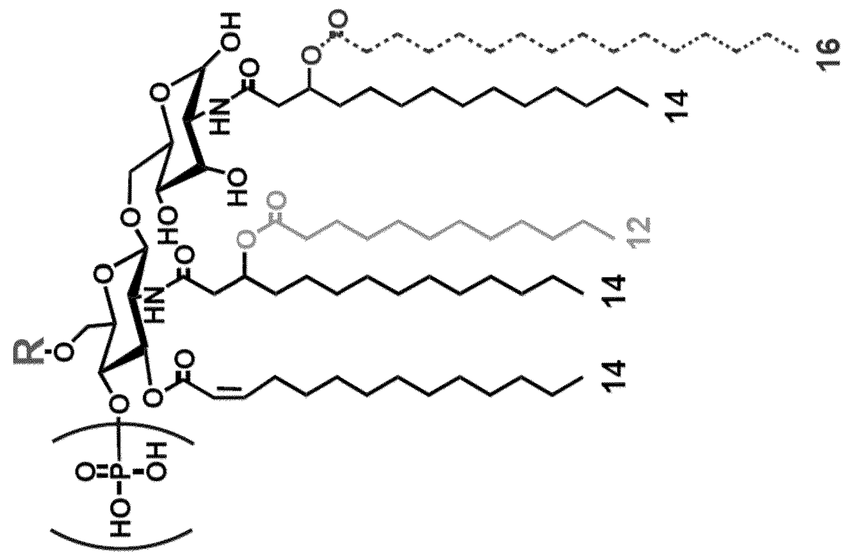

(XIII)(9)(b)

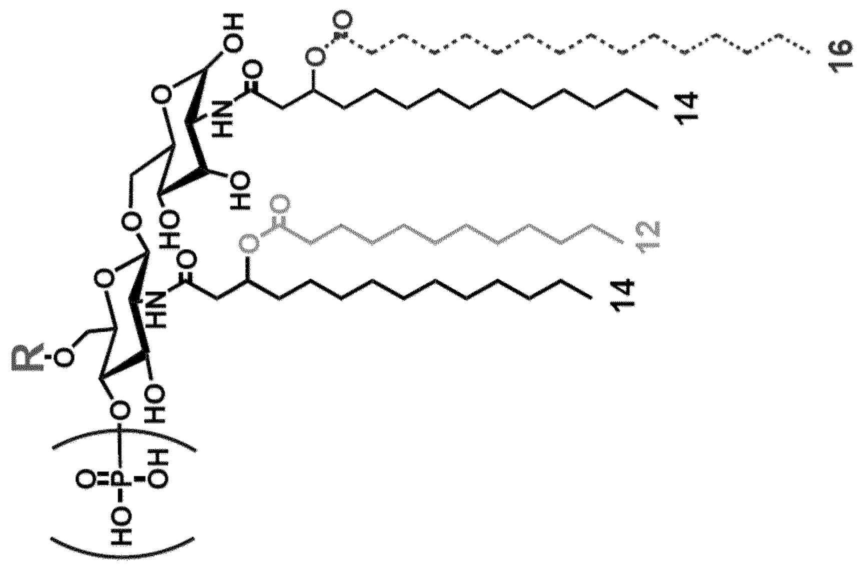

(XIII)(9)(c)

FIG. 43

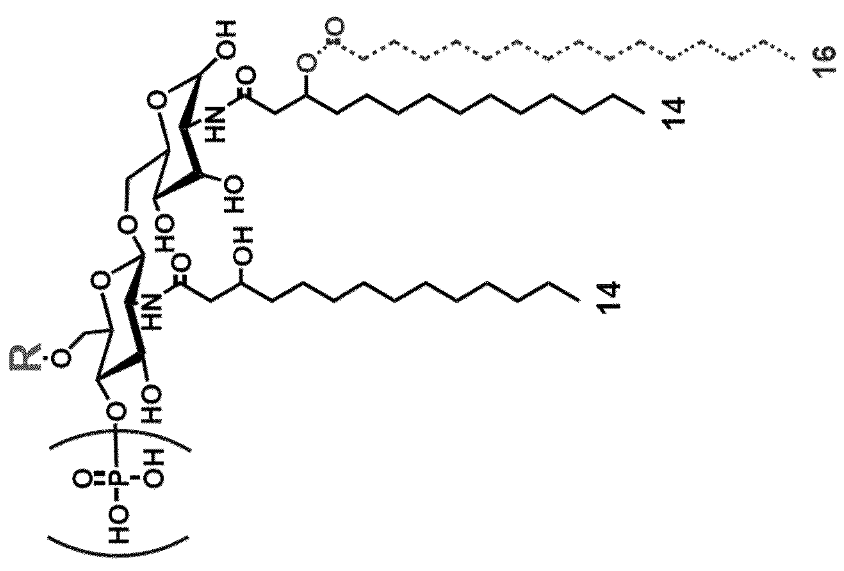

(XIII)(9)(d)

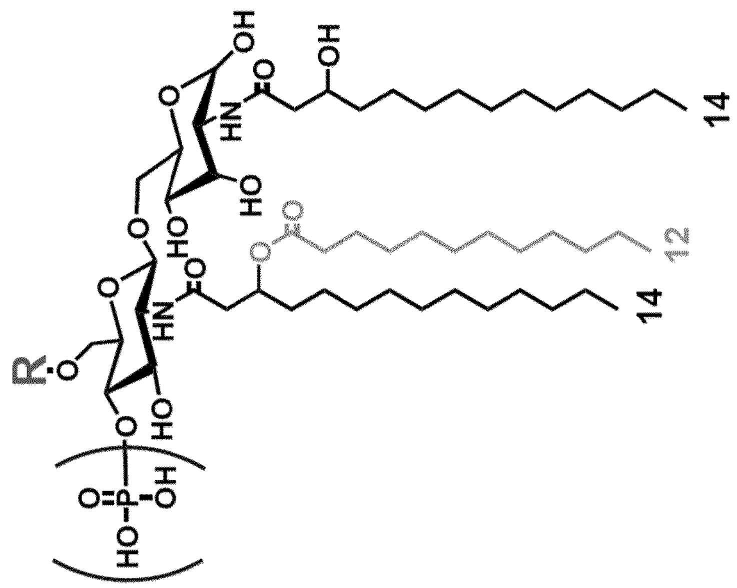

(XIII)(9)(e)

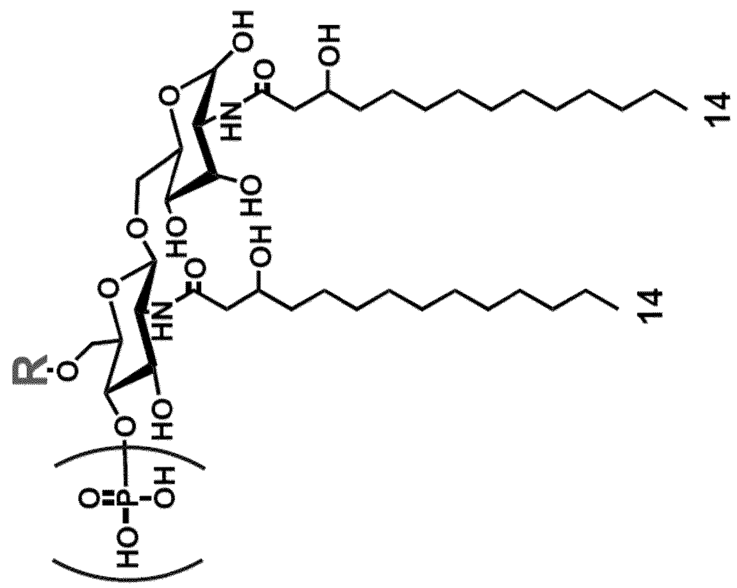

(XIII)(9)(f)

FIG. 43 (cont.)

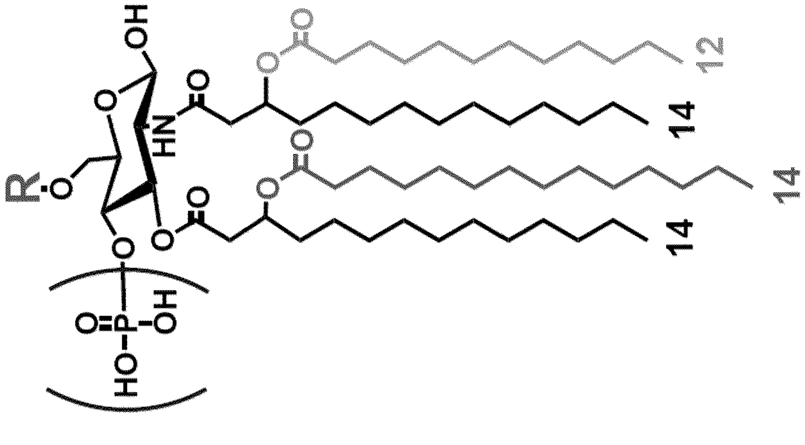

(XIII)(9)(g)

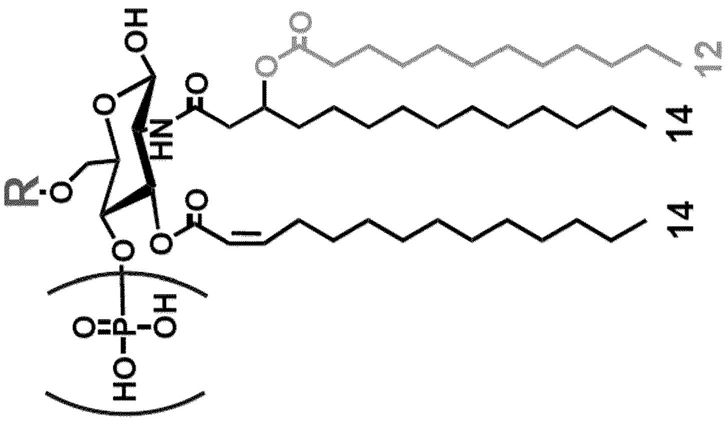

(XIII)(9)(h)

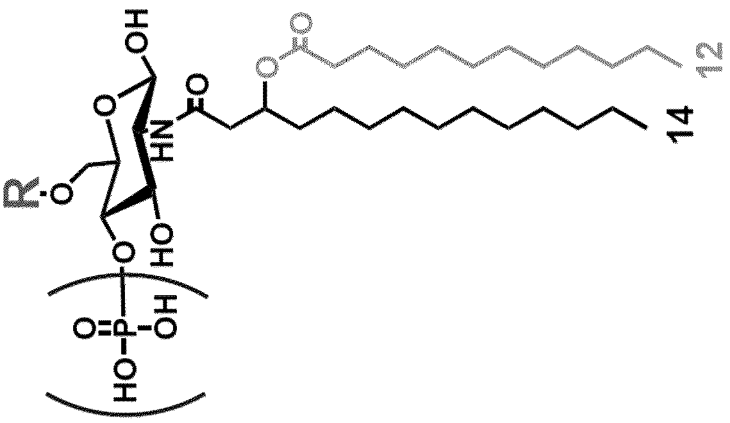

(XIII)(9)(i)

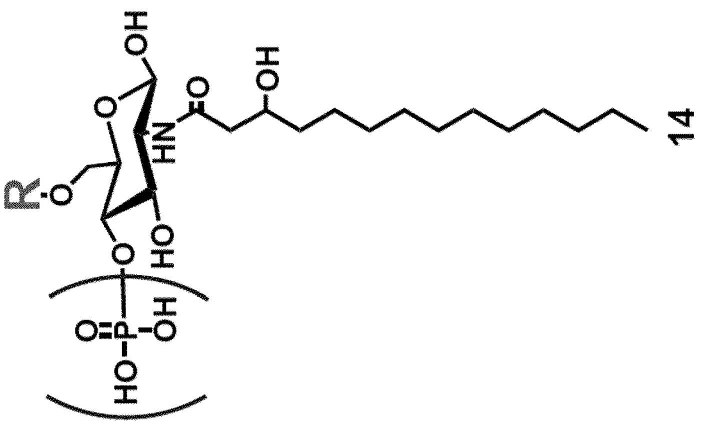

(XIII)(9)(j)

FIG. 43 (cont.)

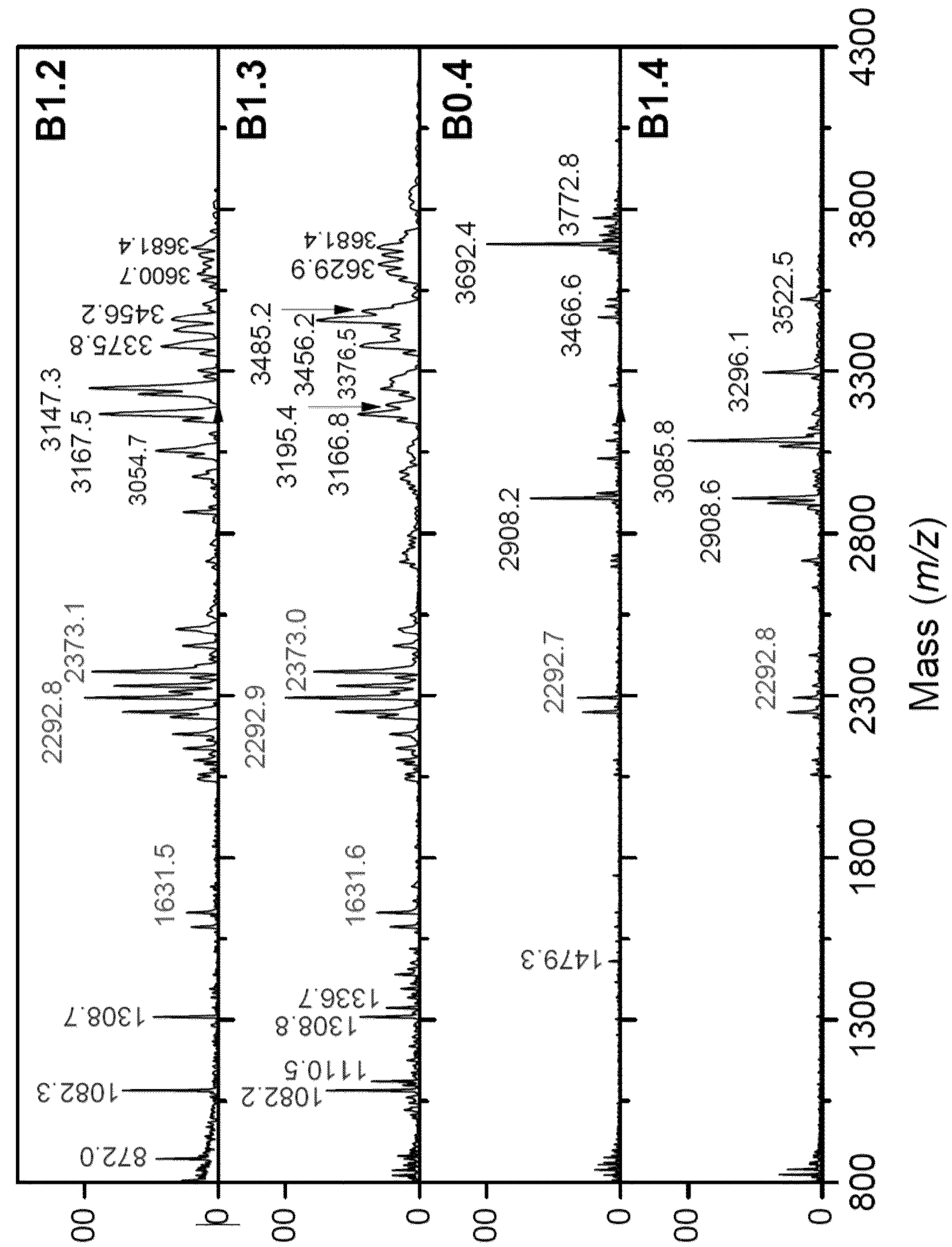
B1.2
B1.3
B0.4
B1.4
Mass (m/z)
800
1300
1800
2300
2800
3300
3800
4300
100
0
872.0
1082.3
1308.7
1631.5
2292.8
2373.1
3054.7
3167.5
3147.3
3375.8
3456.2
3600.7
3681.4
1082.2
1110.5
1308.8
1336.7
1631.6
2292.9
2373.0
3195.4
3166.8
3485.2
3456.2
3376.5
3629.9
3681.4
1479.3
2292.7
2908.2
3466.6
3692.4
3772.8
2292.8
2908.6
3085.8
3296.1
3522.5

FIG. 44

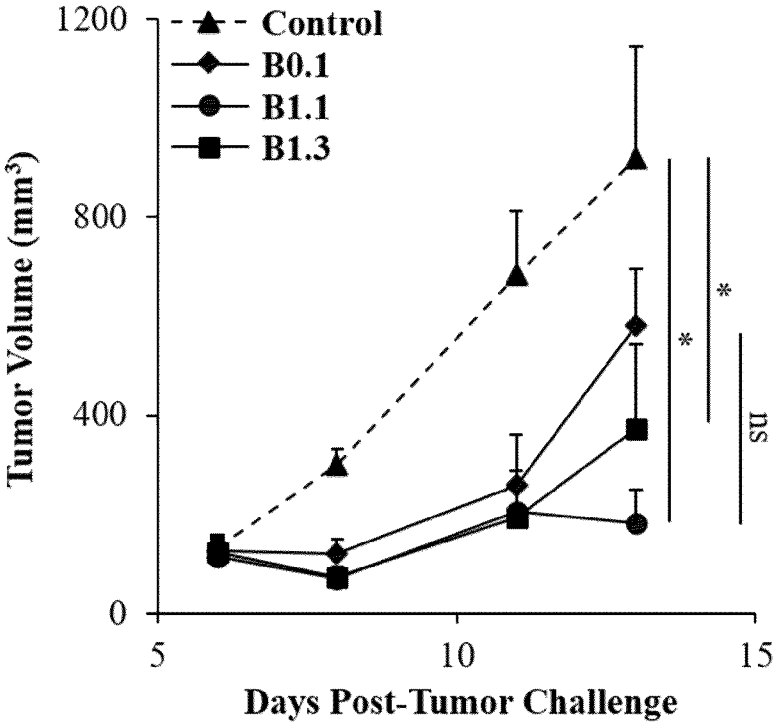


FIG. 45A

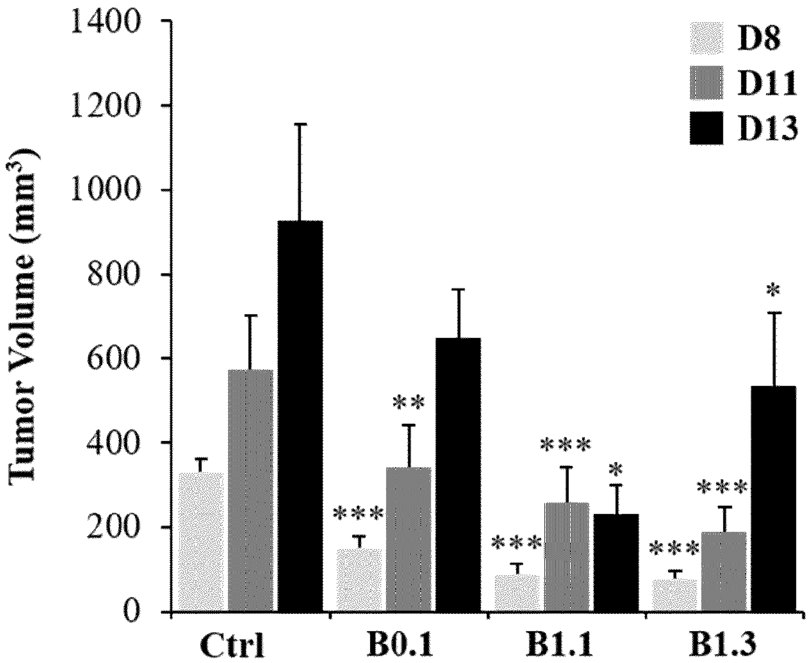


FIG. 45B

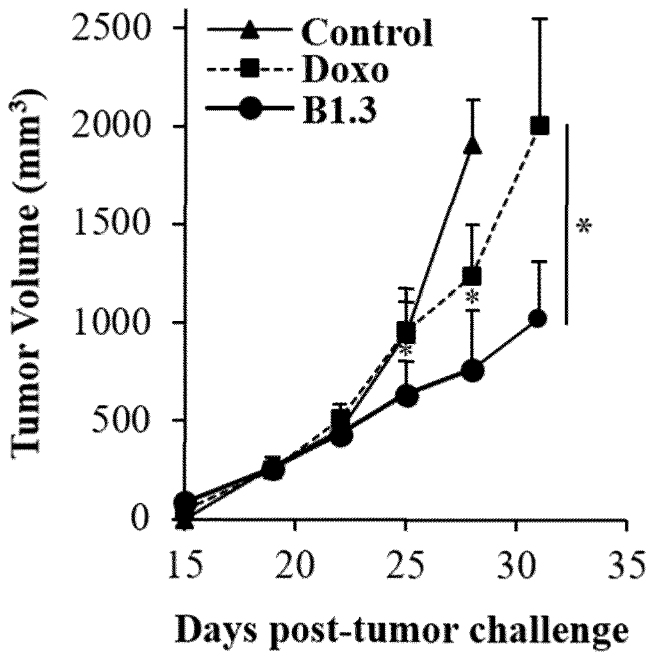


FIG. 46A

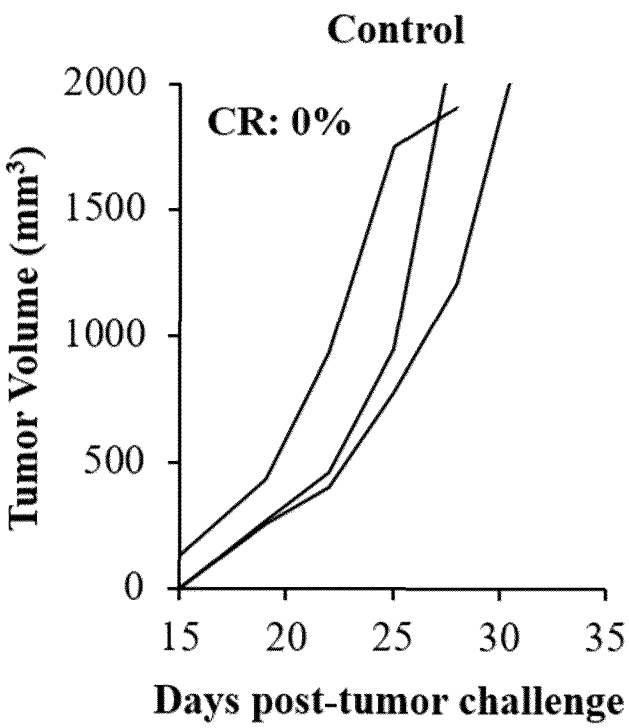


FIG. 46B

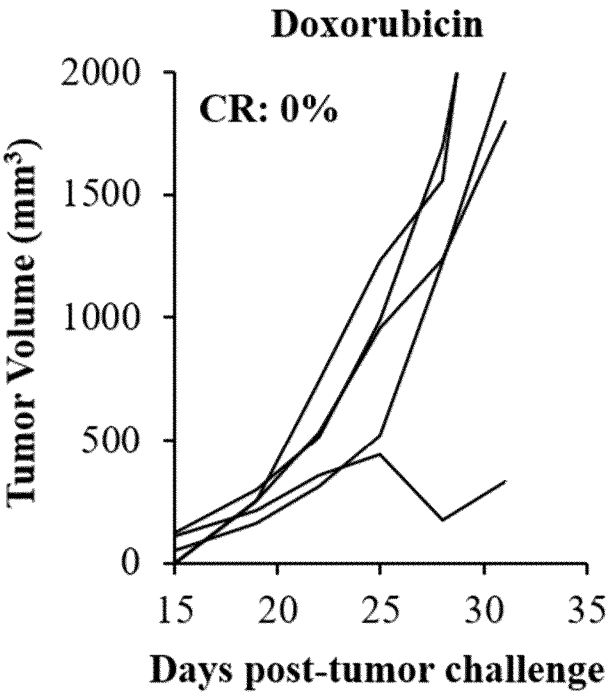


FIG. 46C

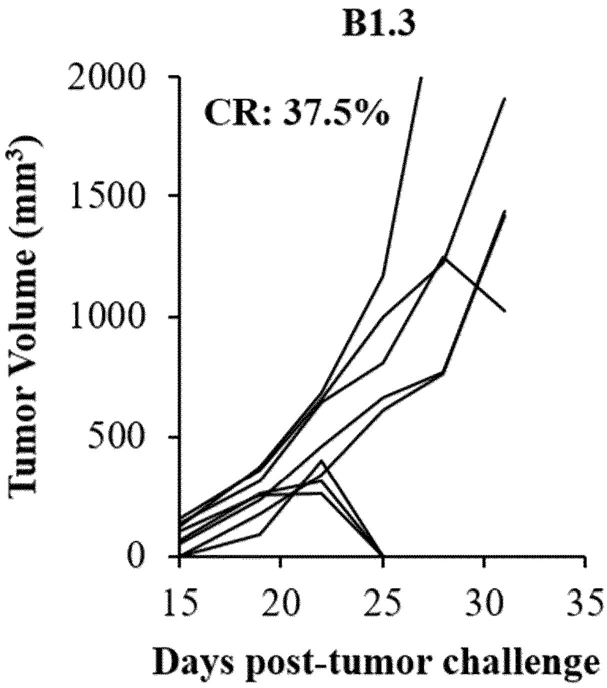


FIG. 46D

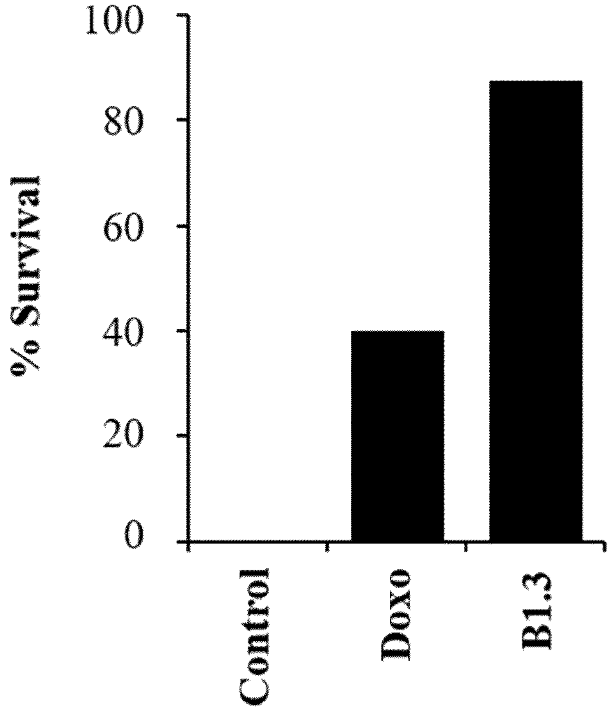


FIG. 46E

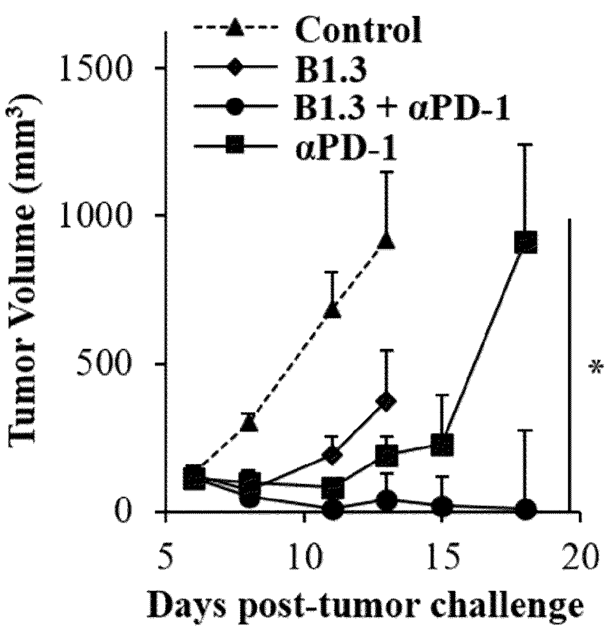


FIG. 47A

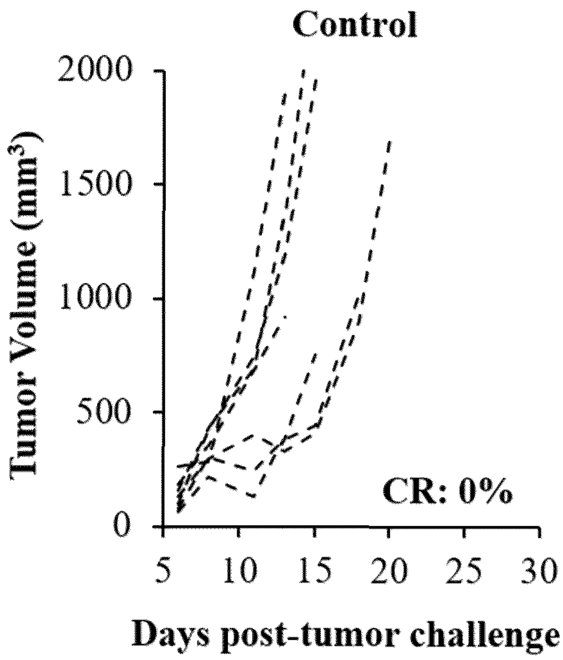


FIG. 47B

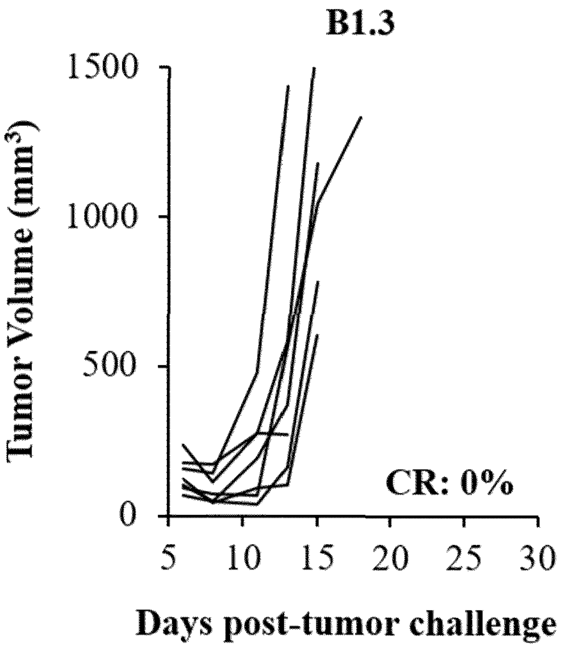


FIG. 47C

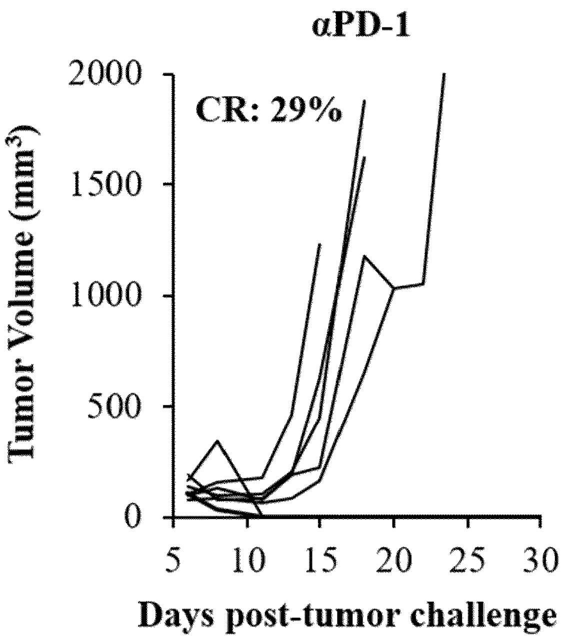


FIG. 47D

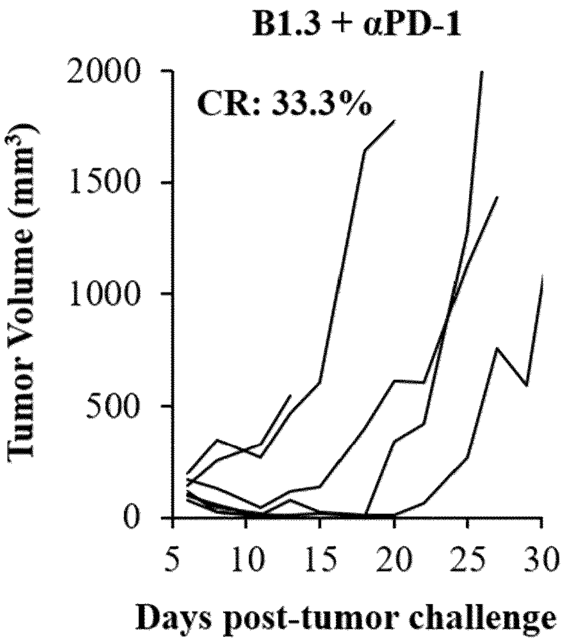


FIG. 47E

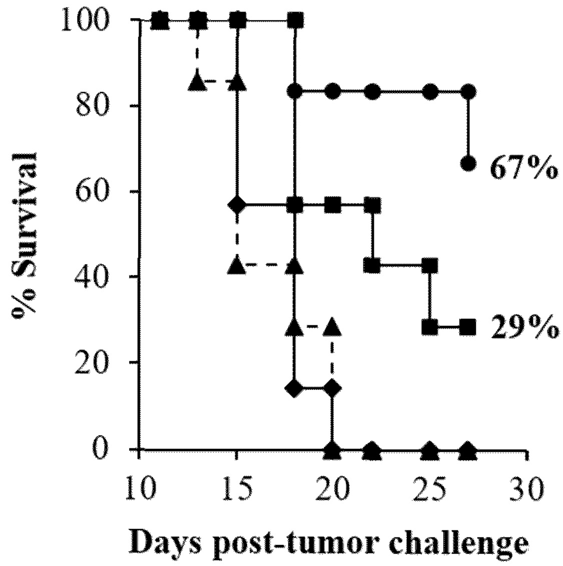


FIG. 47F

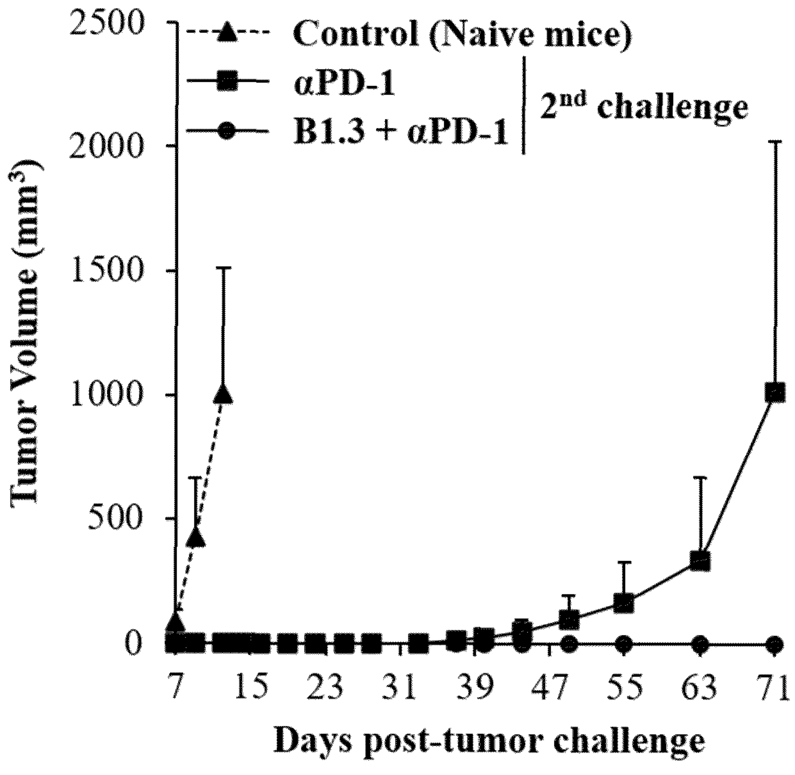


FIG. 48A

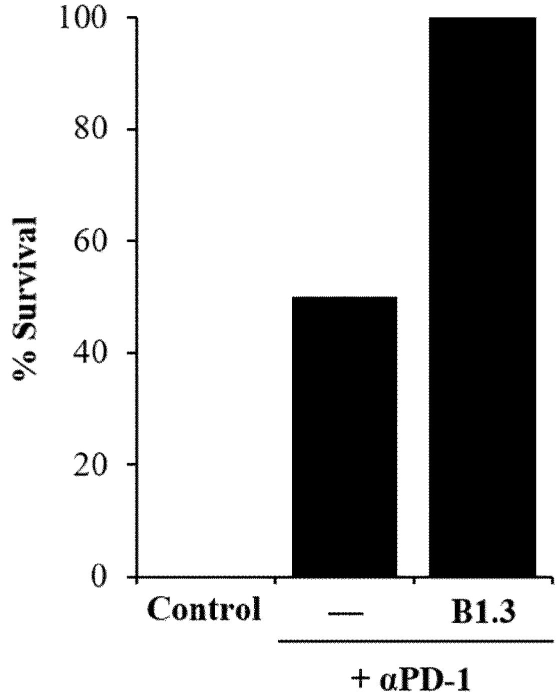


FIG. 48B

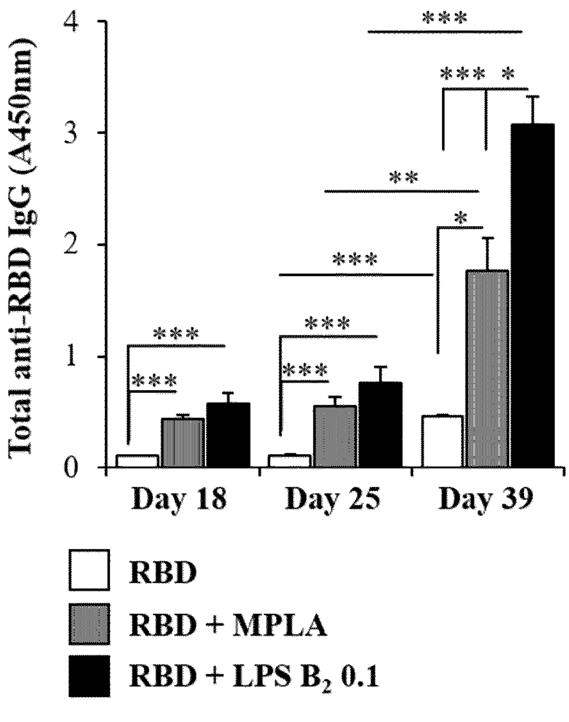


FIG. 49A

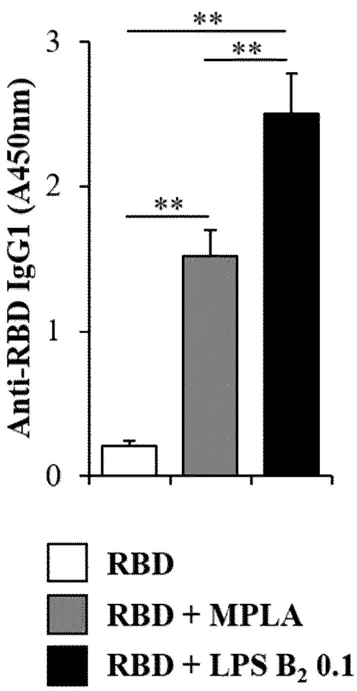


FIG. 49B

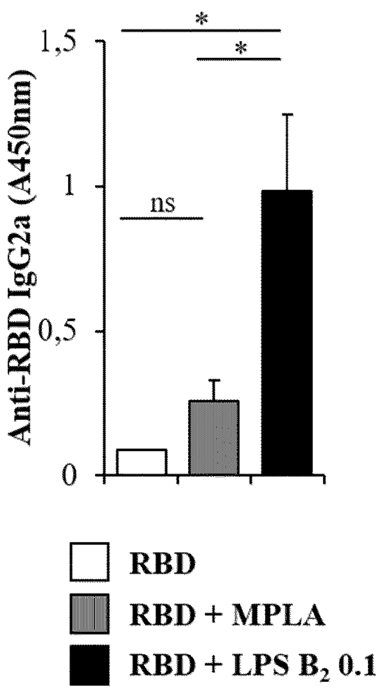


FIG. 49C

82/82

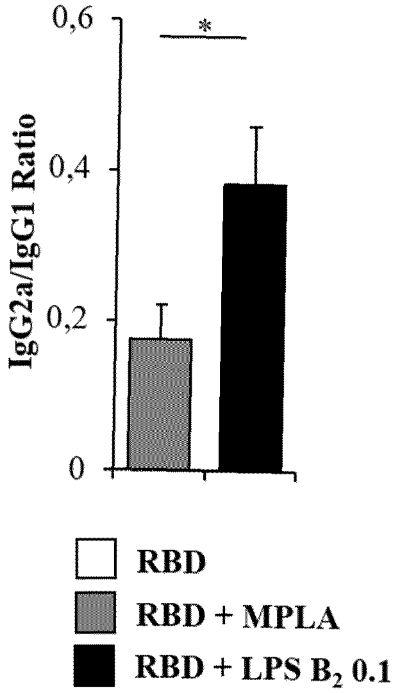


FIG. 49D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- RO 4491533
- WO 2017100305 A
- US 8137935 B


### Non-patent literature cited in the description


- **GARÇON & DI PASQUALE**. *Hum Vaccin Immunother.*, 2017, vol. 13 (1), 19-33
- **LI et al.** *Cancer Immunol Immunother.*, 2004, vol. 53 (3), 139-43
- **NOVELLINO et al.** *Cancer Immunol Immunother.*, 2005, vol. 54 (3), 187-20
- Endotoxins: structure, function and recognition. **WANG et al.** Purification and characterization of lipopolysaccharides. Springer, 2010, vol. 53, 27-51
- **TIRSOAGA et al.** *Appl Environ Microbiol.*, 2007, vol. 73 (6), 1803-8
- **ALLEN**. Ansel's pharmaceutical dosage forms and drug delivery systems. Wolters Kluwer, 2017
- Remington: The science and practice of pharmacy. London: Pharmaceutical Press, 2013
- Handbook of pharmaceutical excipients. London: Pharmaceutical Press, 2017
- **STRAUSS et al.** *J Mol Recognit.*, 2009, vol. 22 (5), 347-55
- **CAROFF** ; **KARIBIAN**. *Appl Environ Microbiol.*, 1990, vol. 56 (6), 1957-9
- **LAEMMLI**. *Nature*, 1970, vol. 227 (5259), 680-5
- **TSAI & FRASCH**. *Anal Biochem.*, 1982, vol. 119 (1), 115-9
- **CHAFCHAOUNI-MOUSSAOUI et al.** *Rapid Commun Mass Spectrom.*, 2011, vol. 25 (14), 2043-8
- *Invivogen, MPLAs technical data sheet, version 19C19-MM*, https://www.invivogen.com/sites/default/files/invivogen/products/files/mplas_tds.pdf
- **MISQUITH et al.** *Colloids Surf B Biointerfaces.*, 2014, vol. 113, 312-319
- **WEARY et al.** *Appl Environ Microbiol.*, 1980, vol. 40 (6), 1148-51
- **CARL R. ALVING**. *Immunobiol.*, 1993
- *A 2-Part Randomized, Double-Blind (Sponsor-Unblinded), Placebo-Controlled, Ascending Dose and Parallel Group Study of TLR4 Agonist (GSK1795091) Administered to Healthy Subjects*, 2017
- **GREISMAN SE et al.** *Proc Soc Exp Biol Med.*, 1969